# EUROPEAN PATENT SPECIFICATION

(11) **EP 1 005 445 B1**
(45) Date of publication and mention of the grant of the patent: **26.05.2004**
(21) Application number: 98945235.4
(22) Date of filing: 13.08.1998
(51) Int. Cl.: C07C 233/87, C07C 235/52, C07C 237/36, C07C 317/50, C07C 323/39, C07D 215/50, C07D 233/32, C07D 295/088, C07D 487/04, A61K 31/165, A61K 31/33

(54) **N-ALKANOYLPHENYLALANINE DERIVATIVES**
N-ALKANOYLPHENYLALANINDERIVATE
DERIVES DE N-ALCANOYLPHENILALANINE

(30) Priority: 22.08.1997 US 56718 P
(43) Date of publication of application: 07.06.2000
(60) Divisional application: 03027533.3
(73) Proprietor: F. HOFFMANN-LA ROCHE AG, 4070 Basel (CH)
(72) Inventor: CHEN, Li, Westfield, NJ 07090 (US); GUTHRIE, Robert, William, Saddle Brook, NJ 07663 (US); HUANG, Tai-Nang, Lexington, MA 02173 (US); HULL, Kenneth, G., Clifton, NJ 07014 (US); SIDDURI, Achytharao, Livingston, NJ 07039 (US); TILLEY, Jefferson, Wright, North Caldwell, NJ 07006 (US)
(74) Representative: Löschner, Thomas
(86) International application number: PCT/EP1998/005135
(87) International publication number: WO 1999/010312

(56) References cited:
- WO-A-95/35296
- WO-A-96/22966
- WO-A-97/36859
- DE-A- 19 654 483
- PATANI G A ET AL: "BIOISOSTERISM: A RATIONAL APPROACH IN DRUG DESIGN" CHEMICAL REVIEWS, vol. 96, no. 8, 1996, pages 3147-3176, XP000652176

## Description

Vascular cell adhesion molecule-1 (VCAM-1), a member of the immunoglobulin (Ig) supergene family, is expressed on activated, but not resting, endothelium. The integrin VLA-4(a₄b₁), which is expressed on many cell types including circulating lymphocytes, eosinophils, basophils, and monocytes, but not neutrophils, is the principal receptor for VCAM-1. Antibodies to VCAM-1 or VLA-4 can block the adhesion of these mononuclear leukocytes, as well as melanoma cells, to activated endothelium *in vitro*. Antibodies to either protein have been effective at inhibiting leukocyte infiltration and preventing tissue damage in several animal models of inflammation. Anti-VLA-4 monoclonal antibodies have been shown to block T-cell emigration in adjuvant-induced arthritis, prevent eosinophil accumulation and bronchoconstriction in models of asthma, and reduce paralysis and inhibit monocyte and lymphocyte infiltration in experimental autoimmune encephalitis (EAE). Anti-VCAM-1 monoclonal antibodies have been shown to prolong the survival time of cardiac allografts. Recent studies have demonstrated that anti-VLA-4 mAbs can prevent insulitis and diabetes in non-obese diabetic mice, and significantly attenuate inflammation in the cotton-top tamarin model of colitis.

Thus, compounds which inhibit the interaction between a₄-containing integrins and VCAM-1 will be useful as therapeutic agents for the treatment of chronic inflammatory diseases such as RA, multiple sclerosis (MS), asthma, and inflammatory bowel disease (IBD).

Accordingly, the present invention relates to new compounds of the formula: 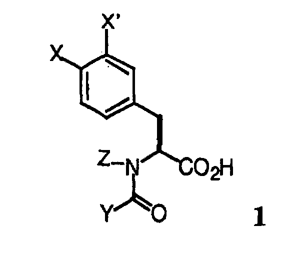 and the pharmaceutically acceptable salts and esters thereof wherein X, X', Z and Y are as defined below which inhibit the binding of VCAM-1 to VLA-4, methods for preparing such compounds, medicaments, a process for the production of such medicaments and the use of the new compounds in the treatment of illnesses, especially inflammatory diseases in which such binding acts to bring on the disease.

As used in this specification, the term "C₁₋₆ alkyl", alone or in combination, means a straight-chain or branched-chain alkyl group containing from one to six carbon atoms, such as methyl, ethyl, n-propyl, isopropyl, n-butyl, sec.butyl, isobutyl, tert.butyl, n-pentyl, n-hexyl and the like. C₁₋₆ alkyl groups may be unsubstituted or substituted by one or more groups selected independently from cycloalkyl, nitro, aryloxy, aryl, hydroxy, halogen, cyano, C₁₋₆ alkoxy, C₁₋₆ alkylcarbonyl, C₁₋₆ alkylthio, C₁₋₆ alkyl sulfinyl, C₁₋₆ alkyl sulfonyl, and substituted amino. Examples of substituted C₁₋₆ alkyl groups include 2-hydroxylethyl, 3-oxobutyl, cyanomethyl, and 2-nitropropyl.

The term "cycloalkyl" means an unsubstituted or substituted 3- to 7-membered carbacyclic ring. Substitutents in accordance with the present invention are hydroxy, halogen, cyano, C₁₋₆ alkoxy, C₁₋₆ alkylcarbonyl, C₁₋₆ alkyl, aroyl, C₁₋₆ alkylthio, C₁₋₆ alkyl sulfinyl, C₁₋₆ alkyl sulfonyl, aryl, heteroaryl and substituted amino.

The term "C₁₋₆ alkoxy" means a straight-chain or branched-chain alkoxy group containing a maximum of six carbon atoms, such as methoxy, ethoxy, n-propoxy, isopropoxy, n-butoxy, tert-butoxy and the like.

The term "C₁₋₆ alkylthio" means a C₁₋₆ alkyl group bonded through a divalent sulfur atom, for example, a methyl mercapto or a isopropyl mercapto group.

The term "aryl" means a mono- or bicylic aromatic group, such as phenyl or naphthyl, which is unsubstituted or substituted by conventional substituent groups. Preferred substituents are C₁₋₆ alkyl, C₁₋₆ alkoxy, hydroxy C₁₋₆ alkyl, hydroxy, hydroxyalkoxy, halogen, C₁₋₆ alkylthio, C₁₋₆ alkylsulfinyl, C₁₋₆ alkylsulfonyl, cyano, nitro, perfluoroalkyl, alkylcarbonyl, aroyl, aryl alkynyl, lower alkynyl and C₁₋₆ alkylcarbonylamino. The especially preferred substituents are C₁₋₆ alkyl, hydroxy, and perfluoro C₁₋₆ alkyl. Examples of aryl groups that may be used in accordance with this invention are phenyl, p-tolyl, p-methoxyphenyl, p-chlorophenyl, m-hydroxy phenyl, m-methylthiophenyl, 2-methyl-5-nitrophenyl, 2,6-dichlorophenyl, 1-naphthyl and the like.

The term "arylalkyl" means a C₁₋₆ alkyl group as hereinbefore defined in which one or more hydrogen atoms is/are replaced by an aryl or heteroaryl group as herein defined. Any conventional aralkyl may be used in accordance with this invention, such as benzyl and the like.

The term "heteroaryl" means an unsubstituted or substituted 5- or 6-membered monocyclic hetereoaromatic ring or a 9- or 10-membered bicyclic hetereoaromatic ring containing 1, 2, 3 or 4 hetereoatoms which are independently N, S or O. Examples of hetereoaryl rings are pyridine, benzimidazole, indole, imidazole, thiophene, isoquinoline, quinzoline and the like. Substitutents as defined above for "aryl" are included in the definition of heteroaryl.

The term "C₁₋₆ alkoxycarbonyl" means a C₁₋₆ alkoxy group bonded via a carbonyl group. Examples of alkoxycarbonyl groups are ethoxycarbonyl and the like.

The term "C₁₋₆ alkylcarbonyloxy" means C₁₋₆ alkylcarbonyloxy groups bonded via an oxygen atom, for example an acetoxy group.

The term "C₁₋₆ alkylcarbonyl" means C₁₋₆ alkyl groups bonded via a carbonyl group and embraces in the sense of the foregoing definition groups such as acetyl, propionyl and the like.

The term "C₁₋₆ alkylcarbonylamino" means C₁₋₆ alkylcarbonyl groups bonded via a nitrogen atom, such as acetylamino.

The term "aroyl" means an mono- or bicyclic aryl or heteroaryl group bonded via a carbonyl group. Examples of aroyl groups are benzoyl, 3-cyanobenzoyl, 2-naphthyl and the like.

The term "aryloxy" means an aryl group, as hereinbefore defined, which is bonded via an oxygen atom. The preferred aryloxy group is phenoxy.

In the first aspect, the present invention relates to a compound of the formula: 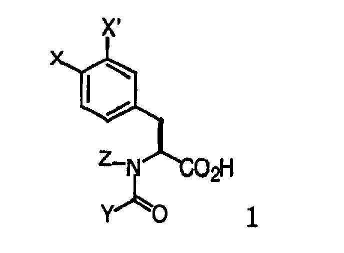 wherein:
one of X and X' is hydrogen, halogen, or C₁₋₆ alkyl, the other is a group of the formula: 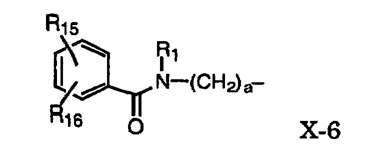
wherein:
R₁ is hydrogen or C₁₋₆ alkyl,
R₁₅ is hydrogen, halogen, nitro, C₁₋₆ alkyl sulfonyl, cyano, C₁₋₆ alkyl, OH, C₁₋₆ alkoxy, C₁₋₆ alkoxycarbonyl, carboxy, C₁₋₆ alkyl aminosulfonyl, perfluoro C₁₋₆ alkyl, C₁₋₆ alkylthio, hydroxy C₁₋₆ alkyl, alkoxy C₁₋₆ alkyl, halo C₁₋₆ alkyl, alkylthio C₁₋₆ alkyl, alkylsulfinyl C₁₋₆ alkyl, alkylsufonyl C₁₋₆ alkyl, C₁₋₆ alkylsulfinyl, C₁₋₆ alkylcarbonyl, aroyl, aryl, aryloxy or a group of the formula R₁₇-C≡C-,
R₁₆ is hydrogen, halogen, nitro, cyano, C₁₋₆ alkyl, OH, perfluoro C₁₋₆ alkyl, aryloxy or C₁₋₆ alkylthio,
R₁₇ is hydrogen, aryl, heteroaryl, or C₁₋₆ alkyl which is unsubstituted or substituted by OH, aryl, or heteroaryl, and
a is 0 or 1;
or one of X and X' is a group of the formula: 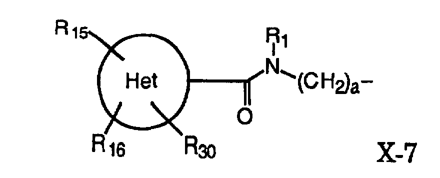 wherein Het is a 5- or 6-membered heteroaromatic ring containing 1, 2 or 3 heteroatoms selected from N,O, and S;
or
Het is a 9- or 10-membered bicyclic heteroaromatic ring containing 1, 2, 3 or 4 heteroatoms selected from O, S, and N,
a, R₁, R₁₅ and R₁₆ are as above, and
R₃₀ is hydrogen or C₁₋₆ alkyl, or is absent;
or one of X and X' is a group of the formula: 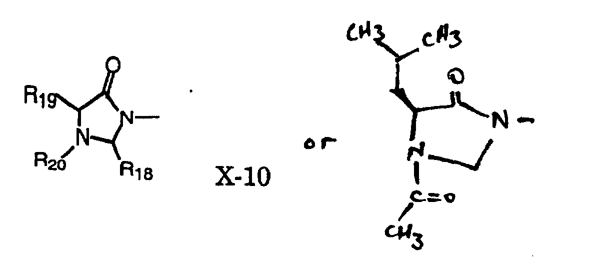 wherein:
R₁₈ is C₁₋₆ alkyl, aryl, heteroaryl, arylalkyl, heteroaryl alkyl,
R₁₉ is C₁₋₆ alkyl, which is unsubstituted or substituted by one or more of halogen, hydroxy, C₁₋₆ alkoxy, aryl, hetereoaryl, alkylthio, or R₁₉ is aryl or heteroaryl, and
R₂₀ is C₁₋₆ alkyl or C₁₋₆ alkylcarbonyl, or
R₁₉ and R₂₀ taken together are tetramethylene; and
Y is a group of the formula: 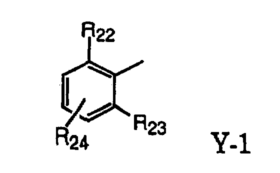 wherein:
R₂₂ and R₂₃ are independently hydrogen, C₁₋₆ alkyl, C₁₋₆ alkoxy, C₁₋₆ alkoxylalkyl, C₁₋₆ alkylamino, aryl, arylalkyl, nitro, cyano, C₁₋₆ alkylthio, C₁₋₆ alkylsulfinyl, C₁₋₆ alkyl sulfonyl, C₁₋₆ alkylcarbonyl, halogen, or perfluoroalkyl and at least one of R₂₂ and R₂₃ is other than hydrogen, and
R₂₄ is hydrogen, hydroxy, C₁₋₆ alkyl, C₁₋₆ alkoxy, C₁₋₆ alkylsulfonyl, amino, aryl, nitro, cyano, halogen, or is a group of the formula: 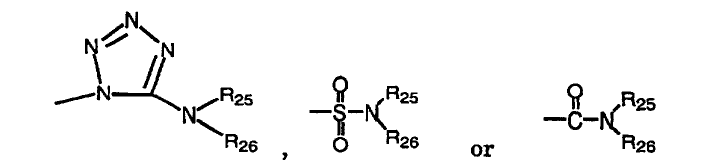 wherein R₂₅ is hydrogen, C₁₋₆ alkyl, aryl, aryl C₁₋₆ alkyl, alkoxy C₁₋₆ alkyl and R₂₆ is hydrogen or C₁₋₆ alkyl, or
R₂₂ and R₂₄ taken together are a fused benzene ring; or
Y is a group Y-2
   which is a five or six membered monocyclic heteroaromatic group containing 1, 2 or 3 heteroatoms selected from N, O, and S, or a 9- or 10-membered bicyclic heteroaromatic group containing 1, 2, 3 or 4 heteroatoms selected from O, S, and N, wherein said heteroaromatic group is bonded via a carbon atom to the amide carbonyl and one or two carbon atoms of said heteroaromatic group are substituted by C₁₋₆ alkyl, halogen, cyano, perfluoroalkyl, or aryl and at least one of said substituted carbon atoms is adjacent to the carbon atom bonded to the amide carbonyl;
and the pharmaceutically acceptable salts and esters thereof;
wherein
aryl refers to a mono- or bicylic aromatic group which is unsubstituted or substituted by C₁₋₆ alkyl, C₁₋₆ alkoxy, hydroxy C₁₋₆ alkyl, hydroxy, hydroxy alkoxy, halogen, C₁₋₆ alkylthio, C₁₋₆ alkylsulfinyl, C₁₋₆ alkylsulfonyl, cyano, nitro, perfluoroalkyl, alkylcarbonyl, aroyl as defined below, aryl alkynyl, lower alkynyl and C₁₋₆ alkylcarbonylamino;
heteroaryl refers to an unsubstituted or substituted 5- or 6-membered monocyclic hetereoaromatic ring or a 9- or 10-membered bicyclic hetereoaromatic ring containing 1, 2, 3 or 4 hetereoatoms which are independently N, S or O;
aroyl refers to an mono- or bicyclic aryl as defined above or heteroaryl as defined above bonded via a carbonyl group; and
aryloxy refers to an aryl group, as defined above, which is bonded via an oxygen atom.

The compounds of the invention can exist as stereoisomers and diastereomers, all of which are encompassed within the scope of the present invention.

In a compound of formula 1 X' is preferably hydrogen which means that then X is a group X-6, X-7 or X-10. When Z is C₁₋₆ alkyl, methyl is preferred. Z is preferably hydrogen.

In a compound of formula 1, wherein the group is Y-1, which is preferred over Y-2, R₂₂ and R₂₃ preferably are independently hydrogen, C₁₋₆ alkyl, nitro, C₁₋₆ alkylthio, C₁₋₆ alkoxy, C₁₋₆ alkylamino, C₁₋₆ alkylsulfinyl, C₁₋₆ alkyl sulfonyl, C₁₋₆ alkylcarbonyl, halogen, or perfluoroalkyl wherein at least one of R₂₂ and R₂₃ is not hydrogen, and
R₂₄ is hydrogen, hydroxy, C₁₋₆ alkyl, C₁₋₆ alkoxy, C₁₋₆ alkylsulfonyl, amino, nitro, halogen or a group of the formula: 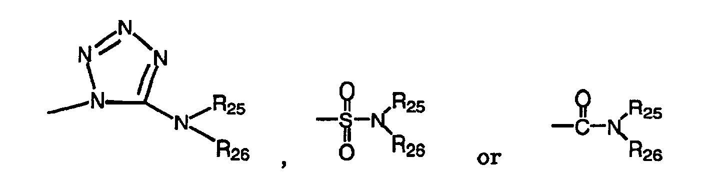 wherein R₂₅ is aryl C₁₋₆ alkyl and R₂₆ is hydrogen or C₁₋₆ alkyl,
or R₂₂ and R₂₄ taken together are a fused benzene ring.

Preferably R₂₂ is hydrogen (when R₂₃ is other than hydrogen), C₁₋₆ alkyl or halogen. R₂₄ is preferably hydrogen, hydroxy, C₁₋₆ alkylsulfonyl, C₁₋₆ alkyl, halogen, amino, nitro or C₁₋₆ alkoxy or a group of the formula: 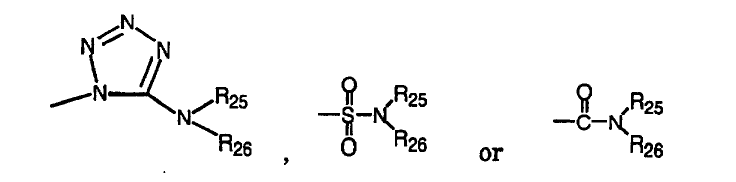 wherein R₂₅ is unsubstituted or hydroxy-substituted phenyl C₁₋₆ alkyl, and R₂₆ is hydrogen,
or R₂₂ and R₂₄ taken together are a fused phenyl ring.

More preferred R₂₄ is hydrogen, hydroxy, amino, methyl, chloro, bromo, nitro, -OCH₃, SO₂CH₃ and R₂₆ is H and R₂₅ is R₂₃ is preferably hydrogen (when R₂₂ is other than hydrogen), C₁₋₆ alkyl, C₁₋₆ alkylamino, halogen, nitro, perfluoro C₁₋₆ alkyl, C₁₋₆ alkoxy, C₁₋₆ alkylcarbonyl, C₁₋₆ alkylthio, C₁₋₆ alkylsulfinyl or C₁₋₆ alkyl sulfonyl. R₂₃ is more preferably methyl, ethyl, iso-propyl, tertbutyl, trifluormethyl, chloro, bromo, fluoro, nitro, -COCH₃, -SCH₃, -SOCH₃, -SO₂CH₃, -NHCH₃ or -OCH₃.

Most preferred Y-1 is selected from the group consisting of:

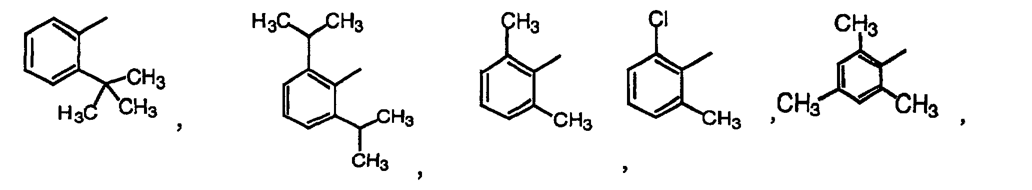

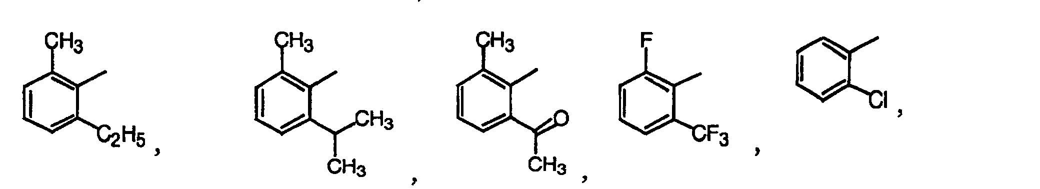

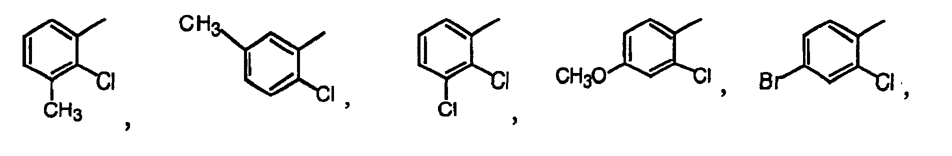

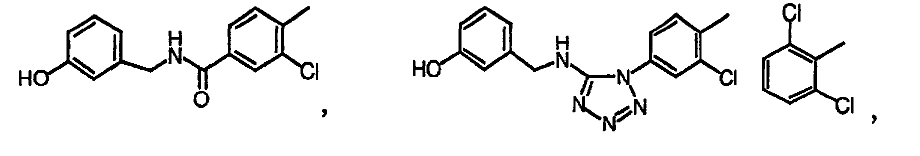

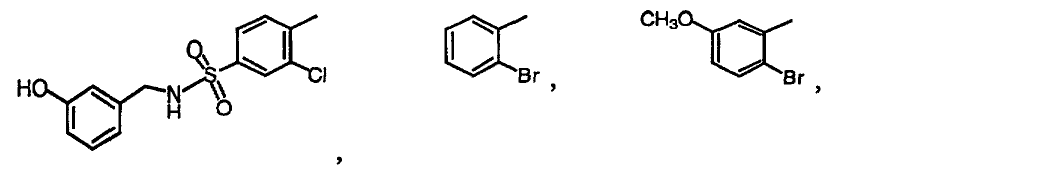

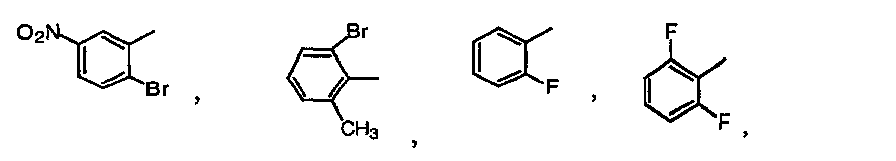

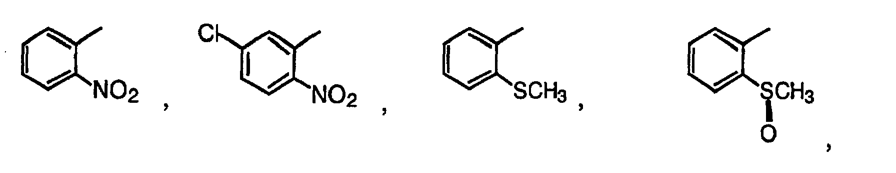

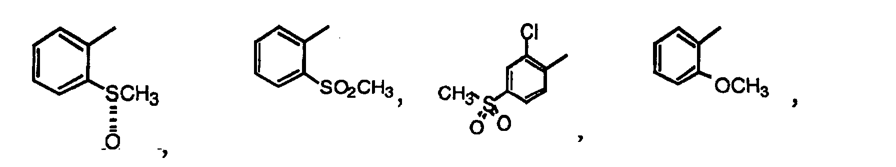

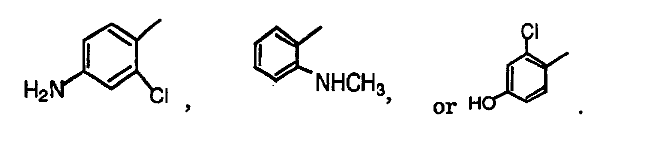

In a compound of formula 1 where Y-2 is a monocyclic heteroaromatic or a 9-or 10-membered bicyclic heteroaromatic group, this heterocycle is preferably selected from the group of:

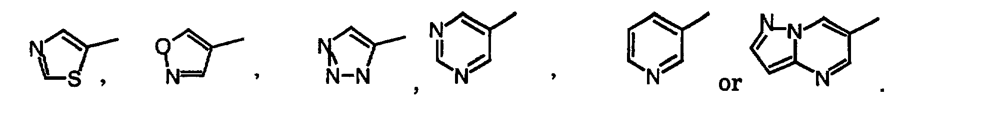

More preferred Y-2 groups are of the formula:

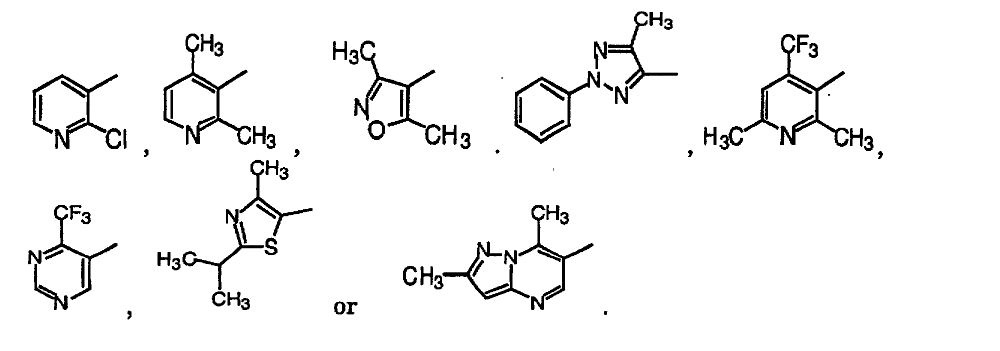

In a compound of formula 1 wherein X is X-6 the groups R₁₅ and R₁₆ preferably are independently hydrogen C₁₋₆ alkyl, nitro, halogen, perfluoro C₁₋₆alkyl, cyano or aryloxy. More preferred R₁₅ or R₁₆ is H, methyl, nitro, chloro, fluoro, trifluormethyl, cyano or phenoxy.

Most preferred X-6 groups are of the formula:

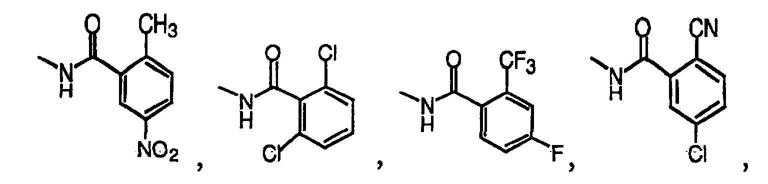

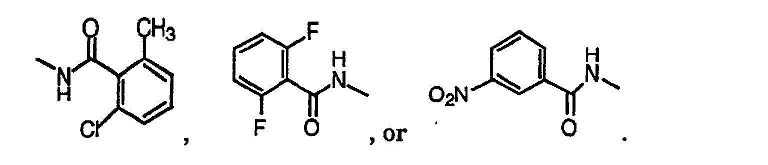

In a compound of formula 1 wherein X is X-7 Het is preferably a 5- or 6-membered monocyclic heteroaromatic ring containing 1, 2 or 3 nitrogens, or a nitrogen and a sulfur, or a nitrogen and an oxygen. More preferred the heteroaromatic ring is

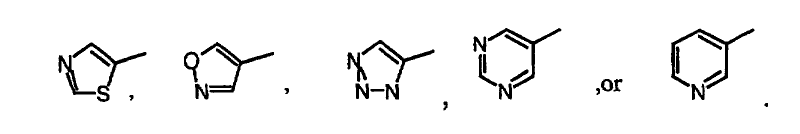

In a compound of formula 1 wherein X is X-7 and Het is a bicyclic heteroaromatic ring it preferably contains from 1 to 3 nitrogens as the heteroatoms. More preferably, the bicyclic heteroaromatic ring is 4-quinolinyl, 1-isoquinolinyl or

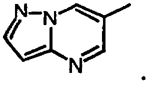

Regarding the substituents in the X-7 heterocycles R₁₅ is preferably hydrogen, nitro, C₁₋₆ alkyl sulfonyl, cyano, C₁₋₆ alkyl, C₁₋₆ alkoxy, perfluoro C₁₋₆ alkyl, C₁₋₆ alkylthio, C₁₋₆ alkylcarbonyl, or aryl. More preferred R₁₅ is isopropyl, methyl or phenyl.

R₁₆ in X-7 heterocycles is preferably hydrogen, halogen, nitro, cyano, C₁₋₆ alkyl or perfluoro C₁₋₆ alkyl. More preferred R₁₆ is methyl or triflouromethyl.
R₃₀ in X-7 is preferably hydrogen or C₁₋₆ alkyl, especially methyl.

Preferred X-7 groups are of the formula:

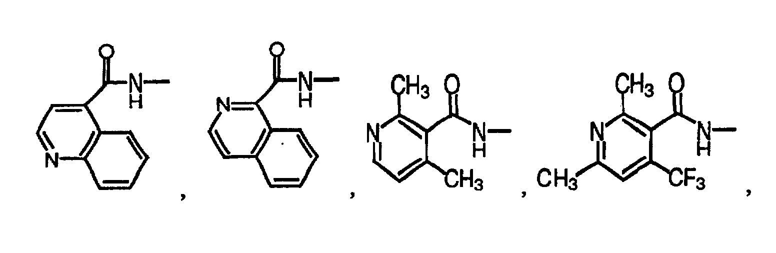

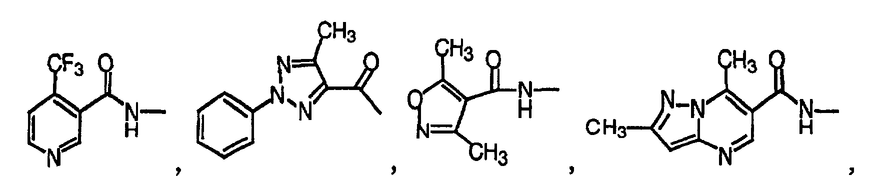

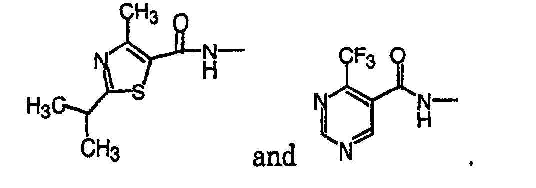

In a preferred embodiment of X-6 or X-7 R₁ is hydrogen. In another a is 0.

In a compound of formula 1 wherein X is X-10 R₁₈ is preferably C₁₋₆ alkyl or phenyl, wherein the phenyl ring is unsubstituted or monosubstituted by halogen, hydroxy, or is phenyl C₁₋₆ alkyl. More preferred R₁₈ is tertbutyl, phenyl, hydroxyphenyl, chlorophenyl or phenylethyl.

R₁₉ is preferably C₁₋₆ alkyl which is unsubstituted or substituted by pyridyl or phenyl wherein the phenyl ring is unsubstituted or monosubstituted by C₁₋₆ alkoxy or halogen. More preferred R₁₉ is methyl, isobutyl, benzyl, 4-chlorobenzyl, 4-methoxybenzyl or 2-pyridylmethyl.

When R₂₀ in X-10 is C₁₋₆ alkyl methyl is preferred. Preferably it is C₁₋₆ alkylcarbonyl, especially acetyl..

Most preferred groups X-10 are of the formula: 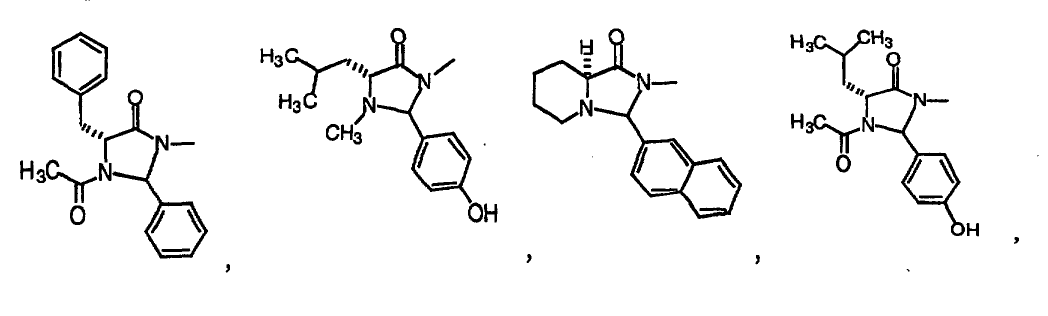 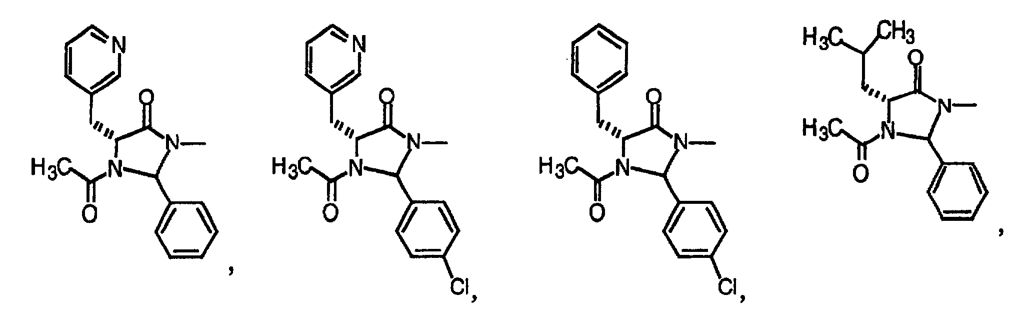 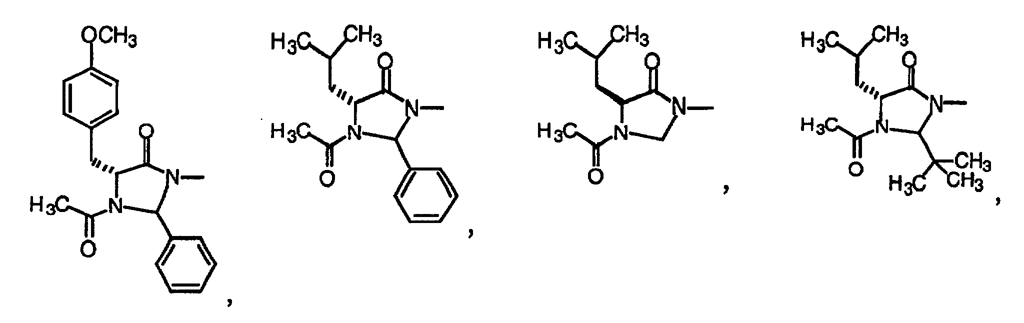 and

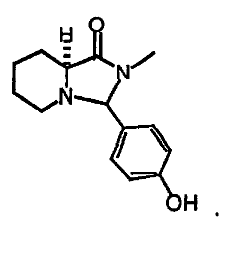

The compounds of the invention include the pharmaceutically acceptable salts and esters thereof. Certain prefered esters of the invention are useful to improve bioavailabilty of compounds of this invention. These preferred esters are of the formula: 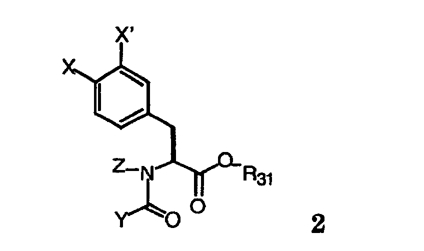 wherein X, X', Z and Y are as described above, and R₃₁ is C₁₋₆ alkyl, or R₃₁ is a group of formula P-1: 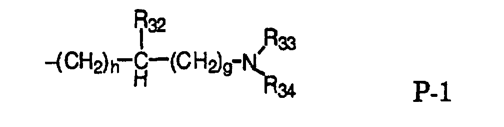 wherein:
R₃₂ is hydrogen or C₁₋₆ alkyl,
R₃₃ is hydrogen, C₁₋₆ alkyl, aryl,
R₃₄ is hydrogen or C₁₋₆ alkyl,
h is an integer from 0 to 2,
g is an integer from 0 to 2,
the sum of h and g is 1 to 3; or
R₃₁ is a group of formula P-2: 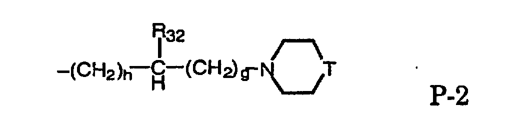 wherein:
R₃₂, g, and h are as previously defined,
T is O, S, -(CH₂)ⱼ-, a bond (when j=0) or a group of the formula N-R₃₅,
R₃₅ is hydrogen, C₁₋₆ alkyl, C₁₋₆ alkylcarbonyl, C₁₋₆ alkoxycarbonyl, and
j is 0, 1 or 2.

Particular C₁₋₆ alkyl ester groups are methyl, ethyl, butyl, 1-methylethyl, 2-methylpropyl, 2-methoxyethyl and 2-hydroxyethyl. A particular P1 group is .2-diethylaminoethyl. Particular P2 group are 2-(4-morpholinyl)-ethyl, 1-methyl-2-(4-morpholinyl)ethyl, 1-methyl-4-piperidinyl, 2-(1-piperazinyl)ethyl and 2-(4-methyl-1-piperazinyl)ethyl.

R₃₁ is preferably methyl, ethyl or 2-(4-morpholinyl)ethyl.

Preferred compounds of formula 1 and 2 are selected from the group:

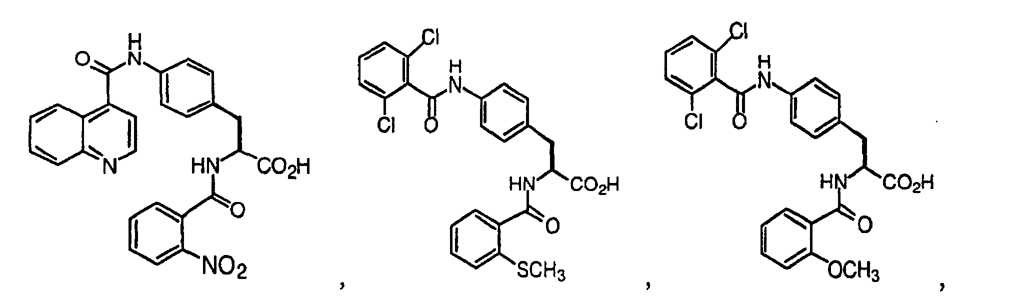

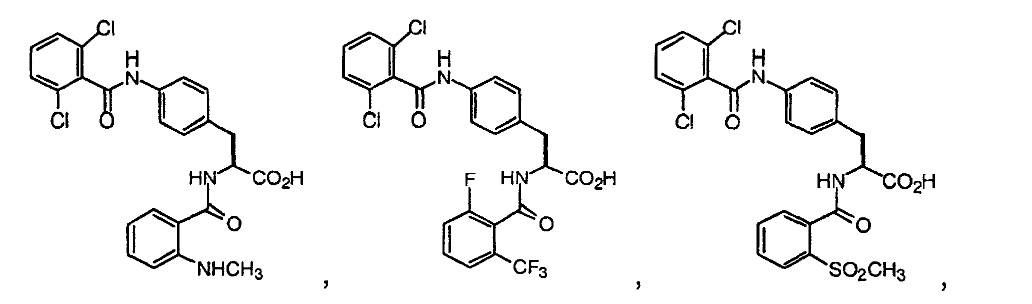

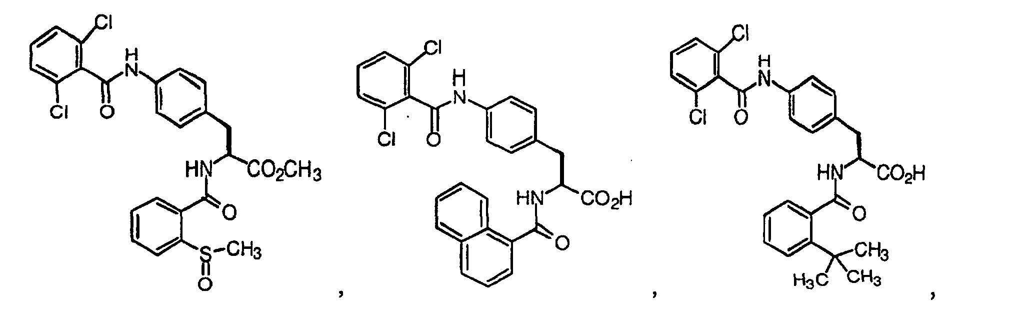

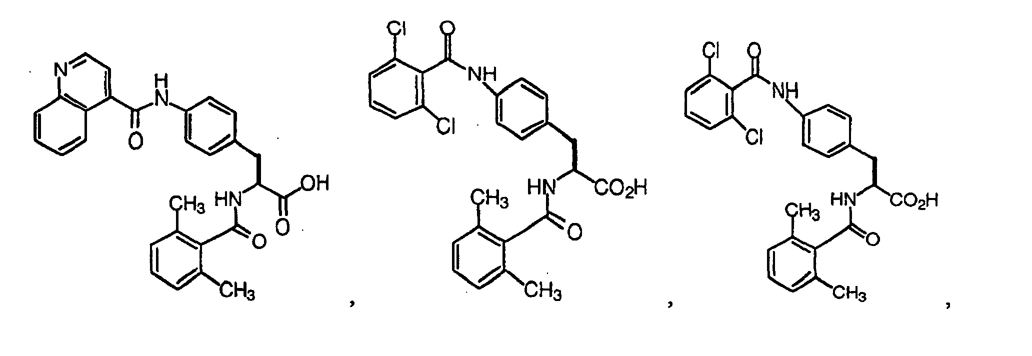

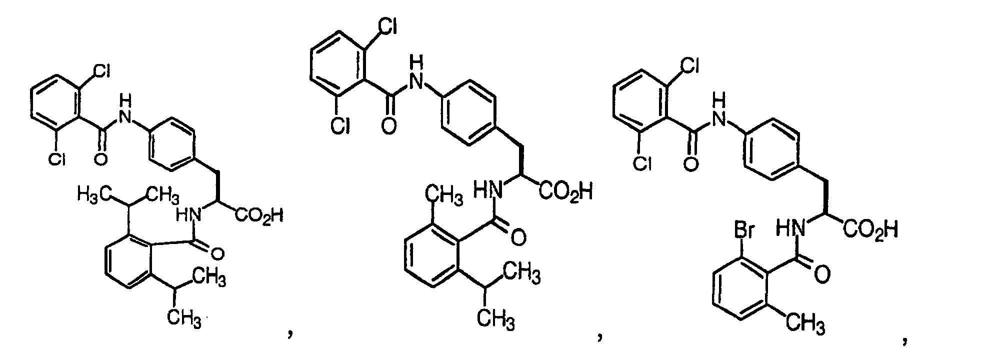

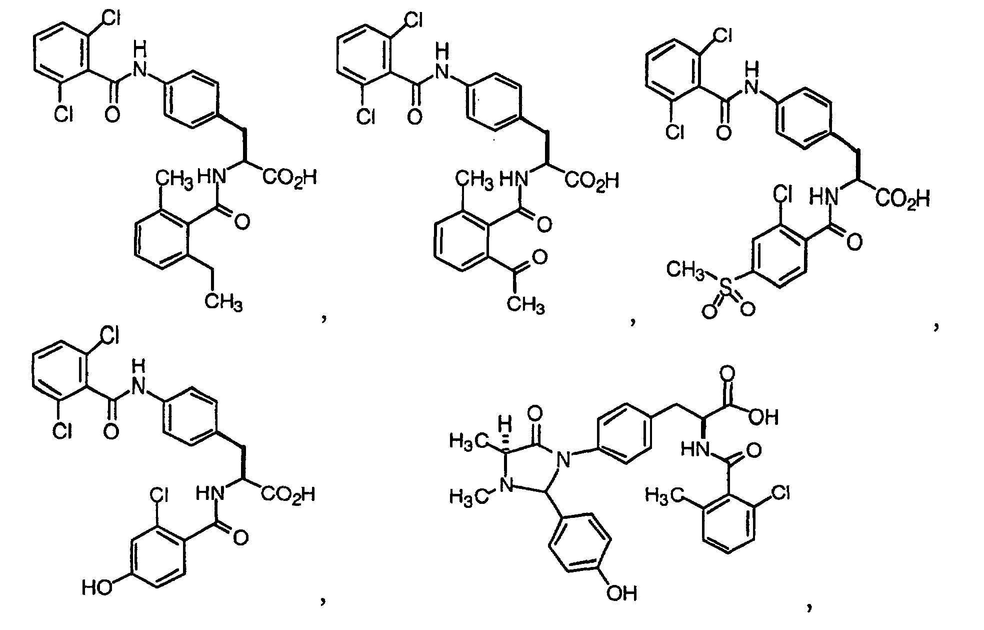

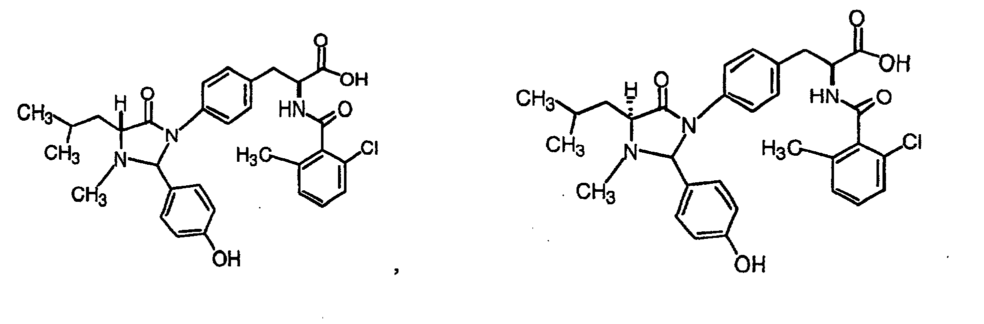

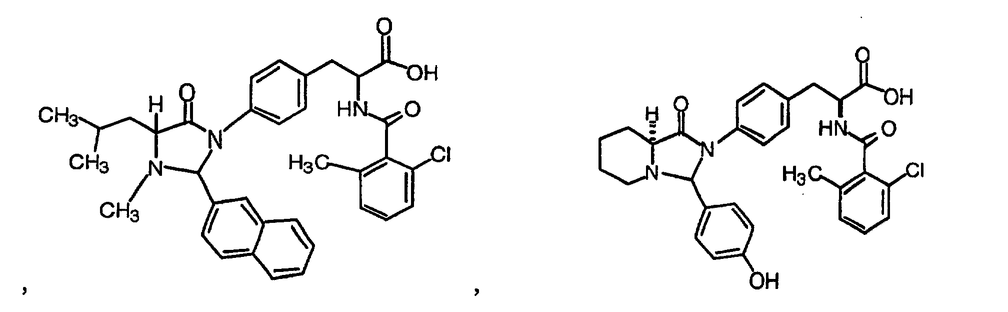

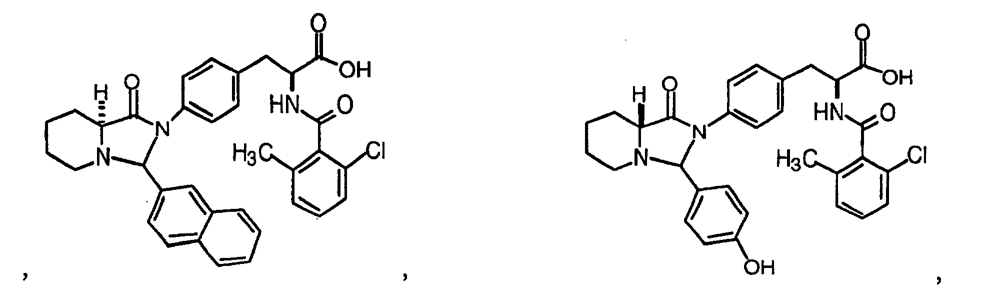

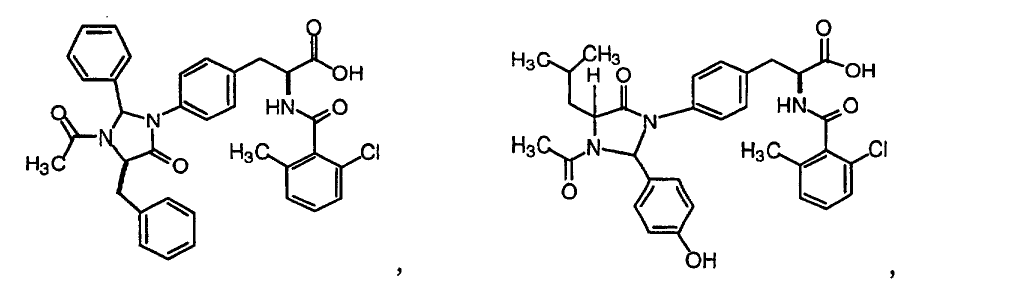

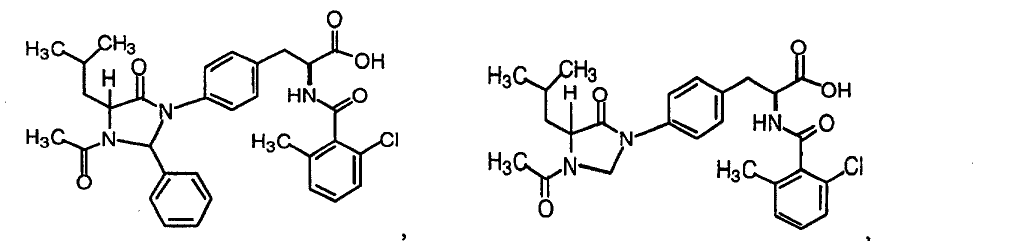

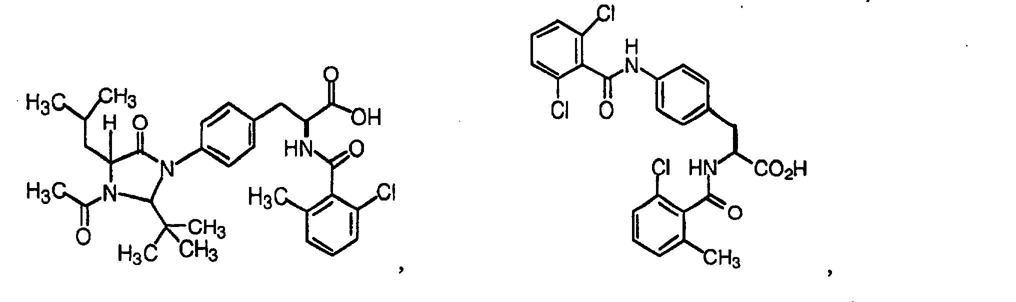

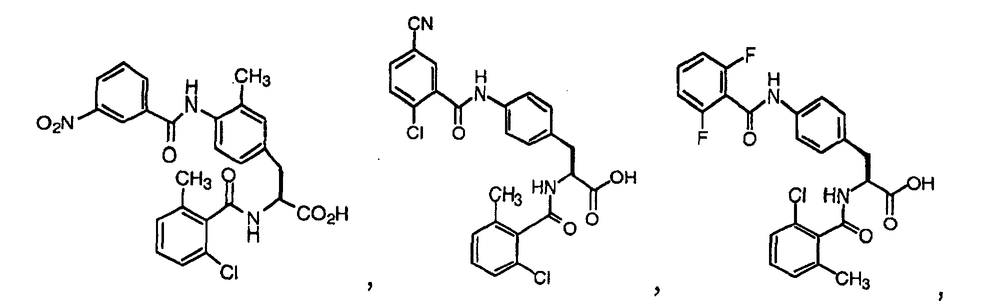

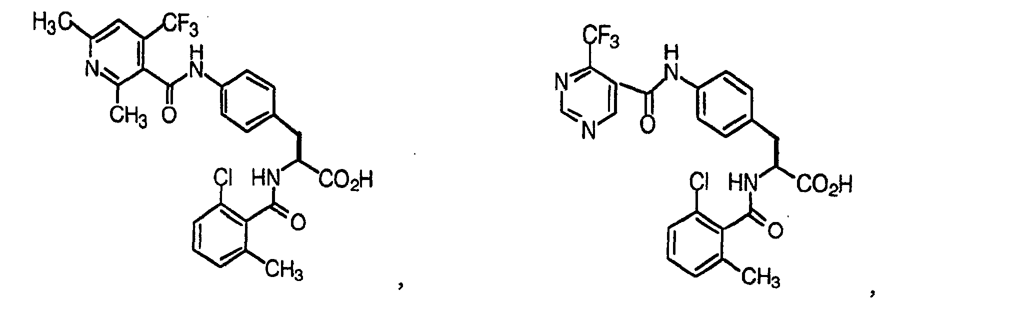

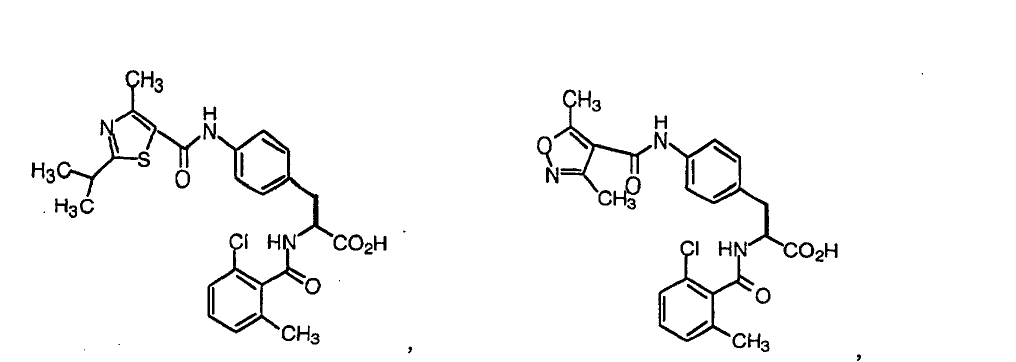

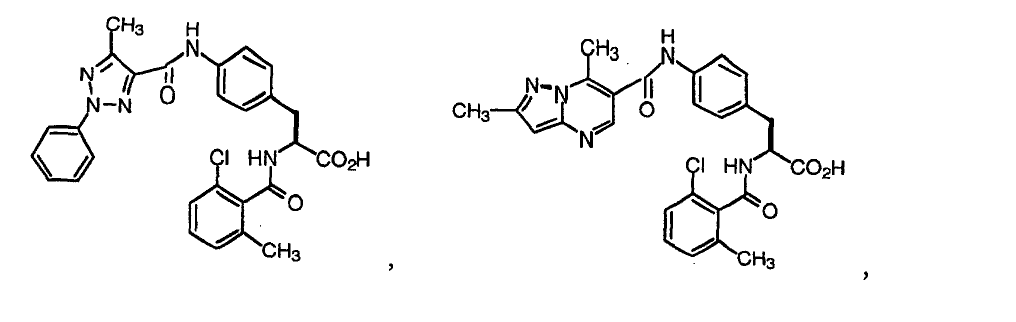

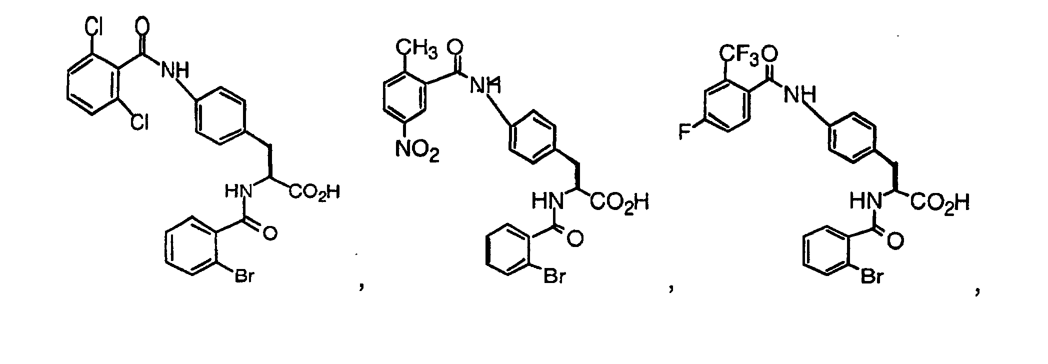

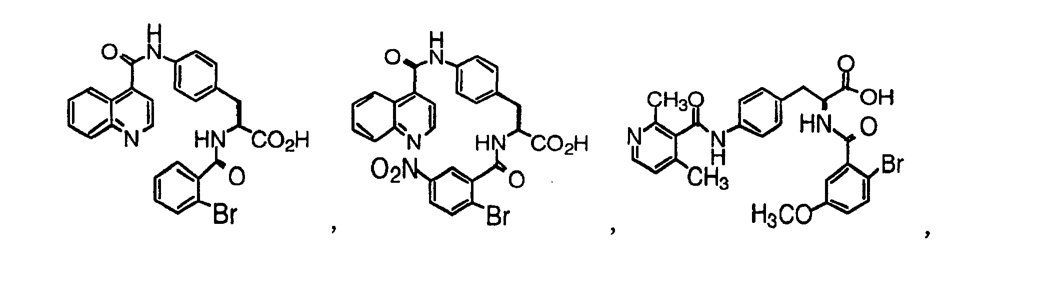

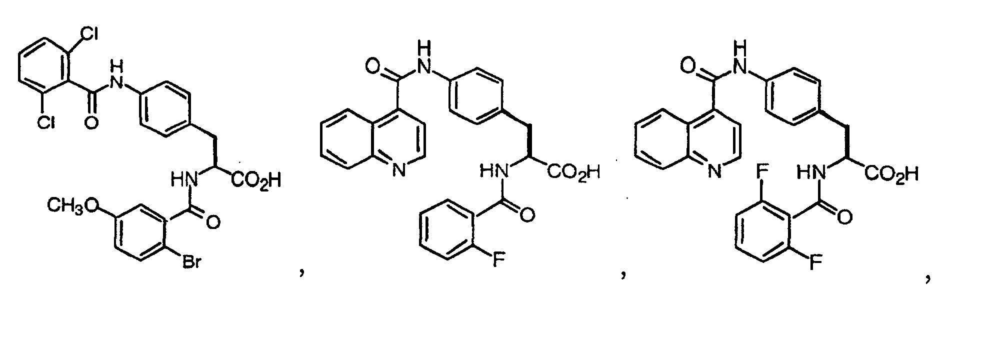

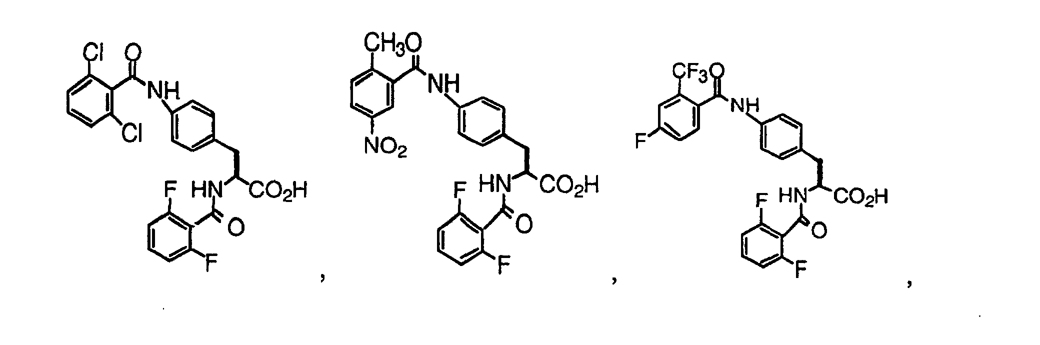

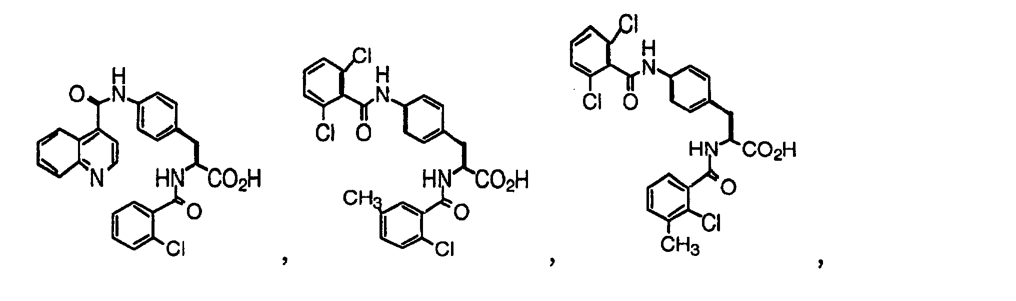

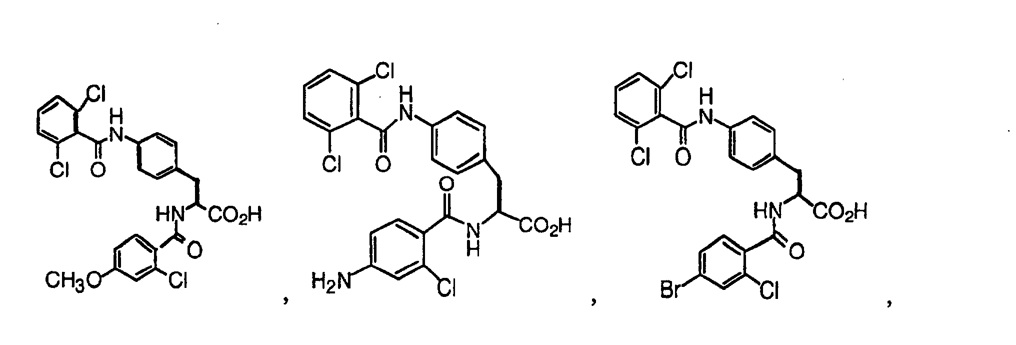

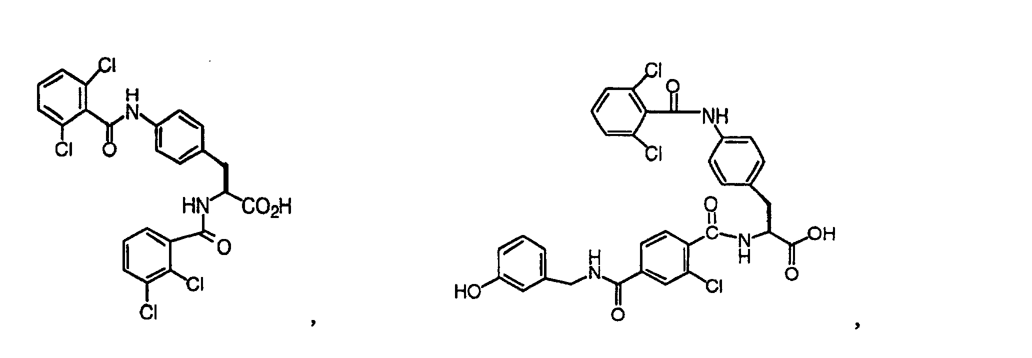

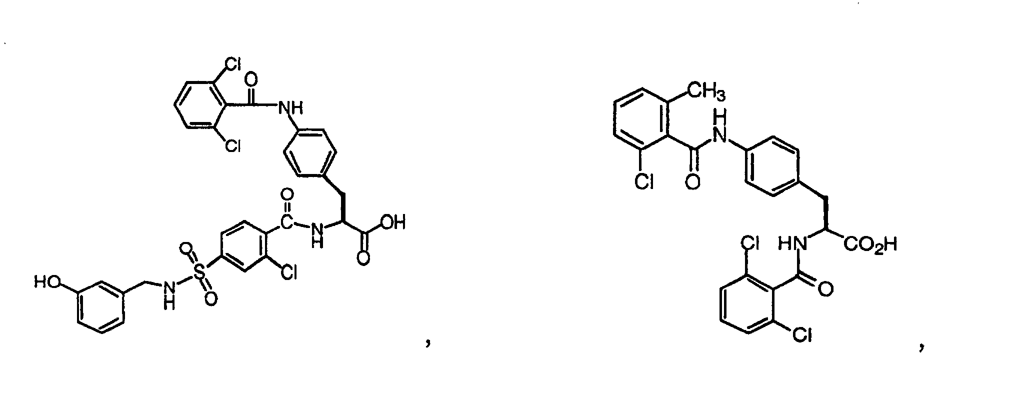

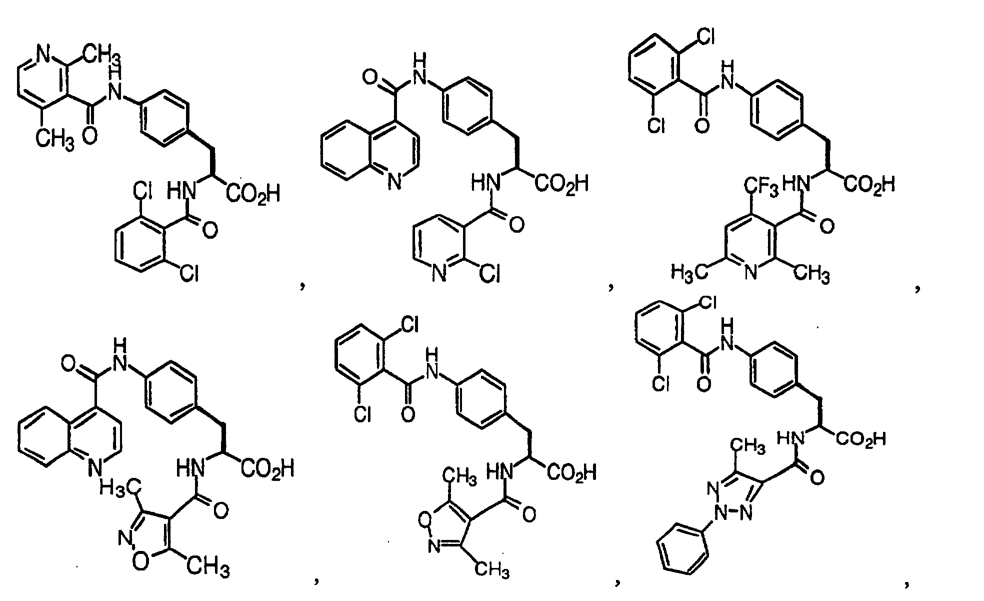

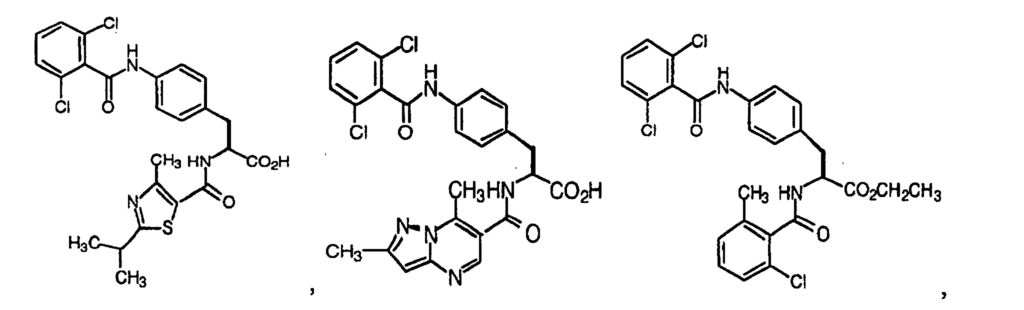

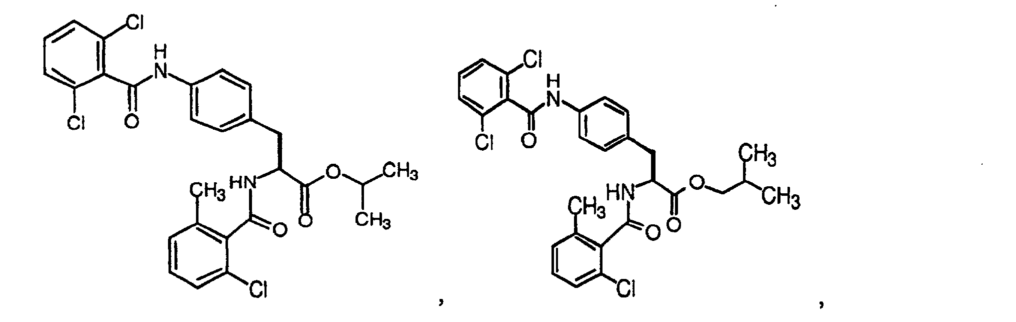

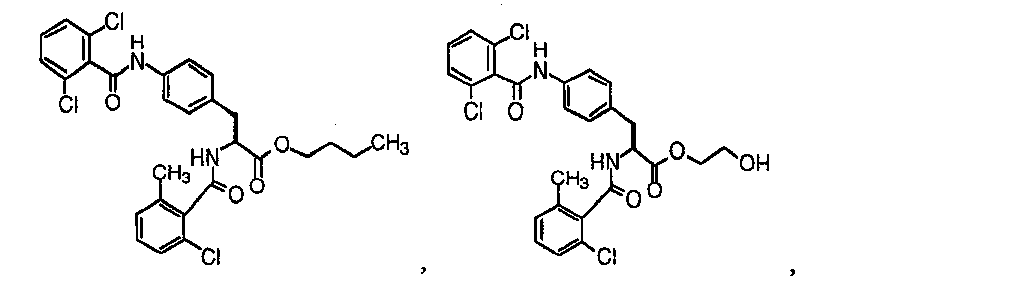

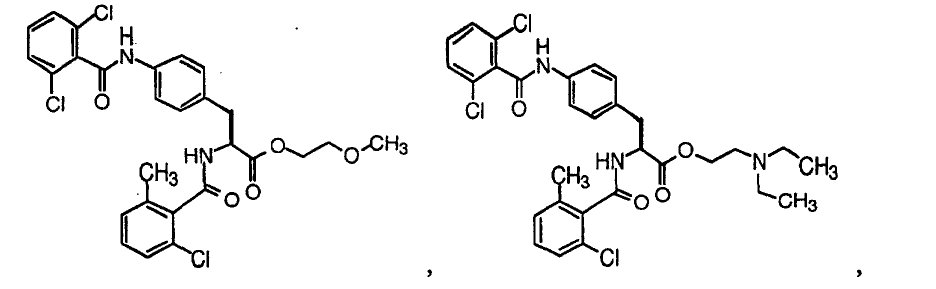

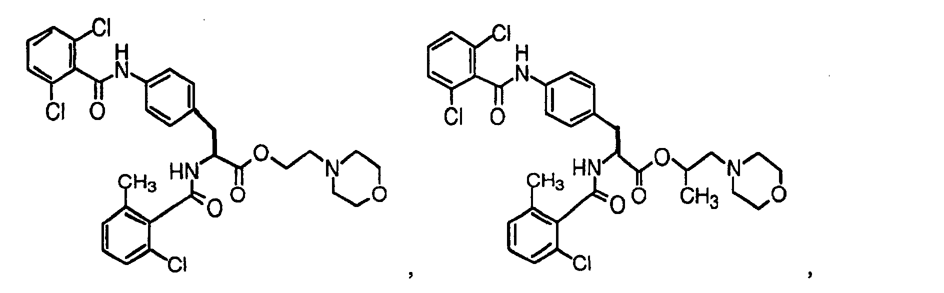

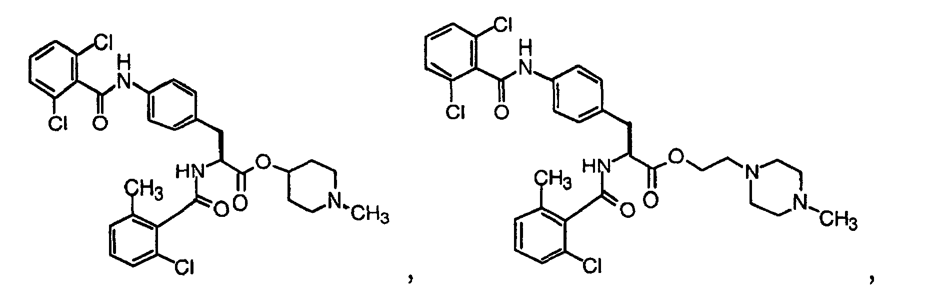

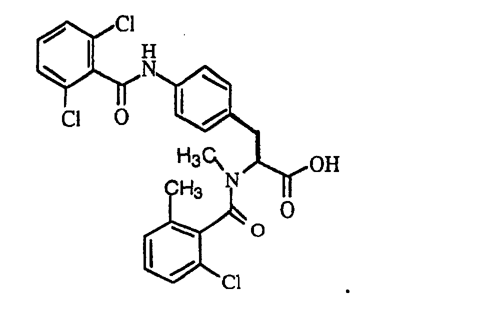

The compounds of the invention inhibit the binding of VCAM-1 and fibronectin to VLA-4 on circulating lymphocytes, eosinophils, basophils, and monocytes ("VLA-4-expressing cells"). The binding of VCAM-1 and fibronectin to VLA-4 on such cells is known to be implicated in certain disease states, such as rheumatoid arthritis, multiple sclerosis, inflammatory bowel disease, and particularly in the binding of eosinophils to pulmonary endothelium which is the cause of the pulmonary inflammation which occurs in asthma. Thus, the compounds of the present invention would be useful for the treatment of asthma.

In another aspect, on the basis of their capability of inhibiting binding of VCAM-1 and fibronectin to VLA-4 on circulating lymphocytes, eosinophils, basophils, and monocytes, the compounds of the invention can be used as medicament for the treatment of disorders which are known to be associated with such binding. Examples of such disorders are rheumatoid arthritis, multiple sclerosis, asthma, and inflammatory bowel disease. The compounds of the invention are preferably used in the treatment of diseases which involve pulmonary inflammation, such as asthma. The pulmonary inflammation which occurs in asthma is related to eosinophil infiltration into the lungs wherein the eosinophils bind to endothelium which has been activated by some asthma-triggering event or substance.

Furthermore, compounds of the invention also inhibit the binding of VCAM-1 and MadCAM to the cellular receptor alpha4-beta7, also known as LPAM, which is expressed on lymphocytes, eosinophiles and T-cells. While the precise role of alpha4-beta7 interaction with various ligands in inflammatory conditions such as asthma is not completely understood, compounds of the invention which inhibit both alpha4-beta1 and alpha4-beta7 receptor binding are particularly effective in animal models of asthma. Furthermore work with monoclonal antibodies to alpha4-beta7 indicate that compounds which inhibit alpha4-beta7 binding to MadCAM or VCAM are useful for the treatment of inflammatory bowel disease. They would also be useful in the treatment of other diseases in which such binding is implicated as a cause of disease damage or symptoms.

The compounds of the invention can be administered orally, rectally, or parentally, e.g., intravenously, intramuscularly, subcutaneously, intrathecally or transdermally; or sublingually, or as opthalmalogical preparations, or as an aerosol for the treatment of pulmonary inflammation. Capsules, tablets, suspensions or solutions for oral administration, suppositories, injection solutions, eye drops, salves or spray solutions are examples of administration forms.

Intravenous, intramuscular, oral or inhalation administration is a preferred form of administration. The dosages in which the compounds of the invention are administered in effective amounts depend on the nature of the specific active ingredient, the age and the requirements of the patient and the mode of administration. Dosages may be determined by any conventional means, e.g., by dose-limiting clinical trials. Thus, the invention further. comprises the use of the present compounds for the manufacture of a medicament for treating a host suffering from a disease in which VCAM-1 or fibronectin binding to VLA-4-expressing cells is a causative factor in the disease symptoms or damage. In general, dosages of about 0.1-100 mg/kg body weight per day are preferred, with dosages of 1-25 mg/kg per day being particularly preferred, and dosages of 1-10 mg/kg body weight per day being espeically preferred.

The invention further relates to pharmaceutical compositions or medicaments which contain a pharmaceutically effective amount of a compound of the invention and a pharmaceutically and therapeutically acceptable carrier. Such compositions may be formulated by any conventional means by bringing a compound according to the present invention into a galenical administration form together with a therapeutically inert carrier material. If desired, one or more additional therapeutically active substances may be added.

Tablets or granulates can contain a series of binders, fillers, carriers or diluents. Liquid compositions can be, for example, in the form of a sterile water-miscible solution. Capsules can contain a filler or thickener in addition to the active ingredient. Furthermore, flavour-improving additives as well as substances usually used as preserving, stabilizing, moisture-retaining and emulsifying agents as well as salts for varying the osmotic pressure, buffers and other additives can also be present.

The previously mentioned carrier materials and diluents can comprise any conventional pharmaceutically acceptable organic or inorganic substances, e.g., water, gelatine, lactose, starch, magnesium stearate, talc, gum arabic, polyalkylene glycols and the like.

Oral unit dosage forms, such as tablets and capsules, preferably contain from 25 mg to 1000 mg of a compound of the invention.

The compounds of the present invention may be prepared by any conventional means. In reaction Scheme 1, a compound of formula **1** in which R₁ is H or C₁₋₆ alkyl, and which is a known compound or can be prepared by standard methodology, is treated with a reducing agent capable of selectively reducing a nitro group in the presence of a benzylic alcohol. This procedure is advantageously carried out in the presence of a derivatizing agent of the formula R₂-OCOX wherein X is a leaving group and R₂ is tert-alkyl, benzyl or the like so as to form a readily cleavable protecting group, thus leading directly to a compound of formula **2**. For example, this procedure can be conveniently carried out by catalytic hydrogenation of 1 over Pd© in ethyl acetate in the presence of di-tert-butyl dicarbonate to give a derivative of **2** in which R₂ is tert-butyl.

Conversion to an aldehyde of formula **3** can be carried out using any one of a variety of oxidizing agents capable of oxidizing a benzylic alcohol to the corresponding aldehyde, for example activated manganese dioxide in a suitable solvent, for example dichloromethane. Reaction of **3** to give a dehydroamino acid of formula **5** can be effected by treatment with a Wittig reagent of formula **4** in which R₃ is C₁₋₆ alkyl and R₄ is an alkoxy group, for example benzyloxy- or tert-butoxy- or represents a portion of one of the acyl groups of the compounds of the invention, for example substituted C₁₋₆ aryl.
For example treatment of **3** with (±)-N-(benzyloxycarbonyl)-a-phosphonoglycine trimethyl ester in the presence of a suitable base for example tetramethyl guanidine leads directly to a dehydroamino acid of formula **5**, R₃ = methyl and R₄ = benzyloxy. Enantioselective reduction of **5** to the L-amino acid **6** can be effected by use of a number of reducing agents suitable for the purpose, for example, the recently described ethyl-DuPHOS rhodium reagent (Burk, M. J., Feaster, J. E.; Nugent, W. A.; Harlow, R. L. *J*. *Am*. *Chem*. *Soc.* **1993,***115*, 10125) using essentially the literature procedure.

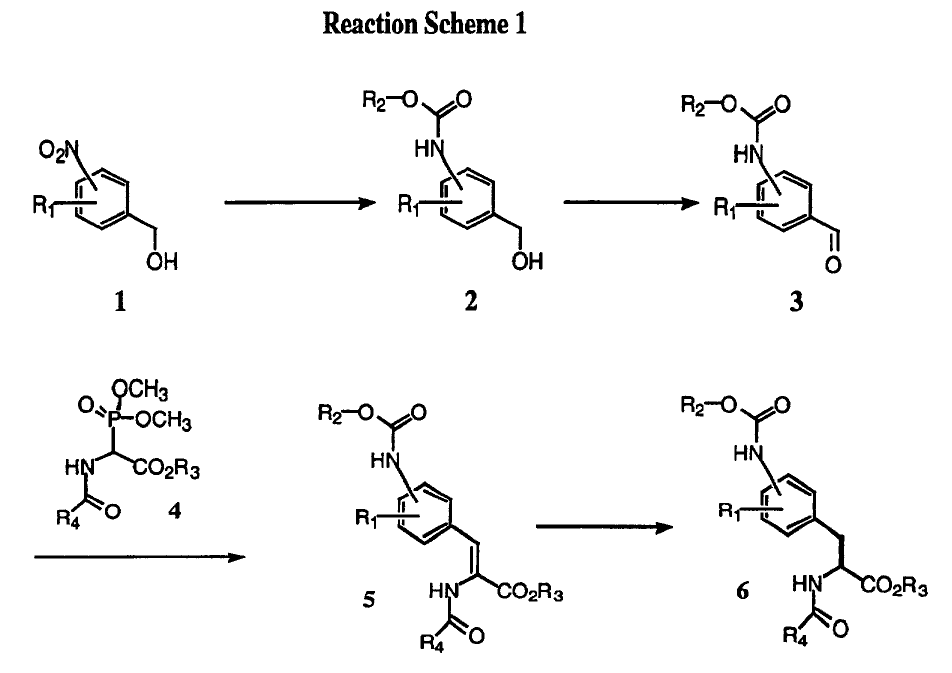

One process for the conversion of compounds of structure **6** into compounds of the invention is shown in Reaction Scheme 2. The protecting group incorporating R₂ can be removed under conditions dependent on the particular choice of R₂ as well as R₃ and R₄. The choice of these groups will be dependent on the particular target compound. A variety of common protecting groups and their use are described in "T. W. Green and P. G. M. Wuts, Protective Groups in Organic Synthesis, 2^{nd} edition, Wiley Interscience, New York, 1991" For example when R₂ is a tert-butyl group and R₃ is C₁₋₆ alkyl and R₄ is either a benzyloxy group or represents a portion of one of the acyl groups of the compounds of the invention, for example ortho-substituted aryl, treatment with trifluoroacetic acid either neat or in dichloromethane solution in the presence of suitable scavengers, for example, triethylsilane or anisol leads to a compound of formula **7**. This compound can be coupled with a carboxylic acid of formula **8** using standard peptide coupling conditions, for example HBTU in the presence of DIPEA in a polar, aprotic solvent such as DMF at a temperature between 0 °C and room temperature to give a compound of formula **9**. In the carboxylic acid of formula **8**, R₅ may represent a substituted alkyl group, a substituted aromatic ring, or a substituted heteroaromatic ring. R₅ may also incorporate suitably protected reactive functionalities to permit final conversion into compounds of the invention. The choice and use of such groups will be apparent to those skilled in the art.

Depending on the choice of R₄ and whether an ester or acid is the final goal of the synthesis, compound **9** may be a compound of the invention or in the case that R₄ is a protecting group, for example, a benzyloxy group, it may be removed under appropriate conditions, for example by catalytic hydrogenation over Pd© in a suitable solvent such as a lower alcohol to give a compound of formula **10**. This intermediate can be coupled with a carboxylic acid of formula **11** using standard peptide coupling conditions, for example HBTU in the presence of DIPEA in a polar, aprotic solvent such as DMF at a temperature between 0 °C and room temperature to give a compound of formula **12**. In the carboxylic acid of formula **11**, R₆ may represent a portion of a compound of the invention, for example ortho-substituted aryl or hetereoaryl. These compounds are known compounds or can be prepared by known methods. R₆ may also incorporate suitably protected reactive functionalities to permit final conversion into compounds of the invention. The choice and use of such groups will be apparent to those skilled in the art. If the acid 13 is the target compound, conversion of a compound of formula **12** can be effected using standard hydrolysis conditions appropriate for the particular choice of R₃ and any functional groups present as part of R₅ and R₆. In the case where R₃ is C₁₋₆ alkyl, treatment with an alkali metal hydroxide, for example lithium hydroxide in aqueous THF is generally effective.

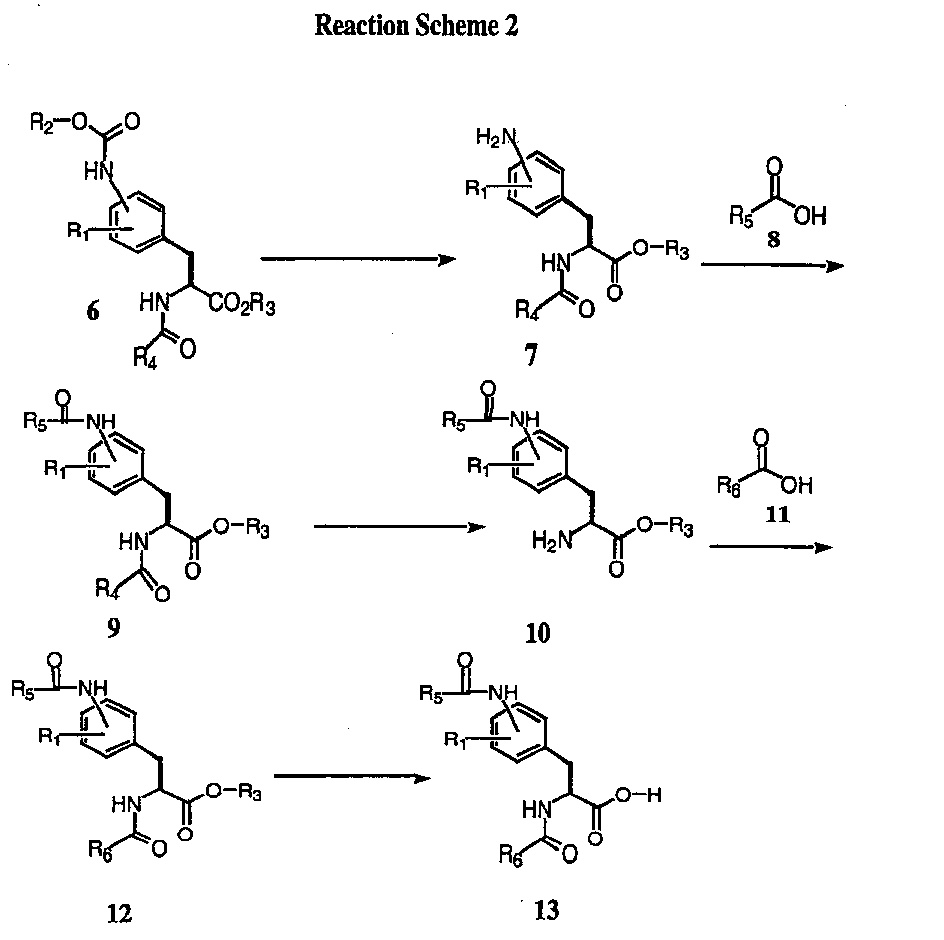

In reaction Scheme 3, a compound of formula **14** in which R₇ is a C₁₋₆ alkyl group which may serve as a protecting group or a group suitable for use in a prodrug for example methyl, ethyl, tert-butyl or the like or represents a connection to a solid phase resin, for example a Wang resin, is coupled with a carboxylic acid of formula **11** using standard peptide coupling conditions, for example HBTU in the presence of DIPEA in a polar, aprotic solvent such as DMF at a temperature between 0 °C and room temperature to give a compound of formula **15**. Reduction of the nitro group of **15** can be effected by catalytic hydrogenation for example using Pd© as a catalyst or by treatment with a standard reducing agent, for example SnCl₂. The resulting compound of structure **16** is useful as a key intermediate for several series of compounds. In the instance highlighted in Scheme 3, it can be coupled with an acid of formula **8** using standard peptide coupling conditions, for example HBTU in the presence of DIPEA in a polar, aprotic solvent such as DMF at a temperature between 0 °C and room temperature to give a compound of formula **17**. Compound **17** may be a compound of the invention depending on the nature of R₇ or may be converted to a compound of the invention by an appropriate hydrolysis procedure, for example in the case where R₇ is C₁₋₆ alkyl, by hydrolysis by treatment with excess alkali metal hydroxide, such as lithium hydroxide in aqueous alcohol. When R₇ represents a resin suitable for solid phase synthesis, appropriate hydrolysis conditions will depend on the choice of resin. In the case of Wang resin, treatment with trifluoroacetic acid in the presence of appropriate scavengers will lead to an acid of formula **18**.

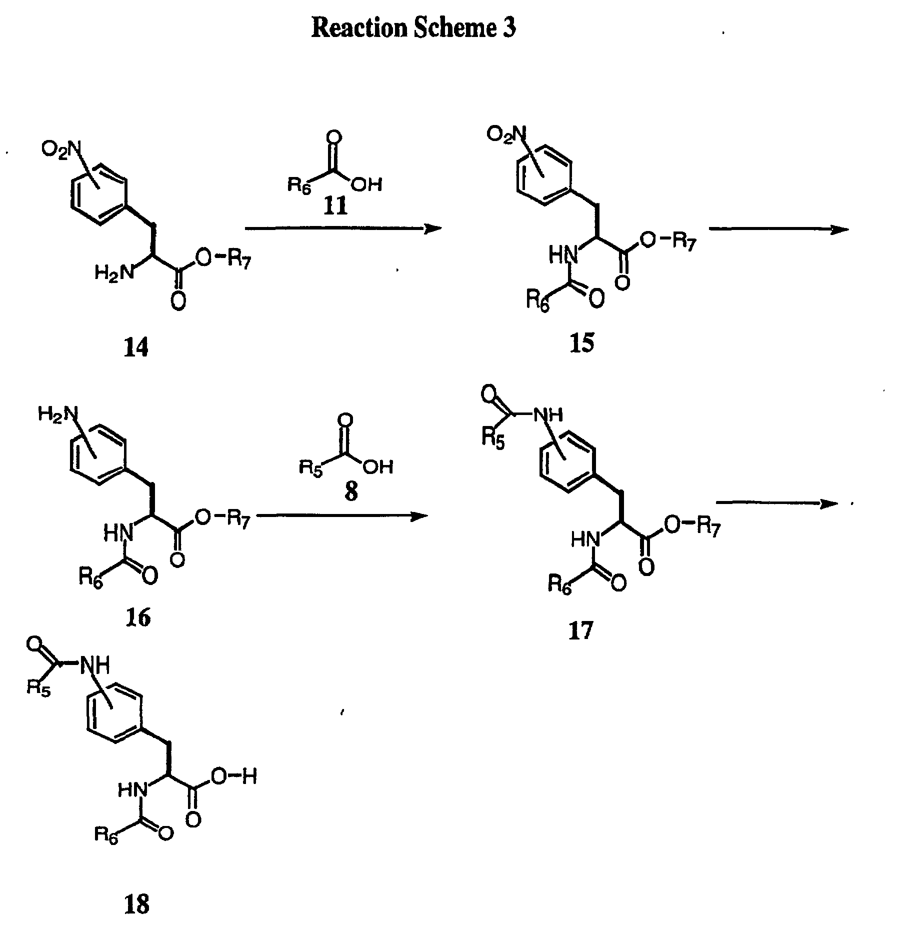

In a method particularly well suited for solid phase synthesis, an N'-Alloc-amino-N^{a}-Fmoc protected phenylalanine derivative of formula **19** can be coupled to a resin suitable for solid phase synthesis, for example, a Wang resin using standard coupling procedures, for example, by forming a mixed anhydride with 2,6-dichlorobenzoyl chloride and carrying out the coupling reaction in a polar, aprotic solvent such as N-methyl pyrrolidinone to give a compound of structure **20** in which R₇, represents the resin. The Alloc group may be removed by standard methods, for example by treatment with a reducing agent such as nBu₃SnH in the presence of a catalyst which is a source of Pd⁰, for instance, Pd(Ph₃P)₂Cl₂ to give an amine derivative of structure **21**. This compound can be coupled with a carboxylic acid of formula 8 using standard peptide coupling conditions, for example HBTU in the presence of DIPEA in a polar, aprotic solvent such as DMF at a temperature between 0 °C and room temperature to give a compound of formula **22**. The Fmoc protecting group may be removed from **22** using standard base treatment well known to those practicing peptide chemistry, for example with piperidine in DMF, to afford an amine of formula **23**. The resulting compound **23** can be coupled with a carboxylic acid of formula **11** using standard peptide coupling conditions, for example HBTU in the presence of DIPEA in a polar, aprotic solvent such as DMF at a temperature between 0 °C and room temperature to give a compound of formula **24**. Finally the compound of structure **24** can be cleaved from the resin under conditions dependent on the particular choice of resin. For example, in the case of a Wang resin, acid treatment with trifluoroacetic acid in dichloromethane in the presence of scavengers as necessary will afford a compound of formula **18**.

Depending on the particular synthetic target, the order of removal of the protecting groups from **19** may be altered so that the Fmoc group is first removed, coupling of the resulting amine with an acid of formula **11** is carried out followed by removal of the Alloc group and coupling of the product with an acid of formula **8** and cleavage from the resin. Also the choice of protecting groups can be modified to reflect the reactivities of the resin or choice of R₇, and the nature of any functional groups incorporated into R₅ and R₆.

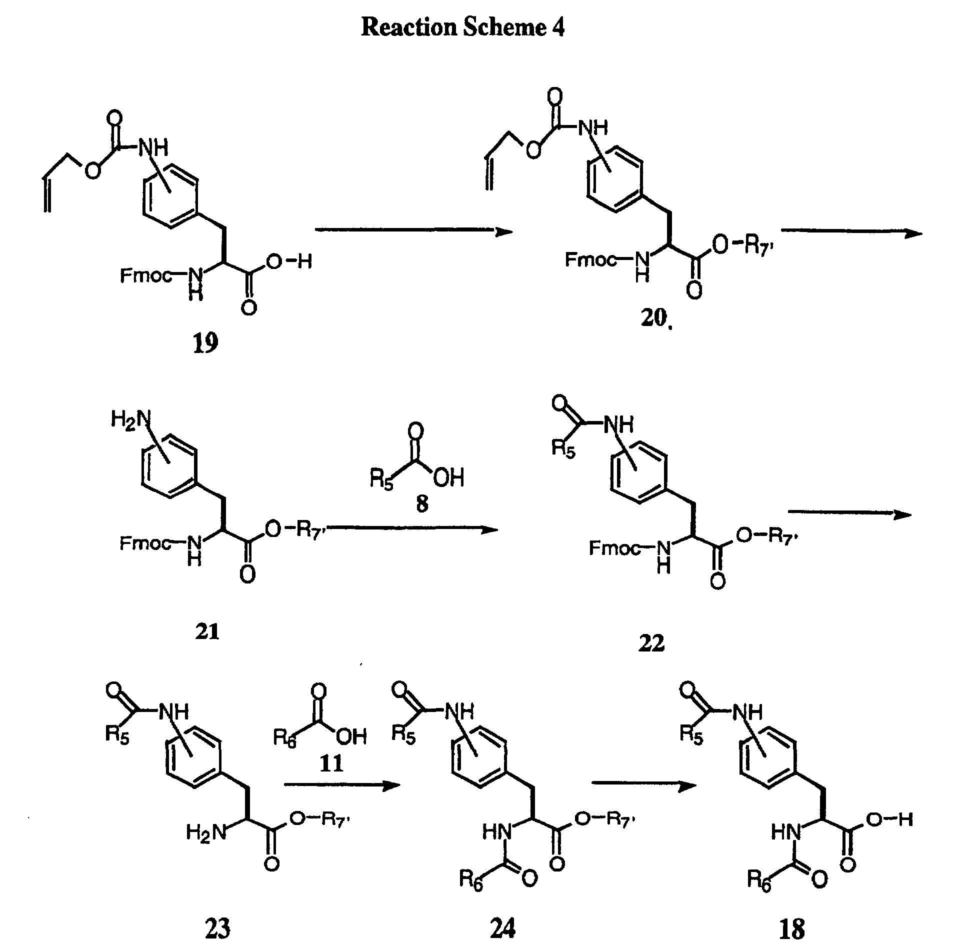

Compounds derived from 3- or 4-(alkylamino)phenylalanine derivatives can be prepared as outlined in Reaction Scheme 5. A compound of formula **16** or **7** may be treated with diazomethane in a suitable solvent, for example, ethyl ether to give products of formulas **25** and **26** respectively in which R₈ is methyl. Alternatively, the compound of structure **16** or **7** may be treated with an lower alkyl aldehyde or ketone, for example acetone, to give an intermediate Schiff's base which is in turn subjected to catalytic hydrogenation or reduction with sodium cyanoborohydride in the presence of an organic acid, for example acetic acid to give a compound of formula 25 or 26 in which R₈ is C₁₋₆ alkyl other than methyl. Conversion of compounds **25** or **26** to prodrug esters **27** or **28** or to the corresponding acids **29** or **30** respectively can be carried out as described above in Reaction Schemes 2 and 3.

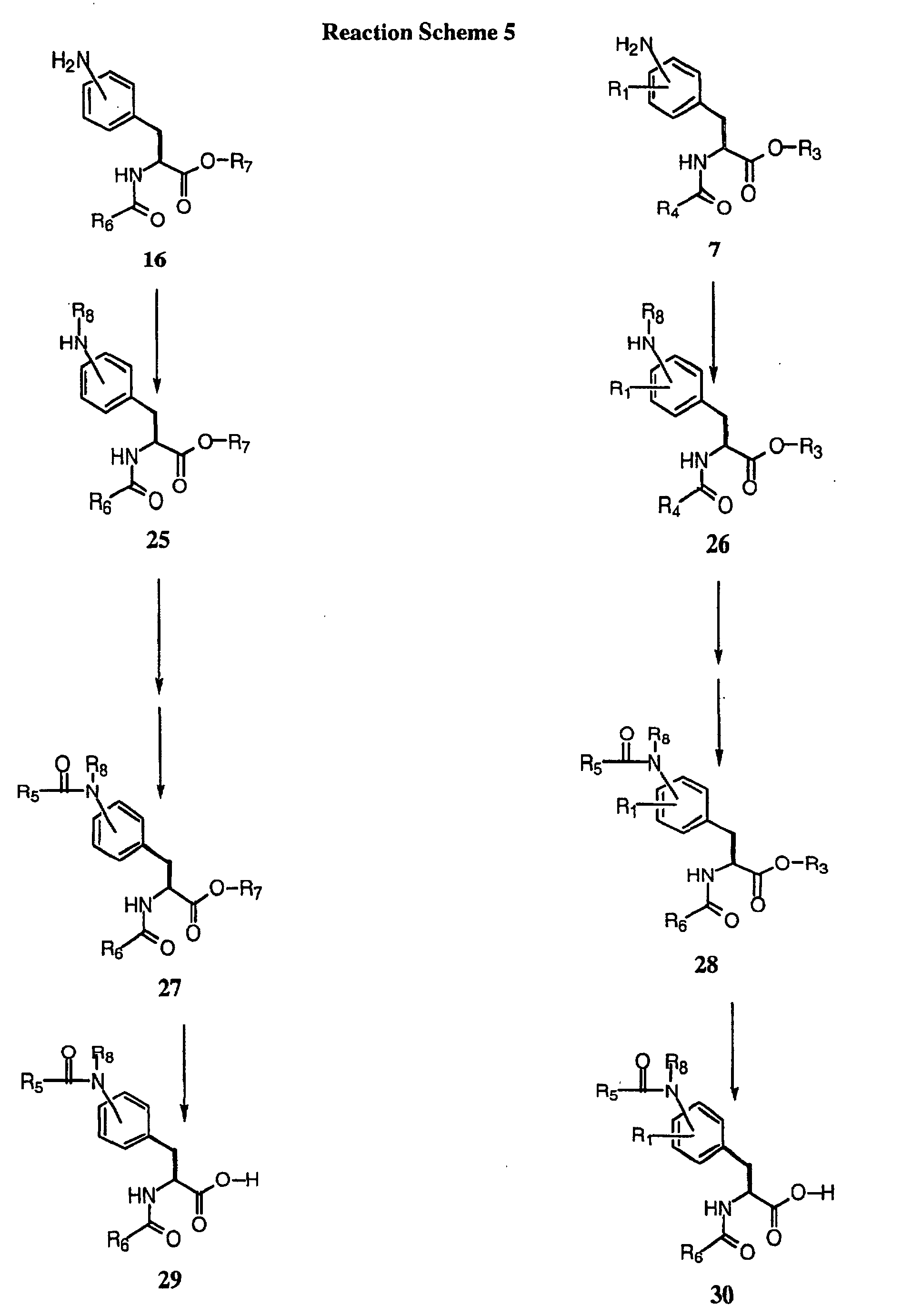

For the preparation of 3- or 4-sulfonylamino phenylalanine derivatives, compounds of formula **7, 16, 25** or **26** may be reacted with a sulfonyl chloride of formula **31,** in which R₉ is a substituted aryl or heteroaryl moiety, in an inert solvent, for example dichloromethane in the presence of a non-nucleophilic base, for example triethylamine or pyridine at about 0 °C to room temperature to give compounds of structure **32** or **33** respectively as illustrated in Reaction Scheme 6 for compounds **7** and **26**. These can be further converted to compounds of formulas **34** and **35** if desired using the general methods described above in Reaction Schemes 2 and 3.

For the preparation of compounds derived from 3- or 4-aminomethyl-phenylalanine, the procedure shown in Reaction Scheme 7 may be employed. A 3- or 4-hydroxymethyl benzoate of formula **36** in which R₁₀ is C₁₋₆ alkyl, which are known compounds, or can be prepared by known methods, is treated with a silylating agent in which R₁₁-R₁₃ are C₁₋₆ alkyl or phenyl, for example tert-butyldimethylsilyl chloride in an inert solvent, for example dimethylformamide in the presence of imidazole at about 0 °C to give a silyl protected compound of formula **37**. Reduction of **37** may be carried out using a variety of suitable reducing agents, for example, lithium aluminum hydride in an inert solvent such as ether or tetrahydrofuran at a temperature of about 0 °C followed by an aqueous workup to give an intermediate alcohol which can be oxidized by any of several oxidizing agents suitable for oxidizing benzyl alcohols to the corresponding aldehydes, for example activated manganese dioxide, to give an aldehyde of formula **38.** Monosilyl protected diols are alternatively available from 3- or 4-hydroxymethylbenzylalcohols by monosilylation and separation of the side products. Alternatively, an ester of formula **37** may be reduced directly to an aldehyde of formula **38** using diisobutylaluminum hydride at low temperature, for example at -78 °C.

Reaction of **38** to give a dehydroamino acid of formula **39** can be effected by treatment with a Wittig reagent of formula 4 in which R₃ is C₁₋₆ alkyl and R₄ is an alkoxy group, for example benzyloxy- or tert-butoxy- or represents a portion of one of the acyl groups of the compounds of the invention, for example ortho-substituted aryl or hetereoaryl. For example treatment of **38** with (±)-N-(benzyloxycarbonyl)-a-phosphonoglycine trimethyl ester in the presence of a suitable base for example tetramethyl guanidine leads directly to a dehydroamino acid of formula **39,** R₃ = methyl and R₄ = benzyloxy. Enantioselective reduction of **39** to the L-amino acid **40** can be effected by use of one of a number of reducing agents suitable for the purpose, for example, the recently described ethyl-DuPHOS rhodium reagent. It will be readily apparent to those skilled in the art that the optimal procedure for the further conversion of **40** into compounds of the invention will depend on the choices of R₄ and R₃. For the case wherein R₃ is C₁₋₆ alkyl and R₄ is benzyloxy, conversion to an amine of formula **41** can be conveniently effected by catalytic transfer hydrogenation of **40** over Pd© in a suitable solvent, for example, methanol in the presence of ammonium formate as the reducing agent. Acylation of **41** with a carboxylic acid of formula **11** can be carried as described above in Reaction Scheme 2 to give a compound of formula **42**. Conditions for removal of the silyl protecting group will depend on the particular choice of R₁₁-R₁₃. In the case of R₁₁, R₁₂ = methyl and R₁₃ = tert-butyl, this group is readily removed by treatment with a strong acid, for example hydrochloric acid in an appropriate solvent for the choice of R₃, for example where R₃ is methyl, methanol.

The resulting benzylic alcohol of formula 43 can be converted to an amine of formula 45 using procedures well established for similar transformations. For example, the alcohol of formula 43 can be converted to a leaving group, for example a mesylate by treatment with methane sulfonyl chloride in the presence of a proton acceptor, for example pyridine, followed by displacement with an alkali metal azide, for example sodium azide in a polar aprotic solvent such as dimethylformamide. Alternatively, the transformation from 43 to an azide of formula 44 can be carried out directly by treatment with diphenyl phosphorazidate as described in: Thompson, A. S.; Humphrey, G R.; DeMarco, A. M.; Mathre, D. J.; Grabowski, E. J. J. *J. Org. Chem*. 1993, *58*, 5886-5888. Reduction of the azide 44 to an amine of formula 45 can be carried out by a number of means suitable for the conversion of azides to amines, for example by treatment with a phosphine, for example triphenyl phosphine in an inert solvent such as dichloromethane or THF followed by an aqueous workup or by catalytic hydrogenation over an appropriate catalyst, for example Pd© in a solvent suitable for catalytic hydrogenations such as a lower alkanol or tetrahydrofuran. The resulting amine of formula 45 can be converted into the corresponding compounds of the invention using the procedures applicable to free amines described in the other reaction schemes. For example, coupling of 45 with a carboxylic acid of formula 8 under the conditions described in Reaction Scheme 2 leads to an amide of formula 46 which may be further converted to an acid of formula 47 if desired by base catalyzed hydrolysis as described in Reaction Scheme 2.

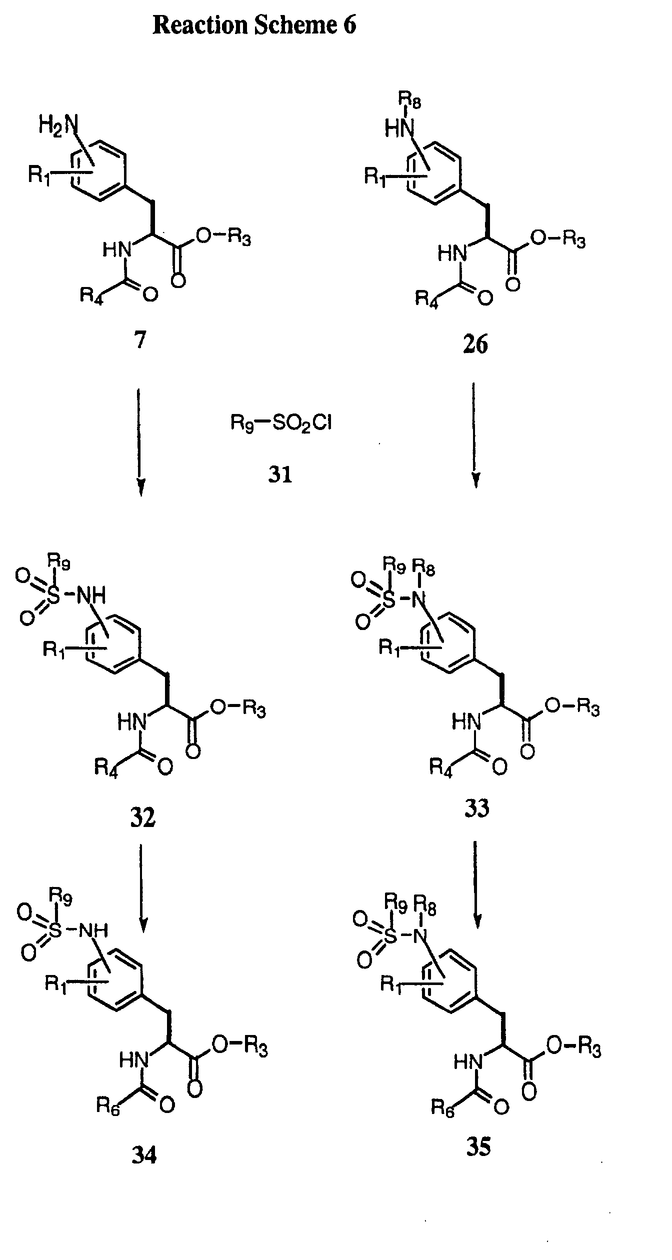

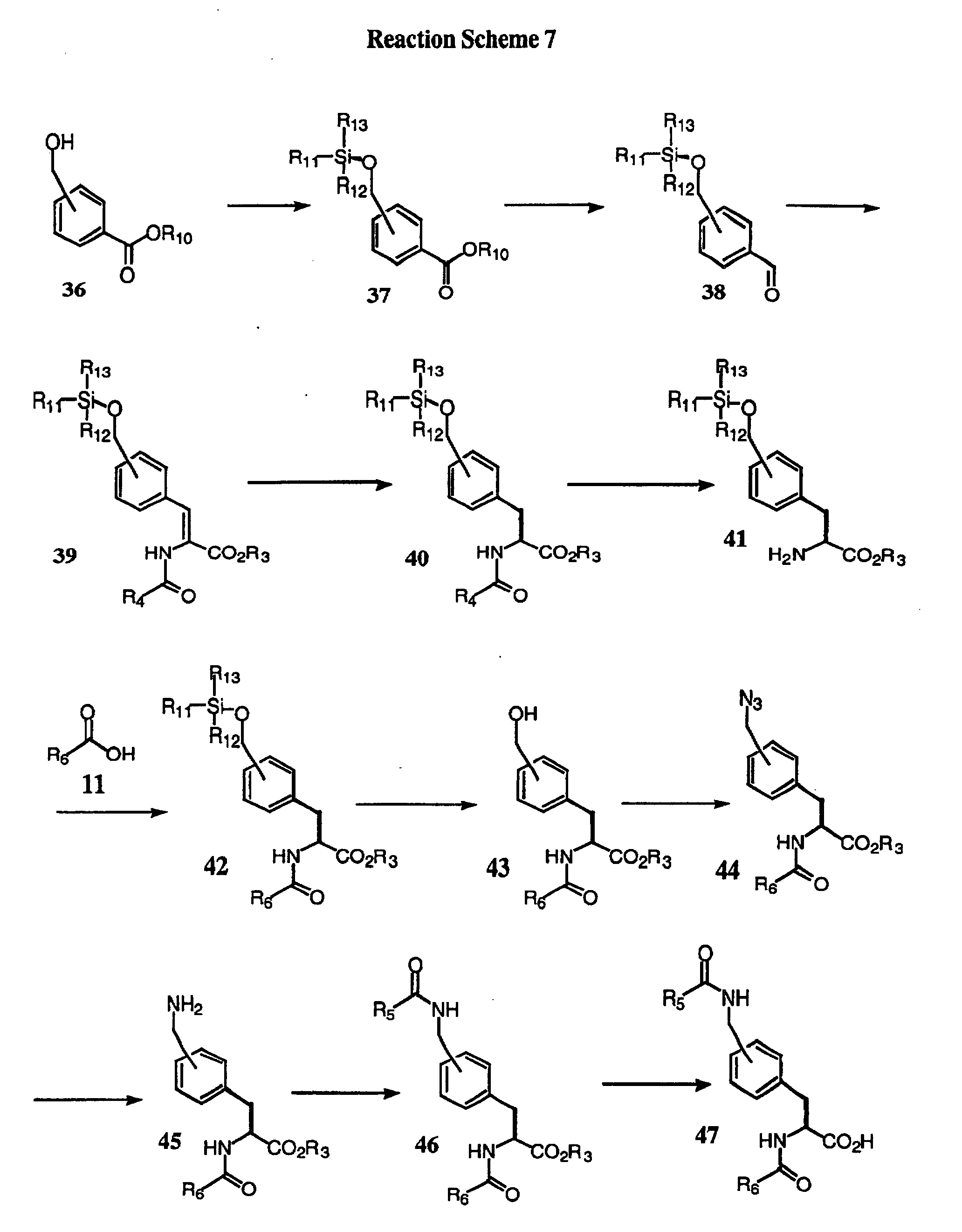

For the synthesis of urea derivatives, a compound of formula **26** can be treated with an isocyanate of formula **49,** wherein R₁₄ is substituted aryl, substituted heteroaryl or substituted C₁₋₆ alkyl with potentially reactive substituents protected as appropriate using conventional protecting group strategies, in a suitable inert solvent, for example dichloromethane, to give a urea of formula **50**. More generally, a compound of formula **26** can be treated with a phosgene equivalent, for example, triphosgene in an inert solvent such as dichloromethane in the presence of a non-nucleophilic proton acceptor, for example diisopropylethylamine, to give an intermediate of formula **48**. Subsequent treatment of a compound of formula **48** with an amine of formula **51** in which R₁₅ and R₁₆ are independently hydrogen, substituted C₁₋₆ alkyl, substituted aryl, substituted heteroaryl or taken together form a substituted 5, 6 or 7 membered ring leads to a compound of formula **52**. Further conversion, if necessary, of **50** or **51** to compounds of the invention can be carried out as described in Reaction Scheme 5.

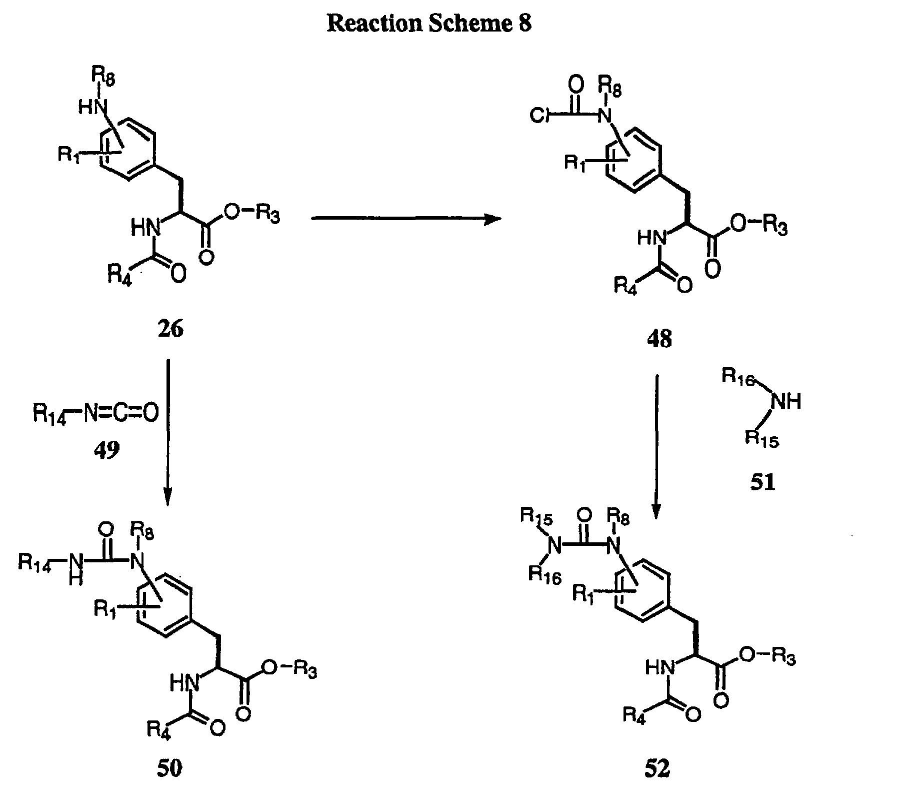

For the synthesis of imides, an aminophenylalanine derivative of structure **53** in which R₁ is H or C₁₋₆ alkyl, R₆ is as previously defined and R₇" is H or a readily cleavable group such as substituted benzyl, tert-butyl, allyl, or the like, or in the event that a prodrug ester is desired as the final product, is that ester group, for example ethyl, is employed. Compounds of formula **53** can be readily obtained from intermediates described above in Reaction Scheme 2. Reaction of a compound of formula **53** with a cyclic anhydride of formula **54** in an inert solvent, for example dichloromethane leads to a ring opened intermediate of formula **55**. The structure implied by **54** includes bicyclic molecules which may incorporate fused aromatic or heteroaromatic rings. In place of **54,** it is also possible to use dicarboxylic acids which are capable of forming cyclic imides. In the latter case, a condensing agent must be employed in the first step, for example carbonyl diimidazole. Treatment of the compound of formula **55** with a reagent such as carbonyl diimidazole capable of effecting cyclodehydration leads to an imide of formula **56.** Further manipulation of functional groups which were present on the anhydride of formula **54** and modification of R_{7"} may be carried out on compound **56** as desired to obtain further analogs using standard chemistry which is compatible with the presence of the imide functionality.

For the synthesis of compounds of the invention in which R₁ is halogen, preferably chloro, the appropriate halogen atom can be inserted at various points during the course of the synthesis depending on the nature of the additional functionality in the molecule. For example a compound of formula 6 in which R₁ is hydrogen can be treated with a mild chlorinating agent, for example, N-chlorosuccinimide in the presence of a proton acceptor, for example, sodium acetate to give the corresponding compound of formula 6 in which R₁ is chloro. In the case where 6 is derived from 3-amino-L-phenylalanine, a mixture of regioisomers may ensue which may be separated at a convenient point in the overall synthesis. Other intermediates described in the above schemes may be more suitable starting materials for halogenation for a particular target molecule. The particular merits of individual candidate starting materials will be apparent to those skilled in the art.

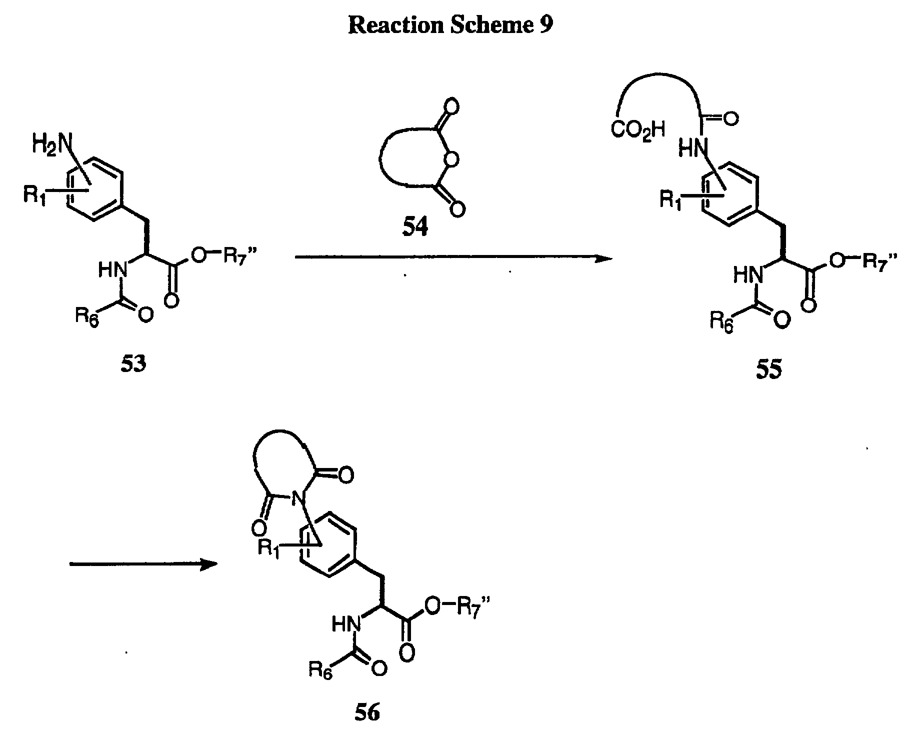

For the synthesis of the thiazolidinones of formula **62** described in reaction scheme 10, an aminophenylalanine derivative of structure **16,** in which R₆ and R₇ are as previously defined may be employed. Reaction of **16** with an a-mercapto carboxylic acid of formula **59** in which R₂₀ can be hydrogen, C₁₋₆ alkyl or aryl, for example a-mercapto acetic acid, and an aldehyde of formula **60** in which R₂₁ can be alkyl, hydroxyalkyl or a substituted aryl group, for example benzaldehyde, in an appropriate solvent such as benzene, THF or a lower alcohol, for example methanol, in the presence of a water scavenger such as 4Å molecular sieves at 60 to 80 °C provides compound of formula **61**. Compound **61** may be a compound of the invention depending on the nature of R₇ or may be converted to a compound of the invention by an appropriate hydrolysis procedure, for example in the case where R₇ is C₁₋₆ alkyl, by treatment with excess alkali metal hydroxide, such as sodium hydroxide in aqueous alcohol. When R₇ represents a resin suitable for solid phase synthesis, the appropriate hydrolysis conditions will depend on the choice of resin. In the case of Wang resin, treatment with trifluoroacetic acid in the presence of appropriate scavengers will lead to an acid of formula 62. The sequence may be initiated with related anilines, for example a compound of formula **7** in which R₁ is C₁₋₆ alkyl or halogen to give the corresponding thiazolidinones.

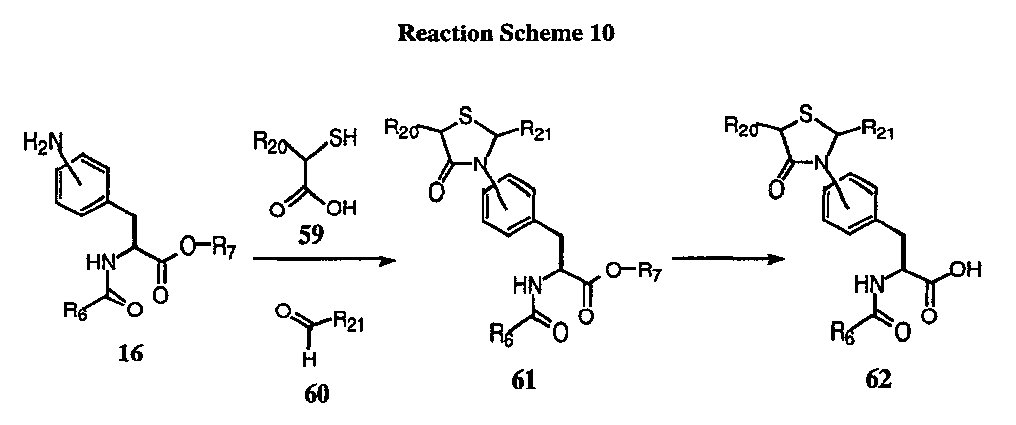

For the synthesis of imidazolidinones of formula **67** shown in reaction scheme 11, an aminophenylalanine derivative of structure **16** in which R₆ and R₇ are as previously defined may be employed. Compound **16** can be readily obtained through the synthesis described in reaction scheme 3. This compound can be coupled with a N-protected a-amino acid of formula **63**, in which R₂₂ can be a C₁₋₆ alkyl or an aryl group, R₂₃ can be a natural or unnatural D- or L-a-amino acid side chain or R₂₂ and R₂₃ together can form a ring, for example a proline or pipicolinic acid ring and R₂₄ may be a standard amine protecting group suitable for the particular selection of R₆, R₇, R₂₂, and R₂₃ for example tert-butoxycarbonyl. The coupling reaction can be effected using standard peptide coupling conditions, for example HBTU in the presence of DIPEA in a polar, aprotic solvent such as DMF at a temperature between 0 °C and room temperature to give a compound of formula **64**. Depending on the nature of protecting group R₂₄, an appropriate deprotection method is employed to give a compound of formula **65**. In the event that the protecting group R₂₄ is a Boc group, the deprotection can be carried out by the reaction of **64** with HCl in dioxane at room temperature. Reaction of compound **65** with an aldehyde of formula **60**, in which the R₂₁ is as defined above, in the presence of a water scavenger such as 4Å molecular sieves at 60 to 80 °C in an appropriate solvent, for example THF, provides a compound of formula **66**. Compound **66** may be a compound of the invention depending on the nature of R₇ or may be converted to a compound of the invention by an appropriate hydrolysis procedure, for example in the case where R₇ is C₁₋₆ alkyl, by hydrolysis by treatment with an alkali metal hydroxide, such as sodium hydroxide in aqueous alcohol to give a carboxylic acid of formula **67**.

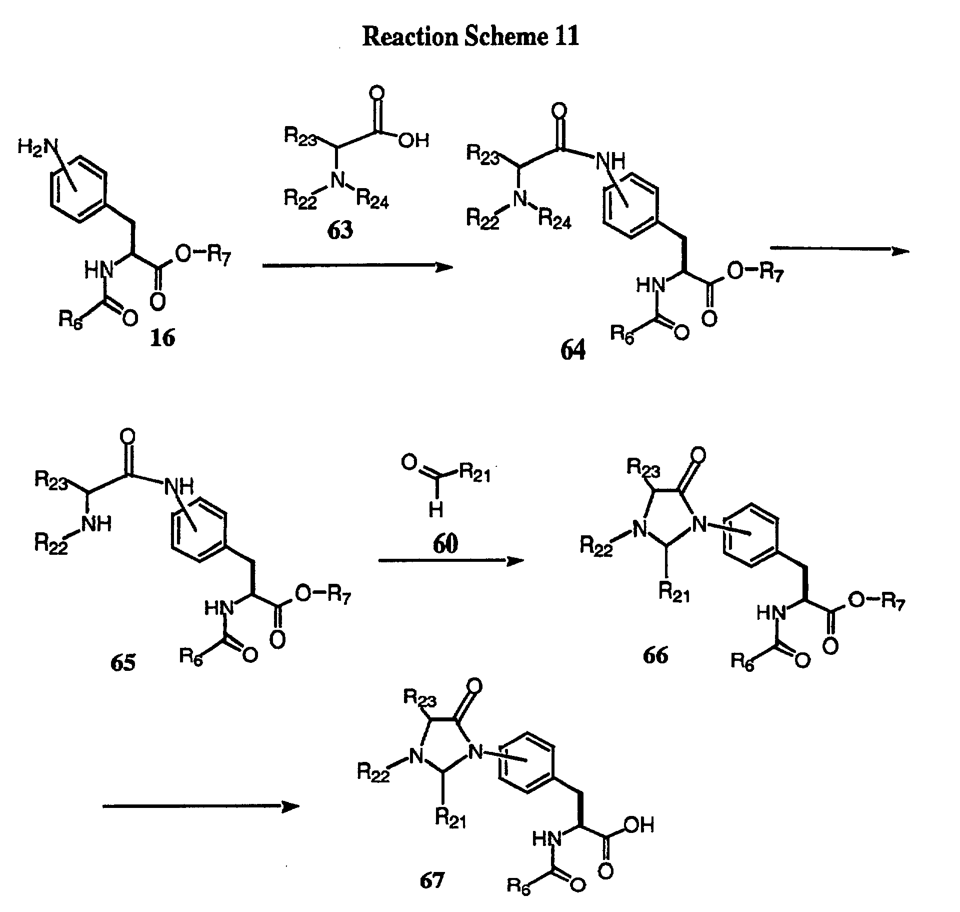

For the synthesis of imidazolidinones of formula **68** described in reaction scheme 12, an aminophenylalanine derivative of structure **16** in which R₆ and R₇ are as previously defined is employed. Compound **16** can be readily obtained through the synthesis described in reaction scheme 3 in the case of R₇ is C₁₋₆ alkyl. This compound can be coupled with a N-protected a-amino acid of formula **69**, in which R₂₅ can be a natural or unnatural, D- or L-a-amino acid side chain and R₂₆ is a nitrogen protecting group of the type conventionally used in peptide chemistry, for example, a Fmoc group, using standard peptide coupling conditions, for example HBTU in the presence of DIPEA in a polar, aprotic solvent such as DMF at a temperature between 0 °C and room temperature to give a compound of formula **70**. Depending on the nature of protecting group R₂₆, an appropriate deprotection method is employed to give compound of formula **71**. In the case of the protecting group R₂₆ is Fmoc group, it may be removed from **70** using standard base treatment well known to those practicing peptide chemistry, for example with piperidine in DMF, to afford an amine of formula **71**. The compound **71** can then react with an aldehyde **60**, in which R₂₁ is as previously defined, in the presence of a water scavenger such as **4A** molecular sieves in an appropriate solvent such as dichloromethane or THF at 25-60°C to give an imine of formula **72**. The imine **72** may then be treated with an acylating agent such as the acyl chloride of formula **74** in which R₂₇ can be an alkyl or aryl group in the presence of a base such DIPEA or DBU in an appropriate solvent such as dichloromethane or THF at 25-60 °C to give an acyl imidazolidinone of formula **73**. Alternatively, other reactive acylating group such as acid anhydrides or mixed anhydrides may be employed in this reaction. Compound **73** may be a compound of the invention, or depending on the nature of R₇ may be converted to a compound of the invention by an appropriate hydrolysis procedure, for example in the case where R₇ is C₁₋₆ alkyl, by hydrolysis by treatment with an alkali metal hydroxide, for example sodium hydroxide in aqueous alcohol to give, after acidification, a carboxylic acid of formula **68**. The sequence may be initiated with related anilines, for example a compound of formula **7** in which R₁ is C₁₋₆ alkyl or halogen to give the corresponding 3-acyl imidazolidinones.

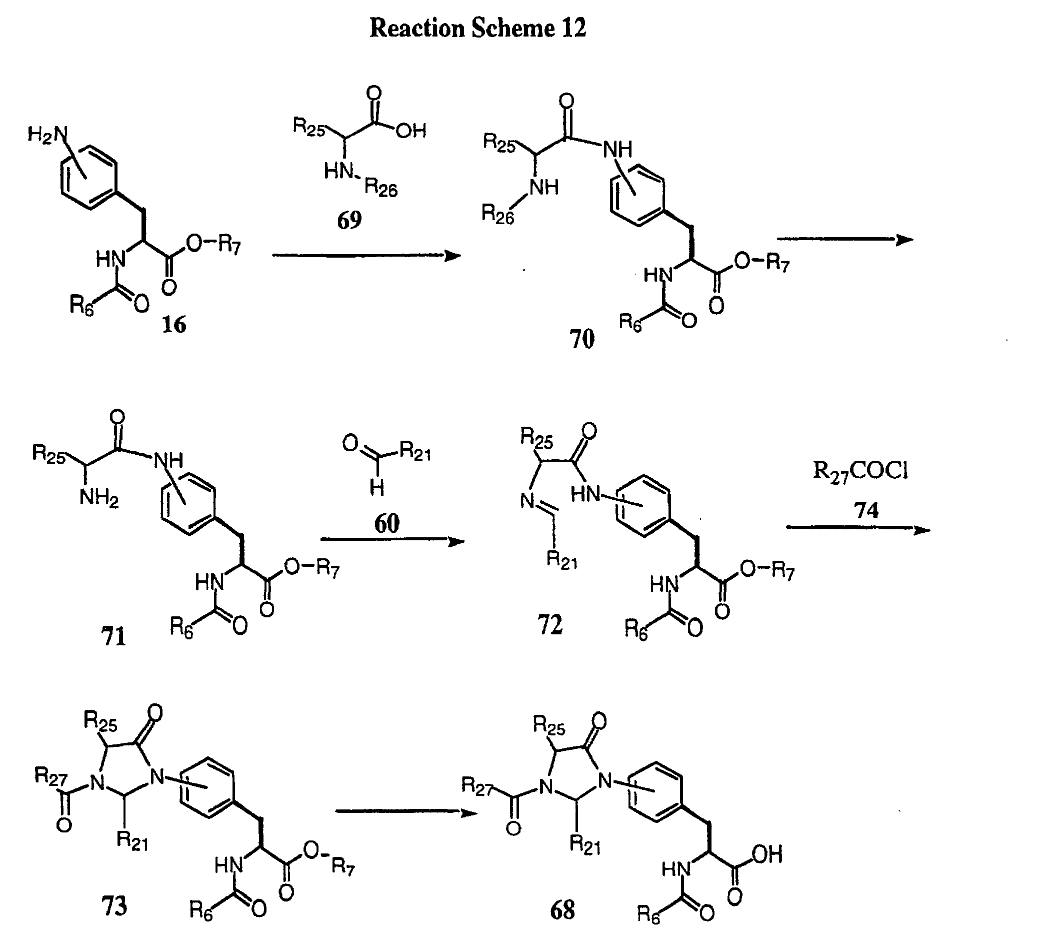

Ortho-substituted benzoic acid derivatives which are not commercially available can be prepared by conventional means. For example ortho-substituted aryl iodides or triflates may be carbonylated in the presence of carbon monoxide and a suitable palladium catalyst. The preparation of such iodide or triflate intermediates is dependent on the particular substitution pattern desired and they may be obtained by direct iodination or diazotization of an aniline followed by treatment with a source of iodide for example, potassium iodide. Triflates may be derived from the corresponding phenols by conventional means such as treatment with trifluoromethane sulfonic anhydride in the presence of a base such as triethylamine or diisopropylethylamine in an inert solvent. Other means of obtaining ortho-substituted benzoic acids involves treatment of an 2-methoxyphenyloxazoline derivative such as **75** with an alkyl Grignard reagent followed by hydrolysis of the oxazoline ring following the general procedure described by Meyers, A. I., Gabel, R., Mihelick, E. D, J. Org. Chem. **1978**, 43, 1372-1379., to give an acid of formula **76**. 2- or 2,6-Disubstituted benzonitriles also serve as convenient precursors to the corresponsing benzoic acids. In the case of highly hindered nitriles, for example 2-chloro-6-methylbenzonitrile, conventional hydrolysis under acidic or basic conditions is difficult and better results are obtained by DIBAL reduction to the corresponding benzaldehyde followed by oxidation using a chromium oxidizing reagent.

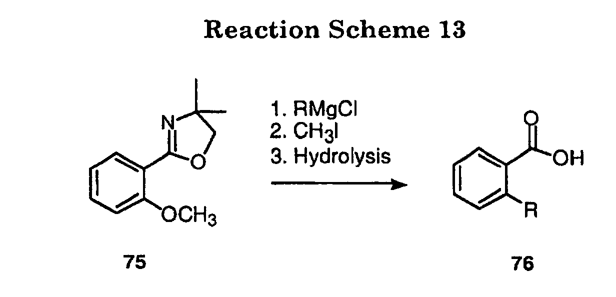

General Melting points were taken on a Thomas-Hoover apparatus and are 241 polarimeter. ¹H-NMR spectra were recorded with Varian XL-200 and Unityplus 400 MHz spectrometers, using tetramethylsilane (TMS) as internal standard. Electron impact (EI, 70 ev) and fast atom bombardment (FAB) mass spectra were taken on VG Autospec or VG 70E-HF mass spectrometers. Silica gel used for column chromatography was Mallinkrodt SiliCar 230-400 mesh silica gel for flash chromatography; columns were run under a 0-5 psi head of nitrogen to assist flow. Thin layer chromatograms were run on glass thin uncorrected. Optical rotations were determined with a Perkin-Elmer model layer plates coated with silica gel as supplied by E. Merck (E. Merck # 1.05719) and were visualized by viewing under 254 nm UV light in a view box, by exposure to I₂ vapor, or by spaying with either phosphomolybdic acid (PMA) in aqueous ethanol, or after exposure to Cl₂, with a 4,4'-tetramethyldiaminodiphenylmethane reagent prepared according to E. Von Arx, M. Faupel and M Brugger, *J. Chromatography*, **1976**, *120*, 224-228.

Reversed phase high pressure liquid chromatography (RP-HPLC)was carried out using either a Waters Delta Prep 4000 employing a 3 x 30 cm, Waters Delta Pak 15 µM C-18 column at a flow of 40 mL/min employing a gradient of acetonitrile:water (each containing 0.75% TFA) typically from 5 tp 95% acetonitrile over 35-40 min or a Rainin HPLC employing a 41.4 x 300 mm, 8 µM, Dynamax™ C-18 column at a flow of 49 mL/min and a similar gradient of acetonitrile:water as noted above. HPLC conditions are typically described in the format (5-95-35-214); this refers to a linear gradient of from 5% to 95% acetonitrile in water over 35 min while monitoring the effluent with a UV detector at a wavelenght of 214 nM.

Methylene chloride (dichloromethane), 2-propanol, DMF, THF, toluene, hexane, ether, and methanol, were Fisher reagent grade and were used without additional purification except as noted, acetonitrile was Fisher hplc grade and was used as is.

### Definitions:

THF is tetrahydrofuran,
DMF is N,N-dimethylformamide,
HOBT is 1-hydroxybenzotriazole,
BOP is [(benzotriazole-1-yl)oxy]tris-(dimethylamino)phosphonium hexafluorophosphate,
HATU is O-(7-azabenzotriazol-1-yl)-1,1,3,3-tetramethyluronium hexafluorophosphate
HBTU is O-benzotriazole-N,N,N',N',-tetramethyluronium hexafluorophosphate,
DIPEA is diisopropylethylamine,
DMAP is 4-(N,N-dimethylamino)pyridine
DPPA is diphenylphosphoryl azide
DPPP is 1,3-bis(diphenylphosphino)propane
DBU is 1,8-diazabicyclo[5.4.0]undec-7-ene
NaH is sodium hydride
brine is saturated aqueous sodium chloride solution
TLC is thin layer chromatography
LDA is lithium diisopropylamide
BOP-Cl is bis(2-oxo-3-oxazolidinyl)phosphinic chloride
NMP is N-methyl pyrrolidinone

### Examples

Example 1. Synthesis of 4-[[(2,6-dichlorophenyl)carbonyllamino]-N-[(1,1-dimethylethoxy)carbonyl]-L-phenylalanine methyl ester.

To a solution of 4-amino-N-[(1,1-dimethylethoxyl)carbonyl]-L-phenylalanine methyl ester (2.6 g, 8.6 mmol) in dichloromethane (20 mL) were added diisopropylethylamine (2.3 mL, 13 mmol) followed by 2,6-dichlorobenzoyl chloride (1.99 g, 9.5 mmol) at room temperature. The mixture was stirred for 15 hr at which time a white precipitate formed. The mixture was diluted with 30 mL of dichloromethane and 50 mL of water. The layers were separated and the aqueous layer was extracted with dichloromethane (2 x 50 mL). The combined extracts were washed with brine and dried over anhydrous magnesium sulfate. Filtration and concentration of the solvent gave 4.03 g (quant) of 4-[(2,6-dichlorophenylcarbonyl)amino]-N-[(1,1-dimethylethoxyl)carbonyl]-L-phenylalanine methyl ester as a white solid: mp 148-151 °C.

Example 2. Synthesis of 4-[[(2,6-dichlorophenyl)carbonyl]amino]-L-phenylalanine methyl ester hydrochloride salt.

4-[[(2,6-Dichlorophenyl)carbonyl]amino]-N-[(1,1-dimethylethoxy)carbonyl]-L-phenylalanine methyl ester (1.86 g, 4.0 mmol) was treated with 10 mL of 4 N hydrochloric acid in dioxane at room temperature. After 5 minutes, the solid went into solution and the mixture was stirred for 1 hr and 25 mL of ethyl ether was added to precipitate the product. The solids were collected by filtration and were washed with hexane. The resulting hydroscopic and gummy solids were dissolved in 50 mL of methanol and concentrated. After drying under high vacuum, 4-[[(2,6-dichlorophenyl)carbonyl]amino]-L-phenylalanine methyl ester hydrochloride salt (1.64 g, 97% ) was obtained as a light yellow solid, mp 158-161 °C.

Example 3. Syntheis of N-(2-chloro-6-methylbenzoyl)-4-[(2,6-dichlorophenyl)carbonyl]amino]-L-phenylalanine methyl ester.

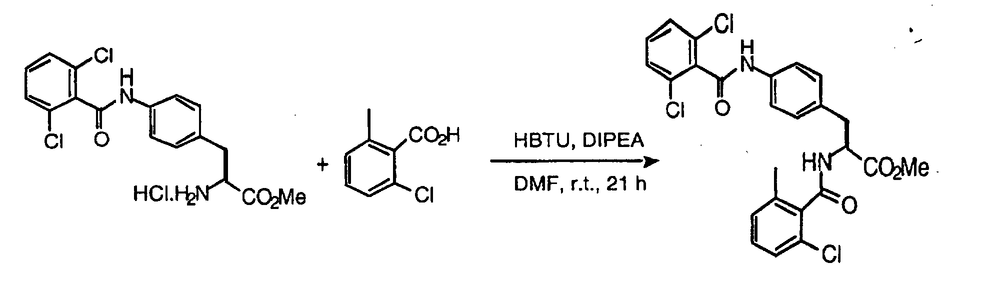

A solution of 4-[[(2,6-dichlorophenyl)carbonyl]amino]-L-phenylalanine methyl ester hydrochloride (1.23 g, 3.05 mmol), 2-chloro-6-methylbenzoic acid (0.50 g, 2.93 mmol), HBTU (1.16 g, 3.05 mmol) and DIPEA (1.33 mL, 7.6 mmol) in DMF (12 mL) was stirred 15 hr at room temperature.The mixture was diluted with ethyl acetate (250 mL) and was washed with 0.5 N HCl (2 x 80 mL), sat. sodium bicarbonate (2 x 80 mL) and brine (2 x 80 mL) and was dried (Na₂SO₄). The solution was filtered and concentrated to a yellow gum which was crystallized from ethyl acetate-hexane to give N-(2-chloro-6-methylbenzoyl)-4- [(2,6-dichlorophenyl)carbonyl]amino]-L-phenylalanine methyl ester (0.75 g), suitable for use in the next step. The mother liquors were concentrated and purified by silica gel chromatography eluting with 1:1 ethyl acetate:hexane to give an additional 0.625 g.

Examples 4 to 12. The compounds shown in below were prepared from 4-[[(2,6-dichlorophenyl)carbonyl]amino]-L-phenylalanine methyl ester hydrochloride and the appropriate benzoic acid derivatives according to the method described in example 3.

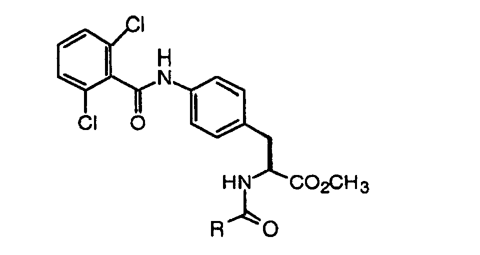

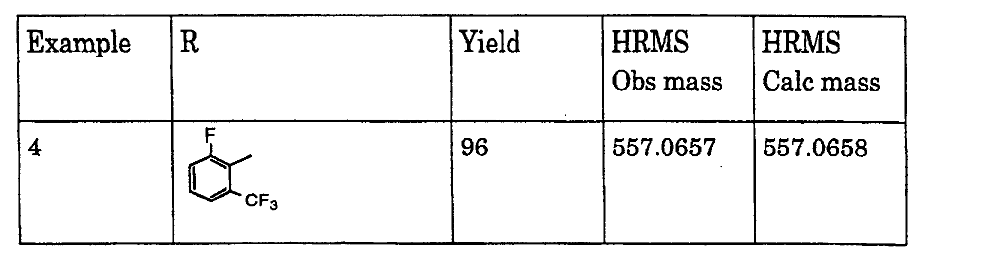

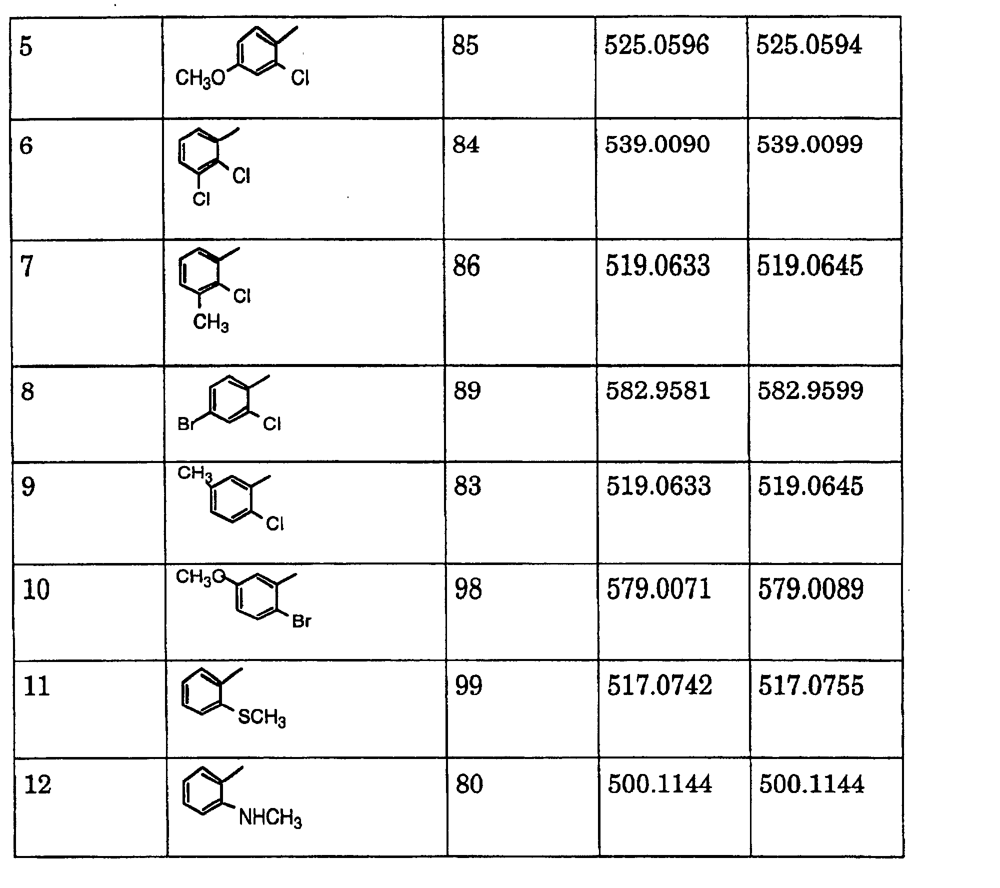

Example 13. Synthesis of N-(2-chloro-6-methylbenzoyl)-4-[(2,6-dichlorophenyl)carbonyl] amino] -L-phenylalanine

A solution of N-(2-chloro-6-methylbenzoyl)-4-[(2,6-dichlorophenyl)carbonyl]amino]-L-phenylalanine methyl ester (1.31 g, 2.6 mmol) in ethanol (45 mL) and 1.0 N sodium hydroxide (45 mL, 45 mmol) was stirred over night at room temperature to give a clear solution. The mixture was neutralized with 1 N hydrochloric acid to precipitate 1.28 g of a white solid. The mother liquor was extracted with ethyl acetate (2 x 50 mL) and the combined extracts were washed with sat. brine, dried (Na₂SO₄) and evaporated to give 0.56 g. Recrystallization of the first crop from ethyl acetate afforded N-(2-chloro-6-methylbenzoyl)-4-[(2,6-dichlorophenyl)carbonyl]amino]-L-phenylalanine (0.77 g). Recrystallization of the second crop from ethyl acetate afforded an additional 0.20 g. FAB HRMS: obs. mass 505.0483. Calcd mass, 505.0488 (M+H).

Example 14. Synthesis of N-(2-chloro-6-methylbenzoyl)-4-[(2,6-dichlorophenyl)carbonyl]amino]-L-phenylalanine sodium salt

A solution of N-(2-chloro-6-methylbenzoyl)-4-[(2,6-dichlorophenyl)carbonyl]amino]-L-phenylalanine (0.15 g) in 1.0 N NaOH (0.3 mL) was applied to a 2 x 20 cm open column of C-18 reversed phase silica gel (40-63 µM, RP Silica Gel60, as supplied by EM Separations, Cat. 10167) eluting with water, then with 40-50% methanol in water to give N-(2-chloro-6-methylbenzoyl)-4-[(2,6-dichlorophenyl)carbonyl]amino]-L-phenylalanine sodium salt (147 mg) as an amorphous white solid after lyophilization.

Examples 15 -30. The compounds shown below were prepared from the corresponding methyl esters using the method described in example 13.

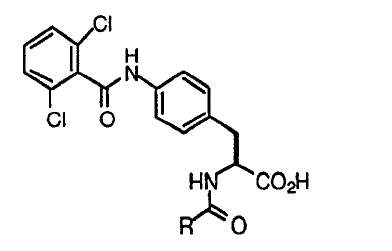

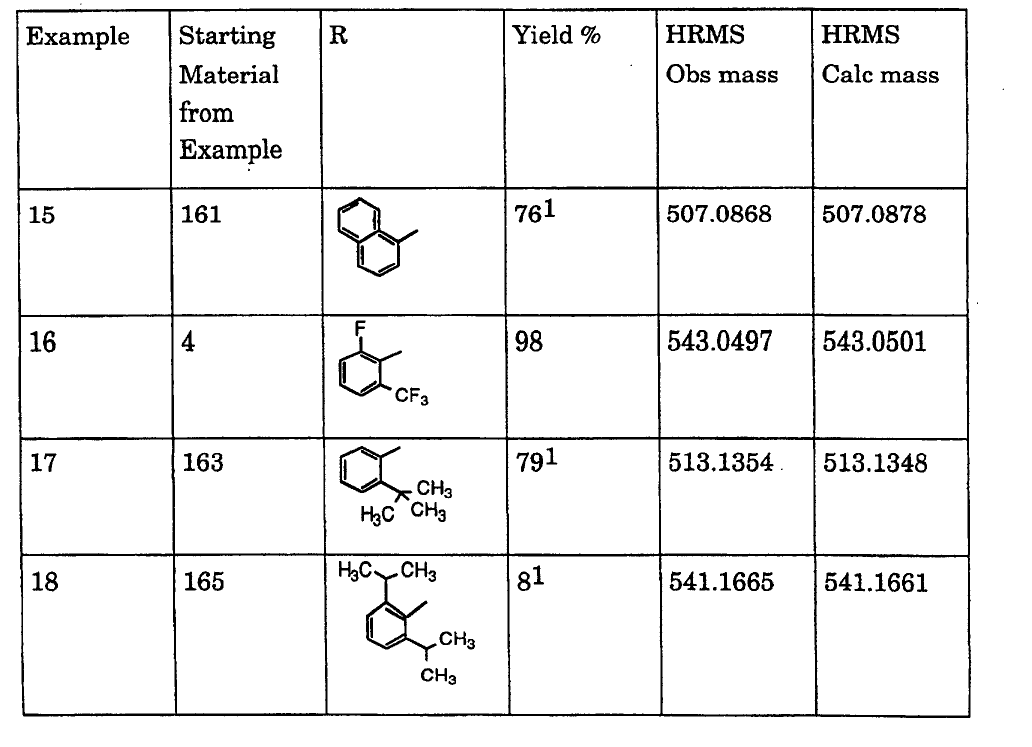

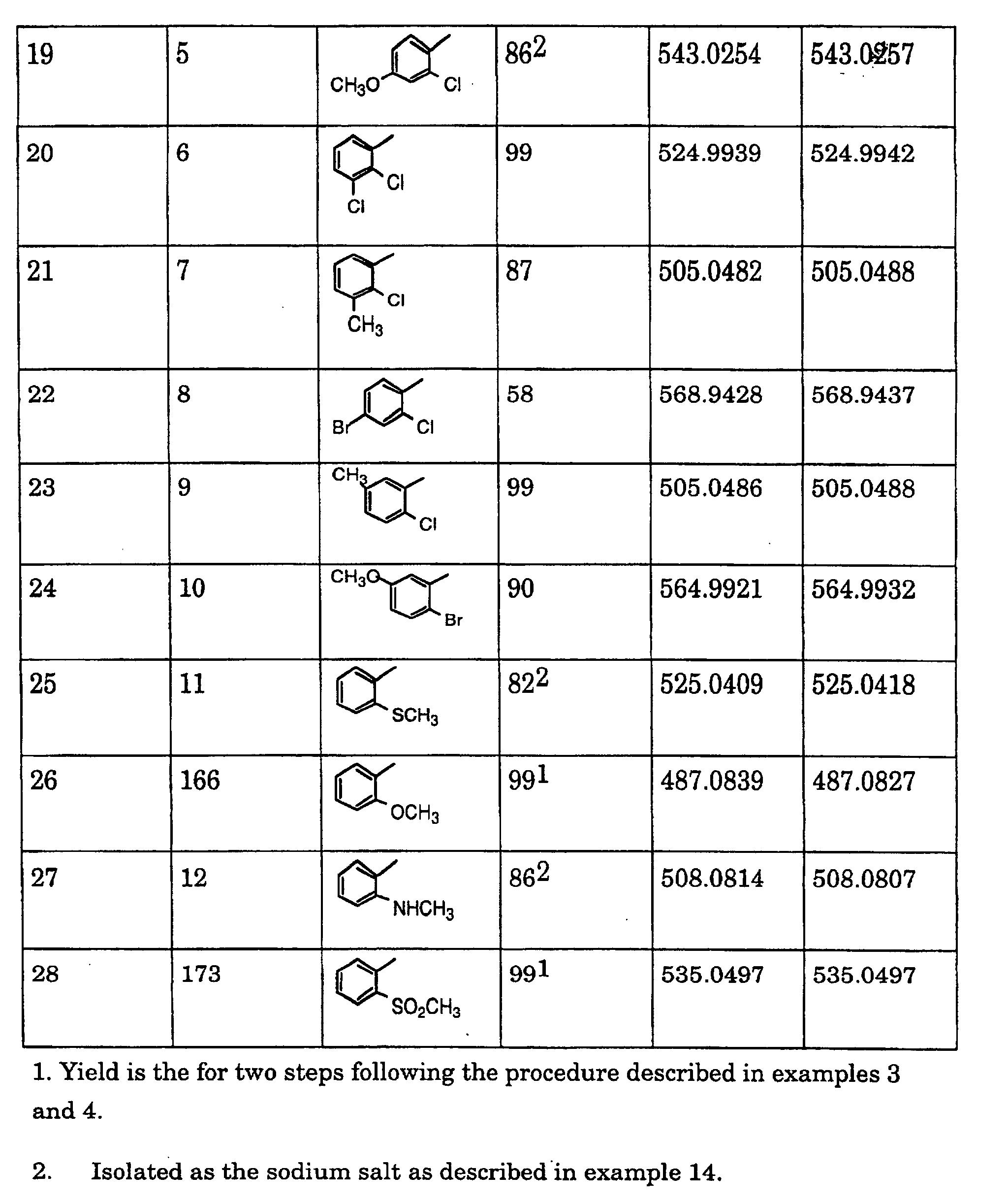

Example 29. Synthesis of 4-[[(2,6-dichlorophenyl)carbonyl]amino]-N-[[2-(methylthio)phenyl]carbonyl]-L-phenylalanine methyl ester

A solution of 4-[(2,6-dichlorophenyl)carbonyl]amino]-N-[(2-(methylthio)phenyl]carbonyl]-L-phenylalanine methyl ester (0.25 g, 0.48 mmol) and oxone (147 mg, 0.24 mmol) in ethyl acetate (12 mL) and water (6 mL) was stirred at room temperature for 2 hr and a second portion of oxone (147 mg, 0.24 mmol) was added. The mixture was stirred over night at which time TLC (20:1 dichloromethane:methanol) suggested the presence of starting material and sulfone in addition to two sulfoxides. The layers were sepatated, the aqueous layer was extracted with ethyl acetate and the combined extracts were washed with sat. brine and were dried (Na₂SO₄). The residue after concentration was chromatogrpaphed on silica gel eluting with 20:1 dichloromethane:methanol to give 4-[[(2,6-dichlorophenyl)carbonyl]amino]-N-[[2-(methylsulfinyl)phenyl]carbonyl]-L-phenylalanine methyl ester (218 mg) as a mixture of diastereomers.

Example 30. Synthesis of 4-[[(2,6-dichlorophenyl)carbonyl]amino]-N-[[2-(methylsulfinyl)phenyl]carbonyl]-L-phenylalanine.

Hydrolysis was carried out as described in example 13. Starting with 4-[(2,6-dichlorophenyl)carbonyl]amino]-N-[[2-(methylsulfinyl)phenyl]carbonyl]-L-phenylalanine methyl ester (214 mg, 0.41 mmol) and isolation of the product by RP HPLC, eluting with acetonitrile:water followed by lyophylization gave the more polar diastereomer 4-[[(2,6-dichlorophenyl)carbonyl]aminol-[[(N-(2-methylsulfinyl)phenyl]carbonyl]-L-phenylalanine (63.6 mg) as an amorphous solid, HR MS: Obs. mass, 541.0385. Calcd. mass, 541.0368 (M+Na) followed by the less polar diastereomer (74.2 mg), HR MS: Obs. mass, 541.0351. Calcd. mass, 541.0368 (M+Na).

Example 31. Synthesis of 4-[[(2,6-dichlorophenyl)carbonyl]amino]-N-[[(2-phenylmethyl)phenyl]carbonyl]-L-phenylalanine
a. A solution of 4-[[(2-propenyloxy)carbonyl]amino]-L-phenylalanine methyl ester (935 mg, 3.54 mmol), HOAT (658 mg, 5.31 mmol), 2-benzylbenzoic acid (1.13 g, 5.31 mmol) and DCC (1.09 g, 5.31 mmol) in DMF (20 mL) was stirred over night at room temperature. The mixture was diluted with water and extracted with ethyl acetate. The combined extracts were washed with water and sat. brine, dried (Na₂SO₄), filtered and evaporated. The residue was recrystallized from ethyl acetate containing small amounts of dichloromethane and methanol to give 4-[[(2-propenyloxy)carbonyl]amino]-N-[[(2-phenylmethyl)phenyl]carbonyl]-L-phenylalanine (1.21g, 74%) suitable for use in the next step.
b. Argon was passed through a solution of 4-[[(2-propenyloxy)carbonyl]amino]-N-[[(2-phenylmethyl)phenyl]carbonyl]-L-phenylalanine methyl ester (1.21 g, 2.63 mmol) and tetrakis(triphenlphosphine)palladium (61 mg, 0.053 mmol) in 45 mL of dichloromethane for 5 min and tributyltin hydride (800 µL, 2.9 mmol) was added. After 1.5 hr at room temperature, the mixture was diluted with dichloromethane (50 mL) and was washed with sat. NaHCO₃ and brine, dried (Na₂SO₄) and concentrated. The residue was dissolved in dichloromethane and ether and hexane were added to precipitate 99 mg of a white solid. The filtrate was concentrated and the residue was recrstallized from dichloromethane to give 4-amino-N-[[(2-phenylmethyl)phenyl]carbonyl]-L-phenylalanine methyl ester (594 mg).
c. A mixture of 4-amino-N-[[(2-phenylmethyl)phenyl]carbonyl]-L-phenylalanine methyl ester (200 mg, 0.52 mmol), 2,6-dichlorobenzoyl chloride (131 mg, 0.62 mmol) and triethylamine (108 µL, 0.78 mmol) in 5 mL of dichloromethane was stirred 6 hr at room temperature. The mixture was diluted with dichloromethane (10 mL) and washed with water and sat. brine. The organic layer was dried (Na₂SO₄) and the residue was chromatographed on silica gel, eluting with 20-60% ethyl acetate in hexane to afford 4-[[(2,6-dichlorophenyl)carbonyl]amino]-N-[[(2-phenylmethyl)phenyl]carbonyl]-L-phenylalanine methyl ester (195 mg) as an off white solid.
d. A solution of 4-[[(2,6-dichlorophenyl)carbonyl]amino]-N-[[(2-phenylmethyl)phenyl]carbonyl]-L-phenylalanine methyl ester (195 mg, 1.4 mmol) and lithium hydroxide (33.5 mg, 1.4 mmol) in THF:methanol:water (6 mL, 3:1:1) was stirred over night at room temperature and was concentrated. The residue was triturated with 1 N aqueous HCl for 10 min and the solids were collected by centrifugation, washing with water and ether to give 4-([(2,6-dichlorophenyl)carbonyl]amino]-N-[[(2-phenylmethyl)phenyl]carbonyl]-L-phenylalanine (165 mg) as a white powder which was 97% pure by hplc analysis. FAB MS 569 (M+Na)(1 Cl), 547 (M+H)(1 Cl).

Example 32. Synthesis of 4-[[(2,6-dichlorophenyl)carbonyl]amino]-N-[[2-chloro-4-[[[(3-hydroxyphenyl)methyl]amino]carbonyl]phenyl]carbonyl]-L-phenylalanine
a. In an inert atmosphere, a solution of 3-chloro-4-methoxycarbonylbenzoic acid (1.13 g; 5.27 mmol), 3-hydroxybenzylamine hydrochloride (0.85g; 5.35 mmol) and HBTU (2.08 g; 5.485 mmol) in dimethylformamide (15 mL) was stirred while DIPEA (3.54 mL; 26.33 mmol) was added. The reaction mixture was stirred overnight at room temperature, then the volatiles were removed *in vacuo*. The amber oily residue was partitioned between ethyl acetate (50 mL) and 0.5 N HCl (30 mL) and the organic extract was washed in turn with brine (30 mL), saturated NaHCO₃ solution (30 mL) and brine (30 mL). The aqueous layers were backwashed in turn with ethyl acetate (30 mL). Evaporation of the combined, dried (MgSO₄) organic layers afforded 1.7 g of crude product. The material was chromatographed (silica gel; 50 g) and eluted with ethyl acetate-hexane (2: 3) to give the amide as an colorless oil (1.3 g). Crystallization from ether-hexane furnished 1.12 g of 2-chloro-4-[[(3-hydroxyphenyl)amino] carbonyl]benzoic acid methyl ester as a colorless solid. FAB HRMS: (C₁₆H₁₄ClNO₄) Obs. Mass 320.0681 Calcd. Mass 320.0689 (M+H).
b. A solution of 2-chloro-4-[[(3-hydroxyphenyl)amino]carbonyl]benzoic acid methyl ester (900 mg; 2.82 mmol) in an aqueous 0.5 N sodium hydroxide solution (20 mL) was stirred at room temperature under argon. After 2 hr, the solution was acidified with 1 N HCl (11 mL) and the resulting colorless solid was filtered, washed with water, and dried *in vacuo* to give 840 mg of 2-chloro-4-[[(3-hydroxyphenyl)amino]carbonyl]benzoic acid. FAB HRMS: (C₁₅H₁₂ClNO₄) Obs. Mass 306.0548 Calcd. Mass 306.0533 (M+H)
c. In an argon atmosphere, to a stirred solution of 2-chloro-4-[[(3-hydroxyphenyl)amino]carbonyl]benzoic acid (45 mg; 0.1472 mmol), 4-(2,6-dichlorobenzoylamino)-L-phenylalanine methyl ester (60 mg; 0.1488 mmol) and HBTU (59 mg; 0.16 mmol) in dimethylformamide (3 mL) was added DIPEA (0.102 mL; 0.585 mmol). The reaction mixture was stirred for 17 hr at room temperature, then was concentrated to dryness *in vacuo* and the residue was partitioned between dichloromethane (25 mL) and 0.5 N HCl (10 mL). The organic layer was washed with water and the aqueous layers were backwashed in turn with dichloromethane. The combined dichloromethane extracts were dried (Na₂SO₄) and evaporated to give 80 mg of crude material that was crystallized from methanol-ethyl acetate to provide 38 mg of N-[[2-chloro-4-[[(3-hydroxyphenyl)amino]carbonyl]phenyl]carbonyl]-4-[[(2,6-dichlorophenyl)carbonyl]amino]-L-phenylalanine methyl ester, mp 230-232 °C. FAB HRMS: (C₃₂H₂₆Cl₃N₃O₆) Obs. Mass 654.0952 Calcd. Mass 654.0965 (M+H).
d. A solution of N-[[2-chloro-4-[[(3-hydroxyphenyl)amino]carbonyl]phenyl]carbonyl]-4-[[(2,6-dichlorophenyl)carbonyl]amino]-L-phenylalanine methyl ester (35 mg; 0.053 mmol) in methanol (0.35 mL) and tetrahydrofuran (0.35 mL) was treated with an aqueous 1N lithium hydroxide solution (0.16 mL) and the mixture was stirred at room temperature under argon for 90 minutes. The solution was concentrated under reduced pressure, then was diluted with water (5 mL) and extracted with diethyl ether (2 x 5 mL). The separated aqueous layer was acidified with 1 N HCl (0.18 mL) and the resulting colorless solid was filtered off, washed with water, and dried to give 29 mg ofN-[[2-chloro-4-[[(3-hydroxyphenyl)amino]carbonyl]phenyl]carbonyl]-4-[[(2,6-dichlorophenyl)carbonyl]amino]-L-phenylalanine. FAB HRMS: (C₃₁H₂₄Cl₃N₃O₆) Obs. Mass 640.0821 Calcd. Mass 640.0809 (M+H).

Example 33. Synthesis of 4-[[(2,6-dichlorophenyl)carbonyl]amino]-N-[[2-chloro-4-[5-[[(3-hydroxyphenyl)methyl]amino]-1H-tetrazol-1-yl]phenyl]carbonyl]-L-phenylalanine
a. A stirred suspension of 3-hydroxyphenylacetic acid (10.2 g; 67 mmol) in acetic anhydride (100 mL; 1.06 mol) under anhydrous conditions was treated with pyridine (0.5 mL). In the mildly exothermic reaction, the solids dissolved within several minutes and the mixture was maintained at 40 °C for five hours. The reaction was concentrated *in vacuo* to about half volume, then water (30 g) in the form of ice chips was added at such a rate that the temperature remained < 45 °C. When the exotherm had subsided, a second portion of water (200 mL) was added slowly and the mixture was stirred for another 30 minutes. The precipitated solid was filtered, washed with water and dried to constant weight *in vacuo* over P₂O₅ to give 3-acetoxyphenylacetic acid (11.7 g) which was used without further purification.
   In an inert atmosphere, a solution of the above 3-acetoxyphenylacetic acid (1.942 g; 10 mmol), diphenylphosphoryl azide (2.8 g; 10.17 mmol) and DIPEA (1.92 mL; 11 mmol) in benzene (25 mL) was stirred at room temperature for 1 hr, then the reaction temperature was slowly raised to 70 °C. Evolution of gas began to be evident as the reaction temperature reached approximately 55 °C and became much more vigorous as the reaction temperature approached 70 °C. Within 30 minutes at that temperature gas evolution had stopped and the reaction solution containing the formed 3-acetoxybenzylisocyanate was cooled to 40 °C. Another portion of DIPEA (3.84 mL; 22 mmol) was added, followed by 4-amino-2-chlorobenzoic acid methyl ester hydrochloride salt (2.95 g; 13.3 mmol) and the brownish purple solution was stirred and heated at reflux under argon overnight. The reaction mixture was cooled, diluted with benzene (50 mL) and washed in turn with 1N HCl (50 mL) and dilute brine. The aqueous layers were re-extracted with benzene, then the combined, dried (MgSO₄) organic extracts were evaporated and the crude residue was purified by HPLC (silica gel; ethyl acetate-hexane-2:3). Evaporation of the appropriate fractions provided 3.24 g of the solid urea which was then crystallized from dichloromethane-ethyl acetate to give 4-[3-(3-acetoxybenzyl)ureido]-2-chlorobenzoic acid methyl ester (2.71 g) as a colorless solid, mp 113-114 °C. FAB HRMS: (C₁₈H₁₇ClN₂O₅) Obs. Mass 377.0898 Calcd. Mass 377.0905 (M+H).
b. In a dry argon atmosphere, a solution of triphenylphosphine (1.684 g; 6.42 mmol), diethyl azodicarboxylate (1.13 g; 6.42 mmol) and 4-[3-(3-acetoxybenzyl)ureido]-2-chlorobenzoic acid methyl ester (1.21 g; 3.21 mmol) in dry THF (30 mL) was treated with trimethylsilyl azide (0.86 mL; 6.48 mmol) and was stirred at room temperature for 24 hr. Examination of the reaction mixture by TLC suggested the presence of considerable starting material, so additional amounts of triphenylphosphine (0.842 g; 3.21 mmol), diethyl azodicarboxylate (0.565 g; 3.21 mmol) and trimethylsilyl azide (0.43 mL; 3.21 mmol) were added. The reaction was stirred at room temperature for an additional 40 hr. After the solvents were removed under reduced pressure, the residue was taken up in dichloromethane (100 mL) and washed with water (2 x 50 mL). The aqueous extracts were backwashed in turn with dichloromethane (50 mL) and the combined, dried (MgSO₄) extracts were evaporated *in vacuo*. From a previous experiment it had been established that the reaction yielded a complex, difficultly separable, mixture of several products, some deacetylated and/or de-esterified. Accordingly, in this experiment, the residue was dissolved in a mixture of methanol ( 30 mL) and 1N lithium hydroxide (15 mL) and the mixture was stirred at room temperature for 2 hr to complete the hydrolyses of both the ester and phenolic acetate groups. Most of the volatiles were removed under reduced pressure then the basic solution was diluted with water (20 mL) and washed with dichloromethane (2 x 30 mL). The aqueous layer was then acidified with 1N HCl (16 mL) and extracted with ethyl acetate (2 x 50 mL). The dried (MgSO₄) ethyl acetate extracts were evaporated and the residual solid (810 mg), approximately a 4:1 mixture of the desired aminotetrazole and its positional isomer, was crystallized from ether to furnish 560 mg of 2-chloro-4-[5-[(3-hydroxyphenyl)amino]tetrazol-1-yl]benzoic acid as a colorless solid. FAB HRMS: (C₁₅H₁₂ClN₅O₃) Obs. Mass 345.0624 Calcd. Mass 345.0629 (M+H).
c. In an argon atmosphere, DIPEA (0.102 mL; 0.585 mmol) was added to a stirred solution of 2-chloro-4-[5-[[(3-hydroxyphenyl)methyl]amino]-1H-tetrazol-1-yl]benzoic acid (51 mg; 0.15 mmol), 4-[[(2,6-dichlorophenyl]carbonyl]amino]-L-phenylalanine methyl ester (60 mg; 0.15 mmol) and HBTU (59 mg; 0.1555 mmol) in dimethylformamide (3 mL). The reaction mixture was stirred for 17 hr at room temperature, then was concentrated under reduced pressure. The residual oil was taken up in dichloromethane (25 mL) and washed in turn with 0.5 N HCl (10 mL) and water (10 mL). The aqueous layers were backwashed in turn with dichloromethane. The combined organic layers were dried (Na₂SO₄) and evaporated to give 85 mg of crude product. This material was crystallized from dichloromethane-diethyl ether to furnish 79 mg of 4-[[(2,6-dichlorophenyl)carbonyl]amino]-N-[[2-chloro-4-[5-[[(3-hydroxyphenyl)methyl]amino]-1H-tetrazol-1-yl]phenyl]carbonyl]-L-phenylalanine methyl ester as a colorless solid, mp 155-158 °C. FAB HRMS: (C₃₂H₂₆Cl₃N₇O₅) Obs. Mass 694.1158 Calcd. Mass 694.1139 (M+H).
d. An aqueous 1N lithium hydroxide solution (0.33 mL) was added to a solution of 4-[[(2,6-dichlorophenyl)carbonyl]amino]-N-[[2-chloro-4-[5-[[(3-hydroxyphenyl)methyl]amino]-1H-tetrazol-1-yl]phenyl]carbonyl]-L-phenylalanine methyl ester (75 mg; 0.108 mmol) in methanol (0.66 mL) and THF (0.66 mL) and the mixture was stirred at room temperature for 90 min. After the solvents were stripped under reduced pressure, the residue was dissolved in water (20 mL) and extracted with diethyl ether (3 x 5 mL). The aqueous layer was filtered through Celite, then acidified with 1 N HCl (0.35 mL). The resulting colorless solid was filtered off, washed with water, and dried *in vacuo* to give 57 mg of 4-[[(2,6-dichlorophenyl)carbonyl]amino]-N-[[2-chloro-4-[5-[[(3-hydroxyphenyl)methyl]amino]-1H-tetrazol-1-yl]phenyl]carbonyl]-L-phenylalanine. FAB HRMS: (C₃₁H₂₄Cl₃N₇O₅) Obs. Mass 680.0981 Calcd. Mass 680.0983 (M+H).

Example 34. Synthesis of 4-[[(2,6-dichlorophenyl)carbonyl]amino]-N-[[2-chloro-4-[[[(3-hydroxyphenyl)methyl]amino]sulfonyl]phenyl]carbonyl]-L-phenylalanine.
a. At room temperature, a stirred solution of 4-amino-2-chlorobenzoic acid methyl ester hydrochloride (1.11 g; 5 mmol) in conc. HCl (10 mL) was treated in one portion with NaNO₂ (0.42 g; 6.09 mmol) in water (3 mL). After 15 minutes, the resulting suspension was added over 2 minutes to a rapidly stirred, saturated solution of SO₂ in acetic acid (15 mL) containing CuCl₂ (0.15g) in water (1 mL). There was an immediate vigorous evolution of gas that subsided after 10 minutes, whereupon the reaction mixture was diluted with ice water (200 mL). The resulting purplish solid was filtered off, washed with water, then was dissolved in dichloromethane. The dried (Na₂SO₄) solution was evaporated *in vacuo* and the residual material was chromatographed over silica gel (50 g). The appropriate fractions, eluted with 30-40% diethyl ether in hexane, were concentrated to dryness under reduced pressure to yield 1.1g of 3-chloro-4-methoxycarbonybenzenesulfonyl chloride as a colorless solid.
   The above 3-chloro-4-methoxycarbonybenzenesulfonyl chloride (0.14g; 0.52 mmol) in dichloromethane (0.5 mL) was added in one portion to a stirred solution of 3-acetoxybenzylamine hydrochloride (0.105g; 0.52 mmol) and triethylamine (0.2 mL; 1.42 mmol) in dichloromethane (0.2 mL). The reaction was allowed to proceed for 90 minutes at ambient temperature, then was diluted with dichloromethane (20 mL) and washed sequentially with 0.5 N HCl (10 mL), brine (10 mL), saturated NaHCO₃ solution (10 mL) and brine (I0 mL). The aqueous layers were backwashed in turn with dichloromethane (10 mL). The combined, dried (Na₂SO₄) organic layers were concentrated to afford 0.2 g of an oil that was chromatographed (silica gel; 15 g). The product was eluted from the column with diethyl ether-hexane (4:1) and diethyl ether to give, after evaporation of the appropriate fractions, 165 mg of 4-[(3-acetoxybenzylamino)sulfonyl]-2-chlorobenzoic acid methyl ester as a colorless solid. FAB HRMS: (C₁₇H₁₆ClNO₆S) Obs. Mass 398.0469 Calcd. Mass 398.0465 (M+H).
b. A stirred solution of 4-[(3-acetoxybenzylamino)sulfonyl]-2-chlorobenzoic acid methyl ester (163 mg; 0.41 mmol) in methanol (3 mL) and tetrahydrofuran (3 mL) was treated at room temperature with an aqueous 1 N lithium hydroxide solution (1.65 mL). After 2 hr the volatiles were removed under reduced pressure and the residual material was dissolved in water ( 15 mL) and the solution filtered through Celite. The filtrate was acidified with 1 N HCl (2 mL) and extracted with ethyl acetate (3 x 10 mL). After the extracts were backwashed in turn with brine, they were combined, dried (Na₂SO₄) and evaporated *in vacuo* to furnish 140 mg of 2-chloro-4-[(3-hydroxybenzylamino)sulfonyl]benzoic acid. A small sample of the product was crystallized from ethyl acetate-hexane to give a colorless solid, mp 167-169 °C. FAB LRMS: (C₁₄H₁₂ClNO₅S) Obs. Mass 342 Calcd. Mass 342 (M+H)
c. A solution of 2-chloro-4-[(3-hydroxybenzylamino)sulfonyl]benzoic acid (50 mg; 0.146 mmol), 4-[[(2,6-dichlorophenyl)carbonyl]amino]-L-phenylalanine methyl ester (60 mg; 0.1486 mmol), HBTU (5 mg; 0.15 mmol) and DIPEA (0.102 mL; 0.585 mmol) in dimethylformamide (3 mL) was stirred for 17 hr under argon at room temperature, then was concentrated to dryness under reduced pressure The residue was partitioned between dichloromethane (25 mL) and 0.5 N HCl (25 mL). The separated aqueous phase was re-extracted with dichloromethane (10 mL), then the organic extracts were washed in turn with water (2 x 25 mL). The combined dichloromethane layers were dried (Na₂SO₄) and evaporated to give 90 mg of the crude product as a dark oil. Chromatography of the oil over silica gel (9 g; 4:1 ethyl acetate-hexane) yielded 55 mg of 4-[[(2,6-dichlorophenyl)carbonyl]amino]-N-[[2-chloro-4-[[[(3-hydroxyphenyl)methyl]amino]sulfonyl]phenyl]carbonyl]-L-phenylalanine methyl ester, as a colorless solid. FAB HRMS: (C₃₁H₂₆Cl₃N₃O₇S) Obs. Mass 690.0639 Calcd. Mass 690.0635 (M+H).
d. An aqueous 1N lithium hydroxide solution (0.25 mL) was added to a solution of 4-[[(2,6-dichlorophenyl)carbonyl]amino]-N-[[2-chloro-4-[[[(3-hydroxyphenyl)methyl]amino]sulfonyl]phenyl]carbonyl]-L-phenylalanine methyl ester (51 mg; 0.0738 mmol) in methanol (0.5 mL) and tetrahydrofuran (0.35 mL). After the reaction was stirred at room temperature for 90 minutes, the solvents were removed under reduced pressure. The crude product in the minimum amount of methanol was then applied to a column of silica gel (5 g) made up in a mixture of chloroform, methanol, acetic acid and water (15:3:1:0.6). The column was eluted with the same solvent mixture and the appropriate fractions were combined and evaporated. The residue was lyophilized from deionized water to give 36 mg of 4-[[(2,6-dichlorophenyl)carbonyl] amino]-N-[[2-chloro-4-[[[(3-hydroxyphenyl)methyl]amino]sulfonyl]phenyl]carbonyl]-L-phenylalanine as an off white solid. FAB HRMS: (C₃₀H₂₄Cl₃N₃O₇S) Obs. Mass 676.0482 Calcd. Mass 676.0479 (M+H).

Example 35. Coupling of N-[(9H-fluoren-9-ylmethoxy)carbonyl]-4-[[(2-propenyloxy)carbonyl]amino]-L-phenylalanine to Wang resin.

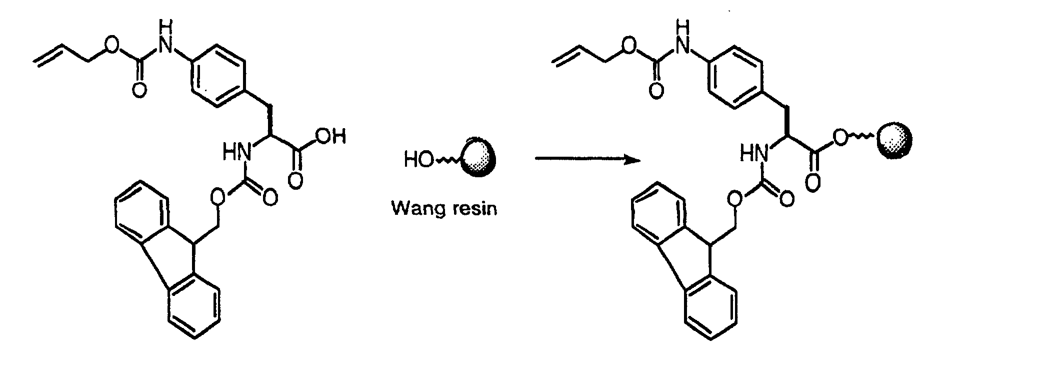

A 250 mL cylindrical glass vessel equipped with a coarse glass frit was charged with 10 g of Wang resin, (loading factor: 1.15 mmol/g, 300 mesh). The resin was washed with dichloromethane (2 x 100 mL), methanol (2 x 100 mL) and dimethylformamide (2 x 100 mL). To the swollen resin was added N-[(9H-fluoren-9-ylmethoxy)carbonyl]-4-[[(2-propenyloxy)carbonyl]amino]-L-phenylalanine (11.2 g, 23 mmol) and 2,6-dichlorobenzoyl chloride (8.06 mL, 57.5 mmol) in N-methylpyrrolidone (70 mL) and the mixture was agitated for 30 minutes. Pyridine (6.45 mL, 80.5 mmol) was added and the resulting mixture was agitated for 24 hours. The substitution was found at 0.75 mmol of N-[(9H-fluoren-9-ylmethoxy)carbonyl]-4-[[(2-propenyloxy)carbonyl]amino]-L-phenylalanine per gram of resin by quantitative UV measurement of the Fmoc present on the resin.

Example 36. Synthesis of 4-amino-N-[(9H-fluoren-9-ylmethoxy)carbonyl]-L-phenylalanine on Wang resin.

A 500 mL cylindrical glass vessel equipped with a coarse glass frit was charged with 10 g of N-[(9H-fluoren-9-ylmethoxy)carbonyl]-4-[[(2-propenyloxy)carbonyl]amino]-L-phenylalanine substituted Wang resin (10 g) obtained from Example 35 and a solution prepared from Pd(Ph₃P)₂Cl₂ (1.6 g, 2.3 mmol) and acetic acid (5 mL, 83 mmol) in dry dichloromethane (150 mL). The resulting mixture was agitated for 30 minutes followed by the addition of tri-n-butyl tin hydride (20 mL, 74.3 mmol). The resulting mixture was agitated for 1 hour. To the mixture was added tri-n-butyl tin hydride (10 mL, 37 mmol). Agitation was continued for 1 hour and the mixture was filtered. To the resulting resin was added a solution prepared from Pd(Ph₃P)₂Cl₂ (1.6 g, 2.3 mmol) and acetic acid (5 mL, 83 mmol) in dried dichloromethane (150 mL). The mixture was agitated for 30 minutes followed by the addition of tri-n-butyl tin hydride (20 mL, 74.3 mmol). The resulting mixture was agitated 1 hour. To the mixture was added additional tri-n-butyl tin hydride (10 mL, 37.15 mmol). Agitation continued for 1 hour. After the second deprotection cycle, the mixture was washed with dichloromethane (2 x 100 mL), methanol (2 x 100 mL) and dimethylformamide (2 x 100 mL) to give 4-amino-N-[(9H-fluoren-9-ylmethoxy)carbonyl]-L-phenylalanine on Wang resin suitable for use in subsequent steps.

Example 37. Synthesis of 4-[[(4-quinolinyl)carbonyl]amino]-L-phenylalanine on Wang resin

A 250 mL cylindrical glass vessel equipped with a coarse glass frit was charged with 4-amino-N-[(9H-fluoren-9-ylmethoxy)carbonyl]-L-phenylalanine on Wang resin (10 g) obtained in Example 36 and a solution prepared from quinoline-4-carboxylic acid (5.2 g, 30 mmol), BOP (13.75 g, 30 mmol) and diisopropylethylamine (6.8 mL) in 70 mL of NMP. The slurry was agitated for 4 hours. The mixture was filtered and washed with dichloromethane (2 x100 mL), methanol (2 x100 mL) and dimethylformamide (2 x 100 mL). To the washed resin was added a solution of 25% piperidine in NMP (80 mL), the mixture was agitated at room temperature for 20 minutes and filtered. The process was repeated and the resulting slurry was filtered and washed with dichloromethane (2 x 100 mL), methanol (2 x 100 mL) and dimethylformamide (2 x 100 mL). Filtration afforded 4-[[(4-quinolinyl)carbonyl]amino]-L-phenylalanine on Wang resin suitable for use in the next step.

Example 38. Synthesis of N-[(2,6-dimethylphenyl)carbonyl]-4-[[(4-quinolinyl)carbonyl] amino] -L-phenylalanine.

4-[[(4-Quinolinyl)carbonyl]amino]-L-phenylalanine on Wang resin (300 mg, 0.20 mmol) was washed with dichloromethane (2 x 10 mL), methanol (2 x 10 mL) and dimethylformamide (2 x 10 mL). To the resin was added a solution prepared from 2,6-dimethylbenzoic acid (150 mg, 1.0 mmol), BOP (450 mg, 1.02 mmol) and diisopropylethylamine (0.23 mL) in 4 mL of N-methylpyrrolidone at room temperature. The resulting mixture was agitated for 2 hr. The reaction mixture was then filtered and washed with dichloromethane (2 x 10 mL), methanol (2 x 10 mL) and dichloromethane (2 x 10 mL). Cleavage was effected by treatment with 90% trifluoroacetic acid (TFA) in dichloromethane for 5 minutes. The mixture was filtered and the TFA was removed under high vaccum. Addition of ether (25 mL) effected precipitation of N-[(2,6-dimethylphenyl)carbonyl]-4-[[(4-quinolinyl)carbonyl)amino]-L-phenylalanine (0.16 g).

Examples 39 - 49. Using the procedure described in Example 38, the compounds shown below were prepared starting from 4-[[(4-quinolinyl)carbonyl]amino]-L-phenylalanine and the appropriate benzoic acid derivates.

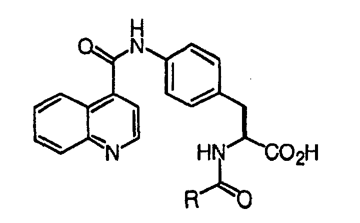

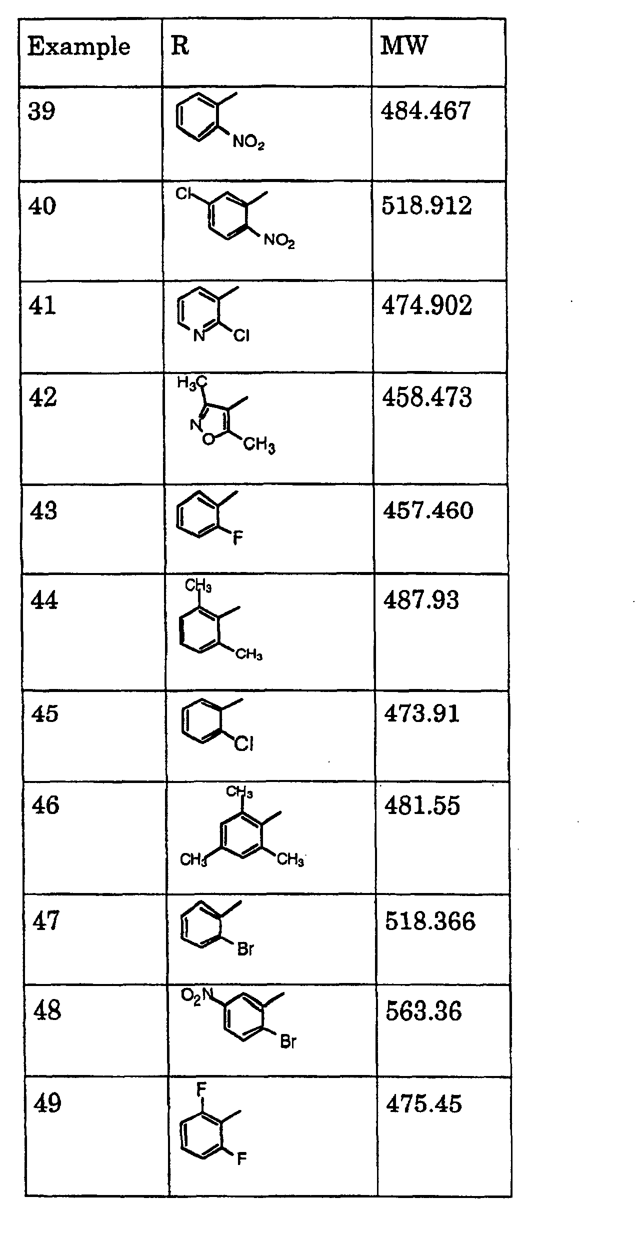

Examples 50 to 61. Using the method described in examples 37 to 38, the following compounds were prepared from 4-amino-N-[(9H-fluoren-9-ylmethoxy)carbonyl]-L-phenylalanine on Wang resin and the appropriate carboxylic acids:

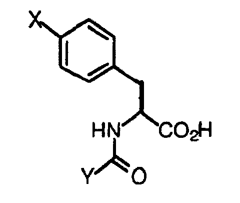

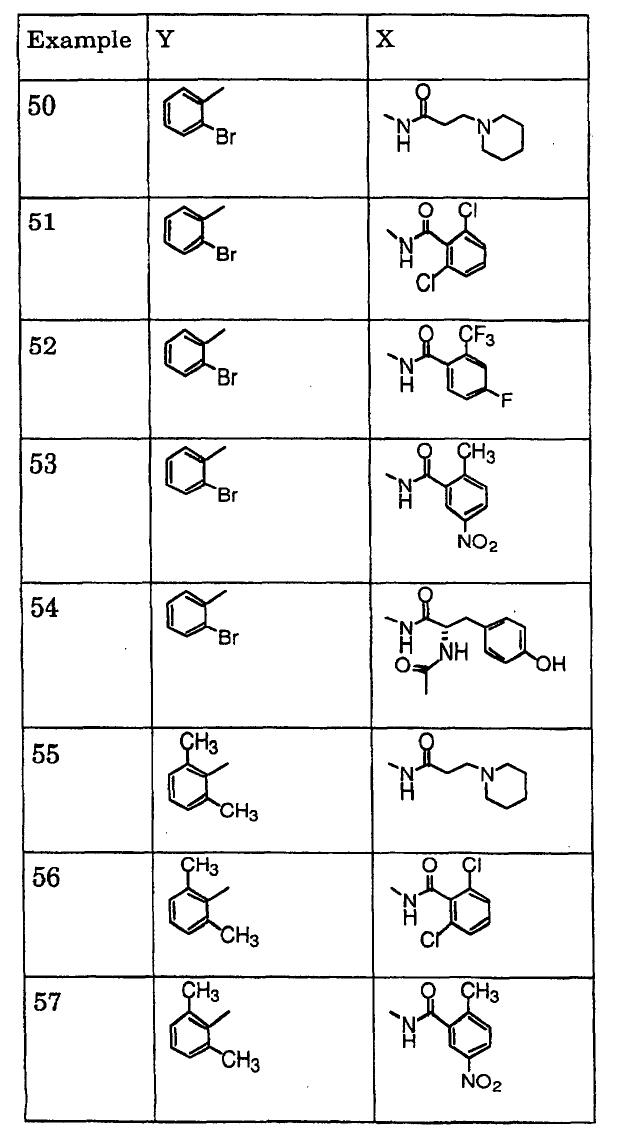

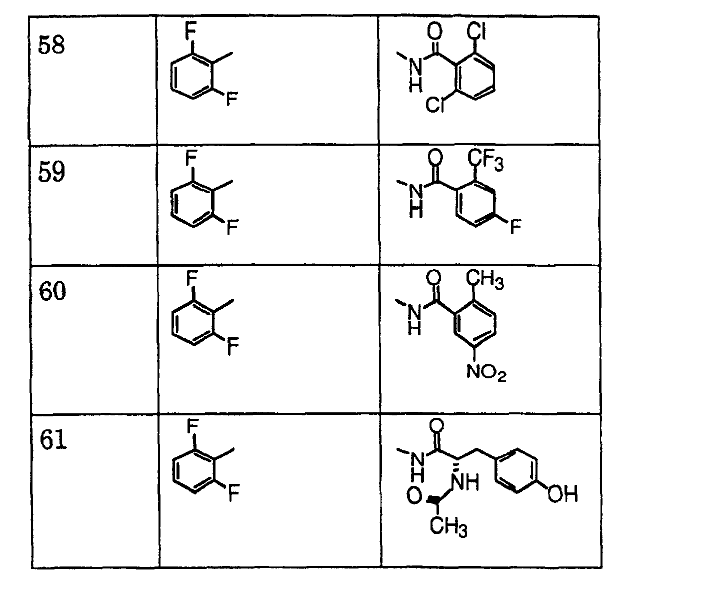

Examples 62. Synthesis of N-[(2,6-dimethylphenyl)carbonyl]-4-[[(2,4,6-trimethylphenyl)sulfonyl]amino]]-L-phenylalanine.

Wang resin loaded with 4-amino-N-[(9H-fluoren-9-ylmethoxy)carbonyl]-L-phenylalanine (3.0 g, 2.28 mmol) in pyridine (15 mL) was cooled to 0 °C and 2,4,6-benzenesulfonyl chloride (2.49 g, 11.4 mmol) was added and the mixture was agitated over night at room temperature. The mixture was filtered and the resin was washed with methanol and dichloromethane. The coupling procedure was repeated. To the washed resin was added a solution of 25% piperidine in N-methylpyrrolidone (10 mL), the mixture was agitated at room temperature for 20 minutes and filtered. The process was repeated and the resulting slurry was filtered and washed with dichloromethane (2 x 10 mL), methanol (2 x 10 mL) and dimethylformamide (2 x 10 mL). Filtration afforded 4-[[(2,4,6-trimethylphenyl)sulfonyl)amino]-L-phenylalanine on Wang resin suitable for use in the next step.

A sample of the above resin (0.3 g, 0.28 mmol) was suspended in N-methylpyrrolidinone (3 mL) and treated with 2,6-dimethylbenzoic acid (171 mg, 1.14 mmol), BOP (0.50 g, 1.14 mmol) and DIPEA (0.26 mL, 1.4 mmol). The mixture was stirred at room temperature for 3 hr, was filtered. and washed with dichloromethane (2 x 10 mL), methanol (2 x 10 mL) and dichloromethane (2 x 10 mL). Cleavage was effected with 90% trifluoroacetic acid (TFA) in dichloromethane for 5 minutes. The mixture was filtered and the TFA was removed under high vaccum. Addition of ether (25 mL) effected precipitation of N-[(2,6-dimethylphenyl)carbonyl]-4-[[(2,4,6-trimethylphenyl)sulfonyl]amino]]-L-phenylalanine.

Example 63. N-(2-Bromobenzoyl)-4-[[(2,4,6-trimethylphenyl)sulfonyl]amino]-L-phenylalanine was prepared from 4-[[(2,4,6-trimethylphenyl)sulfonyl]amino]-L-phenylalanine on Wang resin and 2-bromobenzoic acid using the general method described in example 62..

Example 64. Synthesis of 4-[[(4-cyano-4-phenyl-1-piperidinyl)carbonyl]amino]-N-(2,6-dimethylphenyl)carbonyl]-L-phenylalanine.

4-Amino-N-[(9H-fluoren-9-ylmethoxy)carbonyl]-L-phenylalanine on Wang resin (3.0 g, 2.04 mmol) was placed in a vessel fitted with a glass frit and was suspended in dichloromethane (50 mL) and DIPEA (0.98 mL, 5.6 mmol). The mixture was shaken for 15 min and triphosgene (1.1 g, 3.7 mmol) was added in one portion. The mixture was agitated for 2 hr at room temperature. The mixture was then filtered and washed with dichloromethane (3 x 25 mL). The resin was suspended in dichloromethane (50 mL) and DIPEA (1.0 mL, 5.6 mmol) and 4-cyano-4-phenylpiperidine hydrochloride (2.73 g, 12.2 mmol) was added. The resulting mixture was agitated for 4 hr. The reaction mixture was then filtered and washed with dichloromethane (2 x 50 mL), methanol (2 x 50 mL), dimethylformamide (2 x 50 mL) and methanol (2 x 10 mL). Cleavage of the Fmoc group was effected by treatment with 25% piperidine in N-methylpyrrolidinone (2 x 15 min).

The above resin (0.3 g, 0.20 mmol), 2,6-dimethylbenzoic acid (0.15 g, 1 mmol) was suspended in N-methylpyrrolidinone (3 mL) and treated with BOP-Cl (0.26 g, 1.0 mmol) and DIPEA (0.23 mL, 1.3 mmol). The mixture was stirred at room temperature for 3 hr and was filtered. The reaction mixture was then filtered and washed with dichloromethane (2 x 10 mL), methanol (2 x 10 mL) and dichloromethane (2 x 10 mL). Cleavage was effected with 90% trifluoroacetic acid (TFA) in dichloromethane for 3 minutes. The mixture was filtered and the TFA was removed under high vaccum. Addition of ether (25 mL) effected precipitation of 4-[[(4-cyano-4-phenyl-1-piperidinyl)carbonyl]amino]-N-[(2,6-dimethylphenyl)carbonyl]-L-phenylalanine.

Examples 65 - 66. Using the procedure described in Example 64, the following compounds were prepared:

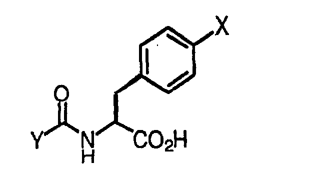

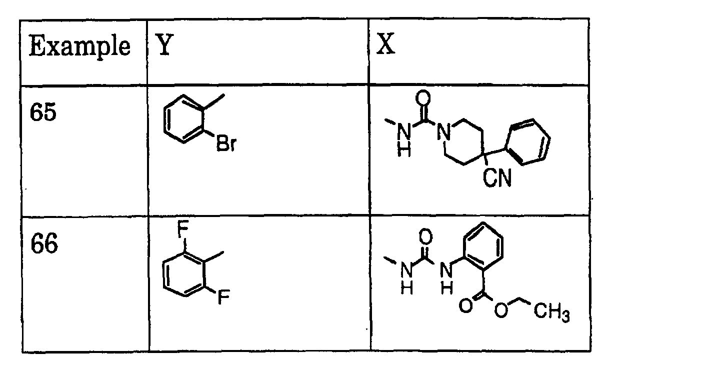

Example 67. Synthesis of N-(2-chloro-6-methylbenzoyl)-4-nitro-L-phenyl alanine methyl ester

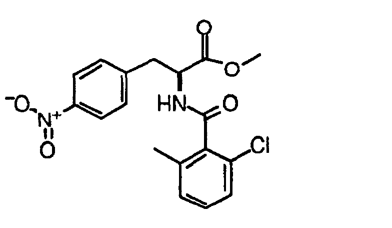

To a solution of 4-nitro-L-phenylalanine methyl ester hydrochloride (1.527 g, 5.86 mmol), 2-chloro-6-methylbenzoic acid (1.0 g, 5.86 mmol) and DIPEA (3.2 mL, 2.3 g, 18 mmol) in DMF (10 mL) was added HBTU (2.22g, 5.86 mmol) at room temperature. After 4 hr at room temperature, the reaction mixture was diltuted with ethyl acetate (200 mL) and the organic layer was washed with water (20 mL), 1N HCl, NaHCO₃ and brine (2 x 30 mL for each solvent) and dried over Na₂SO₄. After removal of the solvent, the residue was purified by chromatography on silica gel eluting with ethyl acetate:hexane (1:2), to give N-(2-chloro-6-methylbenzoyl)-4-nitro-L-phenyl alanine methyl ester (1.71 g, 4.50 mmol, 77.6 %). mp, 123-4 °C. Analysis (C₁₈H₁₇ClN₂O₅) calcd.: C, 57.38. H, 4.55. N, 7.43. Found: C, 57.11. H, 4.58. N, 7.27.

Example 68. Synthesis of 4-amino-N-[(2-chloro-6-methylphenyl)carbonyl]-L-phenylalanine methyl ester

N-[(2-Chloro-6-methylphenyl)carbonyl]-4-nitro-L-phenylalanine methyl ester (1.51g, 4.0 mmol) and SnCl₂•H₂O (4.5g, 20 mmol) were suspended in 30 mL of ethanol. The suspension was stirred at a bath temperature of 97 °C for 1 hr. After it was cooled to room temperature, the solvent was evaporated and the residue was disolved in 15 mL of water. The aqueous solution was then made alkaline by addition of solid K₂CO₃ to pH>10 and was extracted with ethyl acetate (3 x 100 mL). The combined extracts were dried over K₂CO₃ and were concentrated to give 4-amino-N-(2-chloro-6-methylbenzoyl)-L-phenylalanine methyl ester as a light yellow foam (1.37 g).

Example 69. Synthesis of (S)-N-(2-chloro-6-methylbenzoyl)-4-[[[[1-(1,1-dimethylethoxy)carbonyl]-2-piperidinyl]carbonyl]amino]-L-phenylalanine methyl ester

A solution of 4-amino-N-[(2-chloro-6-methylphenyl)carbonyl]-L-phenylalanine methyl ester (347 mg, 1.0 mmol) in DMF (2.0 mL) was treated with (S)-piperidine-1,2-dicarboxylic acid 1-(1,1-dimethy)lethyl ester (347 mg, 1.0 mmol), HBTU (380mg, 1.0 mmol) and DIPEA (0.54 mL, 3.0 mmol) at room temperature for 6 hr. The reaction mixture was diluted to 6 mL with water and the white precipitate was collected by filtration and was washed with water (2 x 2 mL). After drying under vacuum, the light yellow powder was recrystillized from ethyl acetate-hexane to give (S)-N-(2-chloro-6-methylbenzoyl)-4-[[[[1-(1,1-dimethylethoxy)carbonyl]-2-piperidinyl]carbonyl]amino]-L-phenylalanine methyl ester (507 mg, 0.82 mmol, 82%) as a white solid, mp: 87-91 °C. HRMS: calcd. 558.2371. Obs. 558.2359 (M+H).

Example 70. Synthesis of (S)-N-(2-chloro-6-methylbenzoyl)-4-[[(2-piperidinyl)carbonyl]amino]-L-phenylalanine methyl ester hydrochloride

A solution of (S)-N-(2-chloro-6-methylbenzoyl)-4-[[[[1-(1,1-dimethylethoxy)carbonyl]-2-piperidinyl]carbonyl]amino]-L-phenylalanine methyl ester (475 mg, 0.85 mmol) in 2 mL of dicloromethane was treated with 4N HCl in dioxane (2 mL). The solution was stirred at room temperature for 4 hr and the solvent was then removed under vacuum. The residue was then treated with 50 mL of ether and the light yellow precipitate was collected and was dried under vaccum to give (S)-N-(2-chloro-6-methylbenzoyl)-4-[[(2-piperidinyl)carbonyl]amino]-L-phenylalanine methyl ester hydrochloride (440 mg, 0.89 mmol, >100%) as a light yellow powder. ES MS: 458 (100%) (M+H). NMR (DMSO-d6, d, ppm): 10.26 (s, 1H), 9.30 (bd, 1H), 9.00 (d, 1H, J = 9 Hz), 8.80 (bt, 1H), 7.65 ( d, 2H, J = 7.8 Hz), 7.24 (m, 5H), 4.70 (m, 1H), 3.90 (m, 1H), 3.67 (s, 3H), 3.32 (m, 2H), 3.05 (m, 2H), 2.25 (m, 1H), 2.05 (s, 3H), 1.70 (m, 5H).

Example 71. Synthesis of N-[(2-chloro-6-methylphenyl)carbonyl]-4-[(8aS)-hexahydro-3-(4-hydroxyphenyl)-1-oxoimidazo[1,5-a]pyridin-2-yl]-L-phenylalanine.

(S)-N-(2-chloro-6-methylbenzoyl)-4-[[(2-piperidinyl)carbonyl]amino]-L-phenylalanine methyl ester hydrochloride (100 mg, 0.2 mmol), DIPEA (0.10 mL, 0.54 mmol) and 4-hydroxybenzaldehyde (30 mg, 0.25 mmol) was added to a suspension of activated 3Å molecular sieves (100 mg) in THF (1.5 mL). The resulting mixture was stirred room temperature overnight and at 60 °C for 3 hr. After it was cooled to room temperature, the mixture was transfered onto a silica gel column and eluted with ethyl acetate:hexane (2:1) to give N-[(2-chloro-6-methylphenyl)carbonyl]-4-[(8aS)-hexahydro-3-(4-hydroxyphenyl)-1-oxoimidazo[1,5-a]pyridin-2-yl]-L-phenylalanine methyl ester (17.5 mg, 0.031 mmol) as a foam. The methyl ester (17.5 mg, 0.031 mmol) was hydrolyzed with 1N NaOH (0.1 mL, 0.1 mmol) in 0.5 mL of ethanol at room temperature for 6 hr. The reaction mixture was acidified to pH< 2 with TFA and was purified on RP-HPLC to give N-[(2-chloro-6-methylphenyl)carbonyl]-4-[(8aS)-hexahydro-3-(4-hydroxyphenyl)-1-oxoimidazo[1,5-a]pyridin-2-yl]-L-phenylalanine (8.3 mg, 0.015 mmol). in 7.5% yield. HRMS: calcd, 548.1952. Obs. 548.1938 (M+H).

Example 72 - 74. Using the procedure described in example 71, the compounds shown below were prepared.

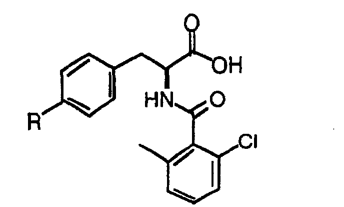

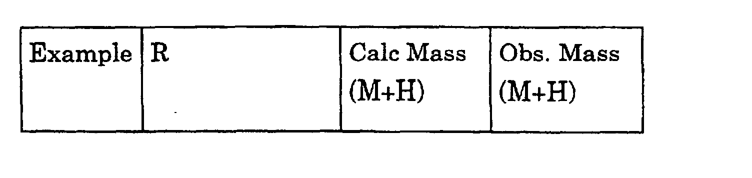

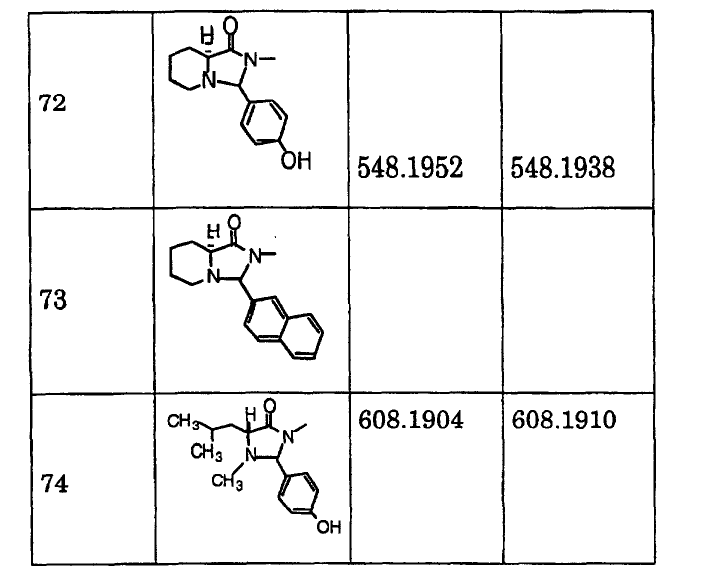

Example 75. Synthesis of N-(2-chloro-6-methylbenzoyl)-4-[[(2R)-2-amino-4-methyl-1-oxopentyl]amino]-L-phenylalanine methyl ester

A solution of 4-amino-N-[(2-chloro-6-methylphenyl)carbonyl]-L-phenylalanine methyl ester (561 mg, 1.61 mmol), prepared using procedure described in Example 68, in DMF (4.5 mL) was treated with N-Boc-D-leucine (393.2 mg, 1.7 mmol), HBTU (644.3 mg, 1.7 mmol) and DIPEA (0.62 mL, 3.50 mmol) at room temperature for 6 hr. The reaction mixture was diluted to 30 mL with water and the white precipitate was collected by filtration and was washed with water (2 x 2 mL). After drying under vacuum, the light yellow powder was recrystillized from ethyl acetate-hexane to give N-(2-chloro-6-methylbenzoyl)-4-[[(2R)-2-[(1,1dimethylethoxy)carbonyl]amino-4-methyl-1-oxopentyl]amino]-L-phenylalanine methyl ester (920 mg) as a white solid. MS 560 (M+H,1 Cl). This solid was dissolved in 4 N HCl in dioxane (5 mL). The solution was stirred at room temperature overnight . After dilution with ether, the white suspension was allowed to stand at -5 °C for 1 hr. The white solid was collected by filtration and was dried under vacuum for 5 hr. The above solid was then dissolved in 20 mL of water and the solution was treated with sodium bicarbonate followed by K2CO3 to PH >9. The mixture was then extracted with dichloromethane (2 x 25 mL) and was dried over sodiun sulfate. After removal of solvent, the residue was then dried under vacuum at 50 °C overnight to give white solid (520 mg, 1.1 mmol) in 70 % overall yield. HRMS: Obs. 460.1997, Calc. 460.2003 (M+H).

Example 76-77.4-[(2S,4R)-3-acetyl-2-phenyl-4-(2-methylpropyl)-5-oxo-imidazolidin-1-yl]-N-(2-chloro-6-methylbenzoyl)-L-phenylalanine and 4-[(2R,4R)-3-acetyl-2-phenyl-4-(2-methylpropyl)-5-oxo-imidazolidin-1-yl]-N-(2-chloro-6-methylbenzoyl)-L-phenylalanine.

(S)-N-(2-chloro-6-methylbenzoyl)-4-[((2R)-2-amino-4-methyl-1-oxopentyl)amino]-L-phenylalanine methyl ester (100 mg, 0.2 mmol) was dissolved in mixture of THF/CH(OMe)3 (1/1, 1.0 mL). To the solution was then added benzaldehyde (21.2 mg, 0.2 mmol) and the solution was stirred at room temperature. After 24 hr, the reaction mixture was heated to 95 °C, acetic anhydride ( 0. 1 mL, 1.0 mmol) was introduced via a syringe and the solution was stirred at 110 °C for 3 hr. After evaporation of solvent, the residue was diluted with ethyl acetate and was washed twice with saturated sodium bicarbonate solution. After removal of the solvent, the residue was dissolved in 3 mL of mixed solvent ( THF/ethanol/H2O = 2/2/1) and was treated with 1N sodium hydroxide (0.2 mL, 0.2 mmol). After 4 hr at room temperature, the reaction was quenched with 0.5 mL of acetic acid and the crude product was purified on RP-HPLC (C18, 5-95-35-214) to give the trans isomer, 4-[(2S,4R)-3-acetyl-2-phenyl-4-(2-methylpropyl)-5-oxoimidazolidin-1-yl]-N-(2-chloro-6-methylbenzoyl)-L-phenylalanine (27 mg, 46 µmol), HRMS (M+H): obs. 576.2251, calc. 576.2265. The corresponding cis-isomer, 4-[(2R,4R)-3-acetyl-2-phenyl-4-(2-methylpropyl)-5-oxoimidazolidin-1-yl]-N-(2-chloro-6-methylbenzoyl)-L-phenylalanine (50.1mg, 86 µmol) HRMS (M+H). calc. 576.2265, obs.576.2250.

Example 78. Synthesis of N-[(2-chloro-6-methylphenyl)carbonyl)-4-[(S)-hexahydro-1,3-dioxoimidazo[1,5-a]pyridin-2-yl)]-L-phenylalanine.

To a solution of N-(2-chloro-6-methylbenzoyl)-4-[[(S)-(2-piperidinyl)carbonyl]amino]-L-phenylalanine methyl ester hydrochloride (50 mg, 0.1 mmol) and DIPEA (0.020 mL, 0.1 mmol) in 0.2 mL of dichloromethane was added carbonyldiimidazole (16.2 mg, 0.1 mmol) at room temperature. The solution was then stirred at this temperature for 6 hr. The reaction mixture was diluted with ethyl acetate to 5 mL and the organic layer was washed with 1N HCl, sat. NaHCO₃ and brine (2 x 1 mL for each solvent) and was dried over Na₂SO₄. The solvent was then removed under vacuum to give a light yellow solid (53.4 mg, 0.11 mmol). The above solid was then disolved in ethanol (1 mL) and was stirred with 1N NaOH (0.1 mL, 0.1 mmol) at room temperature for 6 hr. The reaction mixture was acidified to pH < 2 with TFA and was purified on RP-HPLC to give N-[(2-chloro-6-methylphenyl)carbonyl]-4-[(S)-hexahydro-1,3-dioxoimidazo[1,5-a]pyridin-2-yl)]-L-phenylalanine (27.0 mg, 0.057mmol) in 57% overall yield. HRMS: obs. 470.1465. calcd. 470.1483 (M+H).

Examples 79-84. Using procedures described in Examples 69, 70 and 78, the following compounds were prepared from 4-amino-N-[(2-chloro-6-methylphenyl)carbonyl]-L-phenylalanine methyl ester and the appropriate amino acid derivatives:

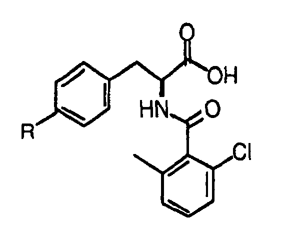

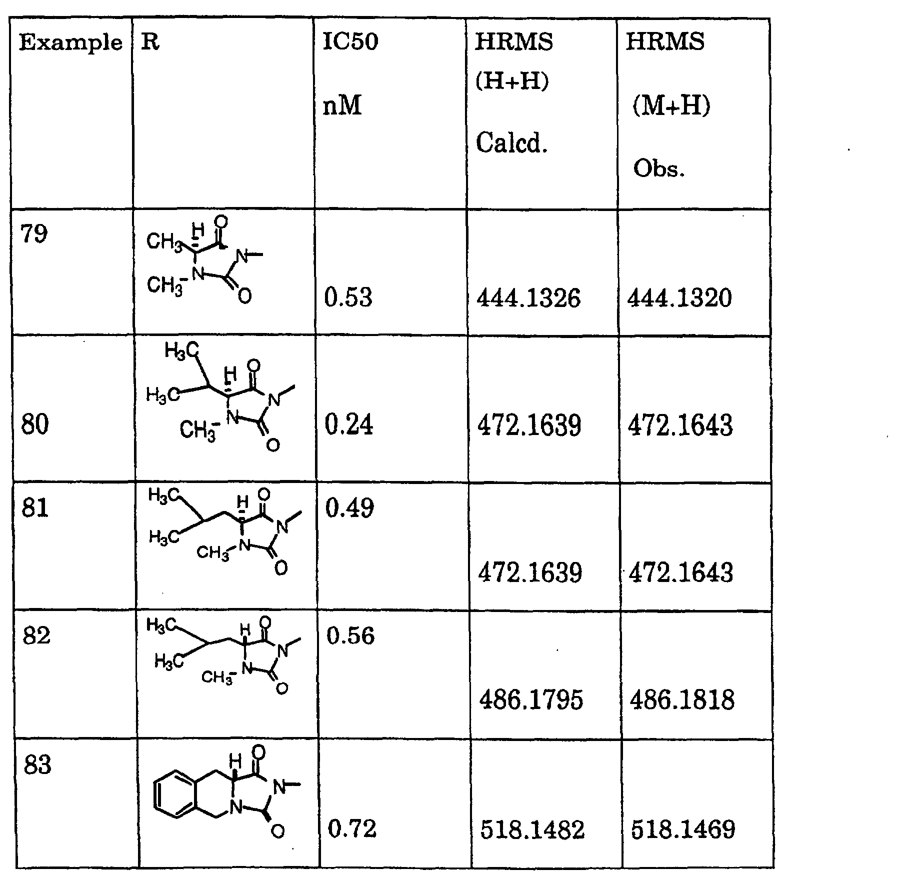

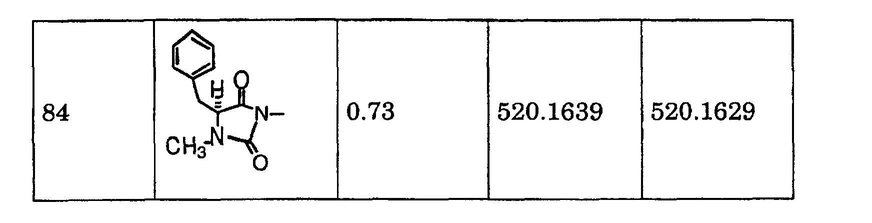

Example 85. Synthesis of 4-nitro-N-[(2,6-dimethylphenyl)carbonyl]-L-phenylalanine methyl ester

A solution of 4-nitrophenylalanine methyl ester (5.21 g, 20 mmol) in 20 mL of dichloromethane and of DIPEA (15 mL) was treated with 2,6-dimethylbenzoyl chloride. After 4 hr, the mixture was concentrated, the residue was taken up in ethyl acetate (200 mL) and washed with 1 N HCl (50 mL), sat. NaHCO₃ (50 mL) and sat. brine (30 mL), and was dried (MgSO₄). Filtration and concentration gave 8.0 g of a solid which was purified by HPLC (Waters Prep 500 -dual silica gel cartridges; 1:1 ethyl acetate-hexane) to give 4-nitro-N-[(2,6-dimethylphenyl)carbonyl]-L-phenylalanine methyl ester (5.26 g, 74 %).

Example 86. 4-Amino-N-[(2,6-dimethylphenyl)carbonyl]-L-phenylalanine methyl ester was prepared using the procedure described in example 68; from 4-nitro-N-[(2,6-dimethylphenyl)carbonyl]-L-phenylalanine methyl ester (5.2 g, 14.6 mmol) there was obtained 4-amino-N-[(2,6-dimethylphenyl)carbonyl]-L-phenylalanine methyl ester (4.6 g, 97 %) as a light yellow glass.

Example 87. Synthesis of 4-[[(4-carboxy-3-pyridinyl)carbonyl]amino]-N-[(2,6-dimethylphenyl)carbonyl]-L-phenylalanine and 4-[[(3-carboxy-4-pyridinyl)carbonyl]amino]-N-[(2,6-dimethylphenyl)carbonyl]-L-phenylalanine

A solution of 4-amino-N-[(2,6-dimethylphenyl)carbonyl]-L-phenylalanine methyl ester (530 mg, 1.162 mmol) and 3,4-pyridinedicarboxylic acid anhydride in dichloromethane (30 mL) was allowed to stir over night and the precipitate was collected. The solids were dissolved in THF (100 mL), filtered and concentrated to give 1.1 g of a mixture of isomeric carboxylic acids. This material was dissolved in ethanol (50 mL) and treated with 1 N NaOH (15 mL, 15 mmol) and stirred for 2.5 hr. The mixture was acidified with excess acetic acid and was purified in 3 batches on the Rainin RP-HPLC to give 0.60 g of a white solid as a mixture of isomeric dicarboxylic acids.

Example 88. Synthesis of 4-(2,3-dihydro-1,3-dioxo-1H-pyrrolo[3,4-c]pyridin-2-yl)-N-(2,6-dimethylbenzoyl)-L-phenylalanine.

A solution of the mixture of acids from example 87 (272 mg, 0.59 mmol) in DMF (10 mL) was treated with carbonyl diimidazole (385 mg, 2.4 mmol) and was allowed to stir over night. The mixture was filtered and purified directly by HPLC on the Rainin instument to afford, after lyophization of the product fraction, 4-(2,3-dihydro-1,3-dioxo-1H-pyrrolo[3,4-c]pyridin-2-yl)-N-(2,6-dimethylbenzoyl)-L-phenylalanine (108 mg, 41 %), FAB HRMS: obs., 444.1548. Calcd., 444.1559 (M+H).

Example 89. Synthesis of N-[(2,6-dimethylphenyl)carbonyl]-4-[(R,S)-2,3,5,6,7,7a-hexahydro-1,3-dioxo-1H-pyrrolo[3,4-c]pyridin-2-yl]-L-phenylalanine.

A solution of 4-(2,3-dihydro-1,3-dioxo-1H-pyrrolo[3,4-c]pyridin-2-yl)-N-(2,6-dimethylbenzoyl)-L-phenylalanine (108 mg, 0.24 mmol) in ethanol:THF (25 mL, 1:1) was hydrogenated over 10 % Pd/C (20 mg) for 4 hr. The mixture was filtered, concentrated and purified by RP-HPLC on a Rainin HPLC. The first product to elute was lyophilized to give N-[(2,6-dimethylphenyl)carbonyl]-4-[(R,S)-2,3,5,6,7,7a-hexahydro-1,3-dioxo-1H-pyrrolo[3,4-c]pyridin-2-yl]-L-phenylalanine (29 mg, 27 %), FAB HRMS: obs., 448.1862. Calcd., 448.1873 (M+H). The second product to elute was lyophilized to give recovered starting material (47 mg, 43 %).

Examples 90 -96. The compounds shown below were prepared using the methods described in example 13 by hydrolysis of 4-[[(2,6-dichlorophenyl)carbonyl]amino]-N-aroyl-L-phenylalanine methyl ester derivatives.

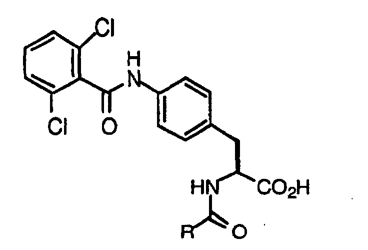

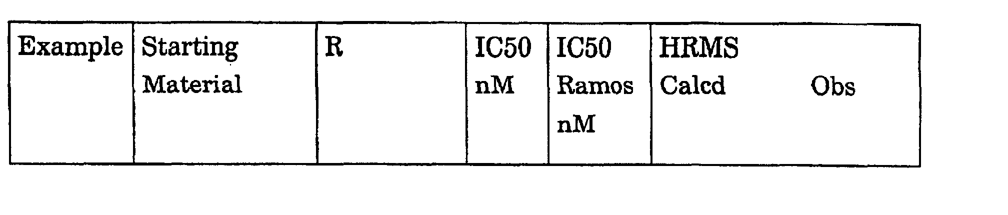

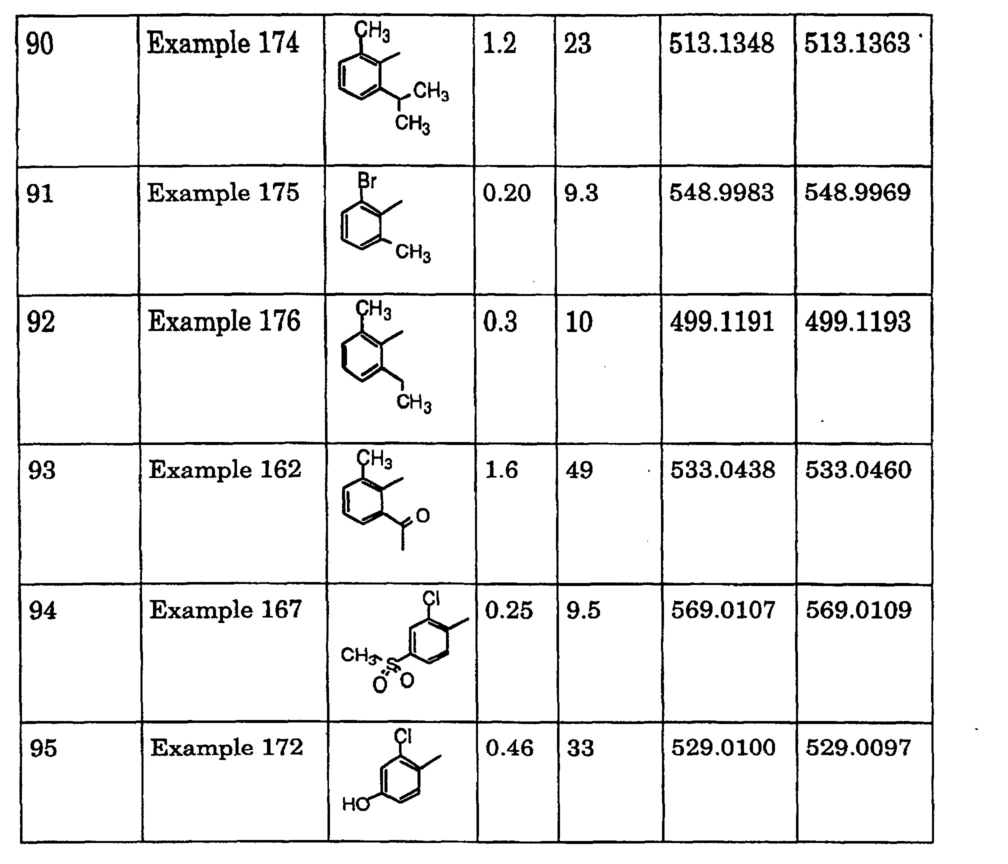

Example 96. N-(2-Chloro-6-methylbenzoyl)-[(R)-2,5-dioxo-3-methyl-4-(1-methylethyl)-1-imidazolininyl]-L-phenylalanine methyl ester was prepared from 4-amino-N-(2-chloro-6-methylbenzoyl)-L-phenylalanine methyl ester and N-[(1,1-dimethylethoxy)carbonyl]-N-methyl-D-valine using the procedure described in examples 69, 70 and 78.

Example 97-102. Using procedures described in the Examples 75, 76 and 77 the following compounds were prepared from 4-amino-N-(2-chloro-6-methylbenzoyl)-L-phenylalanine methyl ester and the appropriate Boc-protected amino acids.

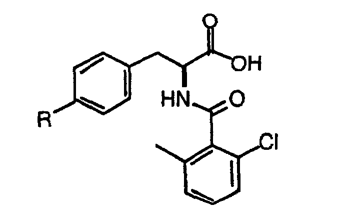

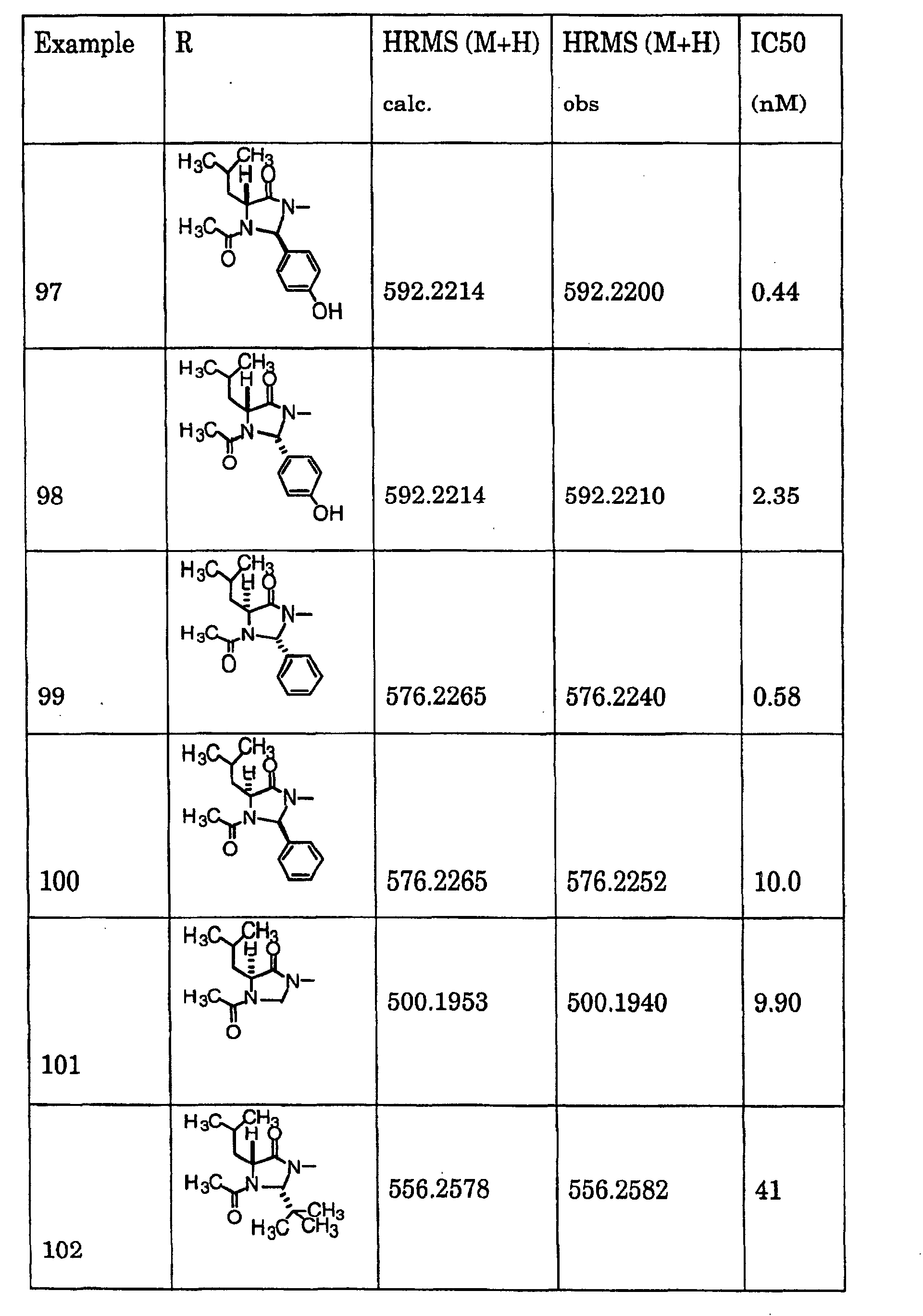

Example 103. Preparation of 2-bromo-6-methylbenzoic acid.

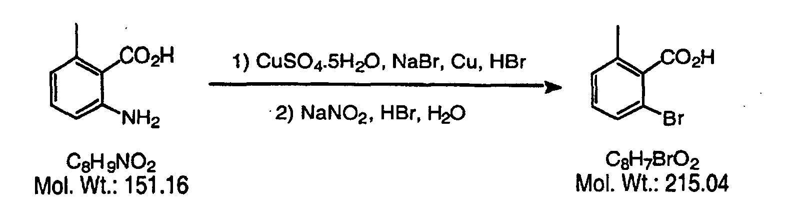

Cuprous bromide was prepared by heating a deep purple solution of CuSO₄.5H₂O (33 mmol, 8.25 g) and NaBr (66 mmol, 6.75 g) in HBr (33 mL, 48%) and adding Cu powder (66 mmol, 4.2 g) in portions until the purple solution became a colorless solution. This solution was then added in portions to a hot solution (*ca*.90°C) of 2-amino-6-methylbenzoic acid (33 mmol, 5 g) in H₂O (80 mL) and HBr (11.5 mL). This was followed by the dropwise addition of a solution of NaNO₂ (99 mmol, 6.85 g) in H₂O (20 mL) to this stirred heated solution over a period of 25 min. The dark-brownish mixture was heated at *ca*.90 °C for 1 hr and then was heated at reflux for another 30 min before it was cooled to room temperature and stirred for 2 hr. Then, the mixture was poured into ice (~500 g), 5% NaOH solution was added until pH 14 was reached and the resulting dark suspension was filtered through celite. The yellow filtrate was acidified with conc. HCl to pH 1. Extractive work-up (Et₂O, 3 x 150 mL) gave a dark residue which was dissolved in Et₂O (100 mL), charcoal was added and the resulting solution was heated to reflux. Filtration and concentration gave a material which was recrystalized from Et₂O/petrolium ether in hexane (100 mL) to afford the 2-bromo-6-methylbenzoic acid (3.5 g, 49%, HR MS: Obs. mass, 213.9633. Calcd. mass, 213.9629, M+) as a crystalline light pink solid; mp 104-106 °C.

Example 104. Preparation of 2-ethyl-6-methylbenzoic acid.

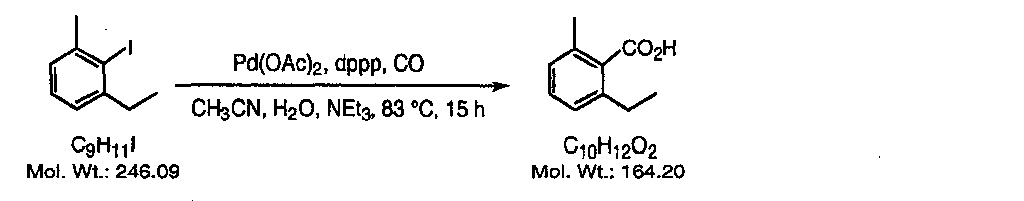

A 250 mL pressure bottle was charged with 2-ethyl-6-methyliodobenzene (30.07 mmol, 7.4 g), Pd(OAc)₂ (1.43 mmol, 334 mg) and dppp (1.43 mmol, 620 mg). The flask was closed with a septum and evacuated three times with argon. Then, acetonitrile (96 mL), triethylamine (189 mmol, 19.0 g, 26.25 mL) and water (19.1 mL) were added successively by the aid of syringe. Then, the rubber septum was replaced with teflon lined cap connected to a carbon monoxide source. The flask was now pressurized with carbon monoxide (40 psi) and the excess pressure was released. This process was repeated three times and finally the mixture was stirred for 5 min under 40 psi carbon monoxide pressure. The flask was then disconnected from the carbon monoxide cylinder and immersed in a preheated oil bath (83-85 °C). The reaction mixture turned black in 1 hr and was stirred for another 14 hr at this temperature. Then, the reaction mixture was cooled to room temperature and the pressure was released. The resulting mixture was diluted with ether (200 mL) and 1.0N NaOH (20 mL). The formed acid was extracted into water (2 x 100 mL). The combined water extracts were neutralized with 1.0N HCl and the acid was extracted into dichloromethane (3 x 100 mL). The combined dichloromethane extracts were washed with brine solution and dried over MgSO₄. Filtration of the drying agent and removal of solvent under vacuum gave 3.58 g (72.5%) of a viscous brown oil which slowly solidfied overnight. HR MS: Obs. mass, 164.0833. Calcd. mass, 164.0837 (M+).

Example 105. Preparation of 2-Chloro-6-acetylbenzoic acid.
a). Preparation of 1-acetyl-3-chloro-2-[[(trifluoromethyl)sulfonyl]oxy]benzene.
   To a solution of 1-acetyl-6-chlorophenol (2.9 mmol, 0.5 g) in dichloromethane (33 mL) was added 4-(N,N-dimethylamino)pyridine (6.54 mmol, 0.8 g) at -70 °C followed by triflic anhydride (4.33 mmol, 1.22 g, 0.73 mL) at -70 °C. After addition, the suspension was stirred for 30 min at this temperature and then warmed to room temperature and stirred for another 3hr, at which time TLC of the reaction mixture indicated the absence of starting material. The mixture was diluted with H₂O (50 mL) and the two layers were separated. The aqueous layer was extracted with dichloromethane (50 mL). The combined dichloromethane extracts were washed with brine solution and were dried over MgSO₄. Filtration of the drying agent and removal of solvent under vacuum gave an yellow oil which was purified by silica gel column chromatography to obtain 0.76 g (86%) of a colorless oil. HR MS: Obs. mass, 301.9617. Calcd. mass, 301.9627 (M+).
b). Preparation of 1-acetyl-3-chlorobenzoic acid.
   A 100 mL pressure bottle was charged with 1-acetyl-3-chloro-2-[[(trifluoromethyl)sulfonyl]oxy]benzene (2.41 mmol, 0.73 g), Pd(OAc)₂ (0.2 mmol, 47 mg) and dppp (0.2 mmol, 87 mg). Then, the flask was closed with a septum and evacuated three times with argon. Then, acetonitrile (96 mL), triethylamine (188.7 mmol, 19.0 g, 26.25 mL) and water (19.1 mL) were added successively by the aid of syringe. Then, the rubber septum was replaced with teflon lined cap connected to a carbon monoxide source. The flask was now pressurized with carbon monoxide (40 psi) and the excess pressure was released. This process was repeated three times and finally the mixture was stirred for 5 min under 40 psi carbon monoxide pressure. The flask was then disconnected from the carbon monoxide cylinder and immersed in a preheated oil bath (83-85 °C) and stirred for 3 hr. The reaction mixture was cooled to room temperature and the pressure was released and the mixture was diluted with ether (200 mL) and 1.0N NaOH (20 mL). The acid was extracted into water (2 x 100 mL). The combined water extracts were neutralized with 1.0N HCl and again the acid was extracted into dichloromethane (3 x 100 mL). The combined dichloromethane extracts were washed with brine solution and dried over MgSO₄. Filtration of the drying agent and removal of solvent under vacuum gave a crude residue which was recrystallized from dichloromethane (~10 mL) and hexane (∼8 mL) and storage in the refrigerator overnight. The precipitated solid was collected by filtration and dried under high vacuum to afford 330 mg (69%) of a colorless solid: mp 128-129 °C. HR MS: Obs. mass, 198.0090. Calcd. mass, 198.0084 (M+).

Example 106. Preparation of 2-*iso*-propyl-6-methylbenzoic acid.
a). Preparation of 2-(1-methylethyl)-6-methyliodobenzene
   To a suspension of 2-(1-methylethyl)-6-methylaniline (15.57 mmol, 14.9 g), in conc. HCl (50 mL) and 30 g of ice, was added dropwise a solution of NaNO₂ (110 mmol, 8 g) in H₂O (35 mL) at -5 °C to 5 °C for 30 min. After addition, the red colored solution was stirred for another 30 min. Then, a solution of KI (200 mmol, 33.2 g) in H₂O (50 mL) was added dropwise over 20 min at 0-5 °C. After the addition, the mixture was allowed to warm to room temperature during which time, an exothermic reaction with gas evolution occurred. The resulting red colored solution was stirred for 18 h. Then, the mixture was extracted with ethyl acetate (3 x 100 mL). The combined extracts were washed with sodium thiosulfate solution ( 200 mL), brine solution and dried over MgSO₄. Filtration of the drying agent and concentration of the solvent under vacuum gave a colored compound which was purified by a silica gel column chromatography to obtain pure 2-(1-methylethyl)-6-methyliodobenzene (17.8 g, 68%) of an yellow oil. HR MS: Obs. mass, 260.0063. Calcd. mass, 260.0062 (M+).
b) Preparation of 2-(1-methylethyl)-6-methylbenzoic acid.
   A 250 mL pressure bottle was charged with 2-(1-methylethyl)-6-methyliodobenzene (25.2 mmol, 6.55 g), Pd(OAc)₂ (1.2 mmol, 280 mg) and dppp (1.2 mmol, 520 mg). Then, the flask was closed with a septum and evacuated three times with argon. Then, acetonitrile (96 mL), triethylamine (188.7 mmol, 19.0 g, 26.25 mL) and water (19.1 mL) were added successively by the aid of syringe. Then, the rubber septum was replaced with teflon lined cap connected to a carbon monoxide source. The flask was now pressurized with carbon monoxide (40 psi) and the excess pressure was released. This process was repeated three times and finally the mixture was stirred for 5 min under 40 psi carbon monoxide pressure. The flask was then disconnected from the carbon monoxide cylinder and immersed in a preheated oil bath (83-85 °C). The reaction mixture turned black in 1 hr and was stirred for another 4 hr at this temperature. Then, the reaction mixture was cooled to room temperature, the pressure was released and the mixture was diluted with ether (200 mL) and 1.0N NaOH (10 mL). The acid was extracted into water (2 x 100 mL). The combined water extracts were neutralized with 1.0N HCl and the acid was extracted into ethyl acetate (2 x 100 mL). The combined organic extracts were washed with brine solution and dried over MgSO₄. Filtration of the drying agent and removal of solvent under vacuum gave 2.8 g (62%) of a viscous yellow oil. HR MS: Obs. mass, 178.0996. Calcd. mass, 178.0994 (M+).

Exmaple 107. N-(2-chloro-6-methylbenzoyl)-4-[(2,4-dimethyl-3-pyridinyl)carbonyl]amino]-L-phenylalanine.
a. Preparation of [[(2,4-dimethyl-3-pyridyl)carbonyl]amino]-L-phenylalanine methyl ester hydrochloride.
   To a solution of 4-amino-N-[(1,1-dimethylethoxyl)carbonyl]-L-phenylalanine methyl ester (1.4 g, 4.8 mmol) in DMF (12 mL) were added 2,4-dimethyl-3-pyridinecarboxylic acid hydrocloride (919 mg, 4.9 mmol), HBTU (1900 mg, 5 mmol) and diisopropylethylamine (2.7 mL, 15 mmol) at room temperature. The mixture was stirred for 15 hr and was diluted with 10 mL of ethyl acetate and 10 mL of water. The layers were separated and the aqueous layer was extracted with ethyl acetate (2 x 20 mL). The combined extracts were washed with brine and dried over anhydrous magnesium sulfate. Filtration and concentration of the solvent gave a crude product which was purified on silica gel eltuing with ethyl acetate:hexane (2:1 to 4:1) to give of 4-[(2,4-dimethyl-3-pyridyl)carbonyl)amino]-N-[(1,1-dimethylethoxyl)carbonyl]-L-phenylalanine methyl ester (226 mg). This compound (220 mg) was treated with 6 mL of 4 N hydrochloric acid in dioxane at room temperature. After 5 minutes, the solid went into solution and the mixture was stirred for 18 hr and was concentrated to give white solid (210 mg). This intermediate was used in the next step synthesis without further purification.
b. Preparation of N-(2-chloro-6-methylbenzoyl)-4- [(2,4-dimethyl-3-pyridyl)carbonyl]amino]-L-phenylalanine.
   A solution of 4-[(2,4-dimethyl-3-pyridyl)carbonyl]amino]-L-phenylalanine methyl ester hydrochloride (50mg, 0.125 mmol), 2-chloro-6-methylbenzoic acid (34 mg, 0.2 mmol), HBTU (76 mg, 0.2 mmol) and DIPEA (0.071 mL, 0.4 mmol) in DMF (0.5 mL) was stirred 15 hr at room temperature.The mixture was diluted with ethyl acetate (10 mL) and was washed with 0.5 N HCl (2 x 8 mL), sat. sodium bicarbonate (2 x 8 mL) and brine (2 x 8 mL) and was dried (Na₂SO₄). The solution was filtered and concentrated to a yellow gum which was hydrolyzed by treatment with 1N NaOH (0.5 mL) in MeOH (3 mL) at rt for 4 hrs. The reaction mixture was then acidified with acetic acid and purified by HPLC using conditions described in Example 76-77 to give a white solid (23.3 mg). MS (M+H): 466 (1 Cl).

Example 108. N-(2-Bromo-5-methoxybenzoyl)-4-[(2,4-dimethyl-3-pyridinyl)carbonyl]amino]-L-phenylalanine was prepared from 4-[(2,4-dimethyl-3-pyridyl)carbonyl]amino]-L-phenylalanine methyl ester hydrochloride and 2-bromo-5-methoxybenzoic acid using the general method described in example 107. MS (M+H) 526 (1Br).

Example 109. Preparation of 4-[[(2-chloro-5-cyanophenyl)carbonyl]amino]-L-phenylalanine methyl ester
a). Preparation of 4-[(2-chloro-5-bromophenylcarbonyl)amino]-N-[(1,1-dimethylethoxy)carbonyl]-L-phenylalanine methyl ester.
   To a mixture of 4-amino-N-[(1,1-dimethyethoxy)carbonyl]-L-phenylalanine methyl ester (20 mmol, 5.88 g), 2-chloro-5-bromobenzoic acid (22 mmol, 5.18 g) and HBTU (22 mmol, 8.34 g) in DMF (70 mL) was added diisopropylethylamine (50 mmol, 8.7 mL) at room temperature. The suspension was stirred for 48 hr at which time TLC analysis of the mixture indicated the absence of starting material. The mixture was diluted with water (100 mL) and the solids were collected by filtration and washed with water (150 mL). After air drying, the crude product was purified by silica gel column chromatography to obtain 1.02 g (10%) of a white solid: mp 158-161 °C. HR MS: Obs. mass, 533.0442. Calcd. mass, 533.0455 (M+Na).
b). Preparation of 4-[[(2-chloro-5-cyanophenyl)carbonyl]amino]-N-[(1,1-dimethylethoxy)carbonyl]-L-phenylalanine methyl ester.
   To a mixture of 4-[(2-chloro-5-bromophenylcarbonyl)amino]-N-[(1,1-dimethylethoxy)carbonyl]-L-phenylalanine methyl ester (2 mmol, 1.02 g), zinc cyanide (1.3 mmol, 152 mg) and Pd(PPh₃)₄ (0.2 mmol, 231 mg) was added distilled and deoxygenated DMF (8 mL) at room temperature. The suspension was heated to 80-85 °C and stirred for 15 hr at which time TLC analysis of the mixture indicated the absence of starting material. The reaction mixture was cooled to room temperature and diluted with ethyl acetate (70 mL) and was washed with 20% aqueous ammonium hydroxide (50 mL), brine solution (50 mL) and was dried over anhydrous magnesium sulfate. Filtration of the drying agent and concentration of the solvent gave a crude product which was purified by silica gel column chromatography to obtain 555 mg (61%) of a white solid: mp 185-187 °C. HR MS: Obs. mass, 480.1301. Calcd. mass, 480.1302 (M+Na).
c). Preparation of 4-[(2-chloro-5-cyanophenylcarbonyl)amino]-L-phenylalanine methyl ester TFA salt.
   To a solution of 4-[(2-chloro-5-cyanophenylcarbonyl)amino]-N-[(1,1-dimethylethoxy)carbonyl]-L-phenylalanine methyl ester (1.2 mmol, 0.55 g) in dichloromethane (12 mL) was added trifluoroacetic acid (3 mL) at room temperature. The reaction mixture was stirred for 15 hr at room temperature at which time TLC analysis of the mixture indicated the absence of starting material. The solvent was removed under vacuum and the residue was azeotrophed with toluene (2 x 10 mL) and dried under high vacuum to afford 0.43 g (100%) of an yellow solid. HR MS: Obs. mass, 358.0963. Calcd. mass, 358.0959 (M+H).

Example 110. Preparation of 4-[[(2-chloro-5-cyanophenyl)carbonyl]amino]-N-[1-(2-chloro-6-methylphenyl)carbonyl]-L-phenylalanine.
a) Preparation of 4-[(2-chloro-5-cyanophenylcarbonyl)amino]-N-[1-(2-chloro-6-methylphenyl)carbonyl]-L-phenylalanine methyl ester.
   Using the procedure described in example 3, 4-[(2-chloro-5-cyanophenylcarbonyl)amino]-N-[1-(2-chloro-6-methylphenyl)carbonyl]-L-phenylalanine methyl ester was prepared in 61% overall yield as a white solid. HR MS: Obs. mass, 510.1003. Calcd. mass, 510.0988, M+H.
b) Preparation of 4-[(2-chloro-5-cyanophenylcarbonyl)amino]-N-[1-(2-chloro-6-methylphenyl)carbonyl]-L-phenylalanine.
   To a mixture of 4-[(2-chloro-5-cyanophenylcarbonyl)amino]-N-[1-(2-chloro-6-methylphenyl)carbonyl]-L-phenylalanine methyl ester (0.146 mmol, 75 mg) and lithium iodide (1.5 mmol, 200 mg) was added pyridine (3 mL) at room temperature. The solution was refluxed for 15 h at which time TLC analysis of the mixutre indicated the absence of starting material. Then, it was cooled to room temperature and was diluted with water (15 mL). The pyridine was removed under reduced pressure on a rotary evaporator and the residue was extracted with ether (2 x 15 mL) to remove any neutral impurities. The aqueous layer was acidified with 1N HCl and the precipitated white solid was collected by filtration and was washed with 20 mL of water and 20 mL of hexane. After air-drying, the crude product was dissolved in ethyl acetate-hexane and stored in the refrigerator overnight. Only traces solid was formed and the solvent was decanted and removed under vacuum to give 55 mg (76%) of as a white solid. HR MS: Obs. mass, 496.0850. Calcd. mass, 496.0831 (M+H).

Example 111. Preparation of 4-[(2-chloro-6-methylphenylcarbonyl)amino]-N-[(1,1-dimethylethoxy)carbonyl]-L-phenylalanine methyl ester.

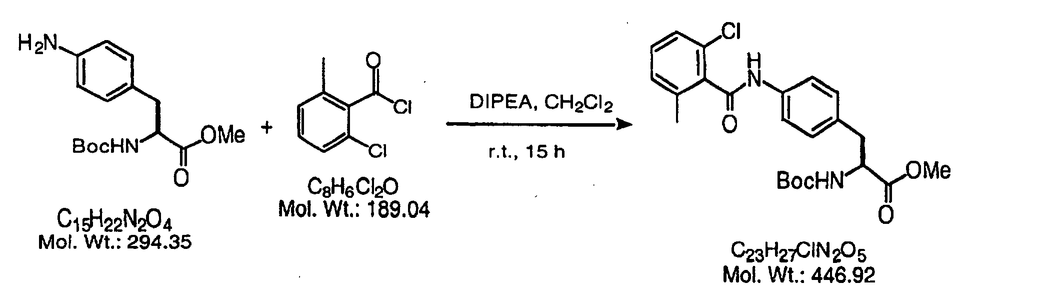

Using the procedure described in example 1, 4-[(2-chloro-6-methylphenylcarbonyl)amino]-N-[(1,1-dimethylethoxy)carbonyl]-L-phenylalanine methyl ester was prepared from 4-amino-N-[(1,1-dimethylethoxy)carbonyl]-L-phenylalanine methyl ester and 2-chloro-6-methylbenzoyl chloride in 83% overall yield as a white solid, mp 154-157 °C. HR MS: Obs. mass, 469.1513. Calcd. mass, 469.1507 (M+Na).

Example 112. Preparation of 4-[(2-chloro-6-methylphenylcarbonyl)amino]-L-phenylalanine methyl ester hydrochloride salt

Using the procedure described in example 2,4-[(2-chloro-6-methylphenylcarbonyl)amino]-L-phenylalanine methyl ester hydrochloride salt was prepared in 99% overall yield from the product of Ex. 111 as a white solid:. HR MS: Obs. mass, 347.1165. Calcd. mass, 347.1162 (M+H).

Example 113. 4-[(2-Chloro-6-methylphenylcarbonyl)amino]-N-[1-(2-methyl-6-ethylphenyl]carbonyl]-L-phenylalanine methyl ester was prepared using the procedure described in example 3 to give a 70% overall yield of a white solid.
HR MS: Obs. mass, 515.1690. Calcd. mass, 515.1714 (M+Na).

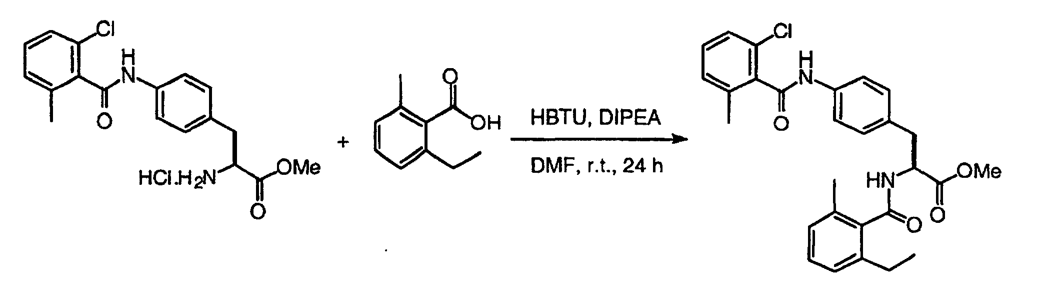

Example 114. N-[1-(2-Chloro-6-methylphenyl)carbonyl]-4-[[(2,6-difluorophenyl)carbonyl]amino]-L-phenylalanine was prepared from 4-amino-N-[(2-chloro-6-methylphenyl)carbonyl]-L-phenylalanine methyl ester and 2,6-difluorobenzoic acid using the procedures described in examples 109 and 13.
HR MS Obs. mass 473.1094. Calcd. mass 473.1079 (M+H).

Example 115. N-[1-(2-Chloro-6-methylphenyl)carbonyl]-4-[[(2,3,4,5,6-pentafluorophenyl)carbonyl]amino]-L-phenylalanine was prepared from 4-amino-N-[(2-chloro-6-methylphenyl)carbonyl]-L-phenylalanine methyl ester and pentafluorobenzoic acid using the procedure described in examples 109 and 13. HR MS Obs. mass, 527.0798. Calcd. mass 527.0797 (M+H).

Example 116. Preparation of (Z)-3-[4-[[(1,1-Dimethylethoxy)carbonyl]amino]-3-methylphenyl]-2-[[(phenylmethoxy)carbonyl]amino]-2-propenoic acid methyl ester.
a. Preparation of 4-[[(1,1-Dimethylethoxy)carbonyl]amino]-3-methylbenzyl alcohol. A solution of 3-methyl-4-nitrobenzyl alcohol (7.0 g, 42 mmol) in ethyl acetate (175 mL) and Boc anhydride (9.1 g, 42.7 mmol) was hydrogenated over 10% palladium on carbon (0.33 g) for 2 hr. The reaction mixture was filtered and the filtrate was concentrated. The residue was recrystallized from ether-hexane to give a white crystalline solid (6.73 g, (68%), mp 73-74 °C. Anal. (C13H19NO3):. C, 65.80; H, 8.07; N, 5.90. Fd. C, 65.74; H, 7.80; N, 5.80.
b. Preparation of 4-[[(1,1-Dimethylethoxy)carbonyl]amino]-3-methylbenzaldehyde. A solution of 4-[[(1,1-dimethylethoxy)carbonyl]amino]-3-methylbenzyl alcohol (7.2 g, 30.4 mmol) in dichloromethane (60 mL) was treated with manganese dioxide (4 x 7 g) at two hr intervals and the mixture was stirred at room temperature for 18 hr. The mixture was filtered through a pad of Celite washing with dichloromethane and the filtrate was concentrated. The residue was recrystallized from ether-hexane to give a white crystalline solid (6.3 g, 87%), mp 109-111 °C. Anal. (C13H17NO3): Calcd. C, 66.36; H, 7.28; N, 5.95. Fd. C, 66.14; H, 7.14; N, 5.85.
c. Preparation of (Z)-3-[4-[[(1,1-Dimethylethoxy)carbonyl]amino]-3-methylphenyl]-2-[[(phenylmethoxy)carbonyl]amino]-2-propenoic acid methyl ester.

A solution of N-[(phenylmethoxy)carbonyl]-2-phosphonoglycine trimethyl ester (11.9 g, 36 mmol) in dichloromethane (60 mL) was treated with tetramethlguanidine (4.5 mL, 36 mmol). After 1 hr, the mixture was cooled to an internal temperature of -30 °C and was treated with a solution of 4-[[(1,1-dimethylethoxy)carbonyl]amino]-3-methylbenzaldehyde (7.02 g, 29.8 mmol) in dichloromethane (25 mL) at such a rate that there was no temperature rise. The reaction mixture was stirred at -30 °C for 30 min and was allowed to warm to room temperature over night. The mixture was diluted with ether (150 mL) and was washed successively with 0.5 N Hcl (2 x 50 mL) and Sat. NaHCO3 (1 x 50 mL) and was dried over MgSO₄. The solution was concentrated and the residue was purified by chromatography on a Biotage Kilo Prep HPLC using a silica gel carttridge and eluting with ethyl acetate:hexane (1:2). Fractions containing the Z-isomer were combined and concentrated, finally under high vacuum to give as a colorless glass (11.48 g, 86%). Anal. (C24H28N2O6): Calcd. C, 65.44; H, 6.41; N, 6.36. Fd. C, 64.81; H, 6.43; N, 6.04. HR MS: Obs. mass, 440.1933. Calcd. mass, 440.1947 (M+H).

Example 117. Preparation of 4-[[(1,1-dimethylethoxy)carbonyl]amino]-3-methyl-N-[[(phenylmethoxy)carbonyl]-L-phenylalanine methyl ester.

A solution of (Z)- 3-[4-[[(1,1-dimethylethoxy)carbonyl]amino]-3-methylphenyl]-2-[[(phenylmethoxy)carbonyl)amino]-2-propenoic acid methyl ester (10 g, 22.7 mmol) from Ex. 116 in methanol (50 mL) and THF (20 mL) was placed in a pressure bottle and a stream of Ar was passed through the mixture over night. (+)-1,2-Bis((2S,5S)-2,5-dimethylphospholano)benzene (cyclooctadiene)rhodium trifluoromethane sulfonate (100 mg, 0.15 mmol) was added and the bottle was pressurized to 50 psi with hydrogen 3 times and the mixture was stirred over night at room temperature under 50 psi of hydrogen. The pressure was released and the solution was concentrated. The residue was treated with activated charcoal and recrystallized from ethyl acetate-hexane to give 6.72 g (67%), mp 120-121 °C. [a]₅₈₉ -5.9° (c = 1%, methanol).
HR MS (C24H30N2O6): Obs. Mass 442.2113. Calcd. Mass 442.2104 (M+).

Example 118. Preparation of 4-[[(1,1-dimethylethoxy)carbonyl]amino]-3-methyl-N-[[(2-chloro-6-methylphenyl)carbonyl]-L-phenylalanine methyl ester.
a. A solution of 4-[[(1,1-dimethylethoxy)carbonyl]amino]-3-methyl-N-[[(phenylmethoxy)carbonyl]-L-phenylalanine methyl ester (3.0 g, 6.8 mmol) in ethanol (40 mL) and cyclohexene (14 mL, 140 mmol) was treated with 10% palladium on carbon (1.5 g) and the mixture was heated to reflux for 20 min and allowed to cool. The mixture was filtered through a pad of celite washing with ethanol and the filtrate was concentrated to give 4-[[(1,1-dimethylethoxy) carbonyl]amino]-3-methyl-L-phenylalanine methyl ester (2.24 g) as a light yellow oil. HR MS (C16H24N2O4): Obs. Mass 309.1819. Calcd. Mass 309.1815 (M+H).
b. A solution of 4-[[(1,1-dimethylethoxy)carbonyl]amino]-3-methyl-L-phenylalanine methyl ester (1.0 g, 3.24 mmol) and 2-chloro-6-methylbenzoic acid (0.66 g, 3.86 mmol) in DMF (8 mL) was treated with HBTU (1.72 g, 4.53 mmol) and DIPEA (3 mL) 17 mmol) and the mixture was stirred over night. The solution was concentrated. The residue was dissolved in ethyl acetate (30 mL) and was washed with sat. NaHCO3 (10 mL), 0.1 N HCl (10 mL), and brine (10 mL) and was dried over Mg2SO4. The residue obtained after filtration and evaporation was purified by silica gel chromatography on 140 g of silica gel, eluting with 1:9 ethyl acetate:dichloromethane to give 1.16 g (78%) of a gum. HR MS (C24H29N2O5Cl): Obs. Mass 461.1858. Calcd. Mass 461.1844 (M+H).

Example 119. Preparation of 4-amino-3-methyl-N-[[(2-chloro-6-methylphenyl)carbonyl]-L-phenylalanine methyl ester hydrochloride salt.

4-[[(1,1-Dimethylethoxy)carbonyl]amino]-3-methyl-N-[[(2-chloro-6-methylphenyl)carbonyl]-L-phenylalanine methyl ester (1.1 g, 2.17 mmol) from Ex. 118 was treated with 4 N HCl in dioxane (20 mL) for 4 hr and was concentrated to dryness. The residue was triturated with ether and filtered to give 0.83 g, 96% as a white solid. HR MS (C19H22N2O3Cl2): Obs. Mass 361.1309. Calcd. Mass 361.1320 (M+H).

Example 120. Preparation of N-[1-(2-chloro-6-methylphenyl)carbonyl]-4-[[3-(3-hydroxyphenyl))-1-oxopropyl]amino]-3-methyl-L-phenylalanine.
a. A solution of 4-amino-3-methyl-N-[[(2-chloro-6-methylphenyl)carbonyl]-L-phenylalanine methyl ester hydrochloride salt (79.5 mg, 0.20 mmol) from Ex. 119, 3-(3-hydroxyphenyl)propanoic acid (33.2 mg, 0.20 mmol) and DIPEA (120 µL, 0.69 mmol) in dichloromethane (3 mL) was cooled to 10 °C and was treated with BOP-Cl (51 mg, 0.20 mmol). The mixture was stirred for 4 hr and was concentrated. The residue was dissolved in dichloromethane (15 mL) and was washed with 5 mL portions of 0.5 N NaCO3, 0.5 N HCl and saturated brine and was dried (MgSO4). The residue obtained after filtration and concentration was purified by chromatography on 25 g of silica gel, eluting with 7:3 ethyl acetate:hexane to give 47 mg of a colorless glass. HR MS: Obs. Mass 509.1849. Calcd. Mass 509.1844 (M+H).
b. A solution of N-[1-(2-chloro-6-methylphenyl)carbonyl]-4-[[3-(3-hydroxyphenyl))-1-oxopropyl]amino]-3-methyl-L-phenylalanine methyl ester (45 mg, 0.088 mmol) in THF (30 mL) was treated with solution of LiOH•H2O (20 mg, 0.47 mmol) in water (1.0 mL). Methanol (0.5 mL) was added for solubility and the mixture was stirred at room temperature for 18 hr. The mixture was acidified with 0.5 mL of acetic acid and was purified directly by RP-HPLC (5-95-35-214) to give, after lyopylization 34.3 mg of a white powder. HR MS (C27H27N2O5Cl): Obs. Mass 495.1697. Calcd. Mass 495.1687 (M+H).

Example 121. N-[1-(2-Chloro-6-methylphenyl)carbonyl]-4-[[2-(3-hydroxyphenyl))-1-oxoethyl]amino]-3-methyl-L-phenylalanine was prepared using the general procedure described in example 120 from 4-amino-3-methyl-N-[[(2-chloro-6-methylphenyl)carbonyl]-L-phenylalanine methyl ester (79.5 mg) and 2-(3-hydroxyphenyl)acetic acid (30 mg 0.2 mmol) to give 23 mg of a colorless glass. HR MS (C26H25N2O5Cl): Obs. Mass 481.1527. Calcd. Mass 481.1530 (M+H).

Example 122. N-[1-(2-Chloro-6-methylphenyi)carbonyl]-4-[[2-(3-nitrophenyl))-1-oxoethyl]amino]-3-methyl-L-phenylalanine was prepared from 4-amino-3-methyl-N-[[(2-chloro-6-methylphenyl)carbonyl]-L-phenylalanine methyl ester (52 mg) and 3-nitrobenzoic acid (32 mg, 0.19 mmol) using the procedure described in example 120 to give 15 mg of a white powder. HR MS (C25H22N3O6Cl): Obs. Mass 496.1288. Calcd. Mass 496.1288 (M+H).

Example 123. N-[1-(2-chloro-6-methylphenyl)carbonyl]-4-[[2,6-dichlorophenyl)carbonyl]amino]-3-methyl-L-phenylalanine was prepared from 4-amino-3-methyl-N-[[(2-chloro-6-methylphenyl)carbonyl]-L-phenylalanine methyl ester (87.4 mg, 0.22 mmol) and 2,6-dichlorobenzoyl chloride using the procedures described in examples 1 and 120 to give 56 mg of a white powder. HR MS (C25H21N2O4Cl3): Obs. Mass 519.0656. Calcd. Mass 519.0645 (M+H).

Example 124. Preparation of N-[(4-amino-2-chlorophenyl)carbonyl]-4-[[(2,6-dichlorophenyl)carbonyl]amino]-L-phenylalanine
a. A solution of 4-amino-2-chlorobenzoic acid (43 mg, 0.25 mmol) and 4-[(2,6-dichlorophenyl)carbonyl]amino]-L-phenylalanine methyl ester hydrochloride (100 mg, 0.25 mmol) and HBTU (100 mg, 0.27 mmol) in DMF (3 mL) was treated with DIPEA (0.20 mL) and the mixture was stirred 2 hr at room temperature. The mixture was diluted with water and was extracted with ethyl acetate. The organic layer was washed with saturated NaHCO3 and dried (MgSO4). The residue after filtration and concentration was chromatographed on 16 g of silica gel eluting with 4:1 ethyl acetate:hexane to give N-[(4-amino-2-chlorophenyl)carbonyl]-4-[[(2,6-dichlorophenyl)carbonyl] amino] -L-phenylalanine methyl ester (66 mg, 51%) of a white foam. HR MS (C24H20Cl3N3O4): Obs. Mass 520.0589. Calcd. Mass 520.0597 (M+H).
b. A solution of N-[(4-amino-2-chlorophenyl)carbonyl]-4-[[(2,6-dichlorophenyl)carbonyl]amino]-L-phenylalanine methyl ester (66 mg, 0.126 mmol) in THF (3mL) was treated with a solution of LiOH•H2O (20 mg, 0.48 mmol) in water (0.5 mL) and the mixture was stirred over night at room temperature. Acetic acid (0.5 mL) was added and the mixture was purified directly by RP-HPLC (5-95-35-214) to give 40 mg of a white solid. HRMS: (C23H18Cl3N3O4): Obs. Mass 506.0461. Calcd. Mass 506.0441 (M+H)

Example 125. Preparation of 4-(4-cyano-1,3-dioxo-2H-isoindol-2-yl)-N-[1-(2-chloro-6-methylphenyl)carbonyl]-L-phenylalanine
a) Preparation of 4-(4-cyano-1,3-dioxo-2H-isoindol-2-yl)-N-[1-(2-chloro-6-methylphenyl)carbonyl]-L-phenylalanine methyl ester 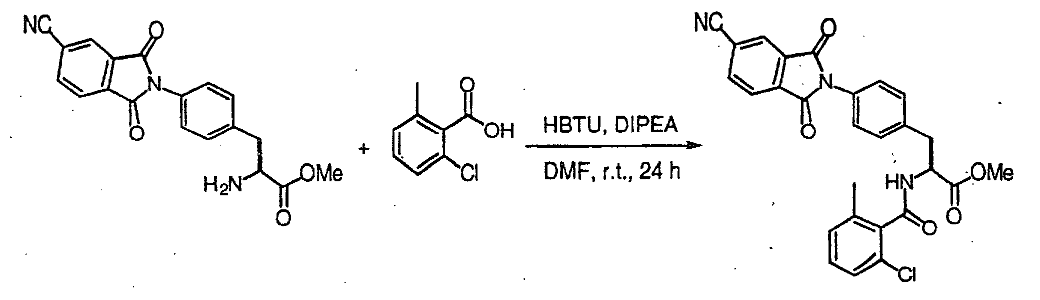
   Using the procedure described in example 3, 4-(4-cyano-1,3-dioxo-2H-isoindol-2-yl)-N-[1-(2-chloro-6-methylphenyl)carbonyl]-L-phenylalanine methyl ester was prepared in 63% overall yield as a white solid: mp 200-202 °C. HR MS:
   Obs. mass, 502.1173. Calcd. mass, 502.1169, M+H.
b) Preparation of 4-(4-cyano-1,3-dioxo-2H-isoindol-2-yl)-N-[1-(2-chloro-6-methylphenyl)carbonyl] -L-phenylalanine.
   Using the procedure described in example 110, 4-(4-cyano-1,3-dioxo-2H-isoindol-2-yl)-N-[1-(2-chloro-6-methylphenyl)carbonyl]-L-phenylalanine was prepared in 26% overall yield as a white solid: mp 170-175 °C. HR MS: Obs. mass, 488.1004. Calcd. mass, 488.1013, M+H.

Example 126. Synthesis of N-(2-chloro-6-methylbenzoyl)-4-[(2,6-dichlorophenyl)carbonyl]amino]-L-phenylalanine ethyl ester

To a solution of sodium salt of N-(2-chloro-6-methylbenzoyl)-4-[(2,6-dichlorophenyl)carbonyl]amino]-L-phenylalanine (1.583 g, 3.0 mmol) in DMF (75 mL) was added excess iodoethane (3.27 g, 21 mmol) at room temperature. The resulting solution was stirred for 24 hr. TLC analysis of the mixture indicated the absence of staring material and the excess iodoethane and some DMF was removed on a rotary evaporator under vaccum. The residue was diluted with 100 mL of ethyl acetate and was washed successively with water (2 x 100 mL), brine solution (100 mL) and dried over MgSO₄. Filtration of the drying agent and removal of the solvent afforded a white solid which was purified by silica gel column chromatography eluting with ethyl acetate:hexane (1:1) to obtain 1.4 g (87%) of ethyl ester as a white solid, mp 230-235 °C. HR MS: Obs. mass, 533.0817. Calcd. mass, 533.0801 (M+H).

Example 127. Syntheis of N-(2-chloro-6-methylbenzoyl)-4-[(2,6-dichlorophenyl)carbonyl]amino]-L-phenylalanine ethyl ester

To a suspension of N-(2-chloro-F-methylbenzoyl)-4-[(2,6-dichlorophenyl)carbonyl]amino]-L-phenylalanine (7.0 g, 13.84 mmol) and powdered sodium bicarbonate (5.88 g, 70 mmol) in DMF (100 mL) was added excess of iodoethane (10.91 g, 70 mmol) at room temperature. The resulting suspension was stirred for 20 h at which time TLC analysis of the mixture indicated the absence of staring material and the excess iodoethane and some DMF was removed on a rotary evaporator under vaccum. The remaining residue was diluted with 150 mL of ethyl acetate and washed successively with water (2 x 100 mL), brine solution (100 mL) and dried over MgSO₄. Filtration of the drying agent and removal of the solvent afforded a white solid which was crystallized from acetonitrile. The resulting crystalline solid was collected by filtration and dried under high vacuum to afford 5.58 g (77%) of N-(2-chloro-6-methylbenzoyl)-4-[(2,6-dichlorophenyl)carbonyl]amino]-L-phenylalanine ethyl ester as a white solid, mp 230-235 °C.

Example 128. Synthesis of of N-(2-chloro-6-methylbenzoyl)-4-[(2,6-dichlorophenyl)carbonyl)amino]-L-phenylalanine 2-morpholinoethyl ester.

To a solution of N-(2-chloro-6-methylbenzoyl)-4-[(2,6-dichlorophenyl)carbonyl]amino]-L-phenylalanine (0.505 g, 1.0 mmol) and 2-(4-morpholino)ethanol (0.262 g, 2.0 mmol) in THF (13 mL) was added dicyclohexylcarbodimide (0.309 g, 1.5 mmol) and 4-dimethylaminopyridine (61 mg, 0.5 mmol) at room temperature. The resulting cloudy solution was stirred for 4 h at which time TLC analysis of the reaction mixture indicated the absence of acid. Then, the mixture was diluted with water (50 mL) and extracted with ethyl acetate (3 x 50 mL). The combined extracts were washed with water (2 x 100 mL) and brine solution (100 mL) and were dried over MgSO₄. Filtration of the drying agent and removal of the solvent gave a white solid which was purified by silica gel column chromatography using dichloromethane:methanol (15:1) as eluent to obtain 0.428 g (69%) of N-(2-chloro-6-methylbenzoyl)-4-[(2,6-dichlorophenyl)carbonyl]amino]-L-phenylalanine 2-(4-morpholino)ethyl ester as a white solid, mp 109-118 °C. HR MS: Obs. mass, 618.1311. Calcd. mass, 618.1329 (M+H).

Example 129. Synthesis of of N-(2-chloro-6-methylbenzoyl)-4-[(2,6-dichlorophenyl)carbonyl]amino]-L-phenylalanine 2-(4-morpholino)ethyl ester.

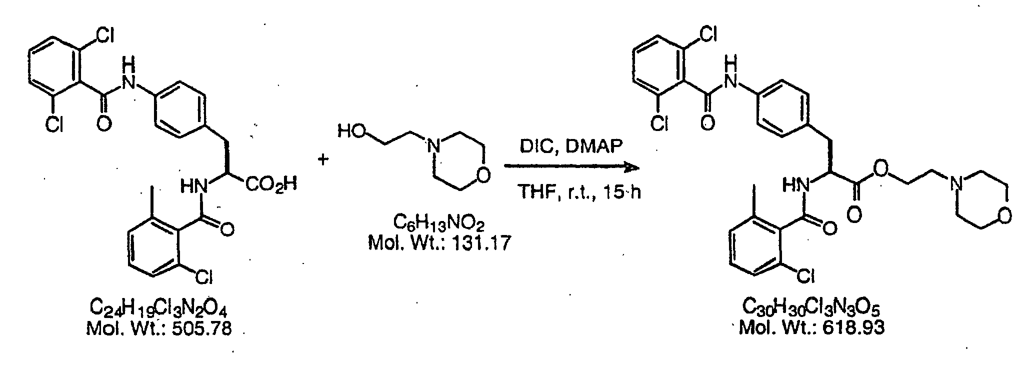

To a solution of N-(2-chloro-6-methylbenzoyl)-4-[(2,6-dichlorophenyl)carbonyl]amino]-L-phenylalanine (0.253 g, 0.5 mmol) and 2-(4-morpholino)ethanol (0.131 g, 1.0 mmol) in THF (5 mL) was added di-isopropylcarbodimide (94.6 mg, 0.75 mmol) and 4-dimethylaminopyridine (30.5 mg, 0.25 mmol) at room temperature. The resulting mixture was stirred for 15 h at room temperature at which time TLC analysis of the reaction mixture indicated the absence of acid. Then, the mixture was diluted with water (50 mL) and the THF was removed under vaccum and the residue was extracted with dichloromethane (3 x 25 mL). The combined extracts were washed with water (2 x 50 mL), brine solution (50 mL) and dried over MgSO₄. Filtration of the drying agent and concentration of the solvent gave a white solid which was purified by silica gel column chromatography using dichloromethane and ethyl acetate (5:1 to 1:1) and pure ethyl acetate as eluent to obtain 0.2 g (65%) of a white solid, mp 109-118 °C.

Example 130-132. Using the procedure described in Example 129, the following ester derivatives were prepared.

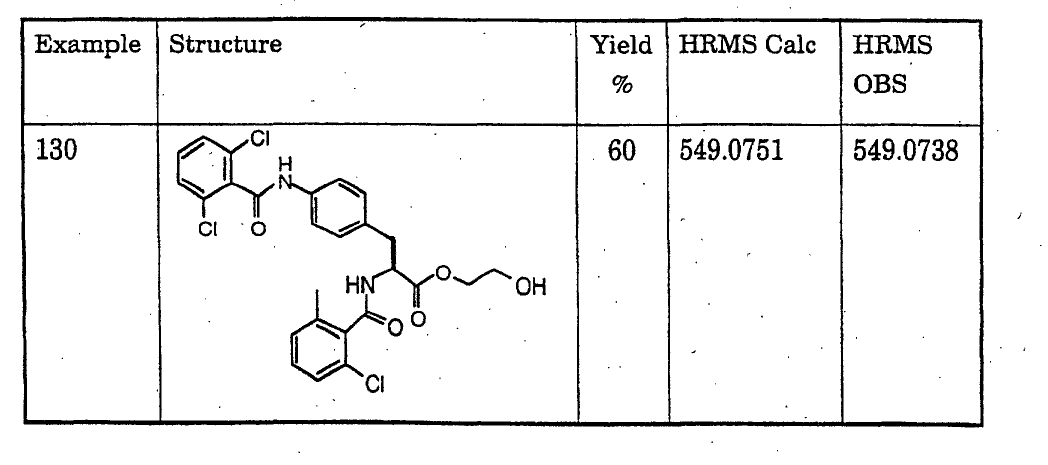

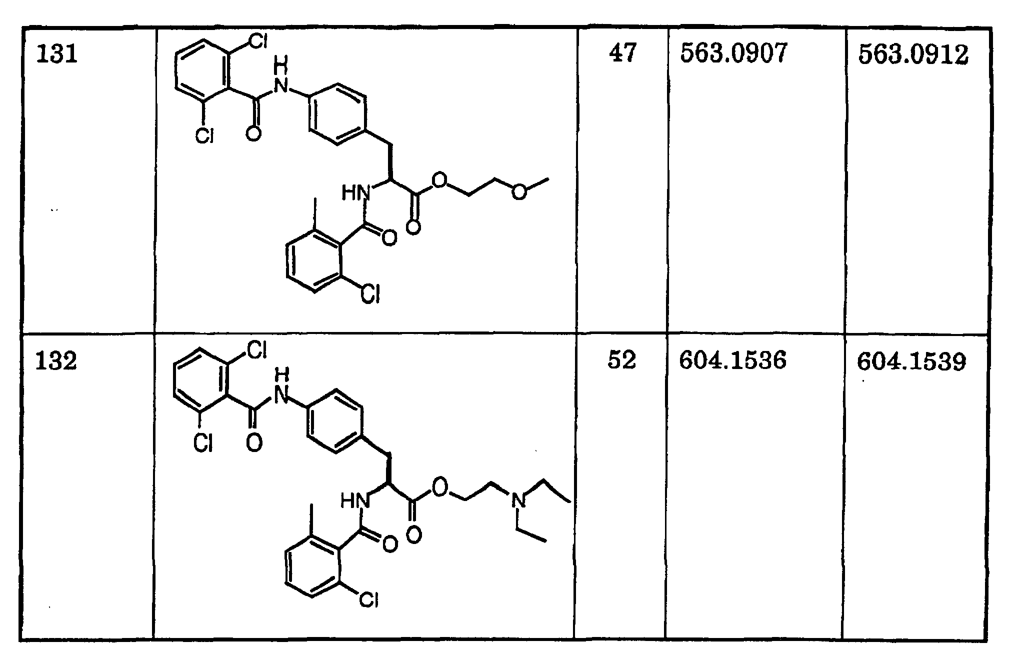

Example 133. Synthesis of N-(2-chloro-6-methylbenzoyl)-4-[(2,6-dichlorophenyl)carbonyl]amino]-L-phenylalanine 1-methyl-2-morpholinoethyl ester was prepared in 32% yield according to the procedure described in Example 129. HRMS Calcd: 632.1484. Obs: 632.1486 (M+H).

Example 134. N-(2-Chloro-6-methylbenzoyl)-4-[(2,6-dichlorophenyl)carbonyl]amino]-L-phenylalanine 1-methylethyl ester was prepared in 60% yield by the procedure described in Example 127. HRMS *m*/*z* Calcd, 569.0778. Obs, 569.0774 (M+Na).

Example 135. N-(2-Chloro-6-methylbenzoyl)-4-[(2,6-dichlorophenyl)carbonyl]amino]-L-phenylalanine 2-methylpropyl ester was prepared by the method described in Example 127. HRMS *m*/*z* Calcd, 561.1114. Obs, 661.1125 (M+H).

Example 136. N-(2-Chloro-6-methylbenzoyl)-4-[(2,6-dichlorophenyl)carbonyl]amino]-L-phenylalanine 1-methyl-4-piperidinyl ester was prepared in 65% by the method described in Example 128. HR MS C30H30Cl3N3O4): Obs, 602.1386-Calcd: 602.1380 (M+H).

Example 137. N-(2-Chloro-6-methylbenzoyl)-4-[(2,6-dichlorophenyl)carbonyl] amino]-L-phenylalanine butyl ester was prepared in 75% yield by the procedure described in Example 127. HR MS (C28H27Cl3N2O4): Obs, 561.1115. Calcd, 561.1114 (M+H).

Example 138. N-(2-Chloro-6-methylbenzoyl)-4-[(2,6-dichlorophenyl)carbonyl]amino]-L-phenylalanine 2-[4-[(1,1-dimethylethoxy)carbonyl]-1-piperazinyl]ethyl ester was prepared in 78% yield from N-(2-chloro-6-methylbenzoyl)-4-[(2,6-dichlorophenyl)carbonyl]amino]-L-phenylalanine and 2-[4-[(1,1-dimethylethoxy)carbonyl]-1-piperazinyl]ethanol using the procedure described in Example 129. HR MS: Obs. mass, 717.1995. Calcd. mass, 717.2013 (M+).

Example 139. N-(2-Chloro-6-methylbenzoyl)-4-[(2,6-dichlorophenyl)carbonyl] amino]-L-phenylalanine 2-(1-piperazinyl)ethyl ester

To a solution of N-(2-chloro-6-methylbenzoyl)-4-[(2,6-dichlorophenyl)carbonyl] amino]-L-phenylalanine 2-[4-[(1,1-dimethylethoxy)carbonyl]-1-piperazinyl]ethyl ester (1.0 mmol, 0.72 g) in dioxane (4 mL) was added a solution of HCl in dioxane (3.0 mmol, 0.75 mL, 4N) at room temperature. The resulting solution was stirred for 2 h at room temperature at which time TLC analysis of the reaction mixture indicated the absence of starting material. Then, the dioxane was removed under vacuum and the solid was triturated with ether (15 mL). The ether was decanted and the solid was dried under high vacuum to obtain 0.68 g (90%) as a white solid. HR MS (C30H31Cl3N4O4): Obs. mass, 617.1464. Calcd. mass, 617.1489 (M+H).

Example 140. Preparation N-(2-chloro-6-methylbenzoyl)-4-[(2,6-dichlorophenyl)carbonyl]amino]-L-phenylalanine 2-(4-methyl-1-piperazinyl)ethyl ester

To a suspension of N-(2-Chloro-6-methylbenzoyl)-4-[(2,6-dichlorophenyl)carbonyl]amino]-L-phenylalanine 2-(1-piperazinyl)ethyl ester dihydrochloride (1.0 mmol, 0.617 g) from Ex. 139 and K2CO3 (8.0 mmol, 1.1 g) in NMP (10 mL) was added methyl iodide (3.0 mmol, 0.43 g) at room temperature. The resulting mixture was stirred for 48 h at room temperature at which time TLC analysis of the reaction mixture indicated the absence of starting material. Then, the mixture was diluted with water (100 mL) and the precipitated solid was collected by filtration and dried under high vacuum. This solid was purified by reverse phase HPLC to obtain 0.35 g (55%) of a white solid. HR MS (C31H33Cl3N4O4): Obs. mass, 631.9208. Calcd. mass, 631.9193 (M+H).

Example 141. Preparation of N-methyl-N-[1-(2-chloro-6-methylphenyl)carbonyl]-4-nitro-L-phenylalanine methyl ester

To a suspension of N-[1-(2-chloro-6-methylphenyl)carbonyl]-4-nitrophenylalanine methyl ester (0.375 mmol, 142 mg) and silver oxide (1.5 mmol, 340 mg) in DMF (2 mL) was added methyl iodide (28 mmol, 1.75 mL) at room temperature. The suspesion was stirred for 2 days at room temperature, at which time TLC analysis of the mixture indicated the absence of starting material, and the solid was filtered. The solution was concetrated and diluted with ethyl acetate (30 mL) and washed with water (20 mL), brine solution (20 mL) and dried over anhydrous magnesium sulfate. Filtration of the drying agent and removal of the solvent gave 99 mg (67%) of a light brown oil. LR MS (C19H19ClN2O5): 390 (M+H).

Example 142. Preparation of 4-amino-N-methyl-N-[1-(2-chloro-6-methylphenyl)carbonyl]-L-phenylalanine methyl ester.

To a mixture of N-methyl-N-[1-(2-chloro-6-methylphenyl)carbonyl]-4-nitro-L-phenylalanine methyl ester (0.5 mmol, 192 mg) from Ex. 141, zinc dust (-325 mesh, 5.0 mmol, 0.33 g, 10 equiv.) and ammonium chloride (7.5 mmol, 0.4 g, 15 equiv.) was added methanol (4 mL) and water (2 mL) at room temperature. After addition of water, the reaction was exothermic. The suspension was stirred for 2 h at room temperature, at which time TLC analysis of the mixture indicated the absence of starting material, and the reaction mixture was filtered through the celite. The filter cake was washed with methanol (30 mL) and water (20 mL). The filtrate was concentrated to remove the methanol and the residue was extracted with ethyl acetate (3 x 20 mL). The combined extracts were washed with brine solution (30 mL) and dried over anhydrous magnesium sulfate. Filtration of the drying agent and concentration of the solvent afforded 148 mg (82%) of a yellow oil. LR MS (C19H21ClN2O3): 361(M+H).

Example 143. Preparation of 4-[(2,6-dichlorophenylcarbonyl)amino]-N-methyl-N-[1-(2-chloro-6-methylphenyl)carbonyl]-L-phenylalanine methyl ester.

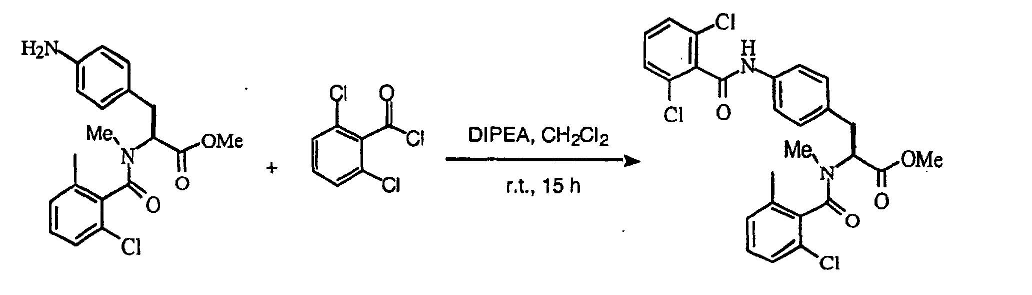

Using the procedure described in example 1, methyl 4-[[(2,6-dichlorophenyl)carbonyl]amino]-N-[1-(2-chloro-6-methylphenmyl)carbonyl]-L-phenylalanine was prepared in 68% overall yield as an amorpous solid. LR MS (C26H23Cl3N2O4) : 534 (M+H).

Example 144. Preparation of 4-[(2,6-dichlorophenylcarbonyl)amino]-N-methyl-N-[1-(2-chloro-6-methylphenyl)carbonyl]-L-phenylalanine

Using the procedure described in example 13, N-[1-(2-chloro-6-methylphenyl)carbonyl]-N-methyl-4-[[(2,6-dichlorophenyl)carbonyl]amino]-L-phenylalanine was prepared from the product of Ex. 143 in 59% overall yield as a white solid. HR MS: Obs. mass, 519.0631. Calcd. mass, 519.0645 (M+H).

Example 145. Preparation of 2-chloro-6-methylbenzoic acid.
a.Preparation of 2-chloro-6-methylbenzaldehyde.
   A 500 mL, three-necked, round bottomed flask equipped with a magnetic stirrer, thermometer, additional funnel, and argon inlet was charged with 75 g (494 mmol) of 2-chloro-6-methylbenzonitrile and 400 mL of toluene (stored over 4 Å molecular sieves). The mixture was cooled to -2 °C (ice + acetone) and a solution of DIBAL-H (593 mmol, 593 mL, 1.0N) in hexanes was added dropwise over a period of 30 min while maintaining the temperature below 0 °C. After the addition, the reaction mixture was stirred for 1 h at 0 °C and then allowed to warm to room temperature. After 2 h at room temperature, TLC analysis indicated the absence of starting material (4:1 hexane:ether, phosphomolybdic acid spray, as analysis by UV fluorescence was misleading). The reaction mixture was poured into a ice (2000 g) and concentrated sulfuric acid (50 mL) and was stirred for overnight. The precipitated solids were collected by filtration and the filtrate was extracted with ether (2 X 200 mL).
   The combined extracts were washed with brine solution and dried over MgSO₄. Filtration of the drying agent and concentration of the solution gave the crude aldehyde which was combined with the above solid to afford 71.31 g (93%) of light yellow solid suitable for use in the next step..
b. Preparation of 2-chloro-6-methylbenzoic acid.
   A 1000 mL, three-necked, round bottomed flask equipped with a magnetic stirrer, thermometer, additional funnel, and argon inlet was charged with 71.31 g (461 mmol, crude obtained from the above experiment) of 2-chloro-6-methylbenzaldehyde and 750 mL of acetonitrile. To this suspension, a solution of monobasic sodium phosphate (115 mmol, 15.9 g, 0.25 eq.) in water 240 mL) was added followed by hydrogen peroxide (50 mL, 30%) at room temperature. Then, a solution of sodium chlorite (73.5 g, 811 mmol, 1.76 eq.) in water (700 mL) was added dropwise at 0 °C while maintaining the temperature below 3 °C. After addition, the yellow suspension was stirred for 15 h at 0 °C to room temperature at which time TLC analysis of the mixture indicated the absence of aldehyde. Then, a solution of sodium bisulfite (73 g, 701 mmol, 1.52 eq.) in water (200 mL) was added dropwise at 0 °C until the yellow color disappear (KI-paper positive). Cooling is essential to control the exothermic reaction. The solvent was removed under vacuum to afford a white solid. The solid was collected by filtration and the filtrate was extracted with ether (200 mL). The above solid also dissolved in this ether solution and was washed with 10% NaOH solution (2 x 200 mL). The basic aqueous solution was neutralized with 10% HCl to pH ∼1. The precipitated white solid was collected by filtration and dried at air to afford 54.88 g (65%, overall in two steps) of 2-chloro-6-methyl benzoic acid as a white solid.

Example 146. Preparation of 4-[(2,6-dichlorophenyl)carbonyl]amino]-L-phenylalanine methyl ester.
a, Preparation of 4-nitro-N-[(1,1-dimethylethoxy)carbonyl]-L-phenylalanine methyl ester.
   To suspesion of 4-nitro-N-[(1,1-dimethylethoxy)carbonyl]-L-phenylalanine (226.2 mmol, 70.2 g) and sodium carbonate (1.13 mol, 95 g) in DMF (500 mL) was added methyl iodide (1.13 mmol, 70.4 mL) at room temperature. The suspesion was stirred for 15 h at room temperature at this time TLC analysis of the mixture indicated the absence of starting acid and the excess methyl iodide and some DMF were removed under high vacuum. The mixture was poured into water (2 L) and stirred at room temperature as a precipitate formed slowly over weekend. The precipitated solids were collected by filtration and washed with water (2 L). After air and vacuum drying, 72 g (98%) of of 4-nitro-N-[(1,1-dimethylethoxy)carbonyl]-L-phenylalanine methyl ester was isolated as a light yellow solid, mp 95-96 °C. ¹H NMR, DMSO-d₆ (400 MHz) δ 8.16 (d, 2H, J = 20 Hz), 7.53 (d, 2H, J = 20 Hz), 7.39 (d, 1H, J = 22 Hz), 4.26-4.28 (m, 1H), 3.6 (s, 3H), 2.96-3.19 (m, 2H), 1.25 (s, 9H). ¹³C NMR, CDCl₃ (100 Mhz) d 172.04, 155.29, 146.27, 145.96, 130.48, 123.18, 78.36, 54.44, 51.9, 36.1, 27.99. HR MS: Obs. mass, 325.1404. Calcd. mass, 325.1400 (M+H).
b. Preparation of 4-amino-N-[(1,1-dimethylethoxy)carbonyl]-L-phenylalanine methyl ester
   To a mixture of 4-nitro-N-[(1,1-dimethylethoxy)carbonyl]-L-phenylalanine methyl ester (222 mmol, 72 g), zinc dust (∼325 mesh, 2.2 mol, 145.2 g, 10 equiv.) and ammonium chloride (3.3 mol, 178.1 g, 15 equiv.) was added methanol (1 L) and water (500 mL) at room temperature. After addition of water, the reaction mixture was exothermic and the temperature raised to 45 to 50 °C. The suspension was stirred for 1 h at room temperature at which time TLC analysis of the mixture indicated the absence of starting material and the reaction mixture was filtered through the celite, washing the filtered cake with methanol (1 L) and water (500 mL). Concentration to remove the methanol and some water resulted in formation of a white solid which was collected by filtration and washed with water. After air drying, 65.5 g (quant) of a white solid, mp 86-89 °C was obtained. ¹H NMR, DMSO-d₆ (400 MHz) δ 6.9 (d, 2H, J = 20 Hz), 6.62 (d, 2H, J = 20 Hz), 7.39 (d, 1H, J = 22 Hz), 4.26-4.28 (m, 1H), 3.68 (s, 3H), 2.96-3.19 (m, 2H), 1.25 (s, 9H). HR MS: Obs. mass, 284.1614. Calcd. mass, 294.1621).
c. Preparation of 4-[(2,6-dichlorophenylcarbonyl)amino]-N-[(1,1-dimethylethoxy)carbonyl]-L-phenylalanine methyl ester.
   To a solution of 4-amino-N-[(1,1-dimethylethoxy)carbonyl]-L-phenylalanine methyl ester (127.6 mmol, 37.57 g) and 2,6-dichlorobenzoyl chloride (140.6 mmol, 29.45 g) in dichloromethane (350 mL) was added diisopropylethylamine (192 mmol, 33.4 mL) at room temperature. The brown solution was stirred for 15 h at room temperature to afford a white suspension. At this time, TLC analysis of the mixture indicated the absence of starting material. The solids were collected by filtration and were washed with dichloromethane (150 mL) and air dried to obtain 52.75 g (88.4%) of a white solid, mp 148-151 °C. ¹H NMR, DMSO-d₆ (400 MHz) δ 10.68 (s, 1H), 7.47-7.6 (m, 5H), 7.2-7.29 (m, 3H), 4.12-4.17 (m, 1H), 3.62 (s, 3H), 2.79-2.99 (m, 2H), 1.33 (s, 9H). ¹³C NMR, CDCl₃ (100 Mhz) d 172.49, 161.82, 155.37, 136.99, 136.36, 131.28, 131.16, 129.48, 128.19, 119.31, 78.27, 55.3, 51.76, 35.9, 27.77. HR MS: Obs. mass, 466.1069. Calcd. mass, 466.1062 (M+H).
d. Preparation of 4-[(2,6-Dichlorophenylcarbonyl)amino]-L-phenylalanine methyl ester hydrochloride salt.
   4-[(2,6-Dichlorophenylcarbonyl)amino]-N-[(1,1-dimethylethoxy)carbonyl]-L-phenylalanine methyl ester (92.97 mmol, 43.45 g) in dioxane (90 mL) was treated with 166 mL of 4 N hydrochloric acid in dioxane at room temperature. After 5 minutes, the solids went into solution and the mixture was stirred for 2 h. Some of the dioxane was removed under vacuum to afford a yellow syrup and 250 mL of ethyl ether was added. A gum was formed which was dissolved in THF (100 mL) and methanol (100 mL). The solvent was removed under vacuum to obtain 43.7 g (100%) of the hydrochloride salt as a white solid. ¹H NMR, DMSO-d₆ (400 MHz) δ 10.81 (s, 1H), 7.76 (d, 2H, J = 22 Hz), 7.58 (d, 2H, J = 18 Hz), 7.51 (t, 1H, J = 15 Hz), 7.24 (d, 2H, J = 22 Hz),4.23-4.26 (m, 1H), 3.56 (s, 3H), 3.14-3.17 (m, 2H). ¹³C NMR, CDCl₃ (100 Mhz) d 169.03, 161.72, 137.56, 136.11, 131.19, 130.95, 129.93, 129.79, 128.06, 119.46, 53.17, 52.6, 35.13. HR MS: Obs. mass, 367.0611. Calcd. mass, 367.0616 (M+).

Example 147. Preparation of 4-[[(2,6-dichlorophenyl)carbonyl]amino]-N-[(2-chloro-6-methylbphenyl)carbonyl]-L-phenylalanine methyl ester.

To a solution of 4-[[(2,6-dichlorophenyl)carbonyl]amino]-L-phenylalanine methyl ester hydrochloride salt (272.5 mmol, 110 g) from Ex. 146 and 2-chloro-6-methyl benzoic acid (276 mmol, 47.15 g) in DMF (600 mL) were added HBTU (276 mmol, 105 g) and diisopropylethylamine (1.24 mol, 119 mL) at room temperature. The clear solution was stirred 48 h at room temperature at which time TLC analysis of the reaction mixture indicated the absence of the starting material. The reaction mixture was poured slowly into 5 L of water which contained some ice to lower the temperature. The white precipitated solid was allowed settle and the solid was collected by filtration. The solid cake was washed with water (1 L) and hexane (1 L) and air dried to obtain 150 g of a crude product. This solid product was dissolved in hot acetonitrile (1 L) and cooled in the refrigerator. The solid was collected by filtration and washed with hexane (500 mL) and air dried to obtain 101.1 g. The mother liquor was concentrated and the residue was purified by silica gel column chromatography eluting with dichloromethane and ethyl acetate (15:1) to obtain another 17.07 g (total = 118.17g, 83%), mp 244-245 °C. ¹H NMR, DMSO-d₆ (400 MHz) δ 10.66 (s, 1H), 8.83 (d, 1H, J = 19 Hz), 7.47-7.6 (m, 5H), 7.15-7.29 (m, 5H), 4.58-4.68 (m, 1H), 3.65 (s, 3H), 3.12 (dd, 1H, J = 17, 13 Hz), 2.87 (dd, 1H, J = 17, 11 Hz), 2.09 (s, 3H). HR MS: Obs. mass, 518.0652. Calcd. mass, 518.0641.

Example 148. Preparation of 4-[[(2,6-dichlorophenyl)carbonyl]amino]-N-[(2-chloro-6-methylphenyl)carbonyl]-L-phenylalanine.

To a suspension of 4-[[(2,6-dichlorophenyl)carbonyl]amino]-N-[(2-chloro-6-methylphenyl)carbonyl]-L-phenylalanine methyl ester (166 mmol, 86.2 g) from Ex. 147 in ethanol (350 mL) was added aqueous 1.0 N sodium hydroxide (250 mL) at room temperature. The mixture was heated to 40-45 °C and the resulting clear solution was stirred for 3-4 h. Then, the mixture was cooled to room temperature and the ethanol was removed on a rotary evaporator. The residue was diluted with 100 mL of water. The neutral impurities was extracted into ether (2 x 100 mL) and the basic aqueous layer was neutralized with 1 N HCl. The precipitated solid was collected by filtration and the solid cake was washed with water (1 L) and dried at air over weekend. The crude solid was dissolved in hot acetontile (2 L) and the resulting solution was stored in the refrigerator for 15 h. The white crystalline solids were collected by filtration and washed with cold acetonitrile (100 mL). After air drying, 79.76 g (95%) of a white solid, mp 212-215 °C was obtained. ¹H NMR, DMSO-d₆ (400 MHz) δ 10.66 (s, 1H), 8.85 (d, 1H, J = 19 Hz), 7.47-7.6 (m, 5H), 7.15-7.29 (m, 5H), 4.58-4.68 (m, 1H), 3.12 (dd, 1H, J = 17, 13 Hz), 2.87 (dd, 1H, J = 17, 11 Hz), 2.09 (s, 3H). HR MS: Obs. mass, 505.0483. Calcd. mass, 505.0488 (M+).

Example 149. Preparation of 2,6-Dimethyl-4-trifluoromethyl-3-pyridinecarboxylic acid.

A solution of 2,6-dimethyl-4-trifluoromethyl-3-pyridinecarboxylic acid ethyl ester in 40 mL of THF and 10 mL of 1 N sodium hydroxide solution was heated to reflux for 48 h. TLC of the mixture (3:7 methanol:dichloromethane) indicated that starting material was consumed. The mixture was acidified with acetic acid (5 mL) and evaporated to dryness. The residue was triturated with THF and the solution was concentrated to give 0.7 g of material containing some THF and acetic acid as indicated by NMR. This material was combined with the product of a similar experiment and was chromatographed on 90 g of silica gel, eluting with (3:7) methanol:dichloromethane to give 1.05 g of a solid. This material was diluted with toluene (6 mL) and evaporated several times to remove most of the acetic acid to afford after drying under high vacuum, 0.9 g of a white foam. LR-ES- MS (C9H6F3NO2): 218 (M-H).

Example 150. Preparation of N-[(2-chloro-6-methylphenyl)carbonyl]-4-[(2,6-dimethyl-4-trifluoromethyl-3-pyridinyl)carbonyl]amino]-L-phenylalanine.
a. To a solution of 2,6-dimethyl-4-trifluoromethylpyridine carboxylic acid (102 mg, 0.6 mmol) from Ex. 149 in dichloromethane (3 mL) was added a drop of DMF and oxalyl chloride (0.78 mmol, 99 mg) at 0 °C (ice bath). The solution was stirred at this temperature for 30 min, warmed to room temperature and stirred for an additional 1 h. Then, the solvent and excess oxalyl chloride was removed under vacuum and the residue was dried under high vacuum. To this 4-amino-N-[(2-chloro-6-methylphenyl)carbonyl]-L-phenylalanine methyl ester (0.5 mmol, 212 mg) was added and the mixture was dissolved in dichloromethane (5 mL). To this clear solution was added DIPEA (2.0 mmol, 0.258 g) at room temperature. The mixture was stirred for 15 h at which time TLC analysis of the mixture indicated the absence of starting material. The mixture was diluted with dichloromethane (20 mL) and water (100 mL). The two layers were separated and the organic layer was washed with saturated sodium bicarbonate solution (20 mL), brine solution (30 mL) and was dried over anhydrous magnesium sulfate. Filtration of the drying agent and removal of the solvent gave a crude product which was used directly in the next step.

Examples 151-155. The N-[(2-chloro-6-methylphenyl)carbonyl]-4-[(heteroaryl)carbonyl]amino]-L-phenylalanine derivatives listed below were prepared by treatment of equimolar amounts of 4-amino-N-[(2-chloro-6-methylphenyl)carbonyl]-L-phenylalanine methyl ester and the appropriate heteroaromatic carboxylic acids using the coupling procedure described in example 109 and the ester hydrolysis procedure described in example 13.

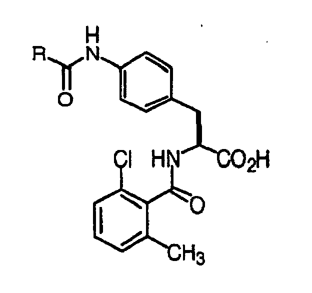

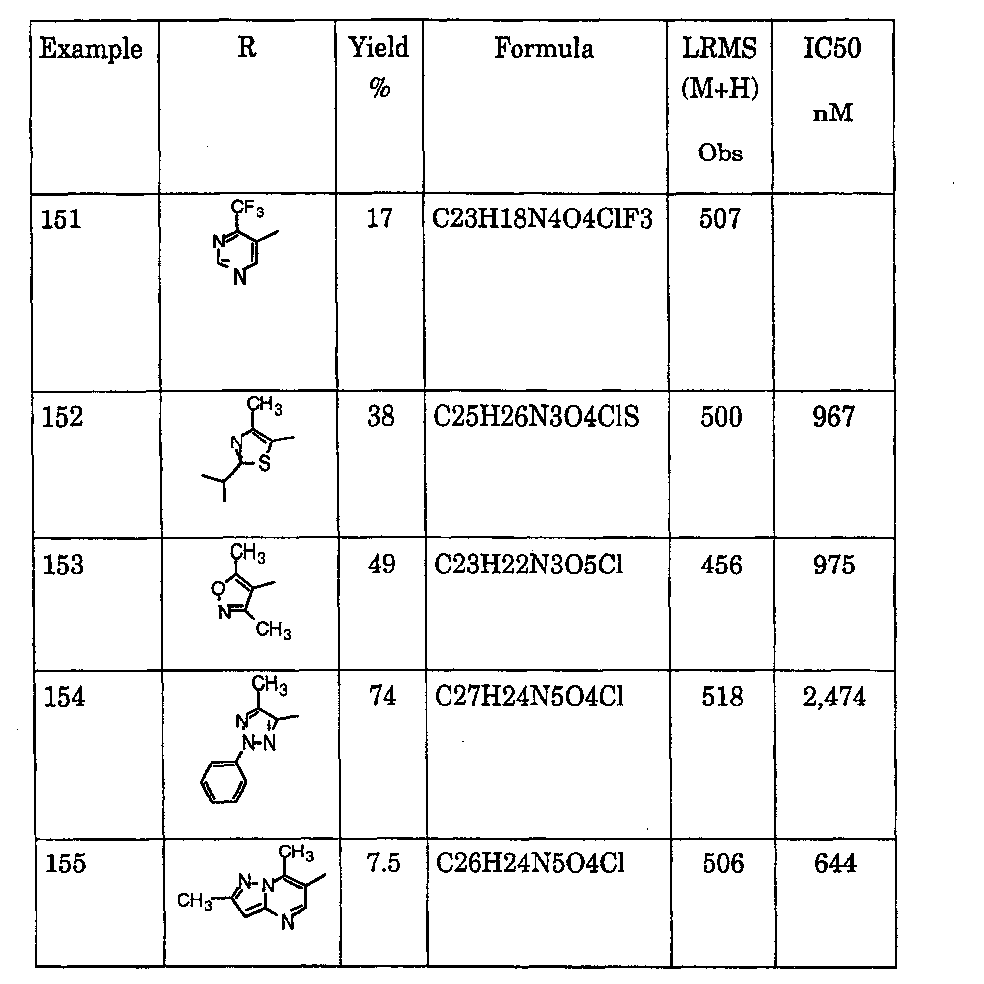

Examples 156-160. The 4-[[(2,6-Dichlorophenyl)carbonyl]amino]-N-[(heteroaryl)carbonyl]-L-phenylalanine derivatives listed below were prepared by coupling of 4-[[(2,6-dichlorophenyl)carbonyl]amino]- L-phenylalanine methyl ester and the appropriate heteroaromatic carboxylic acid using the general procedure described in example 3, followed by ester hydrolysis using the general procedure described in example 13.

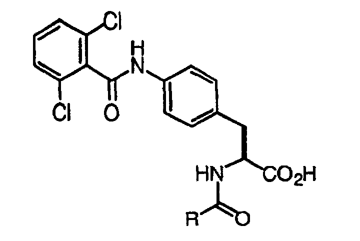

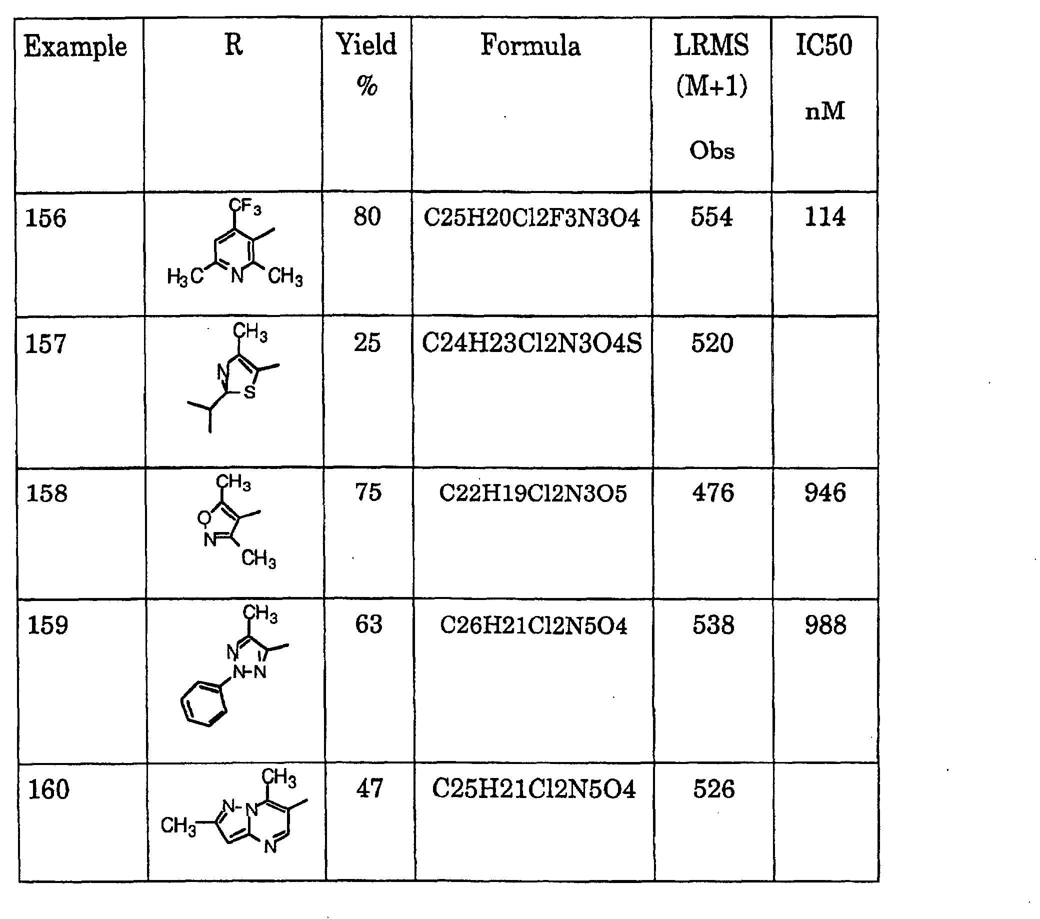

Example 161. 4-[[(2,6-Dichlorophenyl)carbonyl]amino]-N-[(1-naphthyl)carbonyl]-L-phenylalanine methyl ester was prepared in 77% yield from 4-[[(2,6-dichlorophenyl)carbonyl]amino]-L-phenylalanine methyl ester and 1-naphthoic acid using the general procedure described in example 3. HR MS: Obs. mass, 521.1024. Calcd. mass, 521.1053 (M+H).

Example 162. N-[(2-Acetyl-6-methylphenyl)carbonyl]-4-[[(2,6-dichlorophenyl)carbonyl]amino]-L-phenylalanine was prepared in 38 % yield from 4-[[(2,6-dichlorophenyl)carbonyl]amino]-L-phenylalanine methyl ester and 2-acetyl-6-methylbenzoic acid using the general procedure described in example 3. HR MS: Obs. mass, 547.0579. Calcd. mass, 547.0594 (M+Na).

Example 163. 4-[[(2,6-Dichlorophenyl)carbonyl]amino]-N-[[2-(1,1-dimethylethyl)phenyl]carbonyl]-L-phenylalanine methyl ester was prepared from 4-[[(2,6-dichlorophenyl)carbonyl]amino]-L-phenylalanine methyl ester and 2-(1,1-dimethylethyl)benzoic acid using the general procedure described in example 3. HR MS: Obs. mass, 527.1523. Calcd. mass, 527.1573 (M+H).

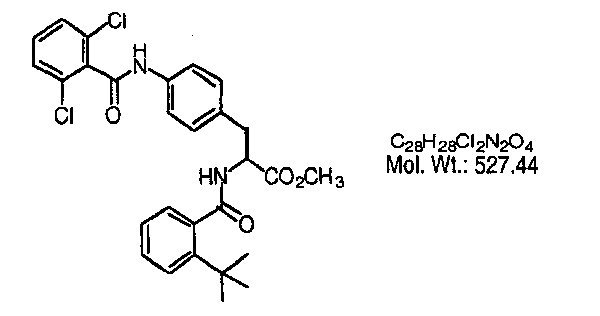

Example 164. 2,6-Bis-(1-methylethyl)benzoic acid was prepared in two steps from 2,6-bis(1-methylethyl)phenol using the two step general procedure described in example 105. HR MS: Obs. mass, 206.0325. Calcd. mass, 206.0342 (M+).

Example 165.4-[[(2,6-Dichlorophenyl)carbonyl]amino]-N-[[2,6-bis-(1-methylethyl)phenyl]carbonyl]-L-phenylalanine methyl ester was prepared from 4-[[(2,6-dichlorophenyl)carbonyl]amino]-L-phenylalanine methyl ester and 2,6-bis-(1-methylethyl)benzoic acid using the general procedure described in example 3. LR MS: 555 (M+).

Example 166. 4-[[(2,6-Dichlorophenyl)carbonyl]amino]-N-[(2-methoxyphenyl)carbonyl]-L-phenylalanine methyl ester was prepared from 4-[[(2,6-dichlorophenyl)carbonyl]amino]-L-phenylalanine methyl ester and 2-methoxybenzoic acid using the general procedure described in example 3. HR MS: Obs. mass, 501.0984. Calcd. mass, 501.0984 (M+H).

Example 167.4-[[(2,6-Dichlorophenyl)carbonyl]amino]-N-[(2-chloro-4-methylsulfonylphenyl)carbonyl]-L-phenylalanine methyl ester was prepared in 73% yield from 4-[[(2,6-dichlorophenyl)carbonyl]amino]-L-phenylalanine methyl ester and 2-chloro-4-methylsulfonylbenzoic acid using the general procedure described in example 3. HR MS: Obs. mass, 583.0263. Calcd. mass, 583.0264 (M+H).

Example 168. N-[(2,6-Dichlorophenyl)carbonyl]-4-[[(2-chloro-6-methylphenyl)carbonyl]amino]-L-phenylalanine methyl ester was prepared from 4-[[(2-chloro-6-methylphenyl)carbonyl]amino]-L-phenylalanine methyl ester and 2,6-dichlorobenzoic acid using the general procedure described in example 3.

Exmample 169. N-[(2,6-Dichlorophenyl)carbonyl]-4-[[(2-chloro-6-methylphenyl)carbonyl]amino]-L-phenylalanine was prepared by hydrolysis of N-[(2,6-dichlorophenyl)carbonyl]-4-[[(2-chloro-6-methylphenyl)carbonyl]amino]-L-phenylalanine methyl ester from Ex. 168 using the general procedure described in example 13.

Example 170. Preparation of 4-[(2S,4R)-3-Acetyl-2-phenyl-4-(phenylmethyl)-5-oxo-1-imidazolinyl]-N-[(2-chloro-6-methylphenyl)carbonyl]-L-phenylalanine and 4-[(2R,4R)-3-acetyl-2-phenyl-4-(phenylmethyl)-5-oxo-1-imidazolinyl]-N-[(2-chloro-6-methylphenyl)carbonyl]-L-phenylalanine. 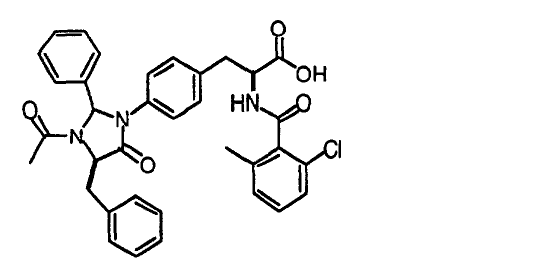
a. Synthesis of N-[(1,1-dimethylethoxy)carbonyl]-4-[[(2R)-2-amino-1-oxo-3-phenylpropyl]amino]-L-phenylalanine methyl ester
   To a solution of 4-amino-N-[(1,1-dimethylethoxy)carbonyl]-L-phenylalanine methyl ester (5.09 g, 17 mmol) in DMF (60 mL) was added Fmoc-D-Phenylalanine (8.70 g, 22.5 mmol), DIPEA (12 mL, 69 mmol) and HBTU (8.50 g, 22.5 mmol). The mixture was then stirred at room temperature for 4 h.
   The reaction mixture was diluted with water (150 mL) and the light yellow solid which precipitated was collected by filtration. This solid was then redissolved in 60 mL of acetone and the solution was treated with 100 mL of water. The solid was collected by filtration and was washed with 1N HCl, H₂O. After drying at 60 °C under vaccum overnight, a light yellow solid was obtained (13.2 g). A portion of this solid (2.51 g, 3.78 mmol) was dissolved in 15 mL of DMF and to the solution was added 1.5 mL of piperidine. The above solution was stirred at room temperature for 45 min. After removal of the solvent, the residue was recrystillized from ethyl acetate-hexane to give N-[(1,1-dimethylethoxy)carbonyl]-4-[[(2R)-2-amino-1-oxo-3-phenylpropyl]amino]-L-phenylalanine methyl ester (1.36 g, 3.0 mmol) in 81.5 % yield. LR MS 442 (M+H).
b. Synthesis of 4- (3-acetyl-5-oxo-2-phenyl-4-phenylmethyl-1-imidazolidinyl)-N-[(1,1,-dimethylethoxy)carbonyl]-L-phenylalanine methyl ester.
   A solution of above amine (1.48, 3.35 mmol) and benzaldehyde (376 µl, 3.7 mmol) in dichloromethane (10 mL) and methyl orthoformate (10 mL) was stirred at room temperature for 3 days. The reaction flask was then warmed to 90_ C and acetic anhydride (neat, 1.8 mL) was added. The resulting mixture was stirred at 110_ C for 4 hr. The solvent was then evaporated and crude product was purified by silica gel chromatography (ethyl acetate:hexane = 1:1) to give 4-(3-acetyl-5-oxo-2-phenyl-4-phenylmethyl-1-imidazolidinyl)-N-[(1,1,-dimethylethoxy)carbonyl]-L-phenylalanine methyl ester diatereomer 1 (417 mg) and diastereomer 2 (1.25 g) These compounds are diastereomeric at the 2-position of the imidazolidinone ring. Both diastereomers gave LR MS (C33H37N3O6): 572 (M+H).
c. Preparation of 4-[(2S,4R)-3-Acetyl-2-phenyl-4-(phenylmethyl)-5-oxo-1-imidazolinyl]-N-[(2-chloro-6-methylphenyl)carbonyl]-L-phenylalanine methyl ester.
   4-(3-acetyl-5-oxo-2-phenyl-4-phenylmethyl-1-imidazolidinyl)-N-[(1,1,-dimethylethoxy)carbonyl]-L-phenylalanine methyl ester (Diastereomer 1) (415 mg, 0.7 mmol) was treated with 10 mL of 4N HCl in dioxane at room temperature for 2 hr. After removal of solvent, the residue was dried overnight under vacuum. The residue (241 mg, 0.471 mmol) was dissolved in DMF (4 mL) and was treated with 2-chloro-6-methylbenzoic acid (105 mg, 0.617 mmol), HBTU (234 mg, 0.617 mmol) and DIEA (246 µL, 1.42 mmol) at room temperature for 4 hr. The mixture was diluted with 30 mL of ethyl acetate, the mixture was washed with 1N HCl, water and brine ( 8 mL each), After it was dried over MgSO4, the solvent was removed and the residue was filtered through silica gel eluting with ethyl acetate:hexane (4:1) to give 4-(3-acetyl-5-oxo-2-phenyl-4-phenylmethyl-1-imidazolidinyl)-N-[(2-chloro-6-methylphenyl)carbonyl]-L-phenylalanine methyl ester diastereomer 1.
d. Preparation of 4-(3-acetyl-5-oxo-2-phenyl-4-phenylmethyl-1-imidazolidinyl)-N-[(2-chloro-6-methylphenyl)carbonyl]-L-phenylalanine diastereomer 1..
   4-(3-acetyl-5-oxo-2-phenyl-4-phenylmethyl-1-imidazolidinyl)-N-[(2-chloro-6-methylphenyl)carbonyl]-L-phenylalanine methyl ester diastereomer 1 (90 mg, 0.128 mmol) in EtOH (3 mL) was treated with NaOH (1N, 0.3 mL) at room temperature for 30 min. The resulting solution was acidified with 1 drop of HOAc and was purified by HPLC (C-18, linear gradent from 5% acetonitrile to 95% in water over 30 min) to give a white solid after lyophization. MS: obs. mass, 609.9 (M+H).

Example 171. 4-[(2S,4R)-3-Acetyl-2-phenyl-4-(3-pyridinylmethyl)-5-oxo-1-imidazolinyl]-N-[(2-chloro-6-methylphenyl)carbonyl]-L-phenylalanine and 4-[(2R,4R)-3-acetyl-2-phenyl-4-(3-pyridinylmethyl)-5-oxo-1-imidazolinyl]-N-[(2-chloro-6-methylphenyl)carbonyl]-L-phenylalanine was prepared from 4-aimino-N-[(2-chloro-6-methylphenyl)carbonyl]-L-phenylalanine methyl ester and Fmoc-D-3-pyridinylalanine using the general procedure described in example 170. The two diastereomers at the 2-position of the imidazoline ring were not readily separated by C-18 RP-HPLC and the compounds were assayed as a mixture. HR MS: obs. 611.2070, calc. 611.2061 (M+H).

Example 172. 4-[[(2,6-Dichlorophenyl)carbonyl]amino]-N-[(2-chloro-4-hydroxyphenyl)carbonyl]-L-phenylalanine methyl ester was prepared in 35% yield from 4-[[(2,6-dichlorophenyl)carbonyl]amino]-L-phenylalanine methyl ester and 2-chloro-4-hydroxybenzoic acid using the general procedure described in example 3. HR MS: Obs. mass, 521.0433. Calcd. mass, 521.0438 (M+H).

Example 173. 4-[((2,6-Dichlorophenyl)carbonyl]amino]-N-[(2-methylsulfonylphenyl)carbonyl]-L-phenylalanine methyl ester was prepared in 99% yield from 4-[[(2,6-dichlorophenyl)carbonyl]amino]-L-phenylalanine methyl ester and 2-methylsulfonylbenzoic acid using the general procedure described in example 3. LR MS: 548 (M+).

Example 174. 4-[[(2,6-Dichlorophenyl)carbonyl]amino]-N-[(2-(1-methyl)ethyl-6-methylphenyl)carbonyl]-L-phenylalanine methyl ester was prepared in 35% yield from 4-[[(2,6-dichlorophenyl)carbonyl]amino]-L-phenylalanine methyl ester and 2-(1-methyl)ethyl-6-methylbenzoic acid using the general procedure described in example 3. HR MS: Obs. mass, 526.1417. Calcd. mass, 526.1426 (M+).

Example 175. 4-[[(2,6-Dichlorophenyl)carbonyl]amino]-N-[(2-bromo-6-methylphenyl)carbonyl]-L-phenylalanine methyl ester was prepared in 64% yield from 4-[[(2,6-dichlorophenyl)carbonyl]amino]-L-phenylalanine methyl ester and 2-bromo-6-methylbenzoic acid using the general procedure described in example 3. HR MS: Obs. mass, 563.0138. Calcd. mass, 563.0140 (M+H).

Example 176. 4-[[(2,6-Dichlorophenyl)carbonyl]amino]-N-[(2-ethyl-6-methylphenyl)carbonyl]-L-phenylalanine methyl ester was prepared in 46% yield from 4-[[(2,6-dichlorophenyl)carbonyl]amino]-L-phenylalanine methyl ester and 2-ethyl-6-methylbenzoic acid using the general procedure described in example 3. HR MS: Obs. mass, 513.1359. Calcd. mass, 513.1348 (M+H).

Example 177. N-[(2,6-Dichlorophenyl)carbonyl]-4-[(2,4-dimethyl-3-pyridinyl)carbonyl]amino]-L-phenylalanine was prepared from 4-[(2,4-dimethyl-3-pyridyl)carbonyl]amino]-L-phenylalanine methyl ester hydrochloride and 2,6-dichlorobenzoic acid using the general method described in example 107. MS (M+H) 486 (2Cl).

Example 178. Preparation of 4-[[(2,6-dichlorophenyl)carbonyl]amino]-N-[(2-chloro-6-methylphenyl)carbonyl]-L-phenylalanine sodium salt.

A suspension of 4-[[(2,6-dichlorophenyl)carbonyl]amino]-N-[(2-chloro-6-methylphenyl)carbonyl]-L-phenylalanine (127.13 mmol, 64.3 g) in water (500 mL) was titrated with aqueous 1.0 N sodium hydroxide (120 mL) at room temperature until the pH of the solution became neutral. In order to effect complete dissolution, the mixture was warmed to 40-45 °Cduring the course of the titration. Some of the water was removed to a approximate volume of 300-350 mL under vacuum and the clear solution was lyopholized under high vaccum for 2 days to obtain 67 g (100%) as a white amorphous solid. Anal. (C24H18ClO3NaO4•0.70 H2O): Calcd. C, 54.62; H, 3.44; N, 5.31; Cl, 20.15; Na, 4.36; H2O, 2.33. Fd: C, 54.37; H, 3.49; N, 5.18; Cl, 20.11; Na, 4.25; H2O, 2.54.

Example 179. VLA-4 / VCAM-1 Screening Assay

VLA-4 antagonist activity, defined as ability to compete for binding to immobilized VCAM-1, was quantitated using a solid-phase, dual antibody ELISA. VLA-4 (a4b1 integrin) bound to VCAM-1 is detected by a complex of anti-integrin b1 antibody: HRP-conjugated anti-mouse IgG: chromogenic substrate (K-Blue). Initially, this entailed coating 96 well plates (Nunc Maxisorp) with recombinant human VCAM-1 (0.4 µg in 100 µl PBS), sealing each plate and then allowing the plates to stand at 4°C for Å18 hr. The VCAM-coated plates were subsequently blocked with 250 µl of 1% BSA/0.02% NaN₃ to reduce non-specific binding. On the day of assay, all plates are washed twice with VCAM Assay Buffer (200 µl/well of 50 mM Tris-HCl, 100 mM NaCl, 1 mM MnCl₂, 0.05% Tween 20; pH 7.4). Test compounds are dissolved in 100% DMSO and then diluted 1:20 in VCAM Assay Buffer supplemented with 1mg/mL BSA (i.e., final DMSO = 5%). A series of 1:4 dilutions are performed to achieve a concentration range of 0.005 nM - 1.563 µM for each test compound. 100 µl per well of each dilution is added to the VCAM-coated plates, followed by 10 µl of Ramos cell-derived VLA-4. These plates are sequentially mixed on a platform shaker for 1 min, incubated for 2 hr at 37 °C, and then washed four times with 200 µl/well VCAM Assay Buffer. 100 µl of mouse anti-human integrin b1 antibody is added to each well (0.6 µg/mL in VCAM Assay Buffer + 1mg/mL BSA) and allowed to incubate for 1 hr at 37°C. At the conclusion of this incubation period, all plates are washed four times with VCAM Assay Buffer (200 µl/well). A corresponding second antibody, HRP-conjugated goat anti-mouse IgG (100 µl per well @ 1:800 dilution in VCAM Assay Buffer + 1mg/mL BSA), is then added to each well, followed by a 1 hr incubation at room temperature and concluded by three washes (200µl/well) with VCAM Assay Buffer. Color development is initiated by addition of 100 µl K-Blue per well (15 min incubation, room temp) and terminated by addition of 100 µl Red Stop Buffer per well. All plates are then read in a UV/Vis spectrophotometer at 650 nM. Results are calculated as % inhibition of total binding (i.e., VLA-4 + VCAM-1 in the absence of test compound). Selected data for compounds of this invention are shown in the table below:

| Example | ELISA IC₅₀ nM |
|---|---|
| 13 | 0.33 |
| 15 | 5.9 |
| 16 | 0.44 |
| 17 | 1.85 |
| 18 | 11 |
| 19 | 1.87 |
| 20 | 2.2 |
| 21 | 1.4 |
| 22 | 1.6 |
| 23 | 0.48 |
| 24 | 0.25 |
| 25 | 0.42 |
| 26 | 8.6 |
| 27 | 1.9 |
| 28 | 3.3 |
| 30 | 2.0 |
| 30 | 1.6 |
| 31 | 0.51 |
| 90 | 1.2 |
| 91 | 0.20 |
| 92 | 0.42 |
| 93 | 1.6 |
| 94 | 0.25 |
| 95 | 0.46 |
| 96 | 0.47 |
| 97 | 0.44 |
| 98 | 2.35 |
| 99 | 0.58 |
| 100 | 10 |
| 101 | 9.9 |
| 102 | 41 |
| 107 | 0.79 |
| 108 | 0.63 |
| 114 | 1.14 |
| 115 | 4.5 |
| 120 | 4.5 |
| 121 | 5.8 |
| 122 | 0.67 |
| 123 | 1.7 |
| 124 | 0.63 |
| 125 | 1.7 |

### Example 180. Ramos (VLA-4) / VCAM-1 Cell-Based Screening Assay Protocol

### Materials:

Soluble recombinant human VCAM-1 (mixture of 5- and 7-Ig domain) was purified from CHO cell culture media by immunoaffinity chromatography and maintained in a solution containing 0.1 M Tris-glycine (pH 7.5), 0.1 M NaCl, 5 mM EDTA, 1 mM PMSF, 0.02% 0.02% NaN₃ and 10 µg/mL leupeptin. Calcein-AM was purchased from Molecular Probes Inc.

### Methods:

VLA-4 (a4b1 integrin) antagonist activity, defined as ability to compete with cell-surface VLA-4 for binding to immobilized VCAM-1, was quantitated using a Ramos-VCAM-1 cell adhesion assay. Ramos cells bearing cell-surface VLA-4, were labeled with a fluorescent dye (Calcein-AM) and allowed to bind VCAM-1 in the presence or absence of test compounds. A reduction in fluorescence intensity associated with adherent cells (% inhibition) reflected competitive inhibition of VIA-4 mediated cell adhesion by the test compound.

Initially, this entailed coating 96 well plates (Nunc Maxisorp) with recombinant human VCAM-1 (100 ng in 100 µl PBS), sealing each plate and allowing the plates to stand at 4°C for Å18 hr. The VCAM-coated plates were subsequently washed twice with 0.05% Tween-20 in PBS, and then blocked for 1hr (room temperature) with 200 µl of Blocking Buffer (1% BSA/0.02% thimerosal) to reduce non-specific binding. Following the incubation with Blocking Buffer, plates were inverted, blotted and the remaining buffer aspirated. Each plate was then washed with 300 µl PBS, inverted and the remaining PBS aspirated.

Test compounds were dissolved in 100% DMSO and then diluted 1:25 in VCAM Cell Adhesion Assay Buffer (4 mM CaCl₂, 4 mM MgCl₂ in 50 mM TRIS-HCl, pH 7.5) (final DMSO = 4%). A series of eight 1:4 dilutions were performed for each compound (general concentration range of 1 nM - 12,500 nM). 100 µl/well of each dilution was added to the VCAM-coated plates, followed by 100 µl of Ramos cells (200,000 cells/well in 1% BSA/PBS). Plates containing test compounds and Ramos cells were allowed to incubate for 45 min at room temperature, after which 165 µl/well PBS was added. Plates were inverted to remove non-adherent cells, blotted and 300 µl/well PBS added. Plates were again inverted, blotted and the remaining buffer gently aspirated. 100 µl Lysis Buffer (0.1% SDS in 50 mM TRIS-HCl, pH 8.5) was added to each well and agitated for 2 min on a rotary shaking platform. The plates were then read for fluorescence intensity on a Cytofluor 2300 (Millipore) fluorecence measurement system (excitation = 485 nm, emission = 530 nm). The results are shown in the following table:

**Table**

| Example | Ramos IC50 nM |
|---|---|
| 13 | 15 |
| 15 | 2,600 |
| 16 | 85 |
| 19 | 351 |
| 20 | 1,630 |
| 21 | 1,270 |
| 22 | 1,320 |
| 23 | 316 |
| 24 | 20 |
| 25 | 103 |
| 90 | 23 |
| 91 | 9.3 |
| 92 | 255 |
| 93 | 49 |
| 94 | 9.5 |
| 95 | 33 |
| 107 | 20 |
| 108 | 22 |
| 115 | 678 |
| 120 | 439 |
| 121 | 515 |
| 122 | 430 |
| 123 | 316 |
| 124 | 985 |
| 150 | 47 |
| 152 | 967 |
| 153 | 975 |
| 154 | 2,474 |
| 155 | 644 |
| 156 | 114 |
| 158 | 946 |
| 159 | 988 |
| 169 | 30 |
| 170 | 33.5 |
| 171 | 13.5 |

### Example 181. Oral Dosage Form

| **Item** | **Ingredients** | **mg/tablet** | | | |
|---|---|---|---|---|---|
| 1 | Compound of invention | 25 | 100 | 250 | 500 |
| 2 | Anhydrous lactose | 83 | 35 | 19 | 38 |
| 3 | Croscarmellose sodium | 6 | 8 | 16 | 32 |
| 4 | Povidone K30 | 5 | 6 | 12 | 24 |
| 5 | Magnesium stearate | 1 | 1 | 3 | 6 |
| | | | | | |
| | Total weight (mg) | 120 | 150 | 300 | 600 |

### Manufacturing procedure:

1. Mix items 1,2 ,3 in a suitable mixer for 15 minutes.
2. Granulate the powder mix from step 1 with 20% PVP K30 solution.
3. Dry the granulation in step 2 at 50° C.
4 Pass the granulation from step 3 through a suitable milling equipment.
5. Add the item 5 to the milled granulation from Step 4 and mix for 3 minutes.
6. Compress the granulation from Step 5 on a suitable press.

Example 182. Aerosol Administration Formulation

| **Ingrdients** | **Qty/mL** |
|---|---|
| Compound of invention | 3-150 mg* |
| Sodium chloride | 8.0 mg |
| Phophate buffer (20 mM) pH 7.0* q.s. | 1.0 mL |

| | |
|---|---|
| * Depending upon activity of the compound | |

pH can be adjusted with Sodium hydroxide solution (1 N) or HCl solution (10%w/v)

### Procedure:

1. Dissolve the drug substance in the buffer.
2. Filter the solution through a 0.22 micron filter.

The particle size distribution after nebulizing the above solution (as measured using Malvern Mastersizer X) is in the range of 1-6 microns.

## Claims

1. A compound of the formula: 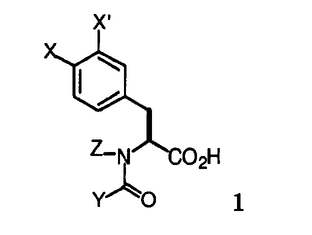 wherein:
one of X and X' is hydrogen, halogen, or C₁₋₆ alkyl, the other is a group of the formula: 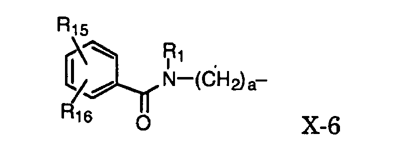
wherein:
R₁ is hydrogen or C₁₋₆ alkyl,
R₁₅ is hydrogen, halogen, nitro, C₁₋₆ alkyl sulfonyl, cyano, C₁₋₆ alkyl, OH, C₁₋₆ alkoxy, C₁₋₆ alkoxycarbonyl, carboxy, C₁₋₆ alkyl aminosulfonyl, perfluoro C₁₋₆ alkyl, C₁₋₆ alkylthio, hydroxy C₁₋₆ alkyl, alkoxy C₁₋₆ alkyl, halo C₁₋₆ alkyl, alkylthio C₁₋₆ alkyl, alkylsulfinyl C₁₋₆ alkyl, alkylsufonyl C₁₋₆ alkyl, C₁₋₆ alkylsulfinyl, C₁₋₆ alkylcarbonyl, aroyl, aryl, aryloxy or a group of the formula R₁₇-C≡C-,
R₁₆ is hydrogen, halogen, nitro, cyano, C₁₋₆ alkyl, OH, perfluoro C₁₋₆ alkyl, aryloxy or C₁₋₆ alkylthio,
R₁₇ is hydrogen, aryl, heteroaryl, or C₁₋₆ alkyl which is unsubstituted or substituted by OH, aryl, or heteroaryl, and
a is 0 or 1;
or one of X and X' is a group of the formula: 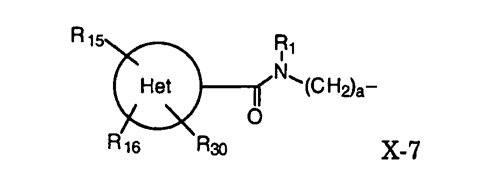
wherein Het is a 5- or 6-membered heteroaromatic ring containing 1, 2 or 3 heteroatoms selected from N,O, and S;
or
Het is a 9- or 10-membered bicyclic heteroaromatic ring containing 1, 2, 3 or 4 heteroatoms selected from O, S, and N,
a, R₁, R₁₅ and R₁₆ are as above, and
R₃₀ is hydrogen or C₁₋₆ alkyl, or is absent;
or one of X and X' is a group of the formula: 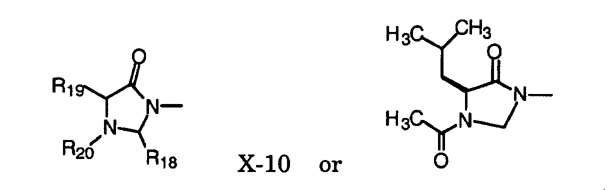 wherein:
R₁₈ is C₁₋₆ alkyl, aryl, heteroaryl, aryl C₁₋₆ alkyl, heteroaryl C₁₋₆ alkyl,
R₁₉ is C₁₋₆ alkyl, which is unsubstituted or substituted by one or more of halogen, hydroxy, C₁₋₆ alkoxy, aryl, heteroaryl, alkylthio, or R₁₉ is aryl or heteroaryl, and
R₂₀ is C₁₋₆ alkyl or C₁₋₆ alkylcarbonyl, or
R₁₉ and R₂₀ taken together are tetramethylene;
and
Y is a group of the formula: 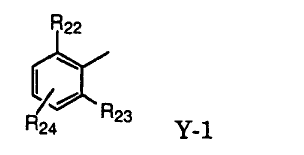
wherein:
R₂₂ and R₂₃ are independently hydrogen, C₁₋₆ alkyl, C₁₋₆ alkoxy, C₁₋₆ alkoxylalkyl, C₁₋₆ alkylamino, aryl, aryl C₁₋₆ alkyl, nitro, cyano, C₁₋₆ alkylthio, C₁₋₆ alkylsulfinyl, C₁₋₆ alkyl sulfonyl, C₁₋₆ alkylcarbonyl, halogen, or perfluoroalkyl and at least one of R₂₂ and R₂₃ is other than hydrogen, and
R₂₄ is hydrogen, hydroxy, C₁₋₆ alkyl, C₁₋₆ alkoxy, amino, aryl, nitro, cyano, C₁₋₆ alkyl sulfonyl, halogen, or is a group of the formula: 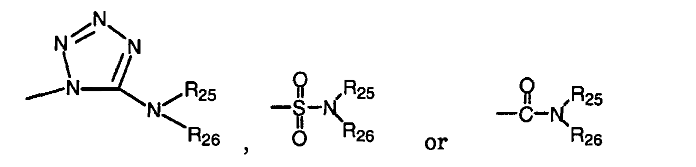
wherein R₂₅ is hydrogen, C₁₋₆ alkyl, aryl, aryl C₁₋₆ alkyl, alkoxy C₁₋₆ alkyl and R₂₆ is hydrogen or C₁₋₆ alkyl, or
R₂₂ and R₂₄ taken together are a fused benzene ring; or
Y is a group Y-2 which is a five or six membered monocyclic heteroaromatic group containing 1, 2 or 3 heteroatoms selected from N, O, and S, or a 9- or 10-membered bicyclic heteroaromatic group containing 1, 2, 3 or 4 heteroatoms selected from O, S, and N, wherein said heteroaromatic group is bonded via a carbon atom to the amide carbonyl and one or two carbon atoms of said heteroaromatic group are substituted by C₁₋₆ alkyl, halogen, cyano, perfluoroalkyl, or aryl and at least one of said substituted carbon atoms is adjacent to the carbon atom bonded to the amide carbonyl;
and Z is hydrogen or C₁₋₆ alkyl;
and the pharmaceutically acceptable salts and esters thereof,
wherein
the term "C₁₋₆ alkyl", alone or in combination, may be unsubstituted or substituted by one or more groups selected independently from cycloalkyl, nitro, aryloxy, aryl, hydroxy, halogen, cyano, C₁₋₆ alkoxy, C₁₋₆ alkylcarbonyl, C₁₋₆ alkylthio, C₁₋₆ alkyl sulfinyl, C₁₋₆ alkyl sulfonyl, and substituted amino;
cycloalkyl refers to an unsubstituted or substituted 3- to 7-membered carbacyclic ring, wherein the substitutents are selected from hydroxy, halogen, cyano, C₁₋₆ alkoxy, C₁₋₆ alkylcarbonyl, C₁₋₆ alkyl, aroyl, C₁₋₆ alkylthio, C₁₋₆ alkyl sulfinyl, C₁₋₆ alkyl sulfonyl, aryl, heteroaryl and substituted amino;
aryl refers to a mono- or bicylic aromatic group which is unsubstituted or substituted by C₁₋₆ alkyl, C₁₋₆ alkoxy, hydroxy C₁₋₆ alkyl, hydroxy, hydroxy alkoxy, halogen, C₁₋₆ alkylthio, C₁₋₆ alkylsulfinyl, C₁₋₆ alkylsulfonyl, cyano, nitro, perfluoroalkyl, alkylcarbonyl, aroyl as defined below, aryl alkynyl, lower alkynyl and C₁₋₆ alkylcarbonylamino;
heteroaryl refers to an unsubstituted or substituted 5- or 6-membered monocyclic hetereoaromatic ring or a 9- or 10-membered bicyclic hetereoaromatic ring containing 1, 2, 3 or 4 hetereoatoms which are independently N, S or O, and the substituents are as defined above for "aryl";
aroyl refers to an mono- or bicyclic aryl as defined above or heteroaryl as defined above bonded via a carbonyl group; and
aryloxy refers to an aryl group, as defined above, which is bonded via an oxygen atom.

2. A compound according to claim 1, wherein Z is hydrogen.

3. A compound according to claim 1 or 2, wherein X' is hydrogen.

4. A compound according to any one of claims 1-3, wherein:
R₂₂ and R₂₃ are independently hydrogen, C₁₋₆ alkyl, nitro, C₁₋₆ alkylthio, C₁₋₆ alkoxy, C₁₋₆ alkylamino, C₁₋₆ alkylsulfinyl, C₁₋₆ alkyl sulfonyl, C₁₋₆ alkylcarbonyl, halogen, or perfluoroalkyl wherein at least one of R₂₂ and R₂₃ is not hydrogen, and
R₂₄ is hydrogen, hydroxy, C₁₋₆ alkyl, C₁₋₆ alkoxy, C₁₋₆ alkylsulfonyl, amino, nitro, halogen or a group of the formula: 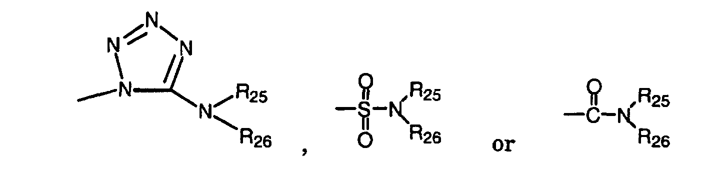
wherein R₂₅ is aryl C₁₋₆ alkyl and R₂₆ is hydrogen or C₁₋₆ alkyl, or R₂₂ and R₂₄ taken together are a fused benzene ring.

5. A compound according to claim 4, wherein R₂₂ is hydrogen (when R₂₃ is other than hydrogen), C₁₋₆ alkyl or halogen.

6. A compound according to claim 4 or claim 5, wherein R₂₄ is hydrogen, hydroxy, amino, C₁₋₆ alkylsulfonyl, C₁₋₆ alkyl, halogen, nitro or C₁₋₆ alkoxy or a group of the formula: 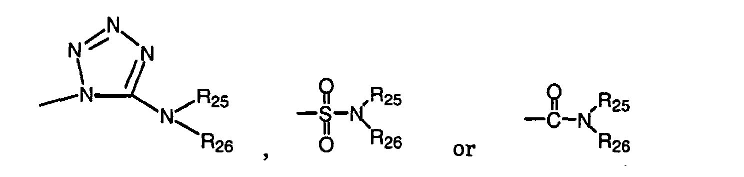 wherein R₂₅ is unsubstituted or hydroxy-substituted phenyl C₁₋₆ alkyl, and R₂₆ is hydrogen,
or R₂₂ and R₂₄ taken together are a fused phenyl ring.

7. A compound according to claim 6, wherein R₂₄ is hydrogen, hydroxy, amino, methyl, chloro, bromo, nitro, -OCH_{3,} SO₂CH₃ and R₂₆ is H and R₂₅ is

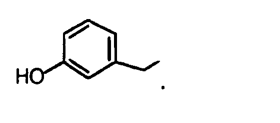

8. A compound according to any one of claims 4-7, wherein R₂₃ is hydrogen (when R₂₂ is other than hydrogen), C₁₋₆ alkyl, C₁₋₆ alkylamino, halogen, nitro, perfluoro C₁₋₆ alkyl, C₁₋₆ alkoxy, C₁₋₆ alkylcarbonyl, C₁₋₆ alkylthio, C₁₋₆ alkylsulfinyl or C₁₋₆ alkyl sulfonyl.

9. A compound according to claim 8, wherein R₂₃ is methyl, ethyl, iso-propyl, tertbutyl, trifluormethyl, chloro, bromo, fluoro, nitro, -COCH₃, -SCH₃,-SOCH₃, -SO₂CH₃, -NHCH₃ or -OCH₃.

10. A compound according to any one of claims 4-9, wherein Y-1 is selected from the group consisting of:

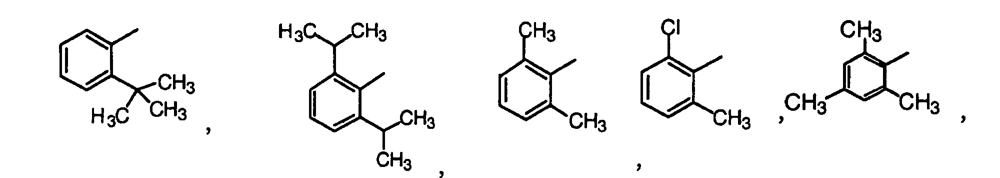

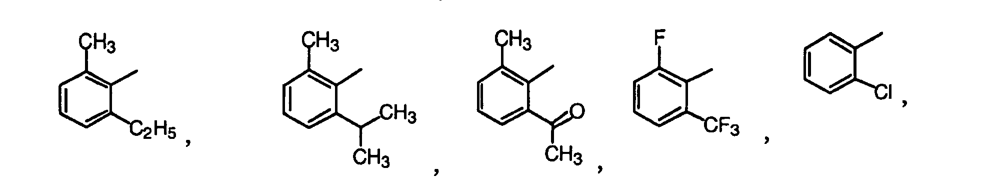

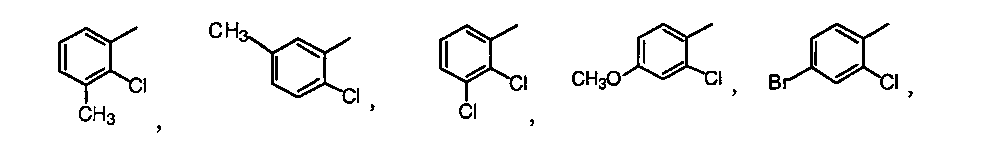

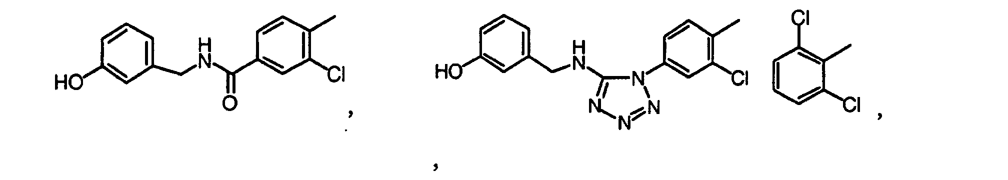

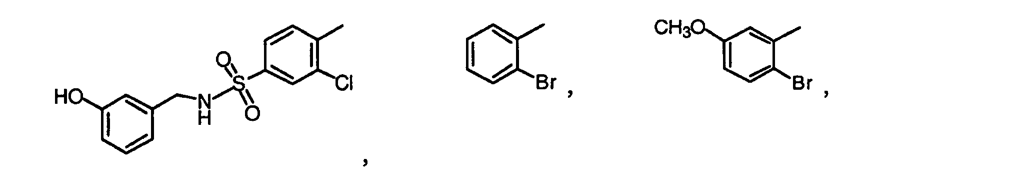

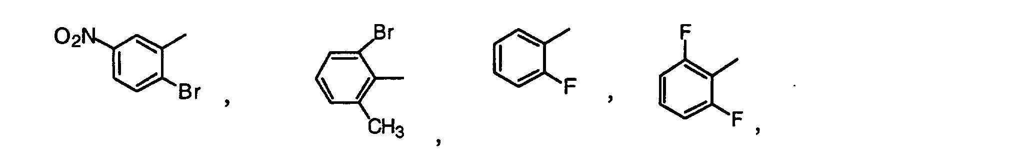

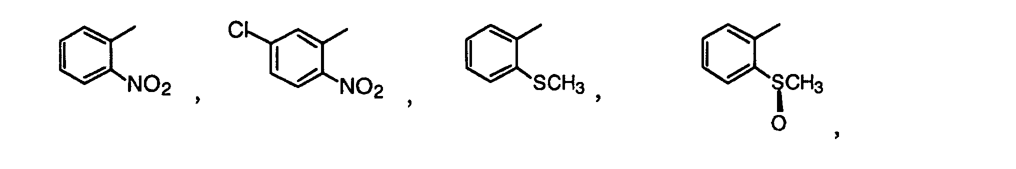

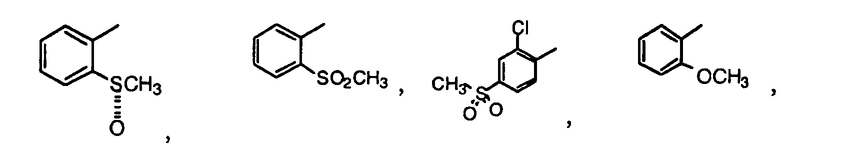

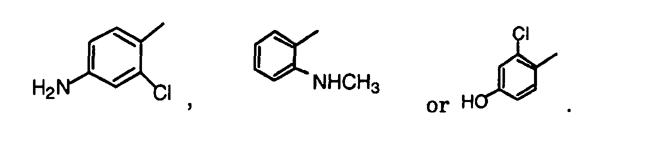

11. A compound according to any one of claims 1-3, wherein in Y-2 the five or six membered monocyclic heteroaromatic or the 9- or 10-membered bicyclic heteroaromatic groups are selected from the group of:

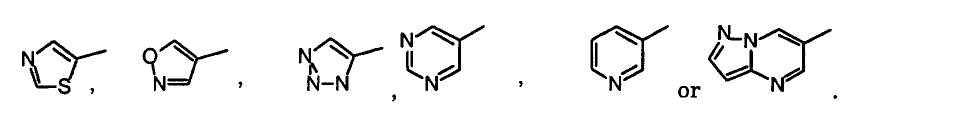

12. A compound according to any one of claims 1-3, wherein groups Y-2 are of the formula:

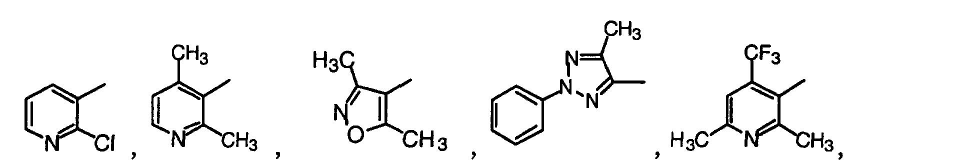

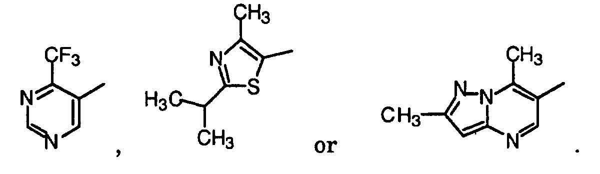

13. A compound according to any one of claims 1-3, wherein in X-6 the groups R₁₅ and R₁₆ independently hydrogen C₁₋₆ alkyl, nitro, halogen, perfluoro C₁₋₆ alkyl, cyano or aryloxy.

14. A compound according to claim 13, wherein R₁₅ or R₁₆ is H, methyl, nitro, chloro, fluoro, trifluormethyl, cyano or phenoxy.

15. A compound according to claim 13 or claim 14, wherein group X-6 is of the formula:

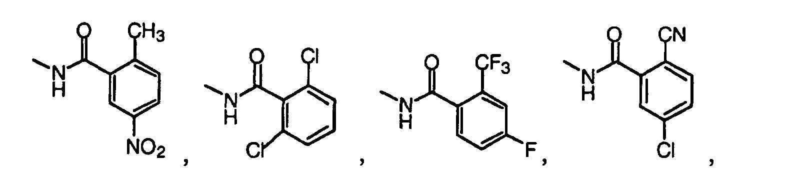

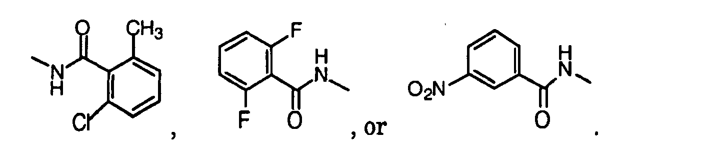

16. A compound according to any one of claims 1-3, wherein in X-7 Het is a 5- or 6-membered monocyclic heteroaromatic ring containing 1, 2 or 3 nitrogens, or a nitrogen and a sulfur, or a nitrogen and an oxygen.

17. A compound according to claim 16, wherein the heteroaromatic ring is

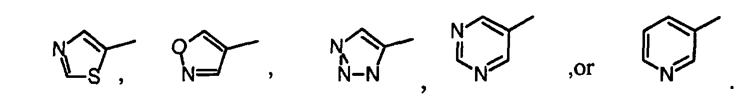

18. A compound according to any one of claims 1-3, wherein in X-7 Het is a bicyclic heteroaromatic ring containing from 1 to 3 nitrogens as the heteroatoms.

19. A compound according to claim 18, wherein the bicyclic heteroaromatic ring is 4-quinolinyl, 1-isoquinolinyl or

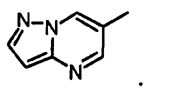

20. A compound according to any one of claims 1-3, wherein in X-7 R₁₅ is hydrogen, nitro, C₁₋₆ alkyl sulfonyl, cyano, C₁₋₆ alkyl, C₁₋₆ alkoxy, perfluoro C₁₋₆ alkyl, C₁₋₆ alkylthio, C₁₋₆.alkylcarbonyl, or aryl.

21. A compound according to claim 20, wherein R₁₅ is isopropyl, methyl or phenyl.

22. A compound according to any one of claims 1-3, wherein R₁₆ in X-7 is hydrogen, halogen, nitro, cyano, C₁₋₆ alkyl or perfluoro C₁₋₆ alkyl.

23. A compound according to claim 22, wherein R₁₆ is methyl or triflouromethyl.

24. A compound according to any one of claims 1-3, wherein R₃₀ in X-7 is hydrogen or C₁₋₆ alkyl.

25. A compound according to any one of claims 1-3, wherein groups X-7 are of the formula:

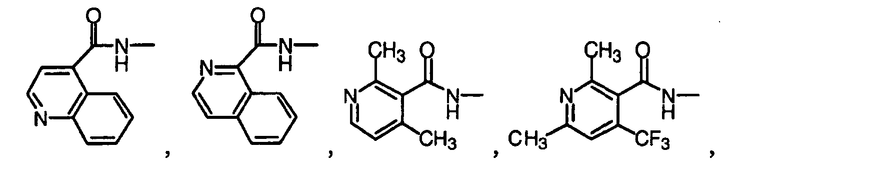

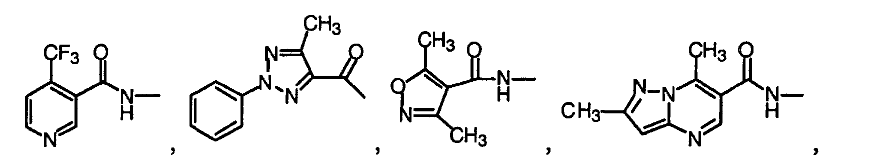

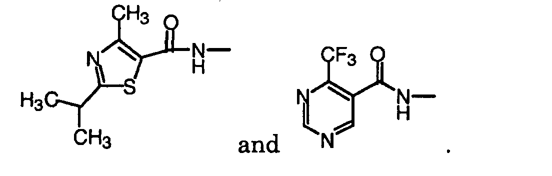

26. A compound according to any one of claims 1-3, wherein in X-6 or X-7 R₁ is hydrogen.

27. A compound according to any one of claims 1-3, wherein in X-6 or X-7 a is 0.

28. A compound according to any one of claims 1-3, wherein in X-10 R₁₈ is C₁₋₆ alkyl or phenyl wherein the phenyl ring is unsubstituted or monosubstituted by halogen, hydroxy or is phenyl C₁₋₆ alkyl.

29. A compound according to claim 28, wherein R₁₈ is tertbutyl, phenyl, hydroxyphenyl, chlorophenyl or phenylethyl.

30. A compound according to any one of claims 1-3, wherein in X-10 R₁₉ is C₁₋₆ alkyl which is unsubstituted or substituted by pyridyl or phenyl wherein the phenyl ring is unsubstituted or monosubstituted by C₁₋₆ alkoxy or halogen.

31. A compound according to claim 30, wherein R₁₉ is methyl, isobutyl, benzyl, 4-chlorobenzyl, 4-methoxybenzyl or 2-pyridylmethyl.

32. A compound according to any one of claims 1-3, wherein in X-10 R₂₀ is C₁₋₆ -alkylcarbonyl.

33. A compound according to claim 32, wherein R₂₀ is acetyl.

34. A compound according to any one of claims 1-3, wherein groups X-10 are of the formula: 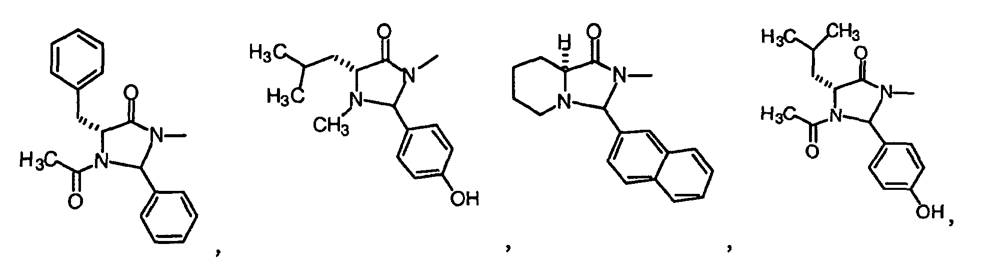 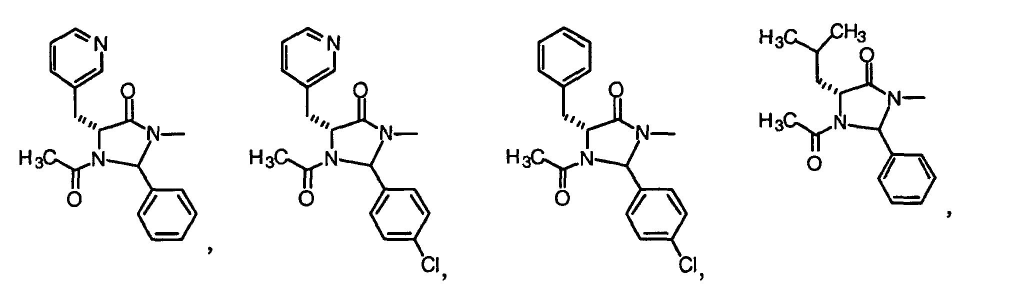 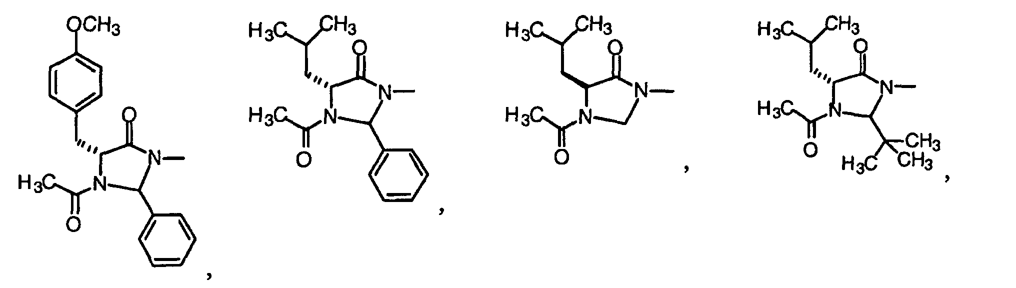 and

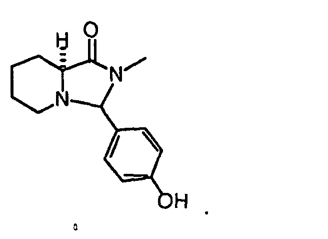

35. A compound selected from the group: 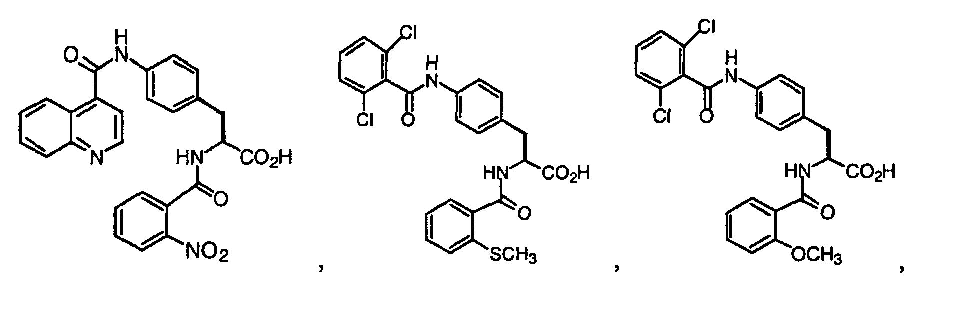 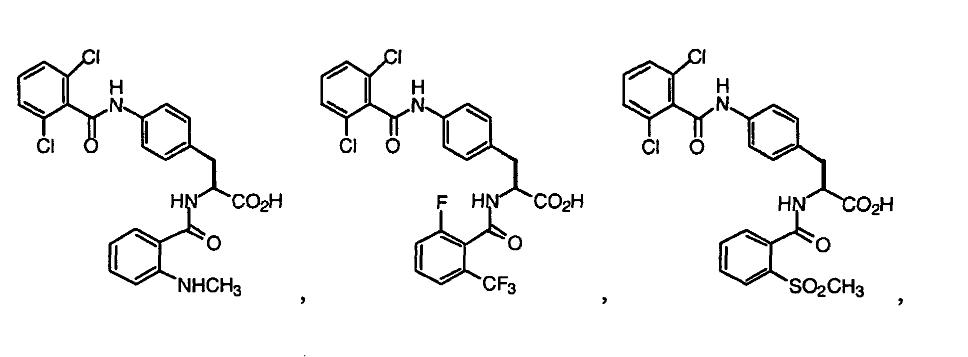 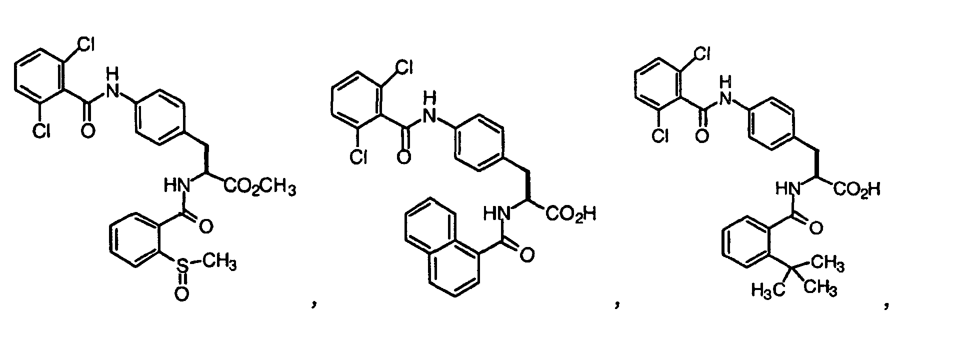 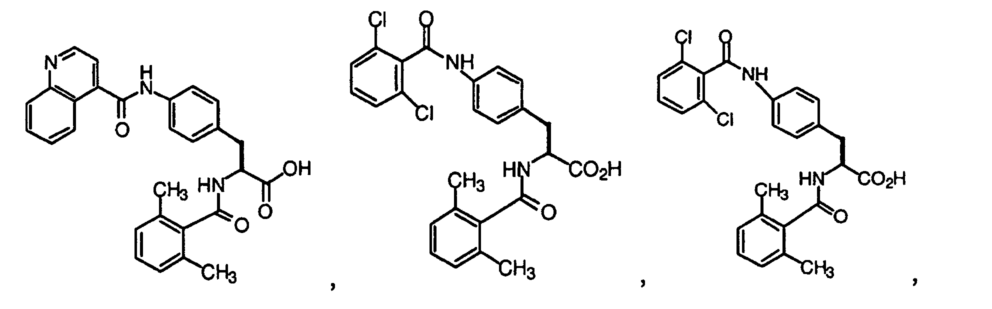 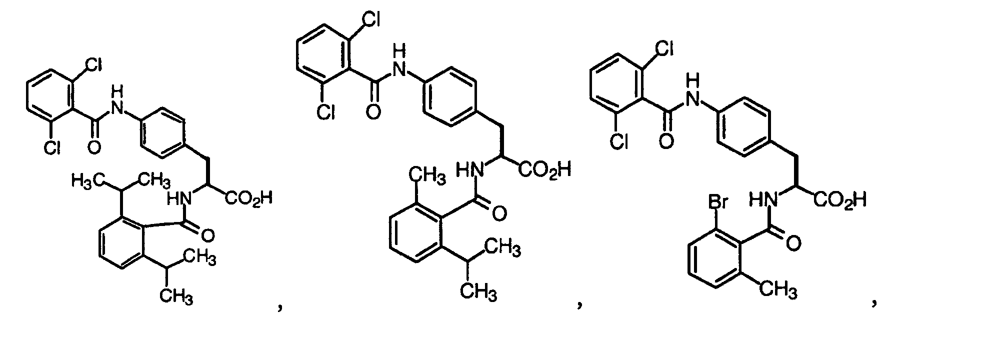 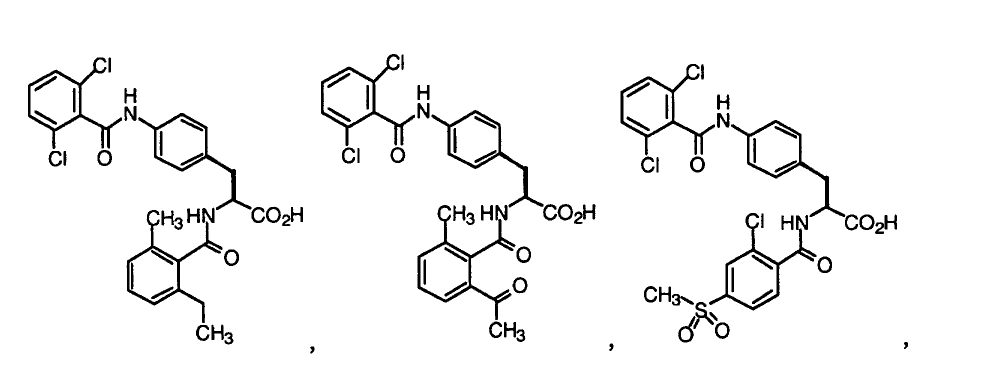 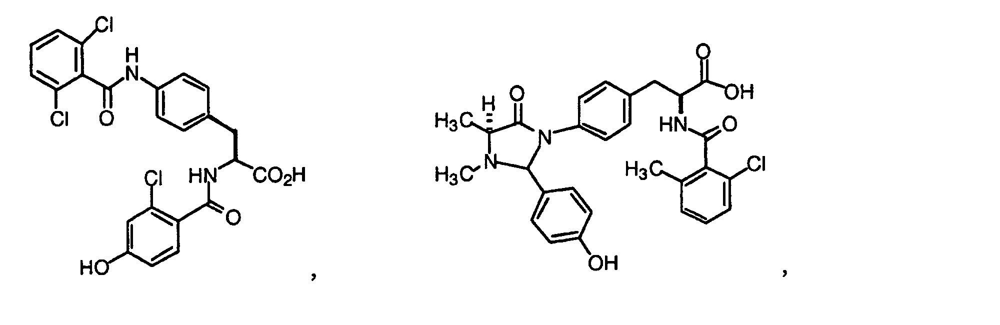 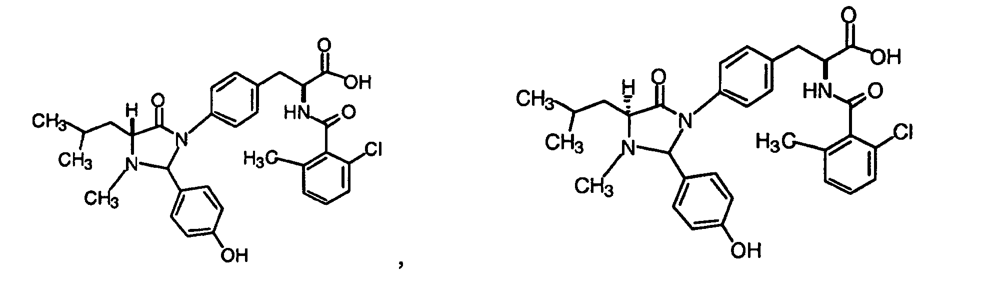 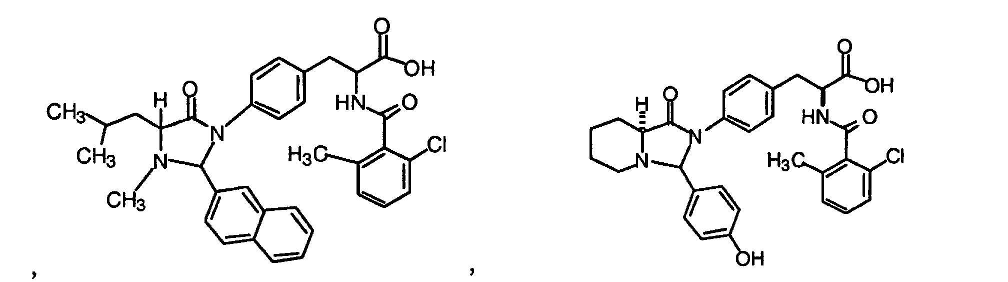 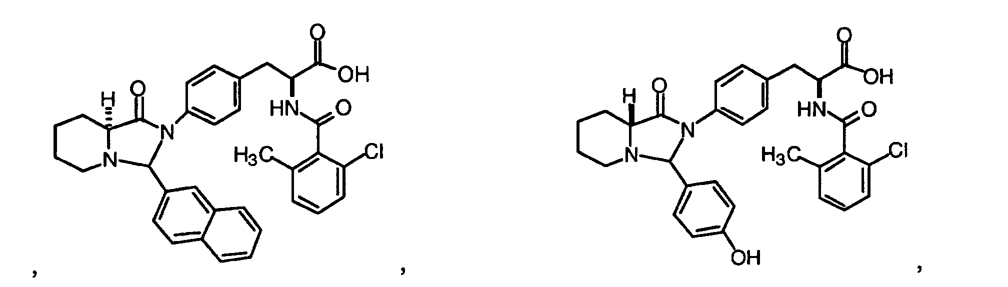 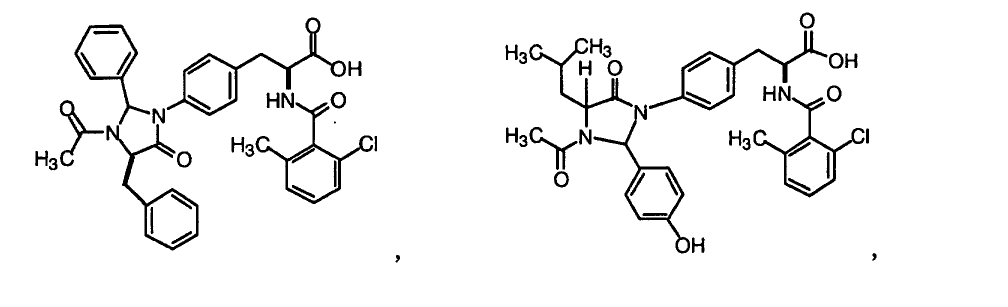 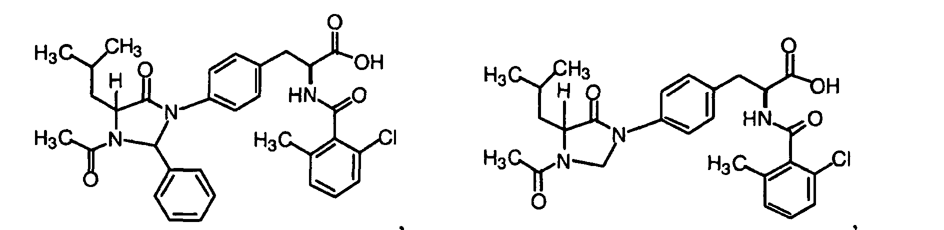 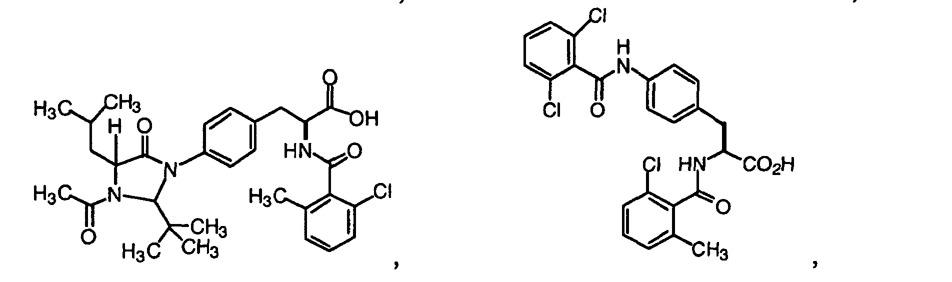 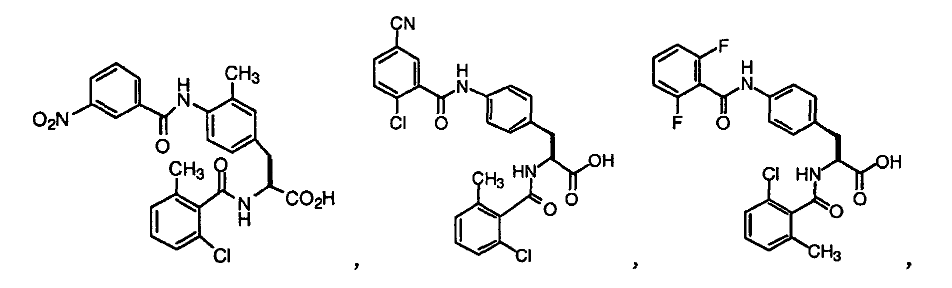 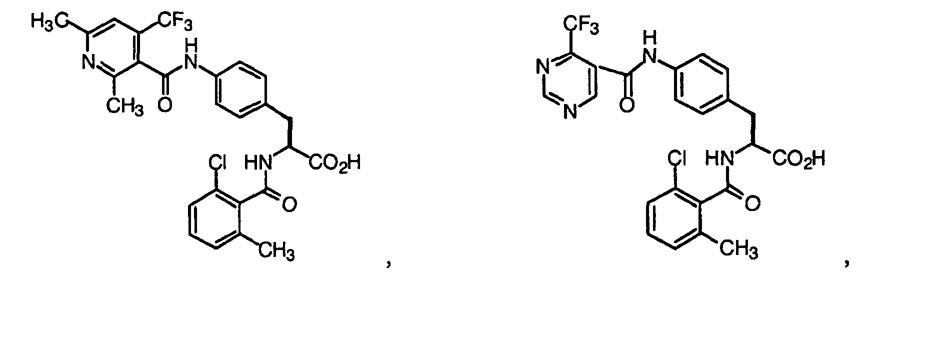 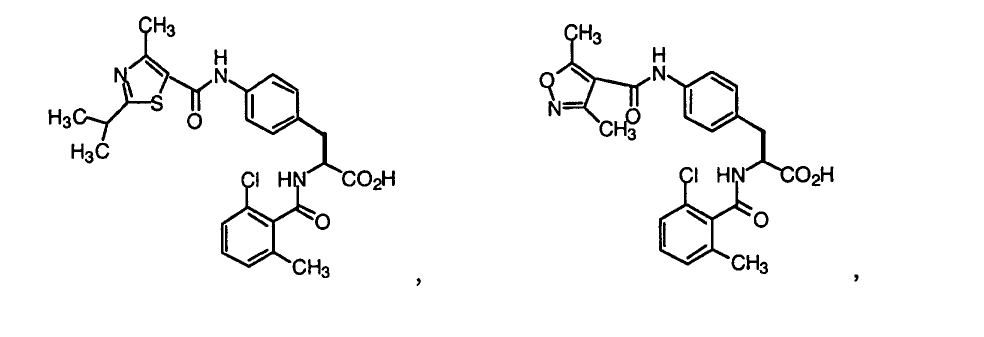 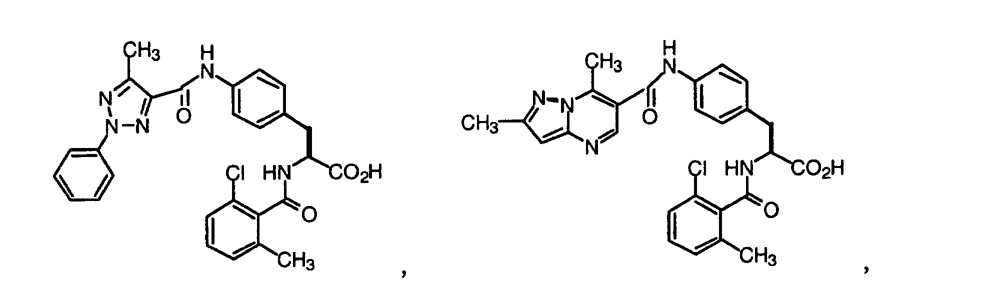 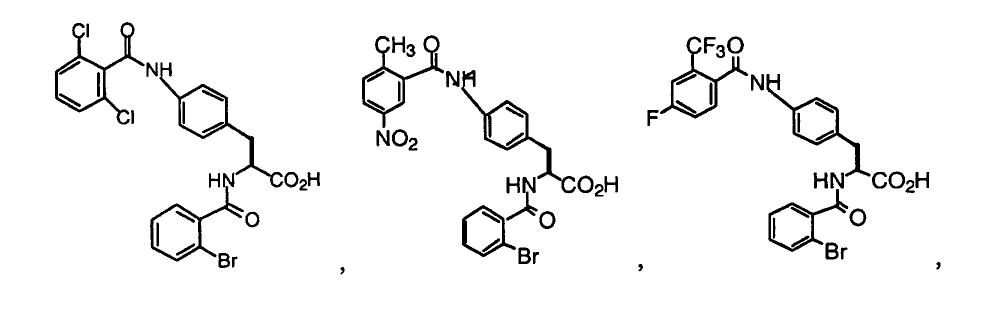 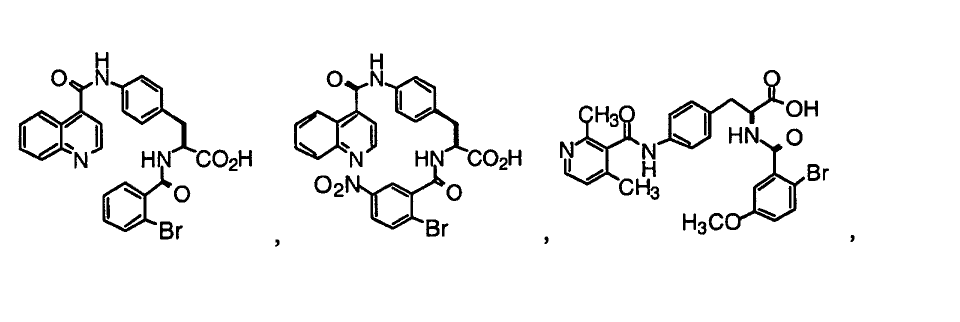 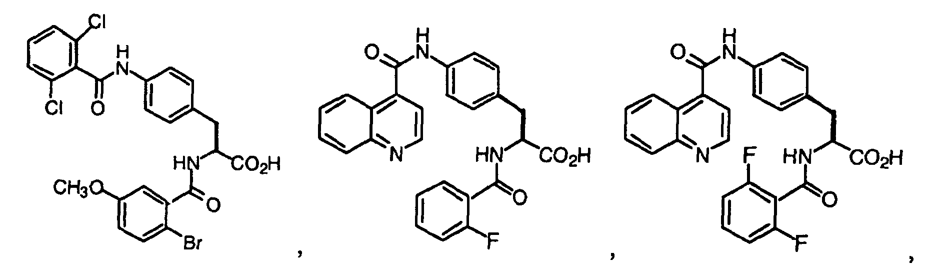 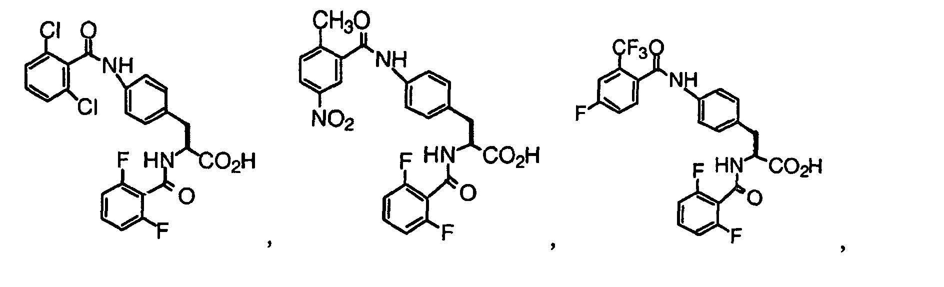 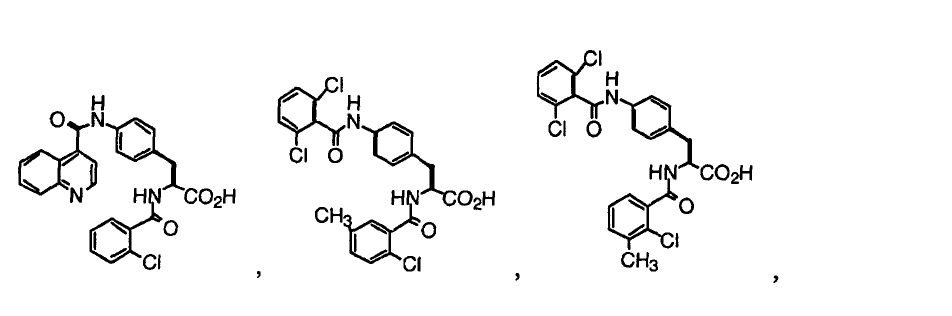 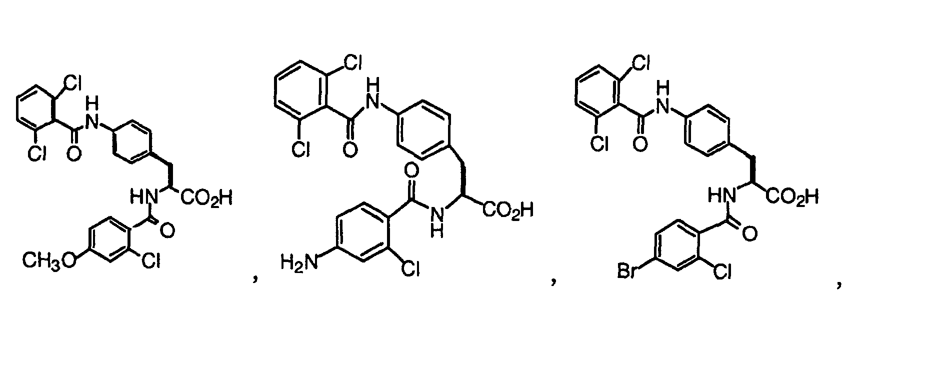 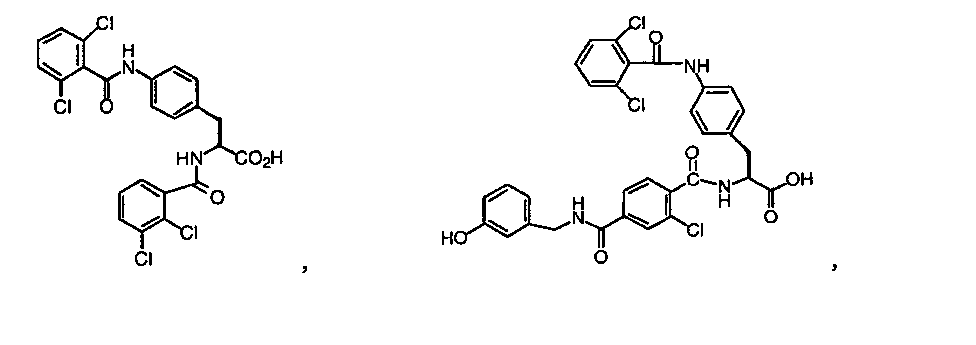 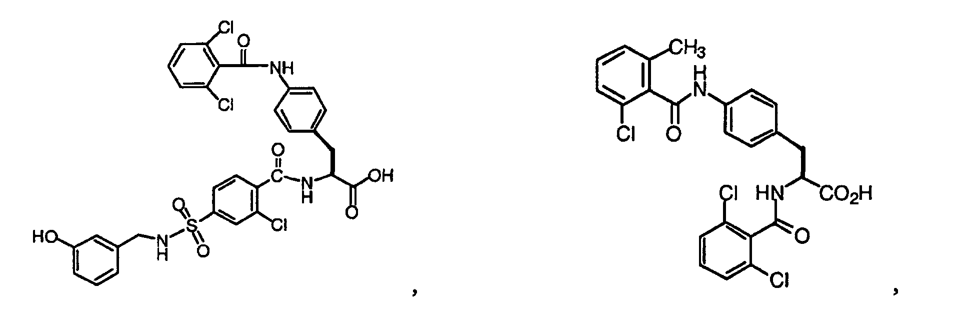 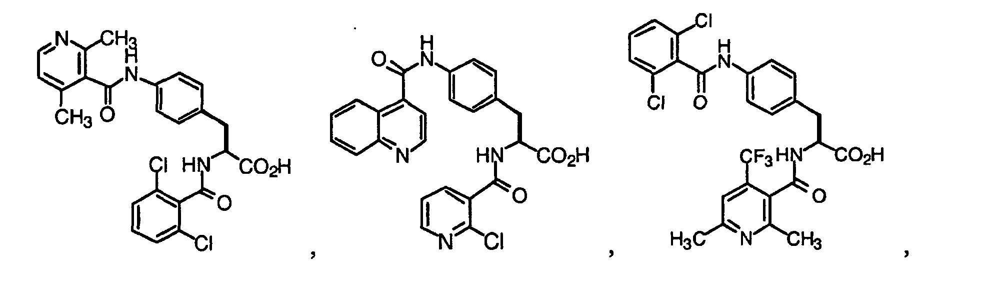 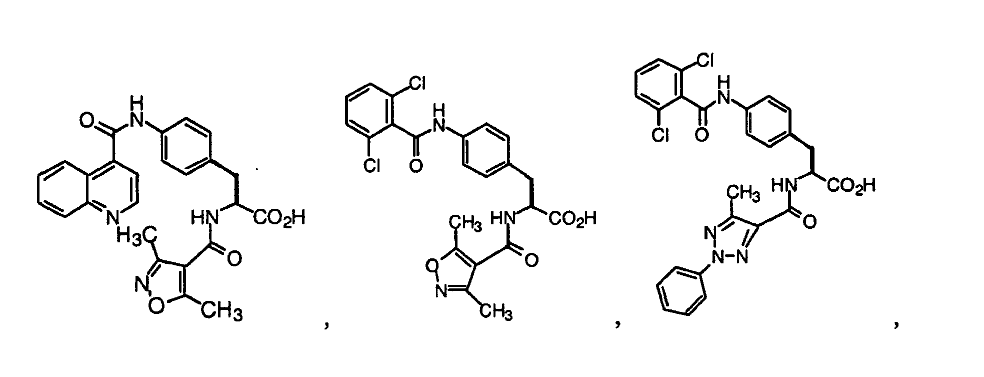 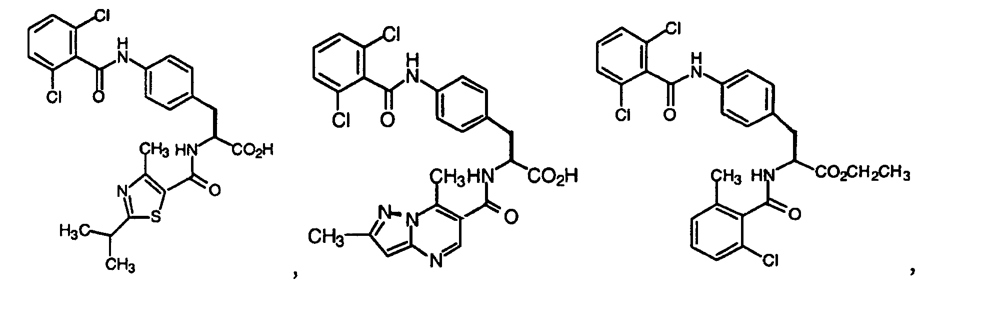 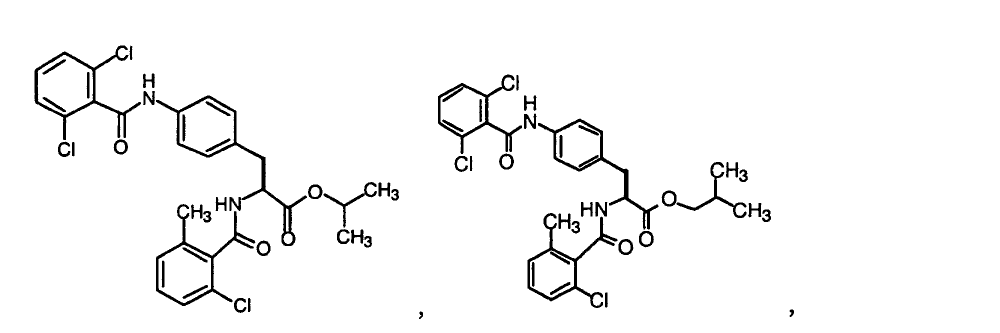 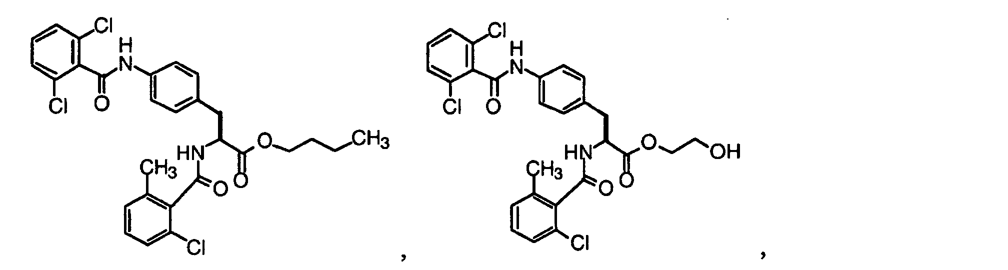 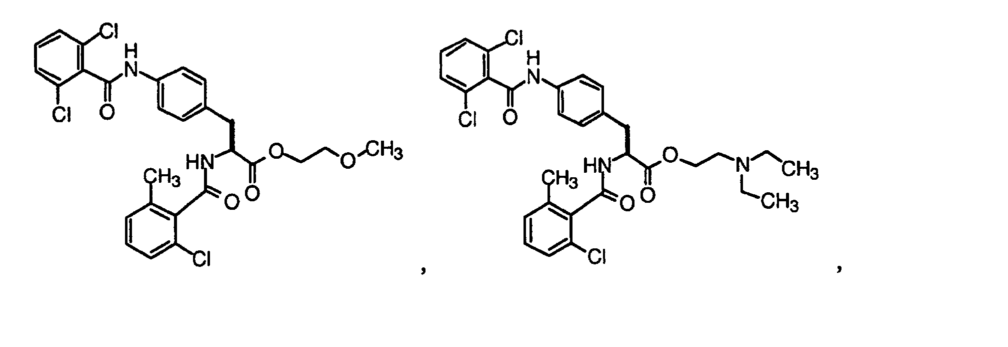 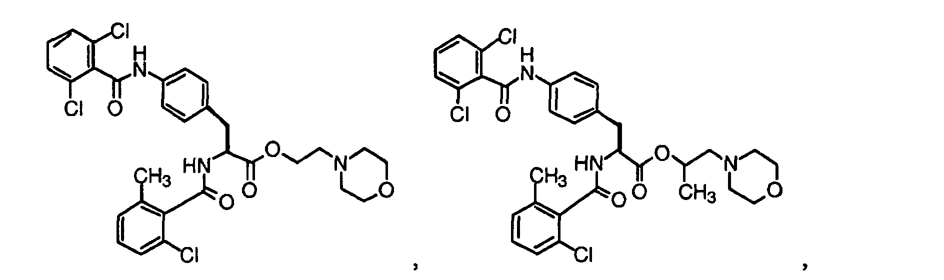 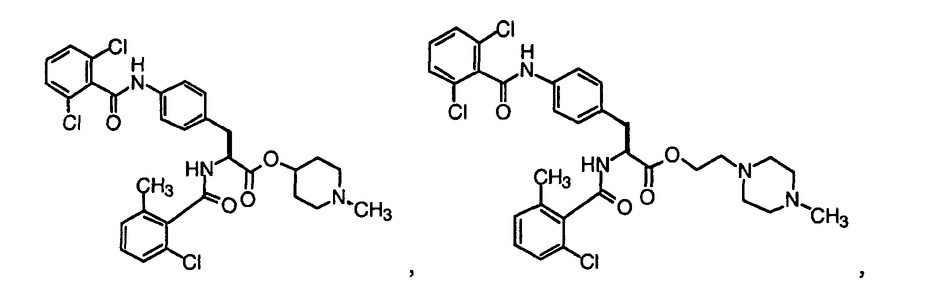 and

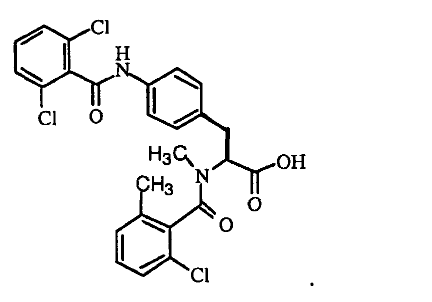

36. A compound according to claim 1 which is N-(2-chloro-6-methylbenzoyl)-4-[[(2,6-dichlorophenyl)carbonyl]amino]-L-phenylalanine or N-(2-chloro-6-methylbenzoyl)-4-[[(2,6-dichlorophenyl)carbonyl]amino]-L-phenylalanine sodium salt.

37. A compound according to claim 1 having the formula: 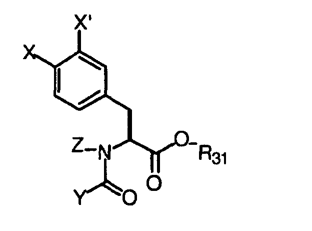 wherein:
X, X', Z and Y are as defined in claim 1,
R₃₁ is C₁₋₆ alkyl; or,
R₃₁ is a group of formula P-1: 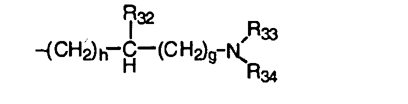 wherein:
R₃₂ is hydrogen or C₁₋₆ alkyl,
R₃₃ is hydrogen, C₁₋₆ alkyl or aryl,
R₃₄ is hydrogen or C₁₋₆ alkyl,
h is an integer from 0 to 2,
g is an integer from 0 to 2, and
the sum of h and g is 1 to 3; or
R₃₁ is a group of formula P-2: 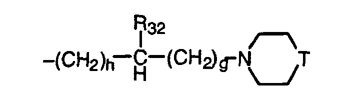 wherein:
R₃₂, g and h are as defined above,
T is O, S, -(CH2)ⱼ-, a bond (when j=0) or a group of the formula N-R₃₅,
R₃₅ is hydrogen, C₁₋₆ alkyl, C₁₋₆ alkylcarbonyl, C₁₋₆ alkoxycarbonyl, and
j is 0, 1 or 2.

38. A compound according to claim 37, wherein R₃₁ is C₁₋₆ alkyl.

39. A compound according to claim 38, which is
N-(2-chloro-6-methylbenzoyl)-4-[[(2,6-dichlorophenyl)carbonyl]amino]-L-phenylalanine methyl ester, or
N-(2-chloro-6-methylbenzoyl)-4-[[(2,6-dichlorophenyl)carbonyl]amino]-L-phenylalanine ethyl ester, or
N-(2-chloro-6-methylbenzoyl)-4-[[(2,6-dichlorophenyl)carbonyl]amino]-L-phenylalanine butyl ester, or
N-(2-chloro-6-methylbenzoyl)-4-[[(2,6-dichlorophenyl)carbonyl]amino]-L-phenylalanine 1-methylethyl ester, or
N-(2-chloro-6-methylbenzoyl)-4-[[(2,6-dichlorophenyl)carbonyl]amino]-L-phenylalanine 2-methylpropyl ester.

40. A compound according to claim 1, which is
N-(2-chloro-6-methylbenzoyl)-4-[[(2,6-dichlorophenyl)carbonyl]amino]-L-phenylalanine 2-methoxyethyl ester or
N-(2-chloro-6-methylbenzoyl)-4-[[(2,6-dichlorophenyl)carbonyl]amino]-L-phenylalanine 2-hydroxyethyl ester.

41. A compound according to claim 37, wherein R₃₁ is P-2.

42. A compound according to claim 41, wherein R₃₁ is 2-(4-morpholinyl)-ethyl, 1-methyl-2-(4-morpholinyl)ethyl, 1-methyl-4-piperidinyl, 2-(1-piperazinyl)ethyl or 2-(4-methyl-1-piperazinyl)ethyl.

43. A compound according to claim 42, which is
N-(2-chloro-6-methylbenzoyl)-4-[[(2,6-dichlorophenyl)carbonyl]amino]-L-phenylalanine 2-morpholinoethyl ester, or
N-(2-chloro-6-methylbenzoyl)-4-[[(2,6-dichlorophenyl)carbonyl]amino]-L-phenylalanine 1-methyl-2-morpholinoethyl ester, or
N-(2-chloro-6-methylbenzoyl)-4-[[(2,6-dichlorophenyl)carbonyl]amino]-L-phenylalanine 1-methyl-4-piperidinyl ester, or
N-(2-chloro-6-methylbenzoyl)-4-[[(2,6-dichlorophenyl)carbonyl]amino]-L-phenylalanine 2-(1-piperazinyl)ethyl ester, or N-(2-chloro-6-methylbenzoyl)-4-[[(2,6-dichlorophenyl)carbonyl]amino]-L-phenylalanine 2-(4-methyl-1-piperazinyl)ethyl ester.

44. A compound according to claim 37, wherein R₃₁ is P-1.

45. A compound according to claim 44, wherein R₃₁ is diethylaminoethyl.

46. A compound according to claim 45 having the formula 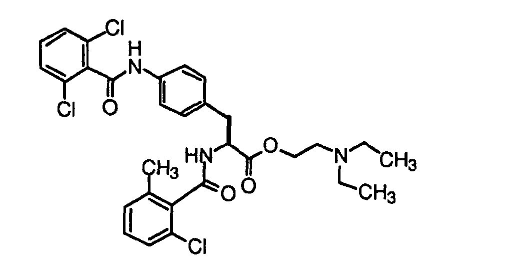 which is N-(2-chloro-6-methylbenzoyl)-4-[[(2,6-dichlorophenyl)carbonyl]amino]-L-phenylalanine 2-diethylaminoethyl ester.

47. Compounds according to any one of the claims 1-46 for use as a medicament.

48. A medicament containing a compound according to any one of claims 1-46 and a therapeutically inert carrier material..

49. A process for the production of a medicament, especially for the treatment of rheumatoid arthritis, multiple sclerosis, inflammatory bowel disease or asthma, which process comprises bringing a compound according to any one of claims 1-46 into a galenical administration form together with a therapeutically inert carrier material and, if desired, one or more additional therapeutically active substances.

50. Use of a compound according to any one of claims 1-46 in the preparation of a medicament for the treatment of rheumatoid arthritis, multiple sclerosis, inflammatory bowel disease or asthma.

## Patentansprüche

1. Verbindung der Formel: 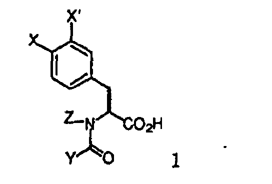 worin:
einer von X und X' Wasserstoff, Halogen oder C₁₋₆-Alkyl darstellt, der andere eine Gruppe der Formel: 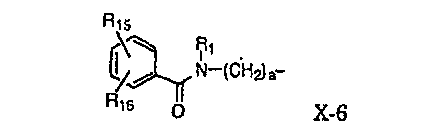
darstellt, worin:
R₁ Wasserstoff oder C₁₋₆-Alkyl darstellt,
R₁₅ Wasserstoff, Halogen, Nitro, C₁₋₆-Alkylsulfonyl, Cyano, C₁₋₆-Alkyl, OH, C₁₋₆-Alkoxy, C₁₋₆-Alkoxycarbonyl, Carboxy, C₁₋₆-Alkylaminosulfonyl, Perfluor-C₁₋₆-alkyl, C₁₋₆-Alkylthio, Hydroxy-C₁₋₆-alkyl, Alkoxy-C₁₋₆-alkyl, Halogen-C₁₋₆-alkyl, Alkylthio-C₁₋₆-alkyl, Alkylsulfinyl-C₁₋₆-alkyl, Alkylsulfonyl-C₁₋₆-alkyl, C₁₋₆-Alkylsulfinyl, C₁₋₆-Alkylcarbonyl, Aroyl, Aryl, Aryloxy oder eine Gruppe der Formel R₁₇-C≡C- darstellt,
R₁₆ Wasserstoff, Halogen, Nitro, Cyano, C₁₋₆-Alkyl, OH, Perfluor-C₁₋₆-alkyl, Aryloxy oder C₁₋₆-Alkylthio darstellt,
R₁₇ Wasserstoff, Aryl, Heteroaryl oder C₁₋₆-Alkyl, das unsubstituiert oder mit OH, Aryl oder Heteroaryl substituiert ist, darstellt, und
a 0 oder 1 ist;
oder einer von X und X' eine Gruppe der Formel: 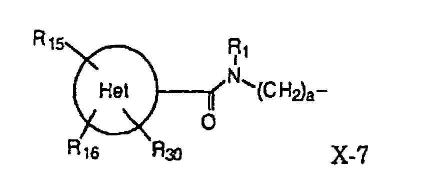 darstellt,
worin Het einen 5- oder 6-gliedrigen heteroaromatischen Ring, der 1, 2 oder 3 Heteroatome, ausgewählt aus N, O und S, enthält, darstellt;
oder
Het einen 9- oder 10-gliedrigen bicyclischen heteroaromatischen Ring, der 1, 2, 3 oder 4 Heteroatome, ausgewählt aus O, S und N, enthält, darstellt,
a, R₁, R₁₅ und R₁₆ wie vorstehend definiert sind und
R₃₀ Wasserstoff oder C₁₋₆-Alkyl darstellt oder nicht vorliegt;
oder einer von X und X' eine Gruppe der Formel: 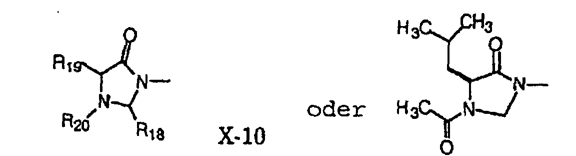 darstellt, worin:
R₁₈ C₁₋₆-Alkyl, Aryl, Heteroaryl, Aryl-C₁₋₆-alkyl, Heteroaryl-C₁₋₆-alkyl darstellt,
R₁₉ C₁₋₆-Alkyl, das unsubstituiert oder mit einem oder mehreren von Halogen, Hydroxy, C₁₋₆-Alkoxy, Aryl, Heteroaryl, Alkylthio substituiert ist, darstellt, oder
R₁₉ Aryl oder Heteroaryl darstellt, und
R₂₀ C₁₋₆-Alkyl oder C₁₋₆-Alkylcarbonyl darstellt, oder
R₁₉ und R₂₀ zusammengenommen Tetramethylen darstellen;
und
Y eine Gruppe der Formel: 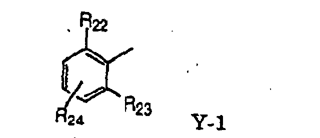 darstellt, worin:
R₂₂ und R₂₃ unabhängig Wasserstoff, C₁₋₆-Alkyl, C₁₋₆-Alkoxy, C₁₋₆-Alkoxylalkyl, C₁₋₆-Alkylamino, Aryl, Aryl-C₁₋₆-alkyl, Nitro, Cyano, C₁₋₆-Alkylthio, C₁₋₆-Alkylsulfinyl, C₁₋₆-Alkylsulfonyl, C₁₋₆-Alkylcarbonyl, Halogen oder Perfluoralkyl darstellen und mindestens einer von R₂₂ und R₂₃ von Wasserstoff verschieden ist, und
R₂₄ Wasserstoff, Hydroxy, C₁₋₆-Alkyl, C₁₋₆-Alkoxy, Amino, Aryl, Nitro, Cyano, C₁₋₆-Alkylsulfonyl, Halogen darstellt, oder eine Gruppe der Formel: 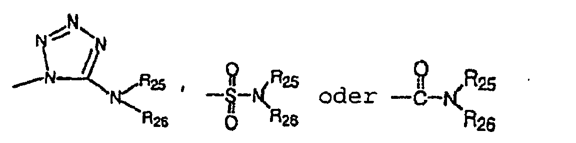 darstellt, worin
R₂₅ Wasserstoff, C₁₋₆-Alkyl, Aryl, Aryl-C₁₋₆-alkyl, Alkoxy-C₁₋₆-alkyl darstellt, und
R₂₆ Wasserstoff oder C₁₋₆-Alkyl darstellt, oder
R₂₂ und R₂₄ zusammengenommen einen kondensierten Benzolring darstellen; oder
Y eine Gruppe Y-2 darstellt, die eine fünf- oder sechsgliedrige monocyclische heteroaromatische Gruppe, die 1, 2 oder 3 Heteroatome, ausgewählt aus N, O und S, enthält, oder eine 9- oder 10-gliedrige bicyclische heteroaromatische Gruppe, die 1, 2, 3 oder 4 Heteroatome, ausgewählt aus O, S und N, enthält, darstellt, wobei die heteroaromatische Gruppe über ein Kohlenstoffatom an das Amidcarbonyl gebunden ist und ein oder zwei Kohlenstoffatome der heteroaromatischen Gruppe mit C₁₋₆-Alkyl, Halogen, Cyano, Perfluoralkyl oder Aryl substituiert sind und mindestens eines der substituierten Kohlenstoffatome zu dem Kohlenstoffatom, das an das Amidcarbonyl gebunden ist, benachbart ist;
und Z Wasserstoff oder C₁₋₆-Alkyl darstellt;
und die pharmazeutisch verträglichen Salze und Ester davon,
wobei der Begriff "C₁₋₆-Alkyl", einzeln oder in Kombination, unsubstituiert oder mit einer oder mehreren Gruppen, unabhängig ausgewählt aus Cycloalkyl, Nitro, Aryloxy, Aryl, Hydroxy, Halogen, Cyano, C₁₋₆-Alkoxy, C₁₋₆-Alkoxycarbonyl, C₁₋₆-Alkylthio, C₁₋₆-Alkylsulfinyl, C₁₋₆-Alkylsulfonyl und substituiertem Amino, substituiert bedeuten kann;
Cycloalkyl sich auf einen unsubstituierten oder substituierten 3- bis 7-gliedrigen carbacyclischen Ring bezieht, wobei die Substituenten ausgewählt sind aus Hydroxy, Halogen, Cyano, C₁₋₆-Alkoxy, C₁₋₆-Alkylcarbonyl, C₁₋₆-Alkyl, Aroyl, C₁₋₆-Alkylthio, C₁₋₆-Alkylsulfinyl, C₁₋₆-Alkylsulfonyl, Aryl, Heteroaryl und substituiertem Amino;
Aryl sich auf eine mono- oder bicyclische aromatische Gruppe bezieht, die unsubstituiert oder mit C₁₋₆-Alkyl, C₁₋₆-Alkoxy, Hydroxy-C₁₋₆-alkyl, Hydroxy, Hydroxyalkoxy, Halogen, C₁₋₆-Alkylthio, C₁₋₆-Alkylsulfinyl, C₁₋₆-Alkylsulfonyl, Cyano, Nitro, Perfluoralkyl, Alkylcarbonyl, Aroyl, wie nachstehend definiert, Arylalkinyl, Niederalkinyl und C₁₋₆-Alkylcarbonylamino substituiert ist;
Heteroaryl sich auf einen unsubstituierten oder substituierten 5- oder 6-gliedrigen monocyclischen heteroaromatischen Ring oder einen 9- oder 10-gliedrigen bicyclischen heteroaromatischen Ring, der 1, 2, 3 oder 4 Heteroatome enthält, die unabhängig N, S oder O darstellen, bezieht; und die Substituenten wie vorstehend für "Aryl" definiert sind;
Aroyl sich auf ein mono- oder bicyclisches Aryl, wie vorstehend definiert, oder Heteroaryl, wie vorstehend definiert, gebunden über eine Carbonylgruppe, bezieht; und
Aryloxy sich auf eine Arylgruppe, wie vorstehend definiert, bezieht, die über ein Sauerstoffatom gebunden ist.

2. Verbindung nach Anspruch 1, worin Z Wasserstoff darstellt.

3. Verbindung nach Anspruch 1 oder 2, worin X' Wasserstoff darstellt.

4. Verbindung nach einem der Ansprüche 1-3, worin:
R₂₂ und R₂₃ unabhängig Wasserstoff, C₁₋₆-Alkyl, Nitro, C₁₋₆-Alkylthio, C₁₋₆-Alkoxy, C₁₋₆-Alkylamino, C₁₋₆-Alkylsulfinyl, C₁₋₆-Alkylsulfonyl, C₁₋₆-Alkylcarbonyl, Halogen oder Perfluoralkyl darstellen, wobei mindestens einer von R₂₂ und R₂₃ nicht Wasserstoff darstellt, und
R₂₄ Wasserstoff, Hydroxy, C₁₋₆-Alkyl, C₁₋₆-Alkoxy, C₁₋₆-Alkylsulfonyl, Amino, Nitro, Halogen oder eine Gruppe der Formel: 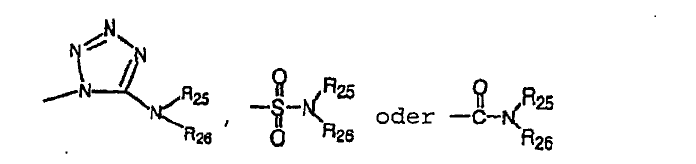 darstellt, worin
R₂₅ Aryl-C₁₋₆-alkyl darstellt und R₂₆ Wasserstoff oder C₁₋₆-Alkyl darstellt,
oder
R₂₂ und R₂₄ zusammengenommen einen kondensierten Benzolring darstellen.

5. Verbindung nach Anspruch 4, worin R₂₂ Wasserstoff (wenn R₂₃ von Wasserstoff verschieden ist), C₁₋₆-Alkyl oder Halogen darstellt.

6. Verbindung nach Anspruch 4 oder Anspruch 5, worin R₂₄ Wasserstoff, Hydroxy, Amino, C₁₋₆-Alkylsulfonyl, C₁₋₆-Alkyl, Halogen, Nitro oder C₁₋₆-Alkoxy oder eine Gruppe der Formel 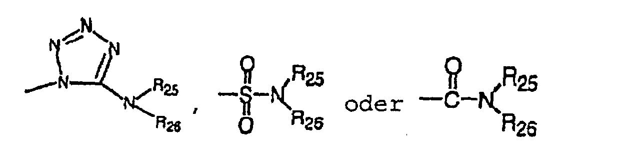 darstellt,
worin R₂₅ unsubstituiertes oder Hydroxy-substituiertes Phenyl-C₁₋₆-alkyl darstellt und R₂₆ Wasserstoff darstellt,
oder R₂₂ und R₂₄ zusammengenommen einen kondensierten Phenylring darstellen.

7. Verbindung nach Anspruch 6, worin R₂₄ Wasserstoff, Hydroxy, Amino, Methyl, Chlor, Brom, Nitro, -OCH₃, -SO₂CH₃ darstellt und R₂₆ H darstellt und R₂₅ 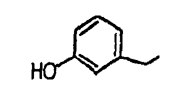 darstellt.

8. Verbindung nach einem der Ansprüche 4-7, worin R₂₃ Wasserstoff (wenn R₂₂ von Wasserstoff verschieden ist), C₁₋₆-Alkyl, C₁₋₆-Alkylamino, Halogen, Nitro, Perfluor-C₁₋₆-alkyl, C₁₋₆-Alkoxy, C₁₋₆-Alkylcarbonyl, C₁₋₆-Alkylthio, C₁₋₆-Alkylsulfinyl oder C₁₋₆-Alkylsulfonyl darstellt.

9. Verbindung nach Anspruch 8, worin R₂₃ Methyl, Ethyl, Isopropyl, tert-Butyl, Trifluormethyl, Chlor, Brom, Fluor, Nitro, -COCH₃, -SCH₃, -SOCH₃, -SO₂CH₃, -NHCH₃ oder -OCH₃ darstellt.

10. Verbindung nach einem der Ansprüche 4-9, worin Y-1 ausgewählt ist aus der Gruppe, bestehend aus:

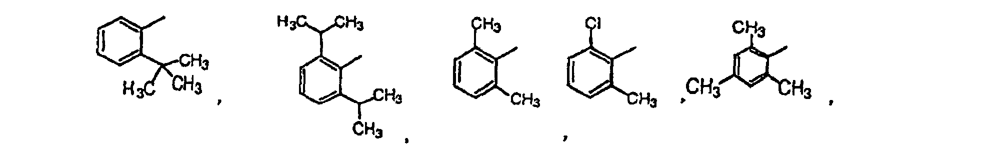

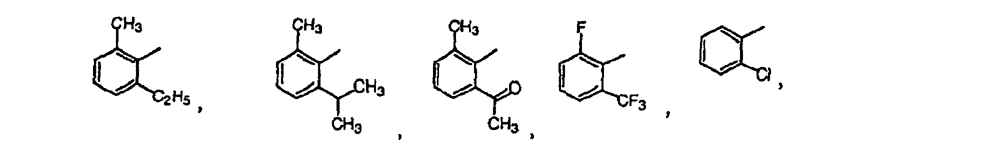

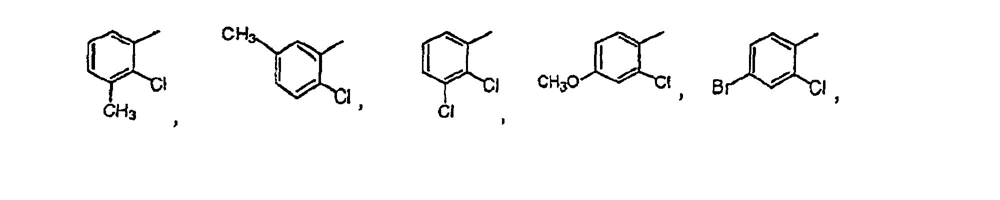

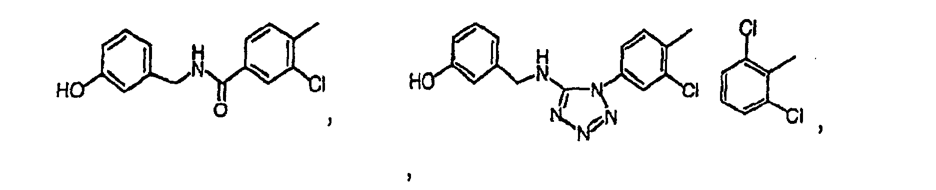

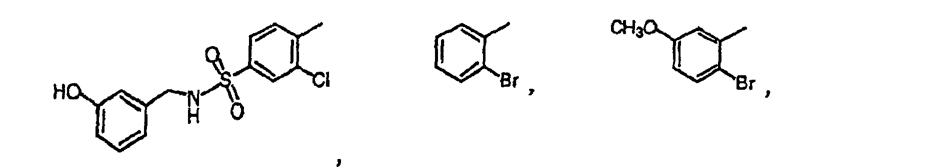

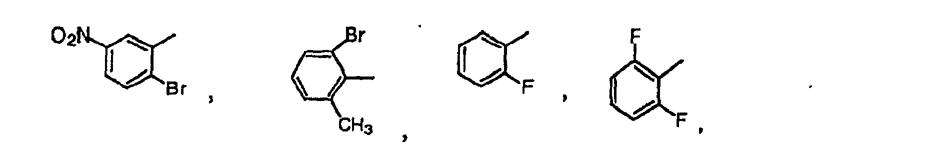

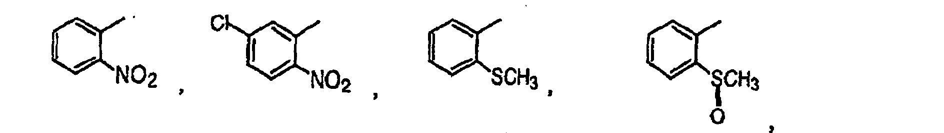

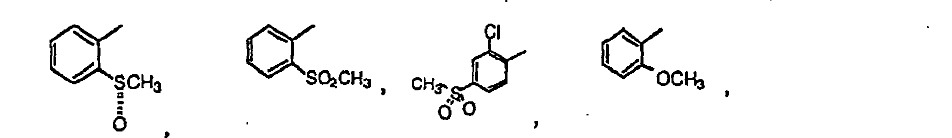

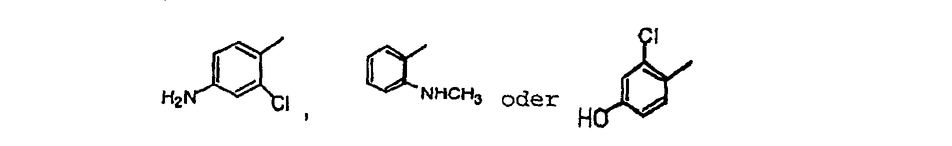

11. Verbindung nach einem der Ansprüche 1-3, worin in Y-2 die fünf- oder sechsgliedrigen monocyclischen heteroaromatischen oder die 9- oder 10-gliedrigen bicyclischen heteroaromatischen Gruppen ausgewählt sind aus der Gruppe von:

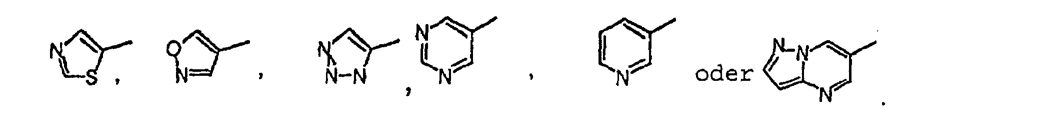

12. Verbindung nach einem der Ansprüche 1-3, worin Gruppen Y-2 die Formel: 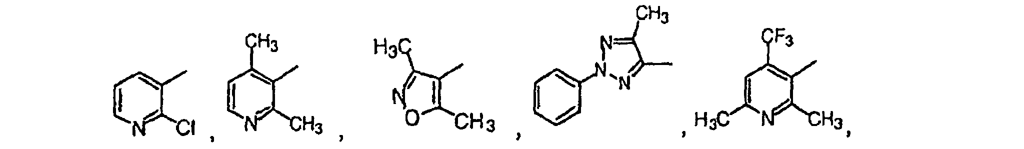 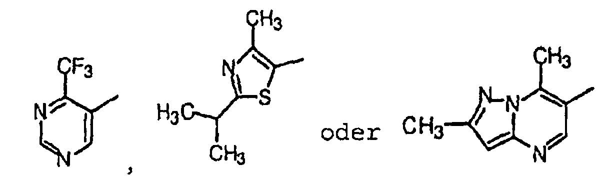 aufweisen.

13. Verbindung nach einem der Ansprüche 1-3, worin in X-6 die Gruppen R₁₅ und R₁₆ unabhängig Wasserstoff, C₁₋₆-Alkyl, Nitro, Halogen, Perfluor-C₁₋₆-alkyl, Cyano oder Aryloxy darstellen.

14. Verbindung nach Anspruch 13, worin R₁₅ oder R₁₆ H, Methyl, Nitro, Chlor, Fluor, Trifluormethyl, Cyano oder Phenoxy darstellen.

15. Verbindung nach Anspruch 13 oder Anspruch 14, worin Gruppe X-6 die Formel: 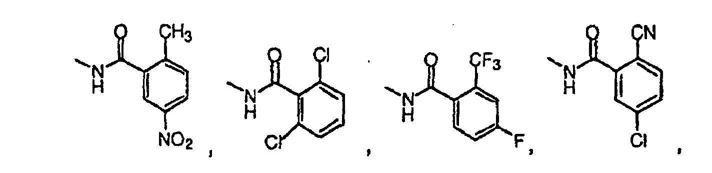 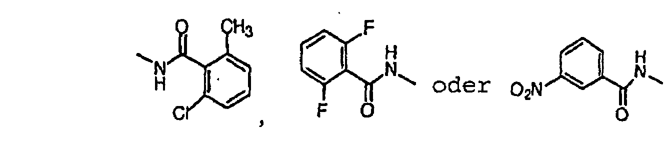 aufweist.

16. Verbindung nach einem der Ansprüche 1-3, worin in X-7 Het einen 5- oder 6-gliedrigen monocyclischen heteroaromatischen Ring darstellt, der 1, 2 oder 3 Stickstoffatome, oder ein Stickstoffatom und ein Schwefelatom, oder ein Stickstoffatom und ein Sauerstoffatom enthält.

17. Verbindung nach Anspruch 16, worin der heteroaromatische Ring 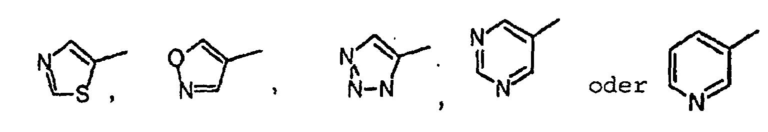 darstellt.

18. Verbindung nach einem der Ansprüche 1-3, worin in X-7 Het einen bicyclischen heteroaromatischen Ring darstellt, der 1 bis 3 Stickstoffatome als die Heteroatome enthält.

19. Verbindung nach Anspruch 18, worin der bicyclische heteroaromatische Ring 4-Chinolinyl, 1-Isochinolinyl oder 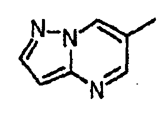 darstellt.

20. Verbindung nach einem der Ansprüche 1-3, worin in X-7 R₁₅ Wasserstoff, Nitro, C₁₋₆-Alkylsulfonyl, Cyano, C₁₋₆-Alkyl, C₁₋₆-Alkoxy, Perfluor-C₁₋₆-alkyl, C₁₋₆-Alkylthio, C₁₋₆-Alkylcarbonyl oder Aryl darstellt.

21. Verbindung nach Anspruch 20, worin R₁₅ Isopropyl, Methyl oder Phenyl darstellt.

22. Verbindung nach einem der Ansprüche 1-3, worin R₁₆ in X-7 Wasserstoff, Halogen, Nitro, Cyano, C₁₋₆-Alkyl oder Perfluor-C₁₋₆-alkyl darstellt.

23. Verbindung nach Anspruch 22, worin R₁₆ Methyl oder Trifluormethyl darstellt.

24. Verbindung nach einem der Ansprüche 1-3, worin R₃₀ in X-7 Wasserstoff oder C₁₋₆-Alkyl darstellt.

25. Verbindung nach einem der Ansprüche 1-3, worin Gruppen X-7 die Formel: 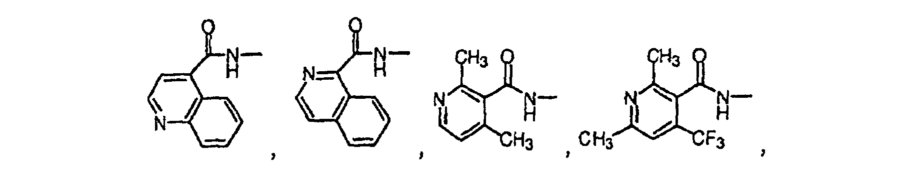 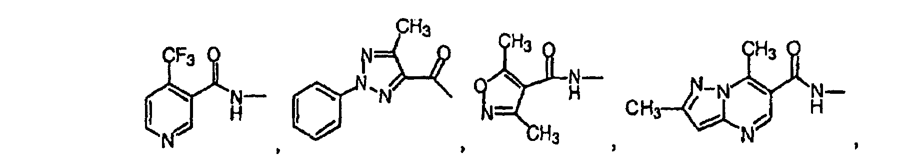 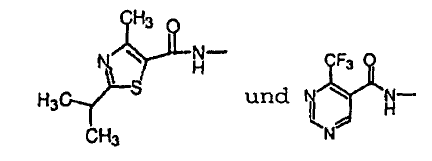 aufweisen.

26. Verbindung nach einem der Ansprüche 1-3, worin in X-6 oder X-7 R₁ Wasserstoff darstellt.

27. Verbindung nach einem der Ansprüche 1-3, worin in X-6 oder X-7 a 0 ist.

28. Verbindung nach einem der Ansprüche 1-3, worin in X-10 R₁₈ C₁₋₆-Alkyl oder Phenyl darstellt, wobei der Phenylring unsubstituiert oder mit Halogen, Hydroxy monosubstituiert ist, oder Phenyl-C₁₋₆-alkyl darstellt.

29. Verbindung nach Anspruch 28, worin R₁₈ tert-Butyl, Phenyl, Hydroxyphenyl, Chlorphenyl oder Phenylethyl darstellt.

30. Verbindung nach einem der Ansprüche 1-3, worin in X-10 R₁₉ C₁₋₆-Alkyl darstellt, das unsubstituiert oder mit Pyridyl oder Phenyl substituiert ist, wobei der Phenylring unsubstituiert oder mit C₁₋₆-Alkoxy oder Halogen monosubstituiert ist.

31. Verbindung nach Anspruch 30, worin R₁₉ Methyl, Isobutyl, Benzyl, 4-Chlorbenzyl, 4-Methoxybenzyl oder 2-Pyridylmethyl darstellt.

32. Verbindung nach einem der Ansprüche 1-3, worin in X-10 R₂₀ C₁₋₆-Alkylcarbonyl darstellt.

33. Verbindung nach Anspruch 32, worin R₂₀ Acetyl darstellt.

34. Verbindung nach einem der Ansprüche 1-3, worin Gruppen X-10 die Formel: 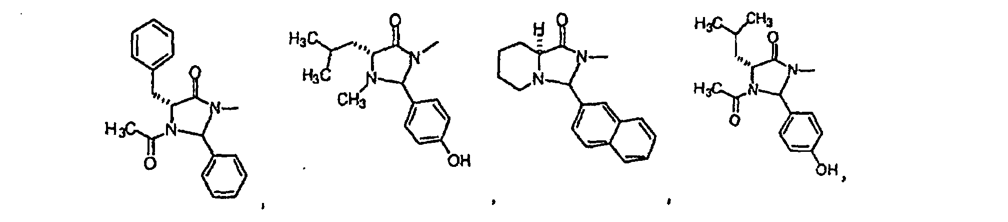 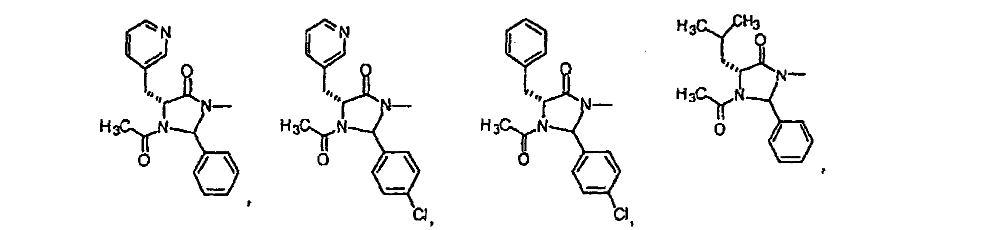 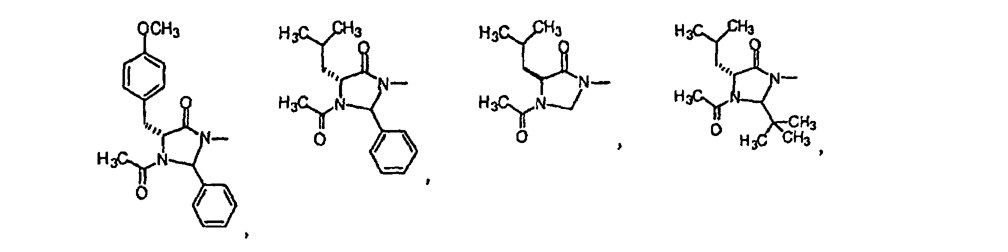 und 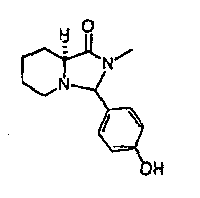 aufweisen.

35. Verbindung, ausgewählt aus der Gruppe: 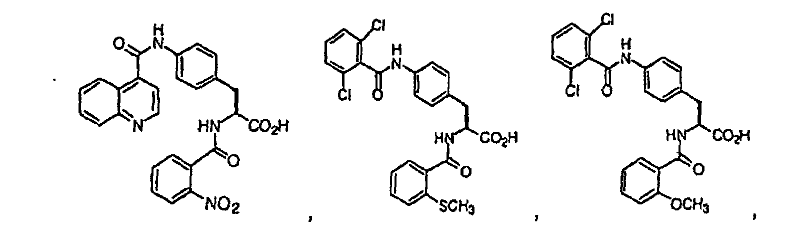 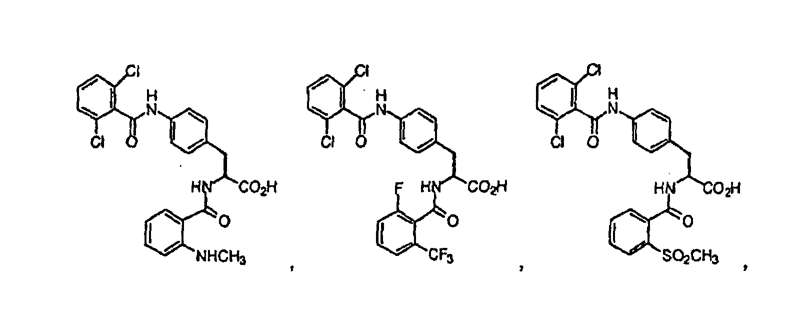 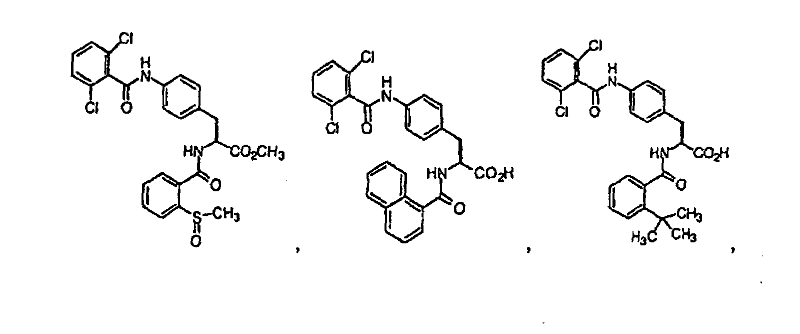 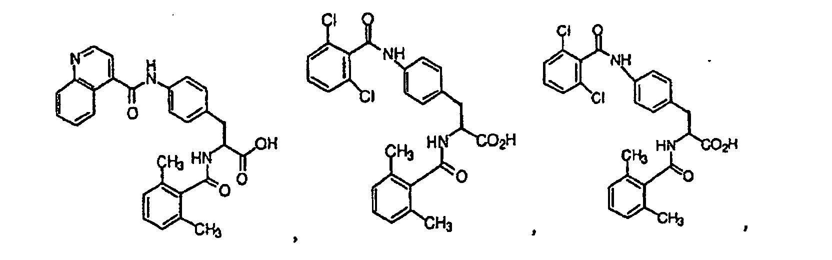 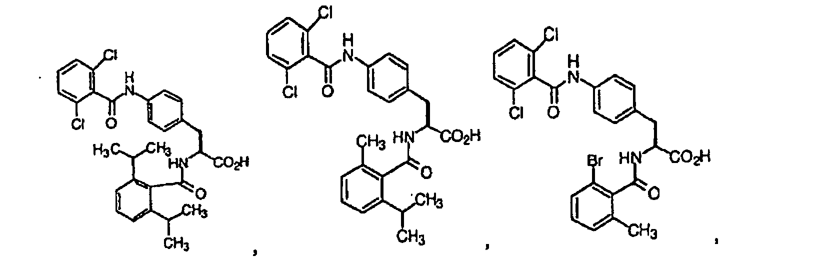 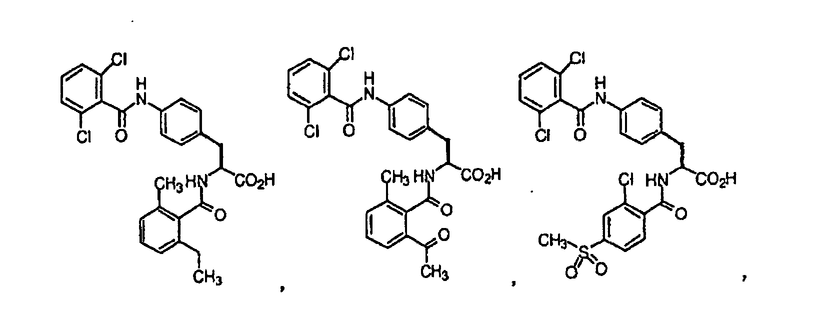 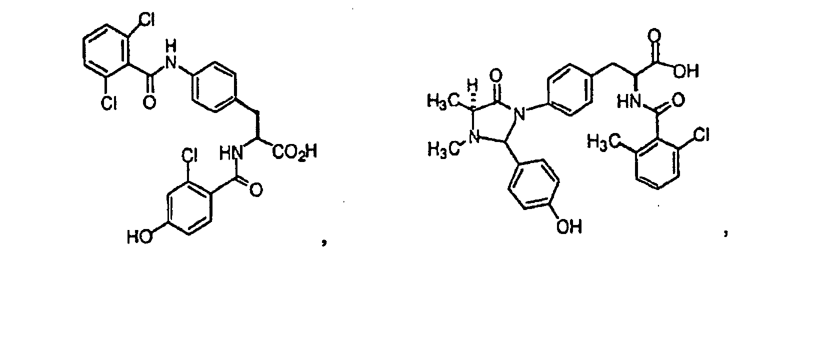 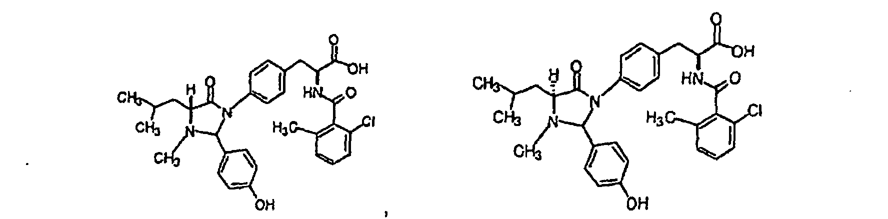 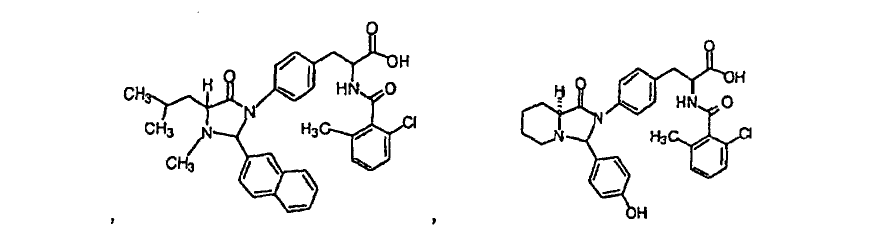 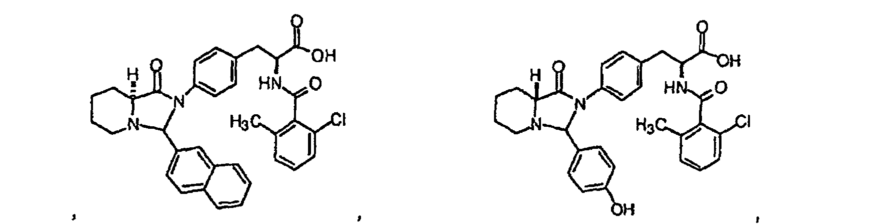 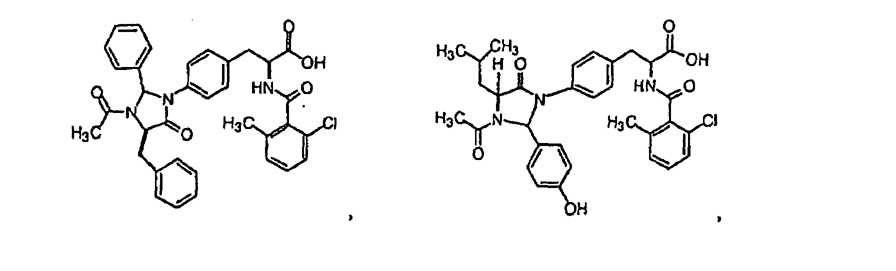 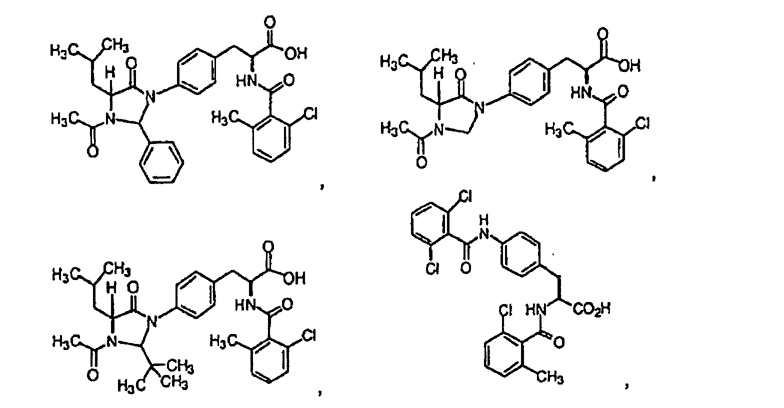 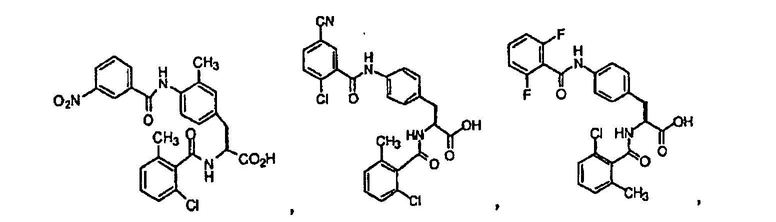 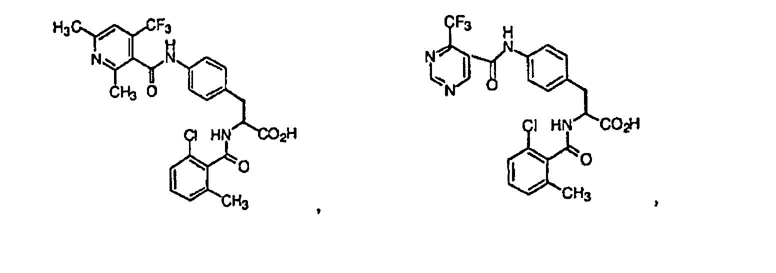 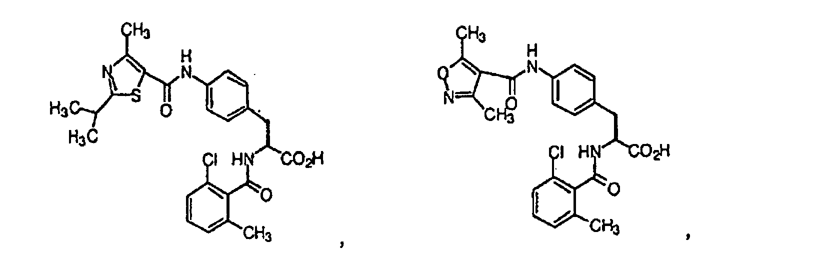 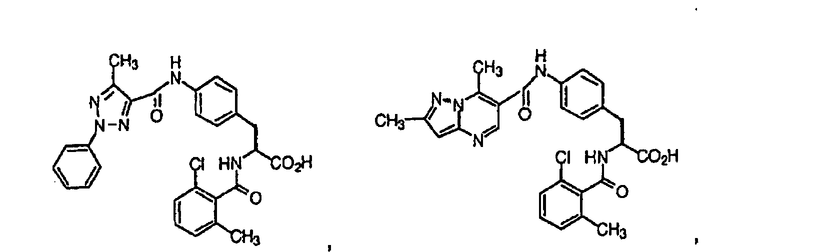 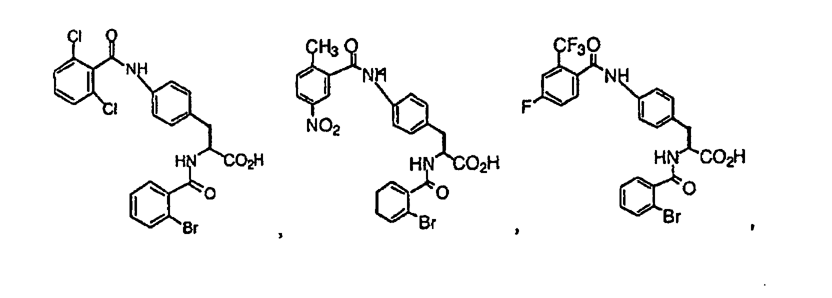 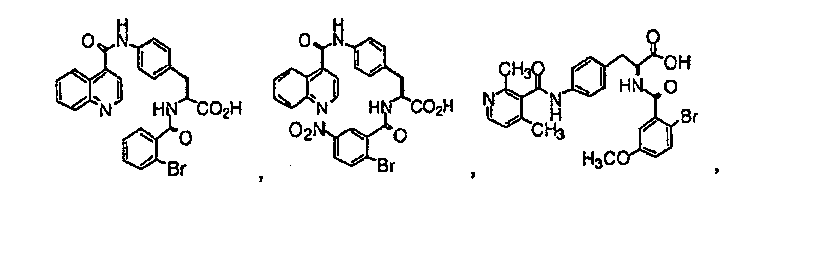 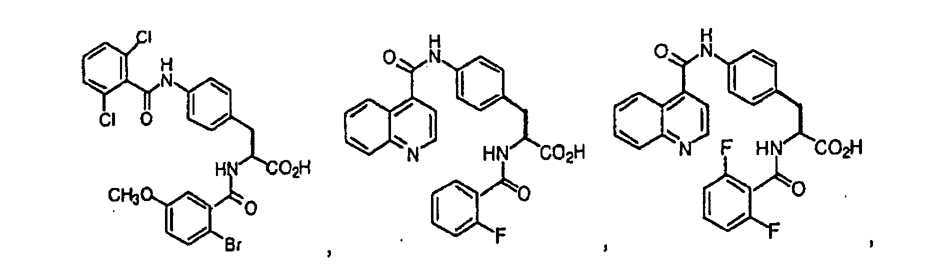 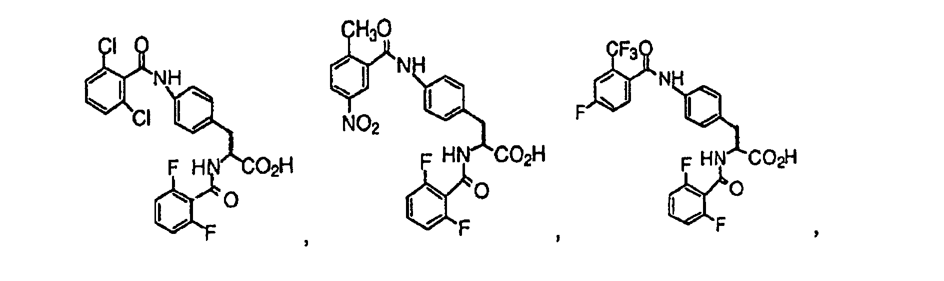 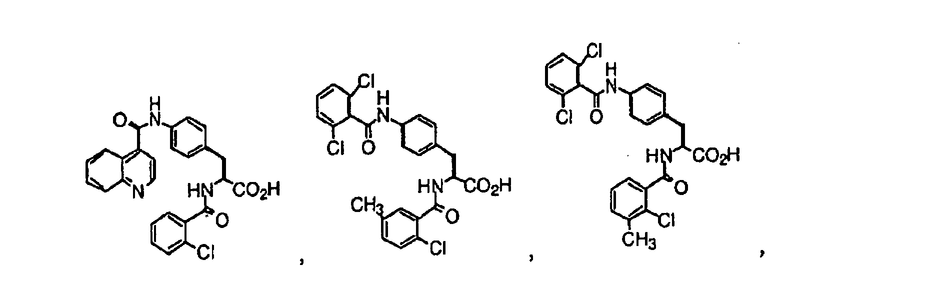 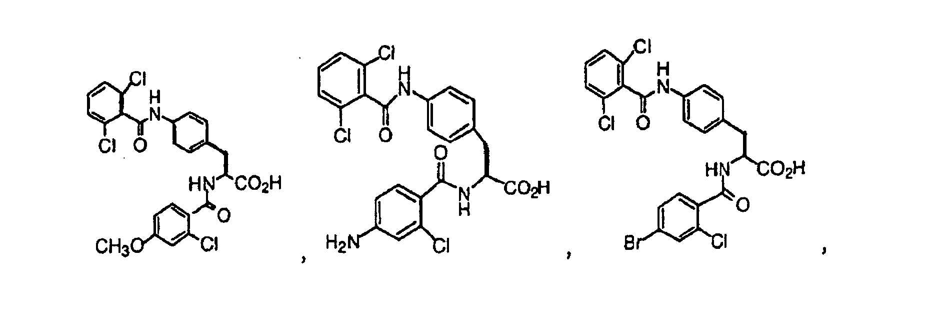 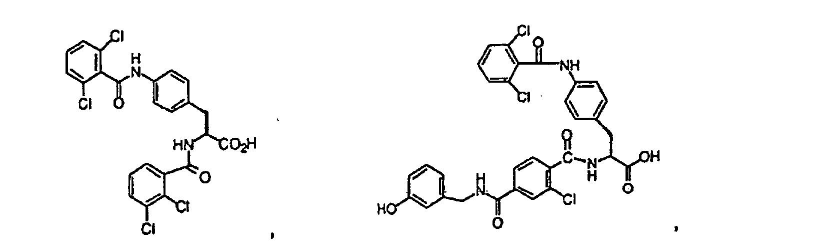 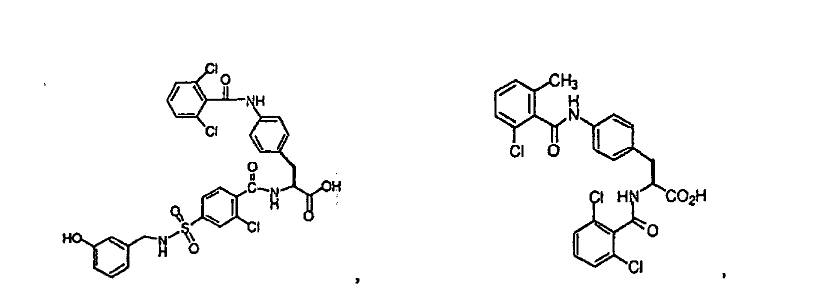 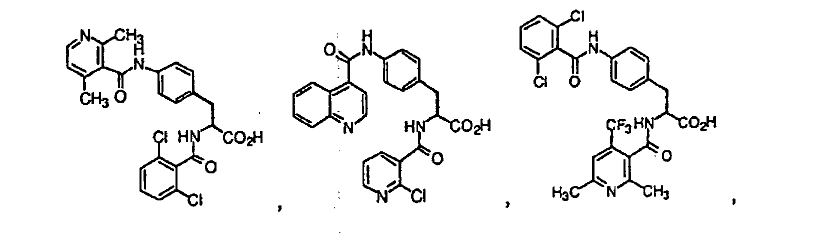 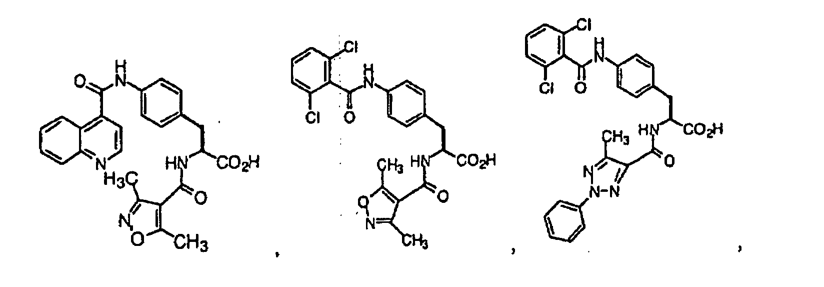 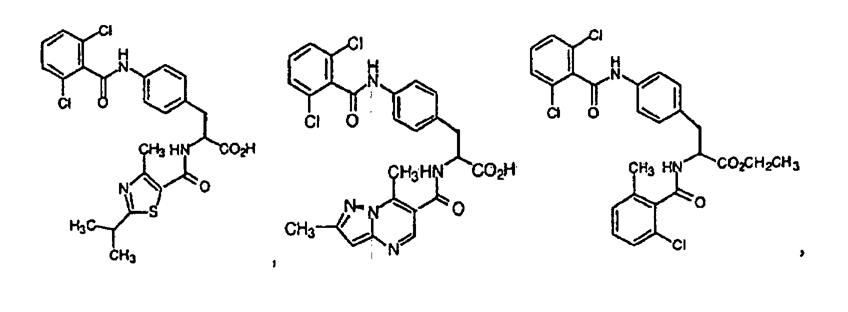 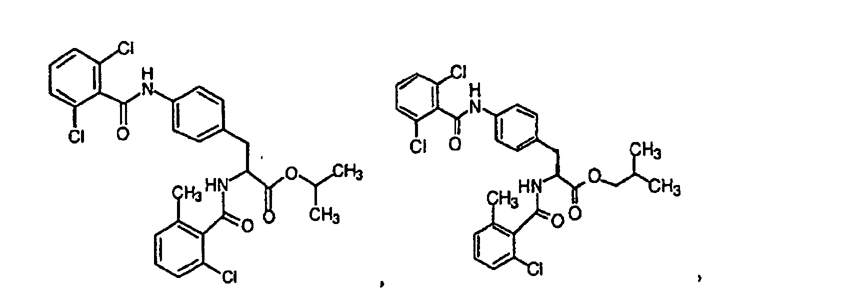 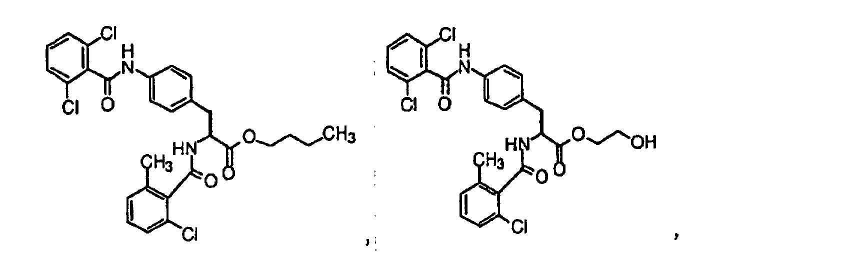 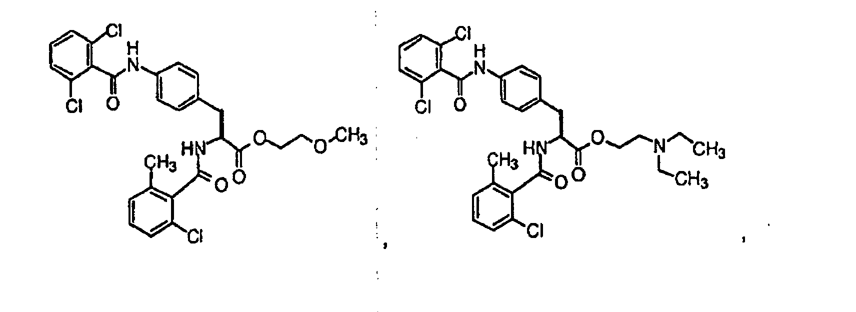 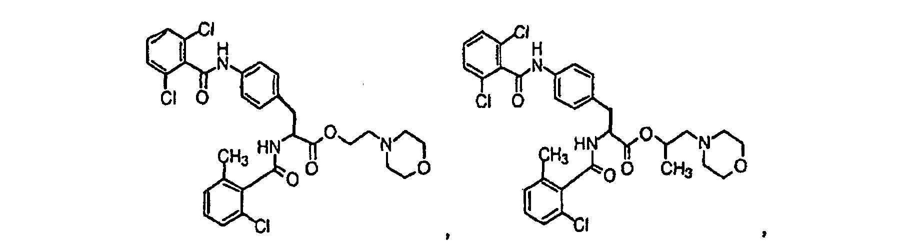 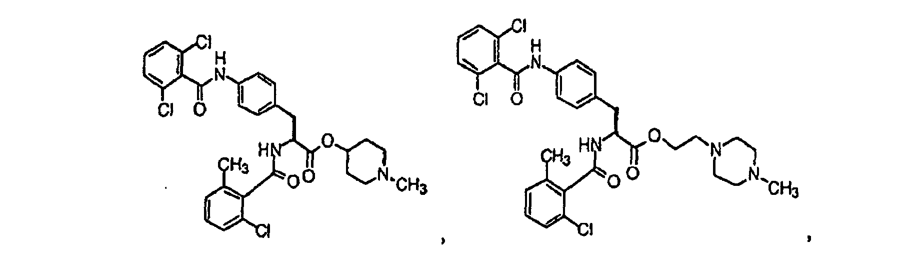 und

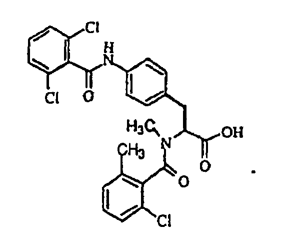

36. Verbindung nach Anspruch 1, nämlich N-(2-Chlor-6-methylbenzoyl)-4-[[(2,6-dichlorphenyl)carbonyl)amino]-L-phenylalanin oder N-(2-Chlor-6-methylbenzoyl)-4-[[(2,6-dichlorphenyl)carbonyl]amino]-L-phenylalaninnatriumsalz.

37. Verbindung nach Anspruch 1 mit der Formel: 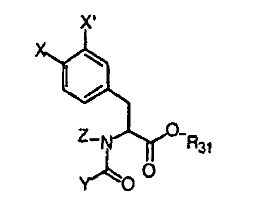 worin:
X, X', Z und Y wie in Anspruch 1 definiert sind,
R₃₁ C₁₋₆-Alkyl darstellt oder
R₃₁ eine Gruppe der Formel P-1 darstellt: 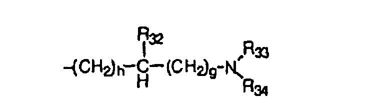
worin:
R₃₂ Wasserstoff oder C₁₋₆-Alkyl darstellt,
R₃₃ Wasserstoff, C₁₋₆-Alkyl oder Aryl darstellt,
R₃₄ Wasserstoff oder C₁₋₆-Alkyl darstellt,
h eine ganze Zahl von 0 bis 2 ist,
g eine ganze Zahl von 0 bis 2 ist und
die Summe von h und g 1 bis 3 ist; oder
R₃₁ eine Gruppe der Formel P-2 darstellt: 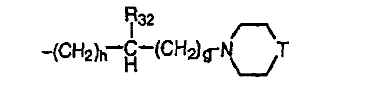
worin:
R₃₂, g und h wie vorstehend definiert sind,
T O, S, -(CH₂)ⱼ-, eine Bindung (wenn j=0) oder eine Gruppe der Formel N-R₃₅ darstellt,
R₃₅ Wasserstoff, C₁₋₆-Alkyl, C₁₋₆-Alkylcarbonyl, C₁₋₆-Alkoxycarbonyl darstellt und j 0, 1 oder 2 ist.

38. Verbindung nach Anspruch 37, worin R₃₁ C₁₋₆-Alkyl darstellt.

39. Verbindung nach Anspruch 38, nämlich
N-(2-Chlor-6-methylbenzoyl)-4-[[(2,6-dichlorphenyl)carbonyl]amino]-L-phenylalaninmethylester oder
N-(2-Chlor-6-methylbenzoyl)-4-[[(2,6-dichlorphenyl)carbonyl]amino]-L-phenylalaninethylester oder
N-(2-Chlor-6-methylbenzoyl)-4-[[(2,6-dichlorphenyl)carbonyl]amino]-L-phenylalaninbutylester oder
N-(2-Chlor-6-methylbenzoyl)-4-[[(2,6-dichlorphenyl)carbonyl]amino]-L-phenylalanin-1-methylethylester oder
N-(2-Chlor-6-methylbenzoyl)-4-[[(2,6-dichlorphenyl)carbonyl]amino]-L-phenylalanin-2-methylpropylester.

40. Verbindung nach Anspruch 1, nämlich
N-(2-Chlor-6-methylbenzoyl)-4-[[(2,6-dichlorphenyl)carbonyl]amino]-L-phenylalanin-2-methoxyethylester oder
N-(2-Chlor-6-methylbenzoyl)-4-[[(2,6-dichlorphenyl)carbonyl]amino]-L-phenylalanin-2-hydroxyethylester.

41. Verbindung nach Anspruch 37, worin R₃₁ P-2 darstellt.

42. Verbindung nach Anspruch 41, worin R₃₁ 2-(4-Morpholinyl)-ethyl, 1-Methyl-2-(4-morpholinyl)ethyl, 1-Methyl-4-piperidinyl, 2-(1-Piperazinyl)ethyl oder 2-(4-Methyl-1-piperazinyl)ethyl darstellt.

43. Verbindung nach Anspruch 42, nämlich
N-(2-Chlor-6-methylbenzoyl)-4-[[(2,6-dichlorphenyl)-carbonyl]amino]-L-phenylalanin-2-morpholinoethylester oder
N-(2-Chlor-6-methylbenzoyl)-4-[[(2,6-dichlorphenyl)-carbonyl]amino]-L-phenylalanin-1-methyl-2-morpholinoethylester oder
N-(2-Chlor-6-methylbenzoyl)-4-[[(2,6-dichlorphenyl)-carbonyl]amino]-L-phenylalanin-1-methyl-4-piperidinylester oder
N-(2-Chlor-6-methylbenzoyl)-4-[[(2,6-dichlorphenyl)-carbonyl]amino]-L-phenylalanin-2-(1-piperazinyl)ethylester oder
N-(2-Chlor-6-methylbenzoyl)-4-[[(2,6-dichlorphenyl)-carbonyl]amino]-L-phenylalanin-2-(4-methyl-1-piperazinyl)-ethylester.

44. Verbindung nach Anspruch 37, worin R₃₁ P-1 darstellt.

45. Verbindung nach Anspruch 44, worin R₃₁ Diethylaminoethyl darstellt.

46. Verbindung nach Anspruch 45 mit der Formel 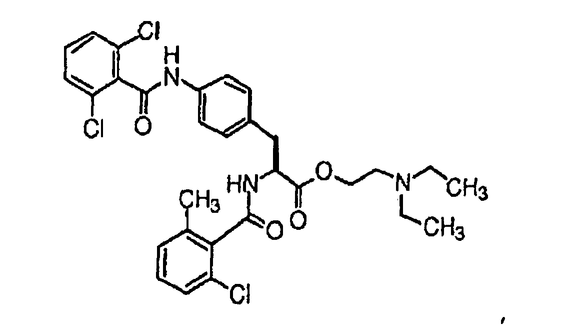 nämlich N-(2-Chlor-6-methylbenzoyl)-4-[[(2,6-dichlorphenyl)carbonyl]amino]-L-phenylalanin-2-diethylaminoethylester.

47. Verbindungen nach einem der Ansprüche 1-46 zur Verwendung als ein Arzneimittel.

48. Arzneimittel, enthaltend eine Verbindung nach einem der Ansprüche 1-46 und ein therapeutisch inertes Trägermaterial.

49. Verfahren zur Herstellung eines Arzneimittels, insbesondere für die Behandlung von rheumatischer Arthritis, Multipler Sklerose, entzündlicher Darmkrankheit oder Asthma, wobei das Verfahren Bringen einer Verbindung nach einem der Ansprüche 1-46, zusammen mit einem therapeutisch inerten Trägermaterial, und, falls erwünscht, einer oder mehreren zusätzlichen therapeutisch wirksamen Substanzen, in eine galenische Verabreichungsform umfasst.

50. Verwendung einer Verbindung nach einem der Ansprüche 1-46 bei der Herstellung eines Arzneimittels für die Behandlung von rheumatischer Arthritis, Multipler Sklerose, entzündlicher Darmkrankheit oder Asthma.

## Revendications

1. Composé de formule 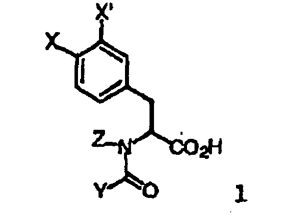 dans laquelle
un des X et X' est de l'hydrogène, un halogène, ou un alkyle en C₁₋₆, l'autre est un groupe de formule : 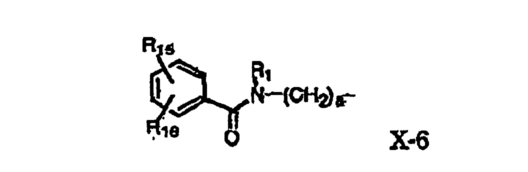 dans laquelle
R₁ est un hydrogène ou un alkyle en C₁₋₆ ;
R₁₅ est un hydrogène, un halogène, un nitro, un alkylsulfonyle en C₁₋₆, un cyano, un alkyle en C₁₋₆, un OH, un alcoxy en C₁₋₆, un alcoxycarbonyle en C₁₋₆, un carboxy, un alkylaminosulfonyle en C₁₋₆, un alkyle en C₁₋₆ perfluoro, un alkylthio en C₁₋₆, un alkyle en C₁₋₆ hydroxy, un alkyle en C₁₋₆ alcoxy, un alkyle en C₁₋₆ halo, un alkyle en C₁₋₆ alkylthio, un alkyle en C₁₋₆ alkylsulfinyle, un alkyle en C₁₋₆ alkylsulfonyle, un alkylsulfinyle en C₁₋₆, un alkylcarbonyle en C₁₋₆, un aroyle, un aryle, un aryloxy ou un groupe de formule R₁₇-C≡C-,
R₁₆ est un hydrogène, halogène, nitro, cyano, alkyle en C₁₋₆, OH, alkyle en C₁₋₆ perfluoro, aryloxy ou alkylthio en C₁₋₆ ;
R₁₇ est un hydrogène, un aryle, un hétéroaryle ou un alkyle en C₁₋₆ qui est non substitué ou substitué par OH, aryle ou hétéroaryle, et
a est 0 ou 1 ;
ou un des X et X' est un groupe de formule : 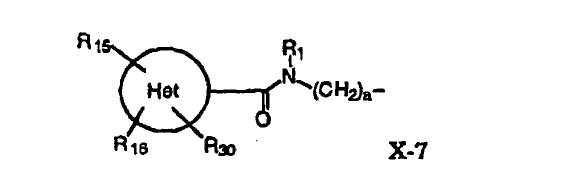 dans laquelle Het est un noyau hétéroaromatique à 5 ou 6 éléments contenant 1, 2, ou 3 hétéroatomes choisis parmi N, O et S ;
ou
Het est un noyau hétéroaromatique bicyclique à 9 ou 10 éléments contenant 1, 2, 3 ou 4 hétéroatomes choisis parmi O, S et N
a, R₁, R₁₅ et R₁₆ sont comme ci-dessus et
R₃₀ est un hydrogène ou un alkyle en C₁₋₆ ou est absent ;
ou un des X et X' est un groupe de la formule : 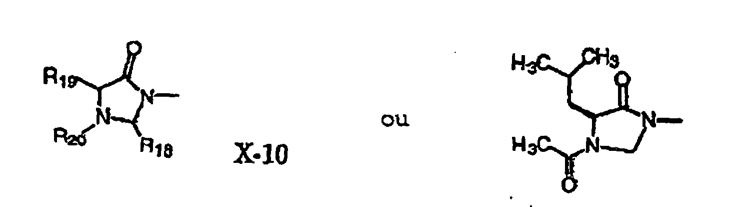 dans laquelle
R₁₈ est un alkyle en C₁₋₆, un aryle, un hétéroaryle, un alkyle en C₁₋₆ aryle, un alkyle en C₁₋₆ hétéroaryle,
R₂₉ est un alkyle en C₁₋₆, qui est non substitué ou substitué par un ou plusieurs des éléments halogène, hydroxy, alcoxy en C₁₋₆, aryle, hétéroaryle, alkylthio, ou R₁₉ est un aryle ou hétéroaryle et
R₂₀ est un alkyle en C₁₋₆ ou un alkylcarbonyle en C₁₋₆ ou
R₁₉ et R₂₀ pris ensemble sont du tétraméthylène ;
et
Y est un groupe de formule : 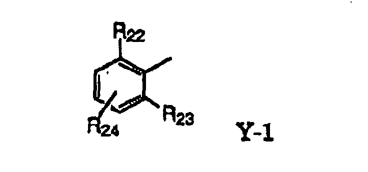 dans laquelle
R₂₂ et R₂₃ sont indépendamment un hydrogène, un alkyle en C₁₋₆, un alcoxy en C₁₋₆, un alcoxylalkyle en C₁₋₆, un alkylamino en C₁₋₆, un aryle, un alkyle en C₁₋₆ aryle, un nitro, un cyano, un alkylthio en C₁₋₆, un alkylsulfinyle en C₁₋₆, un alkylsulfonyle en C₁₋₆, un alkylcarbonyle en C₁₋₆, un halogène ou un perfluoroalkyle et au moins un des R₂₂ et R₂₃ est différent de l'hydrogène, et
R₂₄ est un hydrogène, un hydroxy, un alkyle en C₁₋₆, un alcoxy en C₁₋₆, un amino, un aryle, un nitro, un cyano, un alkylsulfonyle en C₁₋₆, un halogène ou est un groupe de formule : 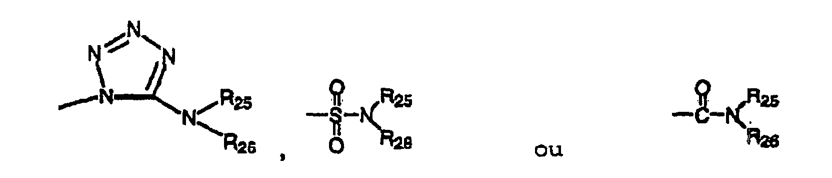 dans laquelle R₂₅ est un hydrogène, un alkyle en C₁₋₆, un aryle, un alkyle en C₁₋₆ aryle, un alkyle en C₁₋₆ alcoxy et R₂₆ est un hydrogène ou un alkyle en C₁₋₆ ou
R₂₂ et R₂₄ pris ensemble sont un noyau de benzène fusionné, ou
Y est un groupe Y-2 qui est un groupe hétéroaromatique monocyclique à cinq ou six éléments contenant 1, 2 ou 3 hétéroatomes choisis parmi N, O et S ou un groupe hétéroaromatique bicyclique à 9 ou 10 éléments contenant 1, 2, 3 ou 4 hétéroatomes choisis parmi O, S et N, dans lequel ledit groupe hétéroaromatique est lié via un atome de carbone au carbonyle amide et un ou deux atomes de carbone dudit groupe hétéroaromatique sont substitués par un alkyle en C₁₋₆, un halogène, un cyano, un perfluoroalkyle, ou un aryle et au moins un desdits atomes de carbone substitués est adjacent à l'atome de carbone lié au carbonyle amide ;
et Z est un hydrogène ou un alkyle en C₁₋₆ ;
et leurs sels et esters pharmaceutiquement acceptables,
dans laquelle le terme « alkyle en C₁₋₆ », seul ou en combinaison, peut être non substitué ou substitué par un ou plusieurs groupes choisis indépendamment parmi le cycloalkyle, nitro, aryloxy, aryle, hydroxy, halogène, cyano, alcoxy en C₁₋₆, alcoxycarbonyle en C₁₋₆, un alkylthio en C₁₋₆, un alkylsulfinyle en C₁₋₆, un alkylsulfonyle en C₁₋₆, et un amino substitué ;
cycloalkyle se réfère à un noyau carbocyclique de 3 à 7 éléments non substitués ou substitués, dans laquelle les substituants sont choisis parmi l'hydroxy, l'halogène, le cyano, l'alcoxy en C₁₋₆, l'alkylcarbonyle en C₁₋₆, l'alkyle en C₁₋₆, l'aroyle, l'alkylthio en C₁₋₆, l'alkylsulfinyle en C₁₋₆, l'alkylsulfonyle en C₁₋₆, l'aryle, l'hétéroaryle et l'amino substitué ;
aryle se réfère à un groupe aromatique mono ou bicyclique qui est non substitués ou substitué par un alkyle en C₁₋₆, un alcoxy en C₁₋₆, un alkyle en C₁₋₆ hydroxy, un hydroxy, un alcoxy hydroxy, un halogène, un alkylthio en C₁₋₆, un alkylsulfinyle en C₁₋₆, un alkylsulfonyle en C₁₋₆, un cyano, un nitro, un perfluoroalkyle, un alkylcarbonyle, un aroyle comme défini ci-dessous, un aryle alcynyle, un alcynyle inférieur et un alkylcarbonylamino en C₁₋₆ ;
hétéroaryle se réfère à un noyau hétéroaromatique monocyclique à 5 ou 6 éléments substitués ou non substitués ou à un anneau hétéroaromatique bicyclique à 9 ou 10 éléments contenant 1, 2, 3 ou 4 hétéroatomes qui sont indépendamment N, S ou O ; les substituants sont comme définis ci-dessus pour « l'aryle »
aroyle se réfère à un aryle mono- ou bicyclique comme défini ci-dessus ou un hétéroaryle comme défini ci-dessus, lié via un groupe carbonyle et
aryloxy se réfère à un groupe aryle, comme défini ci-dessus, qui est lié via un atome d'oxygène.

2. Composé selon la revendication 1, dans lequel Z est de l'hydrogène.

3. Composé selon la revendication 1 où 2, dans lequel X' est de l'hydrogène.

4. Composé selon l'une quelconque des revendications 1-3, dans lequel R₂₂ et R₂₃ sont indépendamment de l'hydrogène, un alkyle en C₁₋₆, un nitro, un alkylthio en C₁₋₆, un alcoxy en C₁₋₆, un alkylamino en C₁₋₆, un alkylsulfinyle en C₁₋₆, un alkylaulfonyle en C₁₋₆, un alkylcarbonyle en C₁₋₆, un halogène, ou un perfluoroalkyle dans lequel au moins un des R₂₂ et R₂₃ n'est pas de l'hydrogène et
R₂₄ est un hydrogène, hydroxy, alkyle en C₁₋₆, alcoxy en C₁₋₆, alkylsulfonyle en C₁₋₆, amino, nitro, halogène ou un groupe de formule : 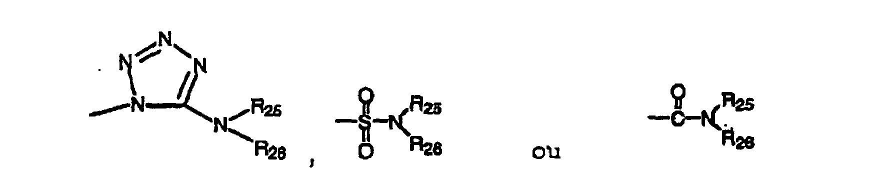 dans laquelle R₂₅ est un alkyle en C₁₋₆ aryle, et R₂₆ est un hydrogène ou un alkyle en C₁₋₆,
ou R₂₂ et R₂₄ pris ensemble sont un noyau de benzène fusionné.

5. Composé selon la revendication 4, dans lequel R₂₂ est un hydrogène (quand R₂₃ est différent de l'hydrogène), un alkyle en C₁₋₆ ou un halogène.

6. Composé selon la revendication 4 ou 5, dans lequel R₂₄ est un hydrogène, un hydroxy, un amino, un alkylsulfonyle en C₁₋₆, un alkyle en C₁₋₆, un halogène, un nitro, ou un alcoxy en C₁₋₆ ou un groupe de formule : 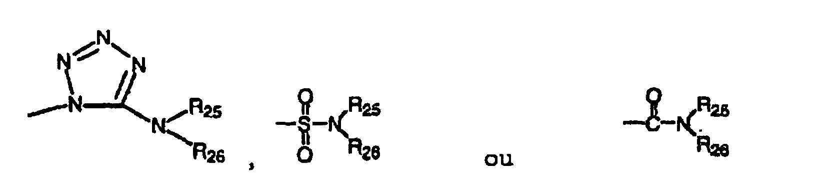 dans laquelle R₂₅ est un alkyle en C₁₋₆ phényle non substitué ou hydroxy-substitué, et R₂₆ est de l'hydrogène
ou R₂₂ et R₂₄ pris ensemble sont un noyau de phényle fusionné.

7. Composé selon la revendication 6, dans lequel R₂₄ est un hydrogène, un hydroxy, un amino, un méthyle, un chloro, un bromo, un nitro, un -OCH₃, un -SO₂CH₃, et R₂₆ est un H et R₂₅ est un

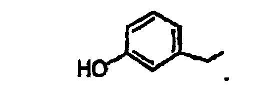

8. Composé selon l'une quelconque des revendications 4-7, dans lequel R₂₃ est un hydrogène (quand R₂₂ est différent de l'hydrogène), un alkyle en C₁₋₆, un alkylamino en C₁₋₆, un halogène, un nitro, un alkyle en C₁₋₆ perfluoro, un alcoxy en C₁₋₆, un alkylcarbonyle en C₁₋₆, un alkylthio en C₁₋₆, un alkylsulfinyle en C₁₋₆ ou un alkylsulfonyle en C₁₋₆.

9. Composé selon la revendication 8, dans lequel R₂₃ est un méthyle, éthyle, isopropyle, tertbutyle, trifluorométhyle, chloro, bromo, fluoro, nitro, -COCH₃,-SCH₃, -SOCH₃, -SO₂CH₃, -NHCH₃ ou -OCH₃.

10. Composé selon l'une quelconque des revendications 4-9, dans lequel Y-1 est choisi dans le groupe composé de :

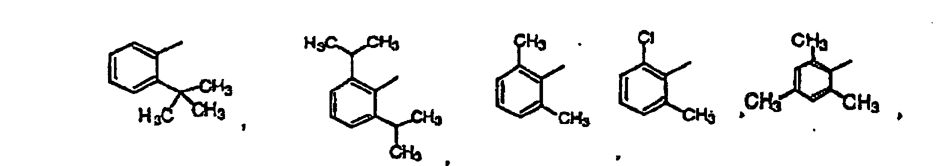

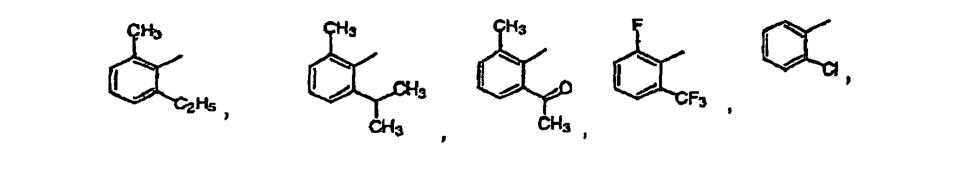

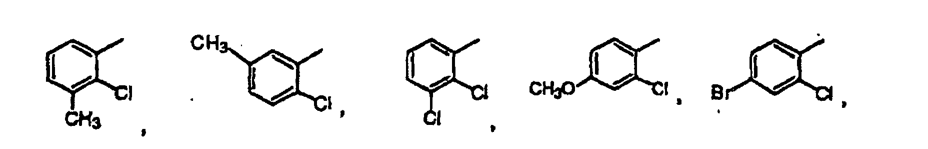

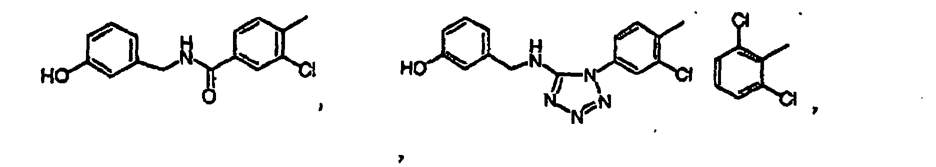

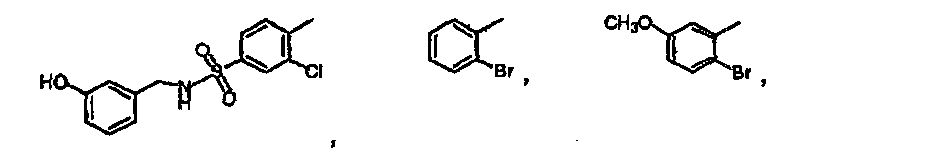

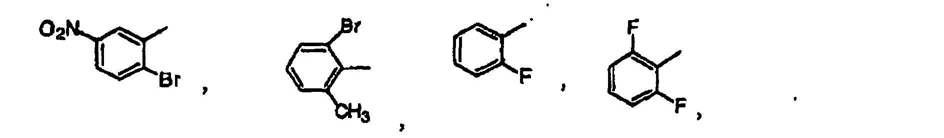

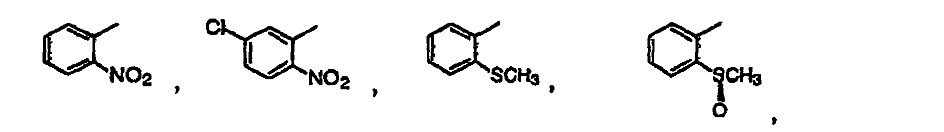

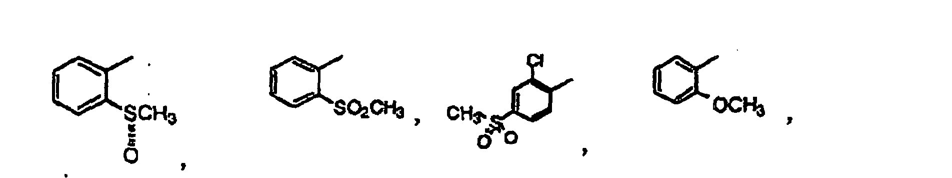

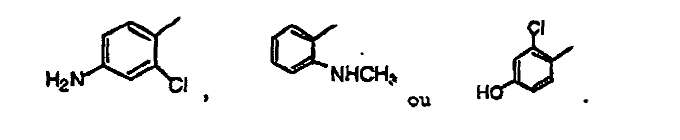

11. Composé selon l'une quelconque des revendications 1-3, dans lequel dans Y-2, les groupes hétéroaromatiques monocycliques à cinq ou six éléments ou les groupes hétéroaromatiques bicycliques à 9 ou 10 éléments sont choisis dans le groupe composé de :

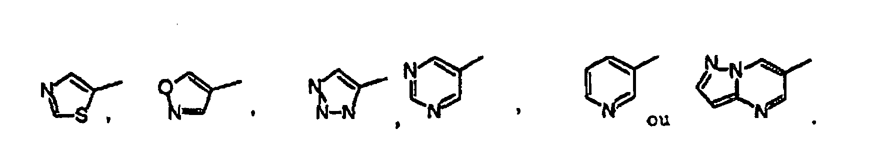

12. Composé selon l'une quelconque des revendications 1-3, dans lequel les groupes Y-2 sont de formule :

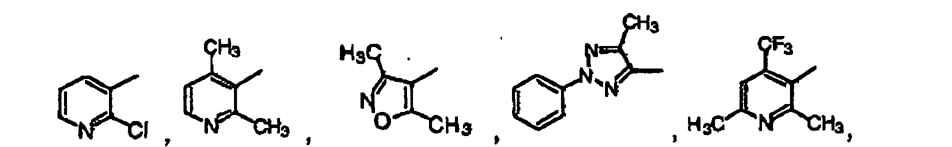

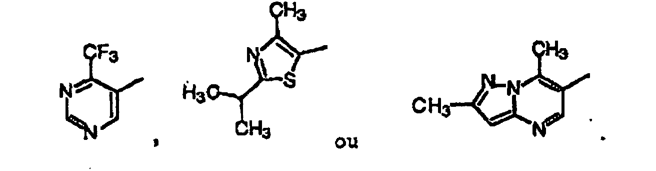

13. Composé selon l'une quelconque des revendications 1-3, dans lequel dans X-6, les groupes R₁₅ et R₁₆ sont indépendamment un alkyle en C₁₋₆ hydrogène, un nitro, un halogène, un alkyle en C₁₋₆ perfluoro, un cyano ou un aryloxy.

14. Composé selon la revendication 13, dans laquelle R₁₅ ou R₁₆ est H, un méthyle, un nitro, un chloro, un fluoro, un trifluorométhyle, un cyano ou un phénoxy.

15. Composé selon la revendication 13 ou 14, dans lequel le groupe X-6 est de formule :

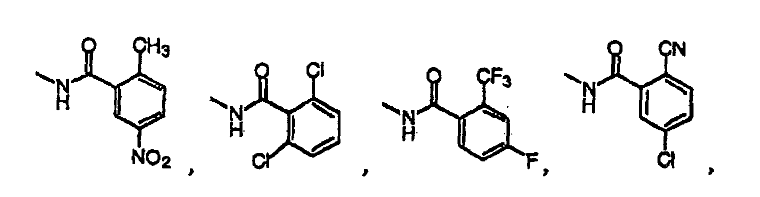

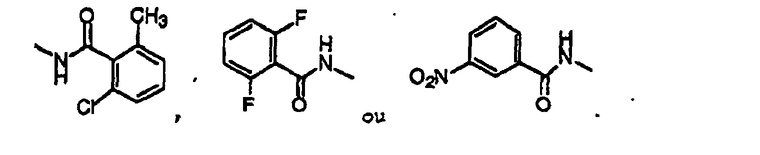

16. Composé selon l'une quelconque des revendications 1-3, dans lequel dans X-7, Het est un noyau hétéroaromatique monocyclique à 5 ou 6 éléments contenant 1, 2 ou 3 atomes d'azote ou un atome d'azote et un de soufre, ou un azote et un oxygène.

17. Composé selon la revendication 16, dans lequel le noyau hétéroaromatique est

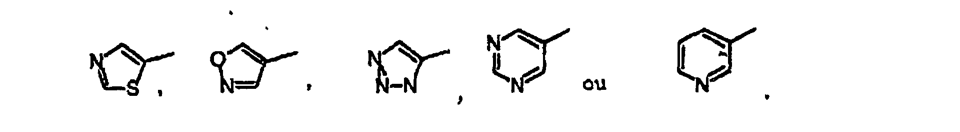

18. Composé selon l'une quelconque des revendications 1-3, dans lequel dans X-7, Het est un noyau hétéroaromatique bicyclique contenant de 1 à 3 atomes d'azote comme hétéroatomes.

19. Composé selon la revendication 18, dans lequel l'anneau hétéroaromatique bicyclique est un 4-quinolinyle, un 1-isoquinolinyle ou

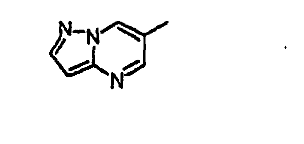

20. Composé selon l'une quelconque des revendications 1-3, dans lequel dans X-7, R₁₅ est un hydrogène, un nitro, un alkylaulfonyle en C₁₋₆, un cyano, un alkyle en C₁₋₆, un alcoxy en C₁₋₆, un alkyle en C₁₋₆ perfluoro, un alkylthio en C₁₋₆, un alkylcarbonyle en C₁₋₆ ou un aryle.

21. Composé selon la revendication 20, dans lequel R₁₅ est un isopropyle, méthyle ou phényle.

22. Composé selon l'une quelconque des revendications 1-3, dans lequel R₁₆, dans X-7, est un hydrogène, halogène, un nitro, un cyano, un alkyle en C₁₋₆ ou un alkyle en C₁₋₆ perfluoro.

23. Composé selon la revendication 22, dans lequel R₁₆ est un méthyle ou trifluorométhyle.

24. Composé selon l'une quelconque des revendications 1-3, dans lequel R₃₀ dans X-7 est un hydrogène ou alkyle en C₁₋₆.

25. Composé selon l'une quelconque des revendications 1-3, dans lequel les groupes X-7 sont de formules :

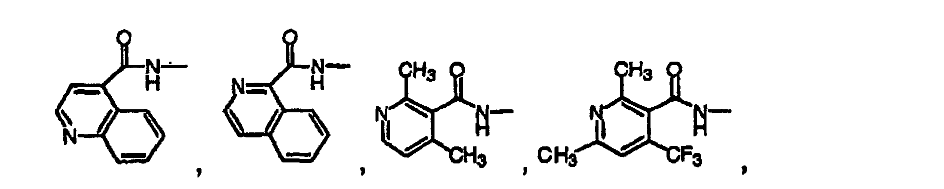

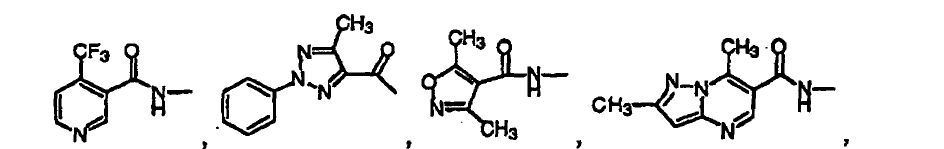

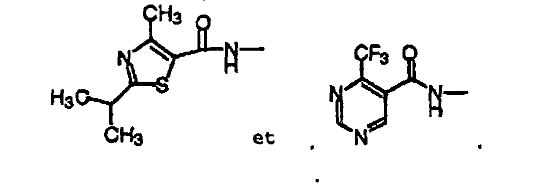

26. Composé selon l'une quelconque des revendications 1-3, dans lequel dans X-6 ou X-7, R₁ est un hydrogène.

27. Composé selon l'une quelconque des revendications 1-3, dans lequel dans X-6 ou X-7, a est égal à 0.

28. Composé selon l'une quelconque des revendications 1-3, dans lequel dans X-10, R₁₈ est un alkyle en C₁₋₆ ou un phényle dans lequel le noyau de phényle est non substitué ou monosubetitué par un halogène, un hydroxy ou est un alkyle en C₁₋₆ phényle.

29. Composé selon la revendication 28, dans lequel R₁₈ est un tert-butyle, un phényle, un hydroxyphényle, un chlorophényle ou un phényléthyle.

30. Composé selon l'une quelconque des revendications 1-3, dans lequel dans X-10, R₁₉ est un alkyle en C₁₋₆ qui est non substitué ou substitué par un pyridyle ou phényle dans lequel le noyau de phényle est non substitué ou monosubstitué par un alcoxy en C₁₋₆ ou halogène.

31. Composé selon la revendication 30, dans lequel R₁₉ est un méthyle, isobutyle, benzyle, 4-chlorobenzyle, 4-méthoxybenzyle ou 2-pyridylméthyle.

32. Composé selon l'une quelconque des revendications 1-3, dans lequel dans X-10, R₂₀ est un alkylcarbonyle en C₁₋₆.

33. Composé selon la revendication 32, dans lequel R₂₀ est un acétyle.

34. Composé selon l'une quelconque des revendications 1-3, dans lequel les groupes C-10 sont de formule : 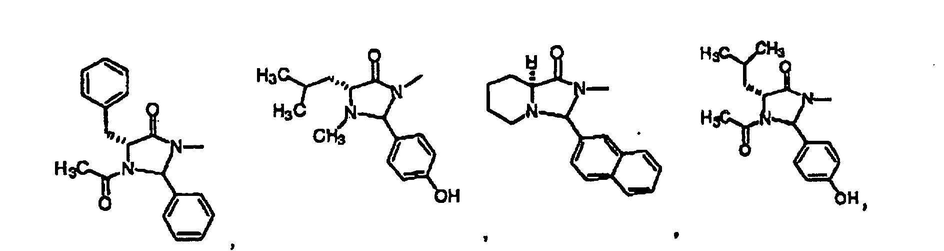 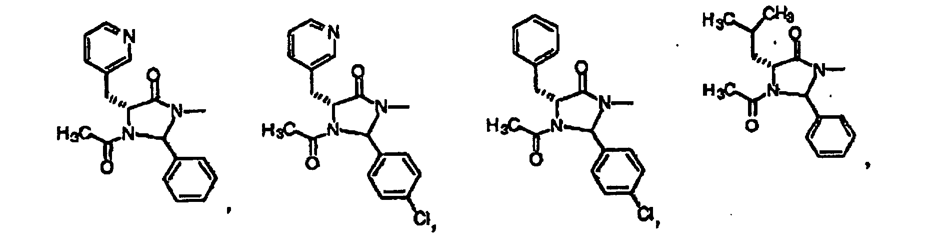 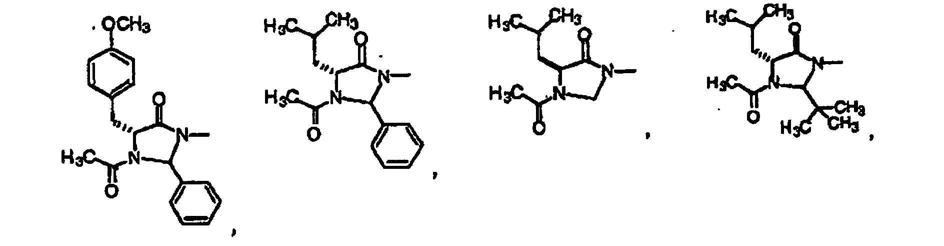 et

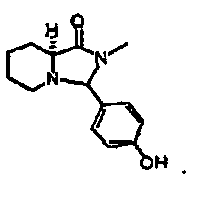

35. Composé choisi dans le groupe 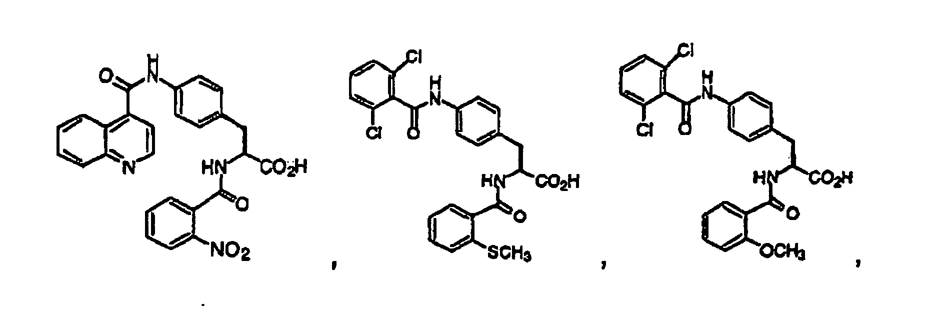 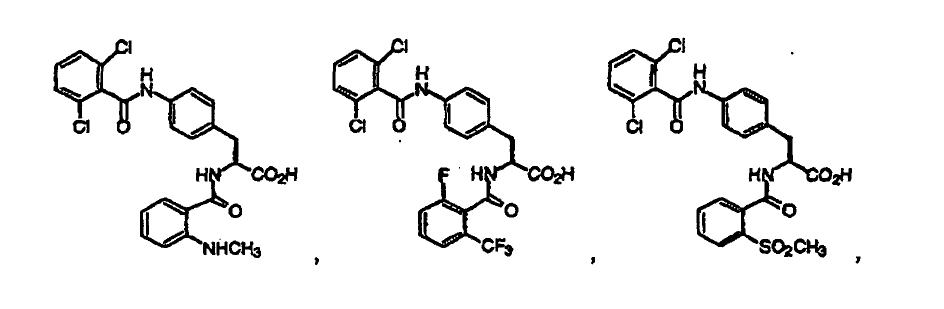 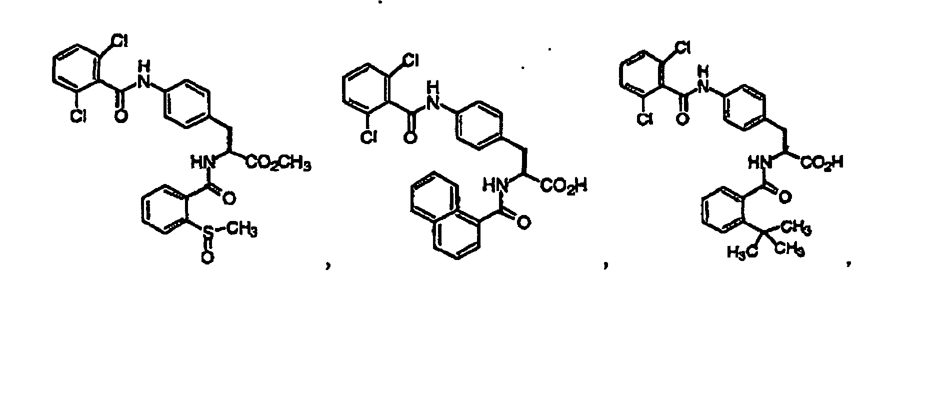 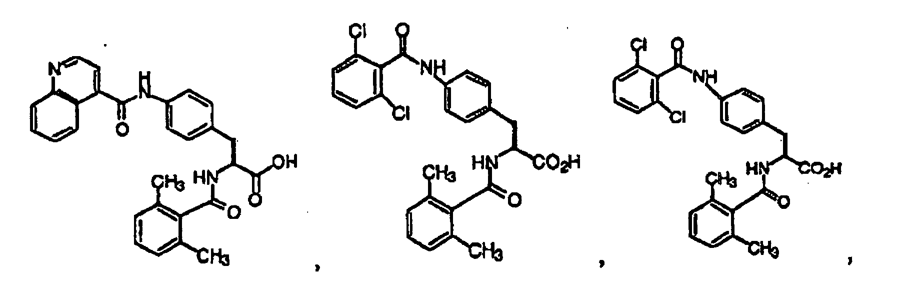 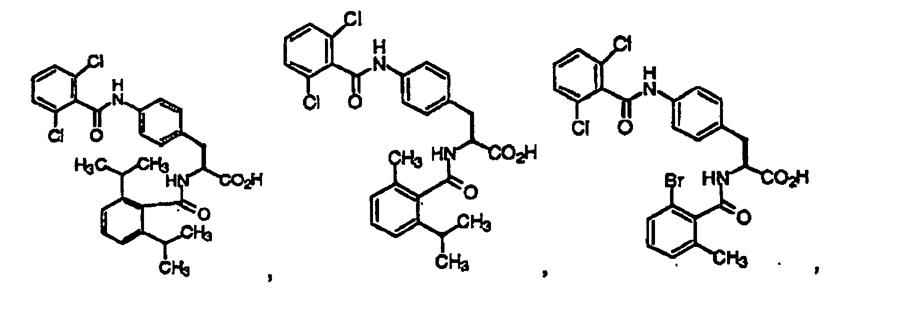 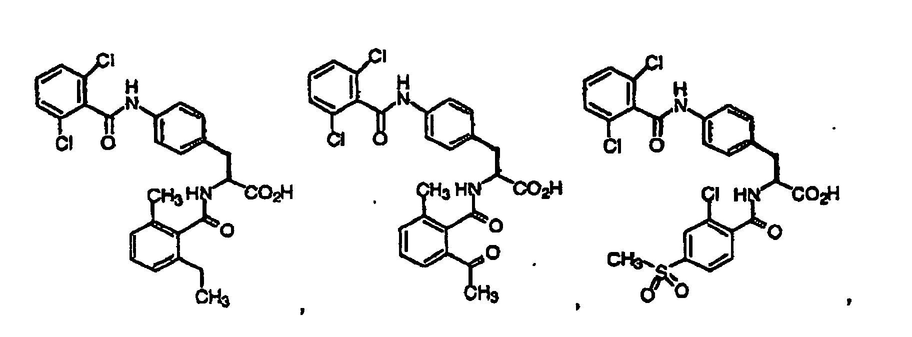 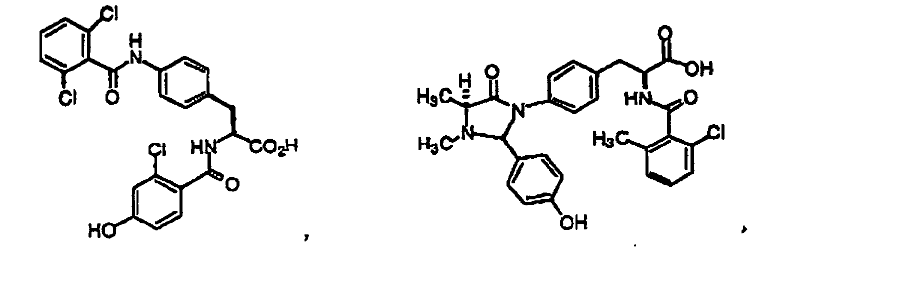 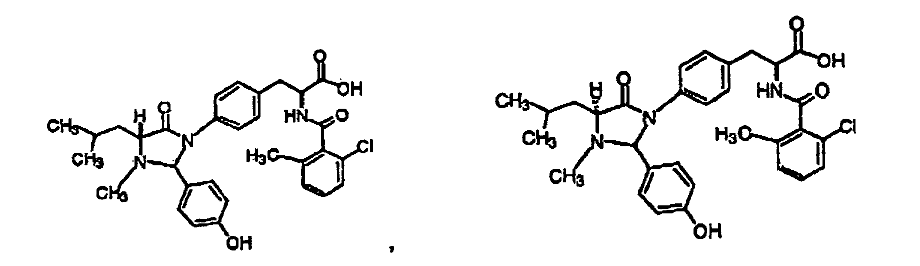 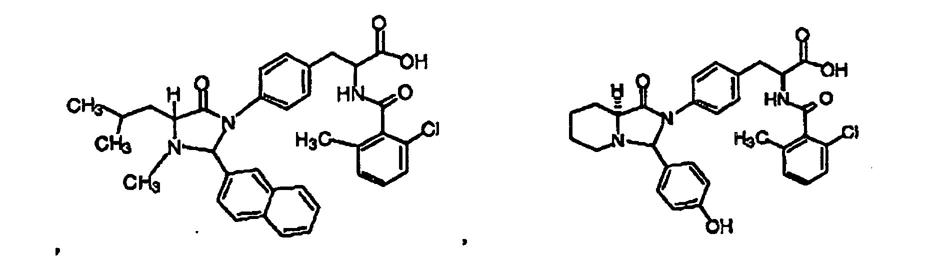 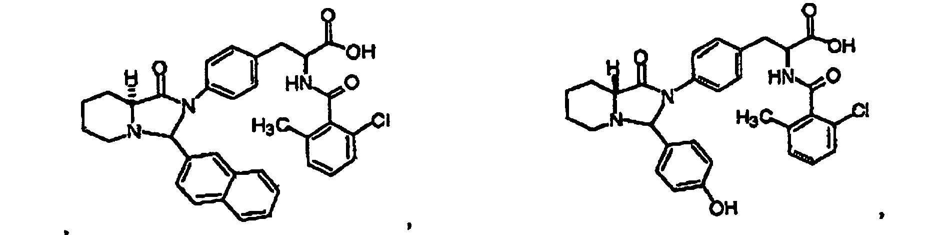 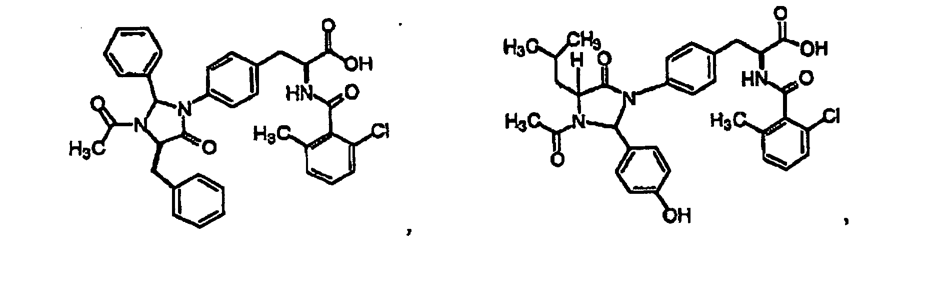 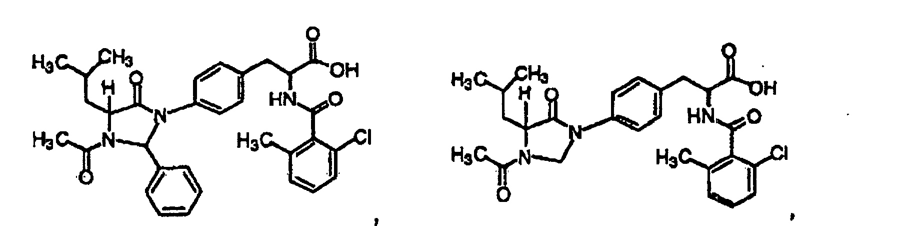 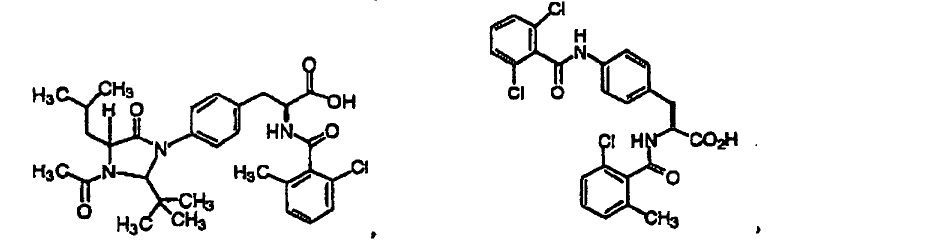 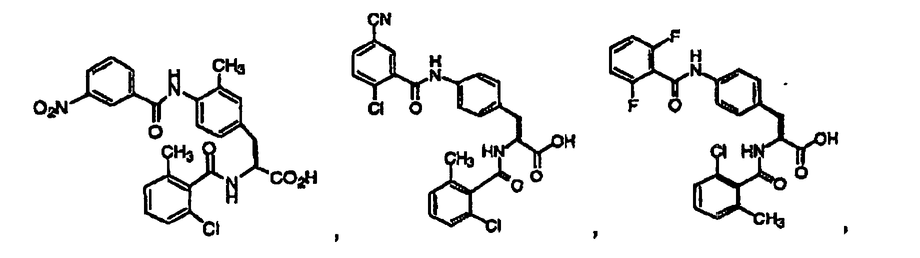 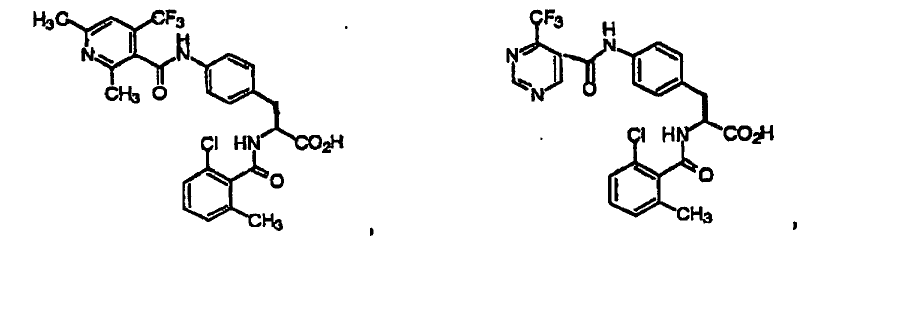 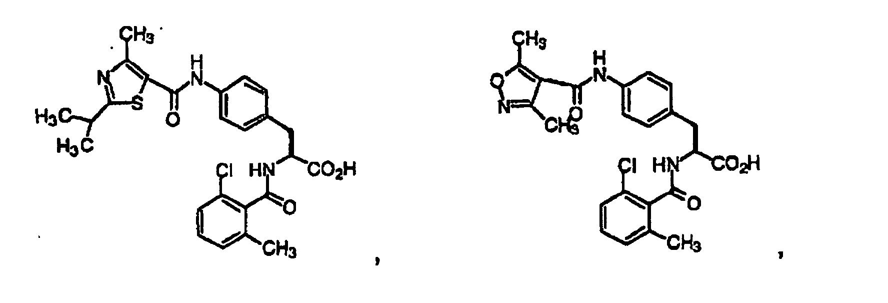 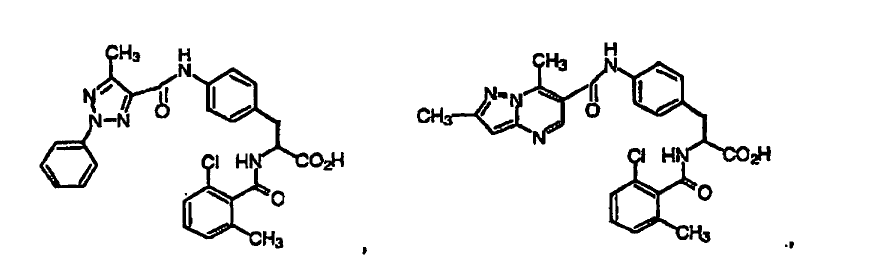 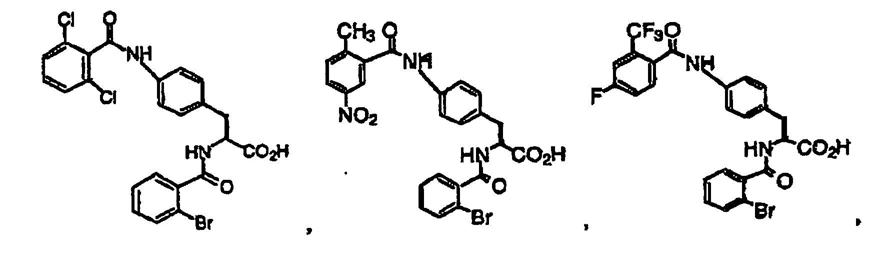 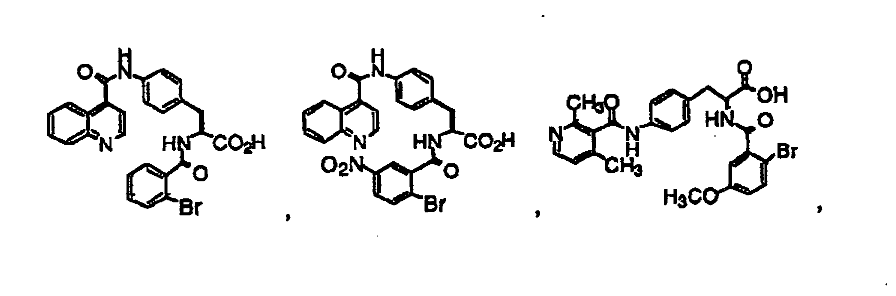 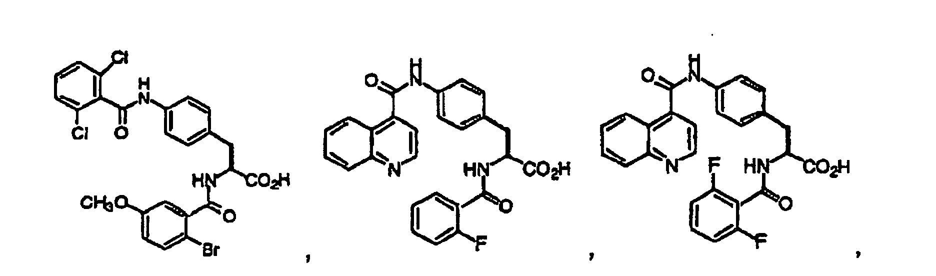 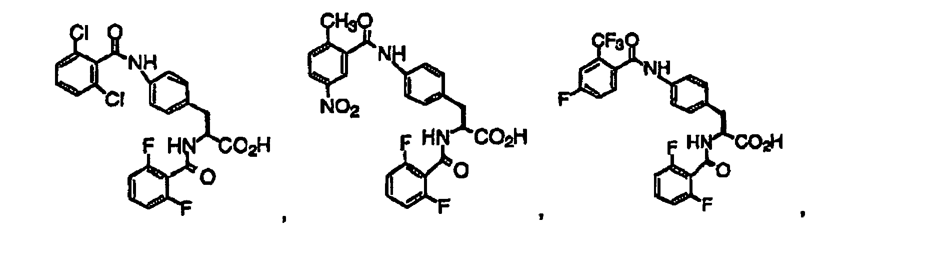 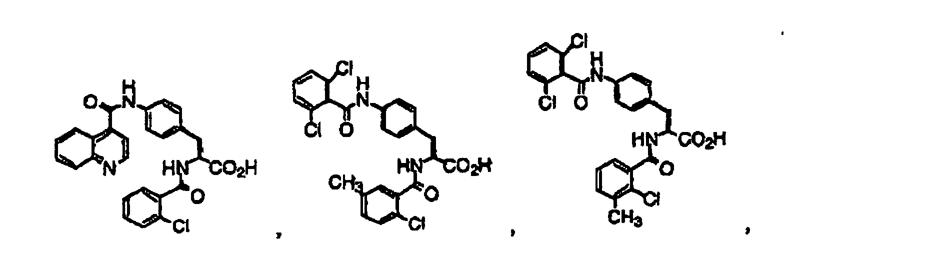 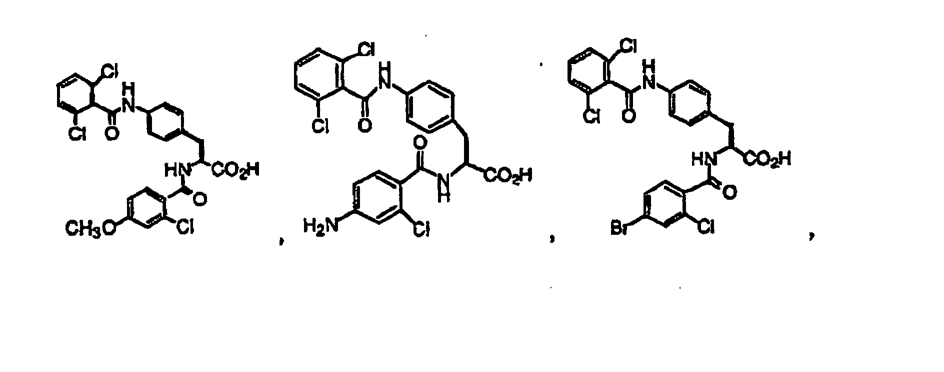 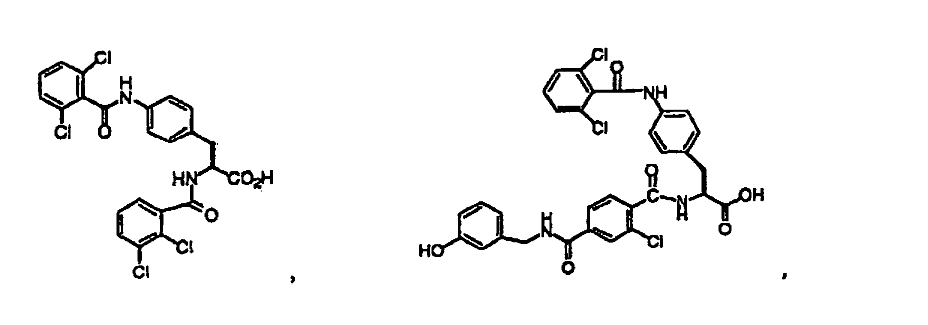 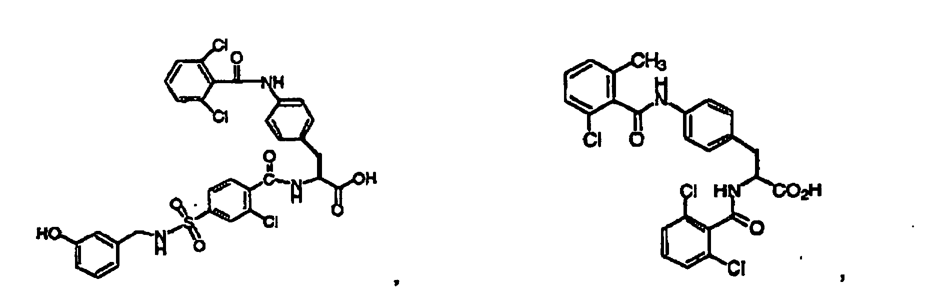 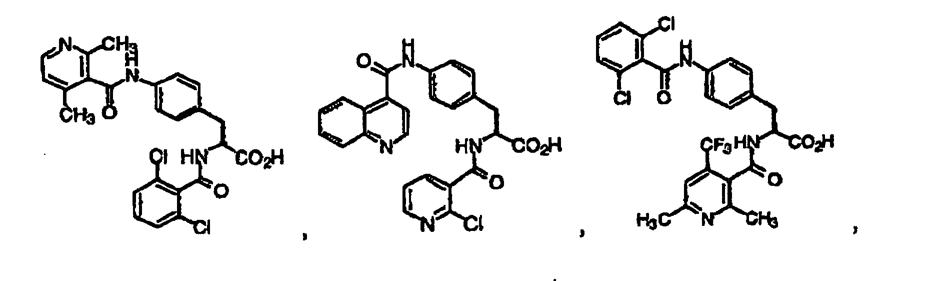 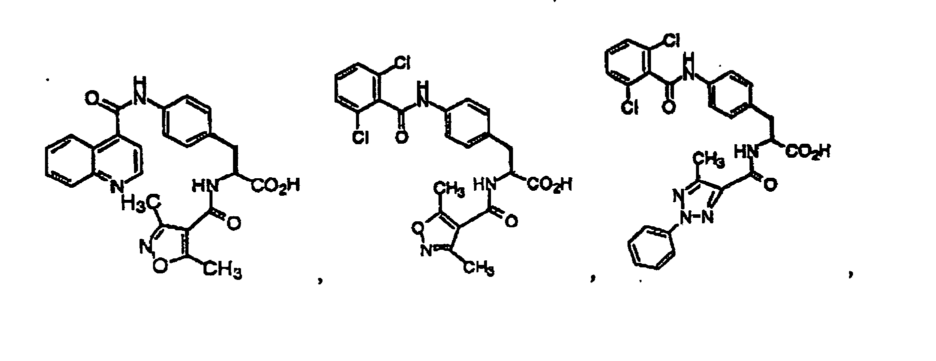 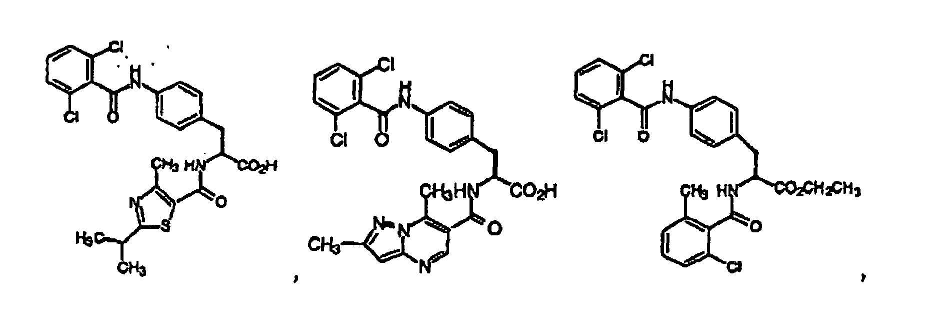 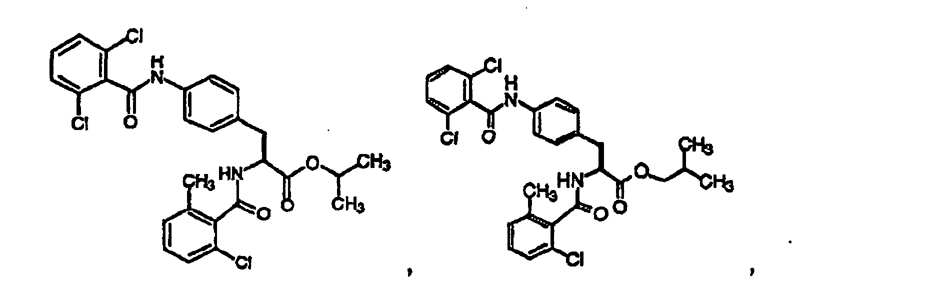 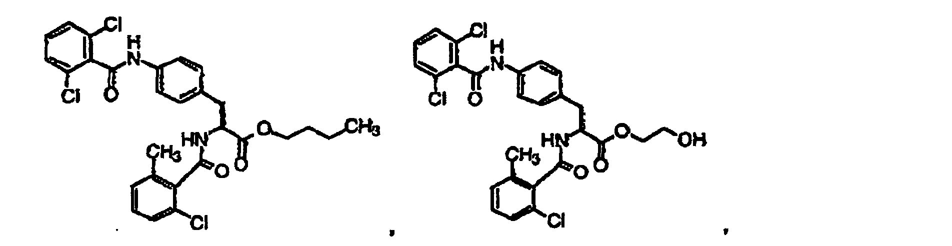 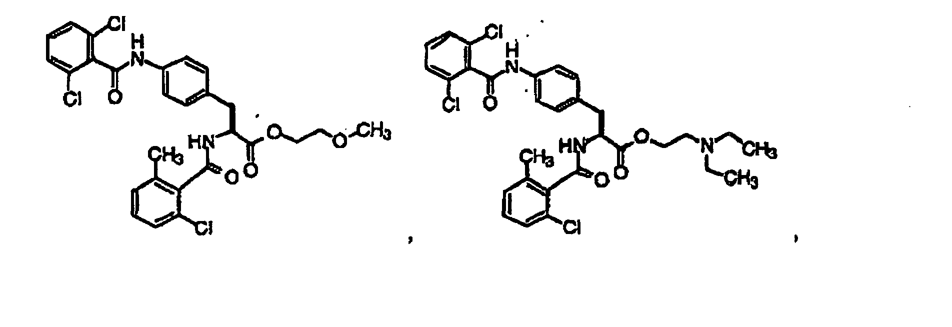 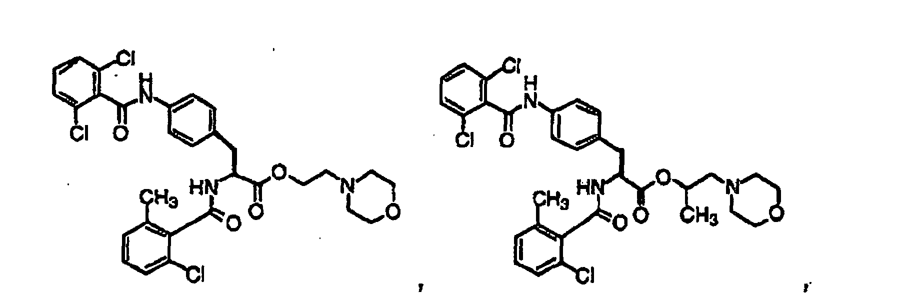 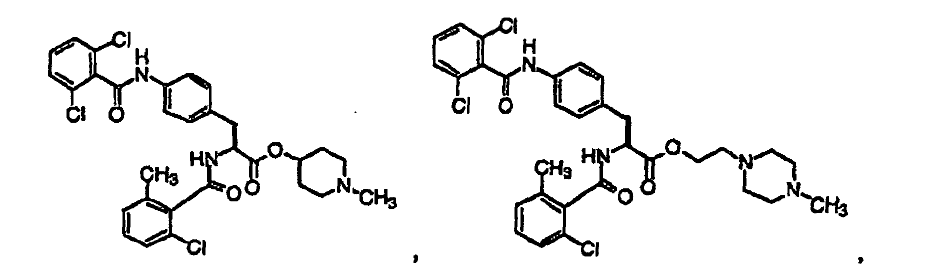 et

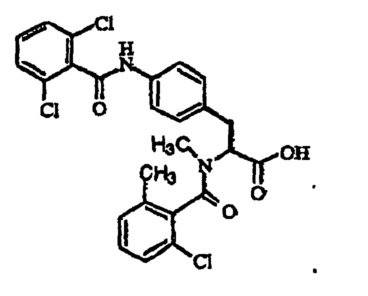

36. Composé selon la revendication 1, qui est un N-(2-chloro-6-méthylbenzoyl)-4-[[(2,6-dichlorophényl) carbonyl]amino]-L-phénylalanine ou un sel de sodium de N-(2(chloro-6-méthylbenzoyl)-4-[[(2,6-dichlorophényl)-carbonyl]amino]-L-phénylalanine.

37. Composé selon la revendication 1 ayant la formule 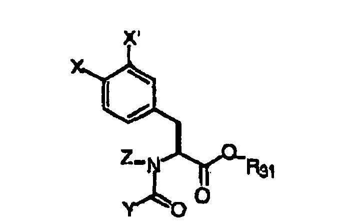 dans laquelle
X, X', Z et Y sont comme définis dans la revendication 1,
R₃₁ est un alkyle en C₁₋₆ ou
R₃₁ est un groupe de la formule P-1 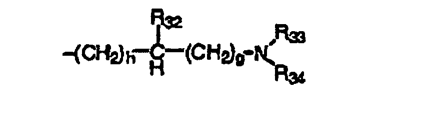 dans laquelle
R₃₂ est un hydrogène ou un alkyle en C₁₋₆
R₃₃ est un hydrogène, un alkyle en C₁₋₆ ou un aryle
R₃₄ est un hydrogène ou un alkyle en C₁₋₆
h est un nombre entier de 0 à 2,
g est un nombre entier de 0 à 2 et
La somme de h et g est de 1 à 3 ou
R₃₁ est un groupe de la formule P-2 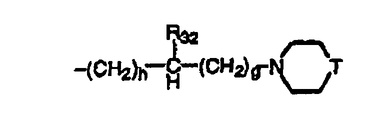 dans laquelle :
R₃₂, g et h sont comme définis ci-dessus,
T est un O, S, -(CH2)ⱼ-, une liaison (quand j = 0) ou un groupe de la formule N-R₃₅,
R₃₅ est un hydrogène, un alkyle en C₁₋₆, un alkylcarbonyle en C₁₋₆, un alcoxycarbonyle en C₁₋₆ et j est égal à 0, 1 ou 2.

38. Composé selon la revendication 37, dans lequel R₃₁ est un alkyle en C₁₋₆.

39. Composé selon la revendication 38, qui est
un méthylester de N-(2-chloro-6-méthylbenzoyl)-4-[[(2,6-dichlorophényl)carbonyl]amino]-L-phénylalanine ou
un éthylester de N-(2-chloro-6-méthylbenzoyl)-4-[[(2,6-dichlorophényl)earbonyl]amino]-L-phénylalanine ou
un butylester de N-(2-chloro-6-méthylbenzoyl)-4-[[(2,6-dichlorophényl)carbonyl]amino]-L-phénylalanine ou
un 1-méthyléthylester de N-(2-chloro-6-méthylbenzoyl)-4-[[(2,6-dichlorophényl)carbonyl]amino]-L-phénylalanine ou
un 2-méthylpropylester de N-(2-chloro-6-méthylbenzoyl)-4-[[(2,6-dichlorophényl)carbonyl]amino]-L-phénylalanine.

40. Composé selon la revendication 1, qui est
un 2-méthoxyéthylester de N-(2-chloro-6-méthylbenzoyl)-4-[[(2,6-dichlorophényl)carbonyl]amino]-L-phénylalanine ou
un 2-hydroxyéthylester de N-(2-chloro-6-méthylbenzoyl)-4-[[(2,6-dichlorophényl)carbonyl]amino]-L-phénylalanine

41. Composé selon la revendication 37, dans lequel R₃₁ est P-2.

42. Composé selon la revendication 41, dans lequel R₃₁ est un 2(4-morpholinyl)-éthyle, 1-méthyl-2-(4-marpholinyl)éthyle, 1-méthyl-4-pipéridinyle, 2-(1-pipérazinyl)éthyle ou 2-(4-méthyl-1-pipérazinyl)éthyle.

43. Composé selon la revendication 42, qui est un
2-morpholinoéthylester de N-(2-chloro-6-méthylbenzoyl)-4-[[(2,6-dichlorophényl)carbonyl]amino]-L-phénylalanine ou
un 1-méthyl-2-morpholinoéthylester de N-(2-chloro-6-méthylbenzoyl)-4[[(2,6-dichlorophényl)carbonyl]amino]-L-phénylalanine ou
un 1-méthyl-A-pipéridinylester de N-(2-chloro-6-méthylbenzoyl)-4[[(2,6-dichlorophényl)carbonyl]amino]-L-phénylalanine ou
un 2-(1-pipérazinyl)éthylester de N-(2-chloro-6-méthylbenzoyl)-4[[(2,6-dichlorophényl)carbonyl]amino]-L-phénylalanine ou
un 2-(4-méthyl-1-pipérazinyl)éthylester de N-(2-chloro-6-méthylbenzoyl)-4[[(2,6-dichlorophényl)carbonyl] amino]-L-phénylalanine

44. Composé selon la revendication 37, dans lequel R₃₁ est P-1.

45. Composé selon la revendication 44, dans lequel R₃₁ est un diéthylaminoéthyle.

46. Composé selon la revendication 45, ayant la formule 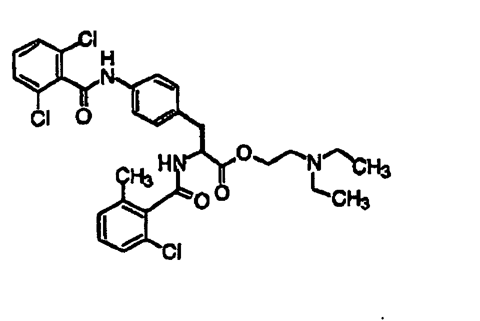 qui est un 2-diéthylaminoéthylester de N-(2-chloro-6-méthylbenzoyl)-4-[[(2,6-dichlorophényl)carbonyl]amino]-L-phénylalanine.

47. Composés selon l'une quelconque des revendications 1 à 46 à utiliser comme un médicament.

48. Médicament contenant un composé selon l'une quelconque des revendications 1 à 46, et un matériau de support thérapeutiquement inerte.

49. Procédé de production d'un médicament, en particulier pour le traitement de la polyarthrite rhumatoide, de la sclérose en plaques, de l'inflammation des intestins, ou de l'asthme, ledit procédé comprenant la fourniture d'un composé selon l'une quelconque des revendications 1 à 46, sous une forme d'administration galénique avec un matériau de support thérapeutiquement inerte et, si on le souhaite, une ou plusieurs substances thérapeutiquement actives supplémentaires.

50. Utilisation d'un composé selon l'une quelconque des revendications 1 à 46, dans la préparation d'un médicament pour le traitement de la polyarthrite rhumatoide, de la sclérose en plaque, de l'inflammation des intestins ou de l'asthme.
